(19) Europäisches Patentamt
European Patent Office
Office européen des brevets

(11) EP 2 158 315 B1

(12) EUROPEAN PATENT SPECIFICATION

(45) Date of publication and mention of the grant of the patent:
**23.03.2016 Bulletin 2016/12**

(21) Application number: **08757278.0**

(22) Date of filing: **25.06.2008**

(51) Int Cl.:
***C12N 15/09*** *(2006.01)* ***A61K 39/395*** *(2006.01)*
***C12N 15/13*** *(2006.01)* ***C07K 16/00*** *(2006.01)*

(86) International application number:
**PCT/CH2008/000285**

(87) International publication number:
**WO 2009/000099 (31.12.2008 Gazette 2009/01)**

(54) **METHODS OF MODIFYING ANTIBODIES, AND MODIFIED ANTIBODIES WITH IMPROVED FUNCTIONAL PROPERTIES**

VERFAHREN ZUR MODIFIKATION VON ANTIKÖRPERN UND MODIFIZIERTE ANTIKÖRPER MIT VERBESSERTEN FUNKTIONSEIGENSCHAFTEN

MÉTHODES DE MODIFICATION D'ANTICORPS ET ANTICORPS MODIFIÉS PRÉSENTANT DES PROPRIÉTÉS FONCTIONNELLES AMÉLIORÉES

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **25.06.2007 US 937112 P**
**12.03.2008 US 69056**

(43) Date of publication of application:
**03.03.2010 Bulletin 2010/09**

(73) Proprietor: **ESBATech, an Alcon Biomedical Research Unit LLC**
**8952 Schlieren (CH)**

(72) Inventors:
- **URECH, David**
  **8708 Männedorf (CH)**
- **BORRAS, Leonardo**
  **8952 Schlieren (CH)**

(74) Representative: **Grünecker Patent- und Rechtsanwälte**
**PartG mbB**
**Leopoldstraße 4**
**80802 München (DE)**

(56) References cited:

| | |
|---|---|
| **WO-A-03/008451** | **WO-A-2005/005604** |
| **WO-A-2006/013468** | **WO-A-2007/070750** |
| **WO-A1-01/85797** | **WO-A2-2005/051998** |

- **EWERT S ET AL: "STRUCTURE-BASED IMPROVEMENT OF THE BIOPHYSICAL PROPERTIES OF IMMUNOGLOBULIN VH DOMAINS WITH A GENERALIZABLE APPROACH" BIOCHEMISTRY, AMERICAN CHEMICAL SOCIETY, EASTON, PA.; US, vol. 42, no. 6, 18 February 2003 (2003-02-18), pages 1517-1528, XP008052212 ISSN: 0006-2960**
- **HONEGGER A ET AL: "Yet Another Numbering Scheme for Immunoglobulin Variable Domains: An Automatic Modeling and Analysis Tool" JOURNAL OF MOLECULAR BIOLOGY, LONDON, GB, vol. 309, no. 3, 8 June 2001 (2001-06-08), pages 657-670, XP004626893 ISSN: 0022-2836**
- **CHOWDHURY P S ET AL: "Improved stability and yield of a Fv-toxin fusion protein by computer design and protein engineering of the Fv" JOURNAL OF MOLECULAR BIOLOGY, LONDON, GB, vol. 281, no. 5, 4 September 1998 (1998-09-04), pages 917-928, XP004457437 ISSN: 0022-2836**
- **JUNG S ET AL: "The Importance of Framework Residues H6, H7 and H10 in Antibody Heavy Chains: Experimental Evidence for a New Structural Subclassification of Antibody VH Domains" JOURNAL OF MOLECULAR BIOLOGY, LONDON, GB, vol. 309, no. 3, 8 June 2001 (2001-06-08), pages 701-716, XP004626896 ISSN: 0022-2836**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- WORN A ET AL: "Stability engineering of antibody single-chain Fv fragments" JOURNAL OF MOLECULAR BIOLOGY, LONDON, GB, vol. 305, no. 5, 2 February 2001 (2001-02-02), pages 989-1010, XP004465987 ISSN: 0022-2836 cited in the application
- FILPULA ET AL: "Antibody engineering and modification technologies" BIOMOLECULAR ENGINEERING, ELSEVIER, NEW YORK, NY, US, vol. 24, no. 2, 1 June 2007 (2007-06-01), pages 201-215, XP022081380 ISSN: 1389-0344
- CARTER P J: "POTENT ANTIBODY THERAPEUTICS BY DESIGN" NATURE REVIEWS. IMMUNOLOGY, XX, XX, vol. 6, 7 April 2006 (2006-04-07), pages 343-357, XP007901440 ISSN: 1474-1733
- ZAHND CHRISTIAN ET AL: "Directed in vitro evolution and crystallographic analysis of a peptide-binding single chain antibody fragment (scFv) with low picomolar affinity" JOURNAL OF BIOLOGICAL CHEMISTRY, AMERICAN SOCIETY OF BIOLOCHEMICAL BIOLOGISTS, BIRMINGHAM,; US, vol. 279, no. 18, 30 April 2004 (2004-04-30), pages 18870-18877, XP002406477 ISSN: 0021-9258
- HOET RENE MICHAEL ET AL: "Generation of high-affinity human antibodies by combining donor-derived and synthetic complementarity-determining-region diversity" NATURE BIOTECHNOLOGY, NATURE PUBLISHING GROUP, NEW YORK, NY, US, vol. 23, no. 3, 1 March 2005 (2005-03-01), pages 344-348, XP002421213 ISSN: 1087-0156

SEQUENCE LISTING

<110> David Urech, Leonard Borras

<120> Methods of Modifying Antibodies, and Modified Antibodies with Improved Functional Properties

<130> P106700PC00

<160> 12

<170> PatentIn version 3.5

<210> 1
<211> 147
<212> PRT
<213> Artificial Sequence

<220>
<223> Fig. 9 - VH1 Family Heavy Chain

<220>
<221> misc_feature
<222> (1)..(1)
<223> xaa is Gln or Glu

<220>
<221> misc_feature
<222> (6)..(6)
<223> Xaa is Gln or Glu

<220>
<221> misc_feature
<222> (11)..(11)
<223> Xaa is Val or Leu

<220>
<221> misc_feature
<222> (12)..(12)
<223> Xaa is Lys or Met

<220>
<221> misc_feature
<222> (13)..(13)
<223> Xaa is Lys, Glu, or Gln

<220>
<221> misc_feature
<222> (18)..(18)
<223> Xaa is Val or Leu

<220>
<221> misc_feature
<222> (20)..(20)
<223> Xaa is Val or Ile

<220>
<221> misc_feature
<222> (26)..(40)
<223> Xaa can be any naturally occurring amino acid

<220>
<221> misc_feature
<222> (30)..(40)
<223> CDR H1

<220>
<221> misc_feature
<222> (55)..(74)
<223> Xaa can be any naturally occurring amino acid

<220>
<221> misc_feature
<222> (55)..(74)
<223> CDR H2

<220>
<221> misc_feature
<222> (88)..(88)

<223> Xaa is Tyr, Phe, Ser, His, or Asp

<220>
<221> misc_feature
<222> (90)..(90)
<223> Xaa is Glu, Asp, Gln

<220>
<221> misc_feature
<222> (93)..(93)
<223> Xaa is Ser, Gly, Thr, Asn

<220>
<221> misc_feature
<222> (96)..(96)
<223> Xaa is Ser, Phe, Thr, Ala, Pro

<220>
<221> misc_feature
<222> (107)..(136)
<223> Xaa can be any naturally occurring amino acid

<220>
<221> misc_feature
<222> (107)..(136)
<223> CDR H3

<400> 1

```
Xaa Val Gln Leu Val Xaa Ser Gly Ala Glu Xaa Xaa Xaa Pro Gly Ser
1               5                   10                  15

Ser Xaa Lys Xaa Ser Cys Lys Ala Ser Xaa Xaa Xaa Xaa Xaa Xaa Xaa
            20                  25                  30

Xaa Xaa Xaa Xaa Xaa Xaa Xaa Xaa Trp Val Arg Gln Ala Pro Gly Gln
        35                  40                  45

Gly Leu Glu Trp Met Gly Xaa Xaa Xaa Xaa Xaa Xaa Xaa Xaa Xaa Xaa
    50                  55                  60

Xaa Xaa Xaa Xaa Xaa Xaa Xaa Xaa Xaa Xaa Arg Val Thr Ile Thr Ala
65                  70                  75                  80

Asp Glu Ser Thr Ser Thr Ala Xaa Met Xaa Leu Ser Xaa Leu Arg Xaa
                85                  90                  95

Glu Asp Thr Ala Val Tyr Tyr Cys Ala Arg Xaa Xaa Xaa Xaa Xaa Xaa
            100                 105                 110
```

Xaa Xaa Xaa Xaa Xaa Xaa Xaa Xaa Xaa Xaa Xaa Xaa Xaa Xaa Xaa Xaa
115 120 125

Xaa Xaa Xaa Xaa Xaa Xaa Xaa Xaa Trp Gly Gln Gly Thr Leu Val Thr
130 135 140

Val Ser Ser
145

<210> 2
<211> 147
<212> PRT
<213> Unknown

<220>
<223> Fig. 10 - VH1B Family Heavy Chain

<220>
<221> misc_feature
<222> (1) .. (1)
<223> Xaa is Gln or Glu

<220>
<221> misc_feature
<222> (9)..(9)
<223> Xaa is Ala, Thr, Asp, Pro, or Val

<220>
<221> misc_feature
<222> (11) .. (11)
<223> Xaa is Val or Leu

<220>
<221> misc_feature
<222> (12)..(12)
<223> Xaa is Lys, Val, Arg, Gln, Met

<220>
<221> misc_feature
<222> (13)..(13)
<223> Xaa is Lys, Arg, Glu, or Met

<220>
<221> misc_feature
<222> (19)..(19)
<223> Xaa is Lys, Asn, Arg, or Thr

<220>
<221> misc_feature
<222> (20)..(20)
<223> Xaa is Val, Leu, Ile, or Phe

<220>
<221> misc_feature
<222> (26)..(40)
<223> Xaa can be any naturally occurring amino acid

<220>
<221> misc_feature
<222> (30)..(40)
<223> CDR H1

<220>
<221> misc_feature
<222> (43)..(43)
<223> Xaa is Arg or Lys

<220>
<221> misc_feature
<222> (45)..(45)
<223> Xaa is Ala, Arg, Thr, Val or Pro,

<220>
<221> misc_feature
<222> (48)..(48)
<223> Xaa is Gln, Lys, Glu, or His

<220>
<221> misc_feature
<222> (53)..(53)
<223> Xaa is Met or Ile

<220>
<221> misc_feature
<222> (55)..(74)
<223> Xaa can be any naturally occurring amino acid

<220>
<221> misc_feature
<222> (55)..(74)
<223> CDR H2

<220>
<221> misc_feature
<222> (75)..(75)
<223> Xaa is Arg or Lys

<220>
<221> misc_feature
<222> (76)..(76)
<223> Xaa is Val, Ala, Ile, or Leu

<220>
<221> misc_feature
<222> (105)..(105)
<223> Xaa is Ala, Asn, or Ser

<220>
<221> misc_feature
<222> (107)..(136)
<223> Xaa can be any naturally occurring amino acid

<220>
<221> misc_feature
<222> (107)..(136)

<223> CDR H3

<400> 2

```
Xaa Val Gln Leu Val Gln Ser Gly Xaa Glu Xaa Xaa Xaa Pro Gly Ala
1               5                   10                  15

Ser Val Xaa Xaa Ser Cys Lys Ala Ser Xaa Xaa Xaa Xaa Xaa Xaa Xaa
            20                  25                  30

Xaa Xaa Xaa Xaa Xaa Xaa Xaa Xaa Trp Val Xaa Gln Xaa Pro Gly Xaa
        35                  40                  45

Gly Leu Glu Trp Xaa Gly Xaa Xaa Xaa Xaa Xaa Xaa Xaa Xaa Xaa Xaa
    50                  55                  60

Xaa Xaa Xaa Xaa Xaa Xaa Xaa Xaa Xaa Xaa Xaa Xaa Thr Met Thr Glu
65                  70                  75                  80

Asp Thr Ser Thr Asn Thr Ala Tyr Met Glu Leu Ser Ser Leu Arg Ser
                85                  90                  95

Glu Asp Thr Ala Val Tyr Tyr Cys Xaa Arg Xaa Xaa Xaa Xaa Xaa Xaa
            100                 105                 110

Xaa Xaa Xaa Xaa Xaa Xaa Xaa Xaa Xaa Xaa Xaa Xaa Xaa Xaa Xaa Xaa
        115                 120                 125

Xaa Xaa Xaa Xaa Xaa Xaa Xaa Xaa Trp Gly Gln Gly Thr Leu Val Thr
    130                 135                 140

Val Ser Ser
145
```

<210> 3
<211> 147
<212> PRT
<213> Unknown

<220>
<223> Fig. 11 - VH3 Family Heavy Chain

<220>
<221> misc_feature
<222> (1)..(1)
<223> Xaa is Glu or Gln

<220>
<221> misc_feature
<222> (6)..(6)
<223> Xaa is Glu or Gln

<220>
<221> misc_feature

<222> (7)..(7)
<223> Xaa is Ser, Thr, or Alia

<220>
<221> misc_feature
<222> (27)..(40)
<223> Xaa can be any naturally occurring amino acid

<220>
<221> misc_feature
<222> (30)..(40)
<223> CDR H1

<220>
<221> misc_feature
<222> (55)..(74)
<223> Xaa can be any naturally occurring amino acid

<220>
<221> misc_feature
<222> (55)..(74)
<223> CDR H2

<220>
<221> misc_feature
<222> (87)..(87)
<223> Xaa is Leu, Val, Ala, or Phe

<220>
<221> misc_feature
<222> (101)..(101)
<223> Xaa is Val, Leu, Ile, Met, Phe, Arg, or Gln

<220>
<221> misc_feature
<222> (107)..(136)
<223> Xaa can be any naturally occurring amino acid

<220>
<221> misc_feature
<222> (107)..(136)
<223> CDR H3

<400> 3

Xaa Val Gln Leu Val Xaa Xaa Gly Pro Gly Leu Val Lys Pro Ser Glu
1               5                   10                  15

Thr Leu Arg Leu Ser Cys Ala Ala Ser Gly Xaa Xaa Xaa Xaa Xaa Xaa
            20                  25                  30

Xaa Xaa Xaa Xaa Xaa Xaa Xaa Xaa Trp Val Arg Gln Ala Pro Gly Lys
        35                  40                  45

Gly Leu Glu Trp Val Ser Xaa Xaa Xaa Xaa Xaa Xaa Xaa Xaa Xaa Xaa
    50                  55                  60

Xaa Xaa Xaa Xaa Xaa Xaa Xaa Xaa Xaa Xaa Arg Phe Thr Ile Ser Arg
65                  70                  75                  80

Asp Asn Ser Lys Asn Thr Xaa Tyr Leu Gln Met Asn Ser Leu Arg Ala
                85                  90                  95

Glu Asp Thr Ala Xaa Tyr Tyr Cys Ala Arg Xaa Xaa Xaa Xaa Xaa Xaa
            100                 105                 110

Xaa Xaa Xaa Xaa Xaa Xaa Xaa Xaa Xaa Xaa Xaa Xaa Xaa Xaa Xaa Xaa
        115                 120                 125

Xaa Xaa Xaa Xaa Xaa Xaa Xaa Xaa Trp Gly Gln Gly Thr Leu Val Thr
    130                 135                 140

Val Ser Ser
145

<210> 4
<211> 149
<212> PRT
<213> Unknown

<220>
<223> Fig. 12 - vk1 Family Light Chain

<220>
<221> misc_feature
<222> (1)..(1)
<223> Xaa is Glu, Asp, or Ile

<220>
<221> misc_feature
<222> (3)..(3)
<223> Xaa is Val, Gln, or Ile

<220>
<221> misc_feature
<222> (4)..(4)
<223> Xaa is Leu, Met, Val, or Ile

<220>

<221> misc_feature
<222> (24)..(24)
<223> Xaa is Gln or Arg

<220>
<221> misc_feature
<222> (25)..(42)
<223> CDR L1; Xaa can be any naturally occurring amino acid

<220>
<221> misc_feature
<222> (47)..(47)
<223> Xaa is Arg, Lys, or Ile

<220>
<221> misc_feature
<222> (50)..(50)
<223> Xaa is Lys, Arg, Glu, Thr, Met, or Gln

<220>
<221> misc_feature
<222> (57)..(57)
<223> Xaa is Ser, Tyr, Phe, or His

<220>
<221> misc_feature
<222> (58)..(72)
<223> CDR L2; Xaa can be any naturally occurring amino acid

<220>
<221> misc_feature
<222> (85)..(86)
<223> Xaa can be any naturally occurring amino acid

<220>
<221> misc_feature
<222> (91)..(91)
<223> Xaa is Phe or Leu

<220>
<221> misc_feature
<222> (103)..(103)
<223> Xaa is Val, Thr, Ser, Gly, or Ile

<220>
<221> misc_feature
<222> (107)..(138)
<223> CDR L3; Xaa can be any naturally occurring amino acid

<400> 4

```
Xaa Ile Xaa Xaa Thr Gln Ser Pro Ser Ser Leu Ser Ala Ser Val Gly
1               5                   10                  15
```

Asp Arg Val Thr Ile Thr Cys Xaa Xaa Xaa Xaa Xaa Xaa Xaa Xaa Xaa
20 25 30

Xaa Xaa Xaa Xaa Xaa Xaa Xaa Xaa Xaa Xaa Trp Tyr Gln Gln Xaa Pro
35 40 45

Gly Xaa Ala Pro Lys Leu Leu Ile Xaa Xaa Xaa Xaa Xaa Xaa Xaa Xaa
50 55 60

Xaa Xaa Xaa Xaa Xaa Xaa Xaa Xaa Gly Val Pro Ser Arg Phe Ser Gly
65 70 75 80

Ser Gly Ser Gly Xaa Xaa Thr Asp Phe Thr Xaa Thr Ile Ser Ser Leu
85 90 95

Gln Pro Glu Asp Phe Ala Xaa Tyr Tyr Cys Xaa Xaa Xaa Xaa Xaa Xaa
100 105 110

Xaa Xaa Xaa Xaa Xaa Xaa Xaa Xaa Xaa Xaa Xaa Xaa Xaa Xaa Xaa Xaa
115 120 125

Xaa Xaa Xaa Xaa Xaa Xaa Xaa Xaa Xaa Xaa Phe Gly Gln Gly Thr Lys
130 135 140

Val Glu Ile Lys Arg
145

<210> 5
<211> 149
<212> PRT
<213> Unknown

<220>
<223> Fig. 13 - Vk3 Family Light Chain

<220>
<221> misc_feature
<222> (2)..(2)
<223> Xaa is Thr or Ile

<220>
<221> misc_feature
<222> (3)..(3)
<223> Xaa is Thr or Val

<220>
<221> misc_feature
<222> (10)..(10)
<223> Xaa is Ile or Thr

<220>
<221> misc_feature
<222> (12)..(12)
<223> Xaa is Tyr or Ser

<220>
<221> misc_feature
<222> (18)..(18)
<223> Xaa is Ser or Arg

<220>
<221> misc_feature
<222> (20)..(20)
<223> Xaa is Ala or Thr

<220>
<221> misc_feature
<222> (25)..(42)
<223> CDR L1; Xaa can be any naturally occurring amino acid

<220>
<221> misc_feature
<222> (56)..(56)
<223> Xaa is Met or Ile

<220>
<221> misc_feature
<222> (58)..(72)
<223> CDR L2; Xaa can be any naturally occurring amino acid

<220>
<221> misc_feature
<222> (74)..(74)
<223> Xaa is Thr, Val, or Ile

<220>
<221> misc_feature
<222> (85)..(86)
<223> Xaa can be any naturally occurring amino acid

<220>
<221> misc_feature
<222> (94)..(94)
<223> Xaa is Asn or Ser

<220>
<221> misc_feature
<222> (101)..(101)
<223> Xaa is ser, Tyr, or Phe

<220>
<221> misc_feature
<222> (103)..(103)
<223> Xaa is Ala, Leu, or Val

<220>
<221> misc_feature
<222> (107)..(138)
<223> CDR L3; Xaa can be any naturally occurring amino acid

<400> 5

```
Glu Xaa Xaa Leu Thr Gln Ser Pro Gly Xaa Leu Xaa Leu Ser Pro Gly
1               5                   10                  15

Glu Xaa Ala Xaa Leu Ser Cys Arg Xaa Xaa Xaa Xaa Xaa Xaa Xaa Xaa
            20                  25                  30

Xaa Xaa Xaa Xaa Xaa Xaa Xaa Xaa Xaa Xaa Trp Tyr Gln Gln Lys Pro
        35                  40                  45

Gly Gln Ala Pro Arg Leu Leu Xaa Tyr Xaa Xaa Xaa Xaa Xaa Xaa Xaa

    50                  55                  60

Xaa Xaa Xaa Xaa Xaa Xaa Xaa Xaa Gly Xaa Pro Asp Arg Phe Ser Gly
65                  70                  75                  80

Ser Gly Ser Gly Xaa Xaa Thr Asp Phe Thr Leu Thr Ile Xaa Arg Leu
                85                  90                  95

Glu Pro Glu Asp Xaa Ala Xaa Tyr Tyr Cys Xaa Xaa Xaa Xaa Xaa Xaa
            100                 105                 110

Xaa Xaa Xaa Xaa Xaa Xaa Xaa Xaa Xaa Xaa Xaa Xaa Xaa Xaa Xaa Xaa
        115                 120                 125

Xaa Xaa Xaa Xaa Xaa Xaa Xaa Xaa Xaa Xaa Phe Gly Gly Gly Thr Lys
    130                 135                 140

Leu Glu Ile Lys Arg
145
```

<210> 6
<211> 149
<212> PRT
<213> Unknown

<220>
<223> Fig. 14 - VL1 Family Light chain

<220>
<221> misc_feature
<222> (1)..(1)
<223> Xaa is Leu, Gln, ser, or Glu

<220>
<221> misc_feature
<222> (2)..(2)
<223> Xaa is Ser, Ala, Pro, Ile, or Tyr

<220>
<221> misc_feature
<222> (4)..(4)
<223> Xaa is Val, Leu, or Met

<220>
<221> misc_feature
<222> (7)..(7)
<223> Xaa is Ser, Glu, or Pro

<220>
<221> misc_feature
<222> (8)..(8)
<223> Xaa can be any naturally occurring amino acid

<220>
<221> misc_feature
<222> (11)..(11)
<223> Xaa is Ala or Val

<220>
<221> misc_feature
<222> (14)..(14)
<223> Xaa is Thr, Ser, or Ala

<220>
<221> misc_feature
<222> (25)..(42)
<223> CDR L1; Xaa can be any naturally occurring amino acid

<220>
<221> misc_feature
<222> (46)..(46)
<223> Xaa is His or Gln

<220>
<221> misc_feature
<222> (53)..(53)
<223> Xaa is Thr, Lys, Ser, Asn, Gln, or Pro

<220>
<221> misc_feature
<222> (58)..(72)
<223> CDR L2; Xaa can be any naturally occurring amino acid

<220>
<221> misc_feature
<222> (82)..(82)
<223> Xaa is Arg, Gln, or Lys

<220>
<221> misc_feature
<222> (85)..(86)
<223> Xaa can be any naturally occurring amino acid

<220>
<221> misc_feature
<222> (92)..(92)
<223> Xaa is Thr, Gly, Asp, or Ala

<220>
<221> misc_feature
<222> (103)..(103)

<223> Xaa is val, Asp, Thr, His, or Glu

<220>
<221> misc_feature
<222> (107)..(138)
<223> CDR L3; Xaa can be any naturally occurring amino acid

<400> 6

```
Xaa Xaa Val Xaa Thr Gln Xaa Xaa Pro Ser Xaa Ser Gly Xaa Pro Gly
1               5                   10                  15

Gln Arg Val Thr Ile Ser Cys Ser Xaa Xaa Xaa Xaa Xaa Xaa Xaa Xaa
            20                  25                  30

Xaa Xaa Xaa Xaa Xaa Xaa Xaa Xaa Xaa Xaa Trp Tyr Gln Xaa Leu Pro
        35                  40                  45

Gly Thr Ala Pro Xaa Leu Leu Ile Tyr Xaa Xaa Xaa Xaa Xaa Xaa Xaa
    50                  55                  60

Xaa Xaa Xaa Xaa Xaa Xaa Xaa Xaa Gly Val Pro Asp Arg Phe Ser Gly
65                  70                  75                  80

Ser Xaa Ser Gly Xaa Xaa Thr Ser Ala Ser Leu Xaa Ile Ser Gly Leu
                85                  90                  95

Gln Ser Glu Asp Glu Ala Xaa Tyr Tyr Cys Xaa Xaa Xaa Xaa Xaa Xaa
            100                 105                 110

Xaa Xaa Xaa Xaa Xaa Xaa Xaa Xaa Xaa Xaa Xaa Xaa Xaa Xaa Xaa Xaa
        115                 120                 125

Xaa Xaa Xaa Xaa Xaa Xaa Xaa Xaa Xaa Xaa Phe Gly Gly Gly Thr Lys
    130                 135                 140

Leu Thr Val Leu Gly
145
```

<210> 7
<211> 236
<212> PRT
<213> unknown

<220>
<223> Fig. 9 - VH1 Family Heavy Chain

<220>
<221> misc_feature
<222> (1) .. (1)
<223> Xaa is Gln or Glu

<220>
<221> misc_feature

<222> (6)..(6)
<223> Xaa is Gln or Glu

<220>
<221> misc_feature
<222> (11)..(11)
<223> Xaa is Val or Leu

<220>
<221> misc_feature
<222> (12)..(12)
<223> Xaa is Lys or Met

<220>
<221> misc_feature
<222> (13)..(13)
<223> Xaa is Lys, Glu, or Gln

<220>
<221> misc_feature
<222> (18)..(18)
<223> Xaa is Val or Leu

<220>
<221> misc_feature
<222> (20)..(20)
<223> Xaa is Val or Ile

<220>
<221> misc_feature
<222> (26)..(29)
<223> Xaa can be any naturally occurring amino acid

<220>
<221> misc_feature
<222> (30)..(79)
<223> CDR H1; at least 3 and up to 50 amino acids can be present or absent; if present, Xaa can be any naturally occurring amino acid

<220>
<221> misc_feature
<222> (94)..(143)
<223> CDR H2; at least 3 and up to 50 amino acids can be present or absent; if present, Xaa can be any naturally occurring amino acid

<220>
<221> misc_feature
<222> (157)..(157)
<223> Xaa is Tyr, Phe, Ser, His, or Asp

<220>
<221> misc_feature
<222> (159)..(159)
<223> Xaa is Glu, Asp, Gln

<220>
<221> misc_feature
<222> (162)..(162)

<223> Xaa is Ser, Gly, Thr, Asn

<220>
<221> misc_feature
<222> (165)..(165)
<223> Xaa is Ser, Phe, Thr, Ala, Pro

<220>
<221> misc_feature
<222> (176)..(225)
<223> CDR H3; at least 3 and up to 50 amino acids can be present or absent; if present, Xaa can be any naturally occurring amino acid

<400> 7

```
Xaa Val Gln Leu Val Xaa Ser Gly Ala Glu Xaa Xaa Xaa Pro Gly Ser
1               5                   10                  15

Ser Xaa Lys Xaa Ser Cys Lys Ala Ser Xaa Xaa Xaa Xaa Xaa Xaa Xaa
            20                  25                  30

Xaa Xaa Xaa Xaa Xaa Xaa Xaa Xaa Xaa Xaa Xaa Xaa Xaa Xaa Xaa Xaa
        35                  40                  45

Xaa Xaa Xaa Xaa Xaa Xaa Xaa Xaa Xaa Xaa Xaa Xaa Xaa Xaa Xaa Xaa
    50                  55                  60

Xaa Xaa Xaa Xaa Xaa Xaa Xaa Xaa Xaa Xaa Xaa Xaa Xaa Xaa Xaa Trp
65                  70                  75                  80

Val Arg Gln Ala Pro Gly Gln Gly Leu Glu Trp Met Gly Xaa Xaa Xaa
                85                  90                  95

Xaa Xaa Xaa Xaa Xaa Xaa Xaa Xaa Xaa Xaa Xaa Xaa Xaa Xaa Xaa Xaa
```

```
                100                 105                 110

Xaa Xaa Xaa Xaa Xaa Xaa Xaa Xaa Xaa Xaa Xaa Xaa Xaa Xaa Xaa Xaa
        115                 120                 125

Xaa Xaa Xaa Xaa Xaa Xaa Xaa Xaa Xaa Xaa Xaa Xaa Xaa Xaa Xaa Arg
    130                 135                 140

Val Thr Ile Thr Ala Asp Glu Ser Thr Ser Thr Ala Xaa Met Xaa Leu
145                 150                 155                 160

Ser Xaa Leu Arg Xaa Glu Asp Thr Ala Val Tyr Tyr Cys Ala Arg Xaa
                165                 170                 175

Xaa Xaa Xaa Xaa Xaa Xaa Xaa Xaa Xaa Xaa Xaa Xaa Xaa Xaa Xaa Xaa
            180                 185                 190

Xaa Xaa Xaa Xaa Xaa Xaa Xaa Xaa Xaa Xaa Xaa Xaa Xaa Xaa Xaa Xaa
        195                 200                 205

Xaa Xaa Xaa Xaa Xaa Xaa Xaa Xaa Xaa Xaa Xaa Xaa Xaa Xaa Xaa Xaa
    210                 215                 220

Xaa Trp Gly Gln Gly Thr Leu Val Thr Val Ser Ser
225                 230                 235
```

<210> 8
<211> 236
<212> PRT
<213> Unknown

<220>
<223> Fig. 10 - vH1B Family Heavy Chain

<220>
<221> misc_feature
<222> (1)..(1)
<223> Xaa is Gln or Glu

<220>
<221> misc_feature
<222> (9)..(9)
<223> Xaa is Ala, Thr, Asp, Pro, or Val

<220>
<221> mi sc_feature
<222> (11)..(11)
<223> Xaa is Val or Leu

<220>
<221> misc_feature
<222> (12)..(12)
<223> Xaa is Lys, Val, Arg, Gln, Met

<220>
<221> misc_feature
<222> (13)..(13)
<223> Xaa is Lys, Arg, Glu, or Met

<220>
<221> misc_feature
<222> (19)..(19)
<223> Xaa is Lys, Asn, Arg, or Thr

<220>
<221> misc_feature
<222> (20)..(20)
<223> Xaa is Val, Leu, Ile, or Phe

<220>
<221> misc_feature
<222> (26)..(29)
<223> Xaa can be any naturally occurring amino acid

<220>
<221> misc_feature
<222> (30)..(79)
<223> CDR H1; at least 3 and up to 50 amino acids can be present or absent; if present, Xaa can be any naturally occurring amino aci

<220>
<221> misc_feature
<222> (82)..(82)
<223> Xaa is Arg or Lys

<220>
<221> misc_feature
<222> (84)..(84)
<223> Xaa is Ala, Arg, Thr, Val or Pro

<220>
<221> misc_feature
<222> (87)..(87)
<223> Xaa is Gln, Lys, Glu, or His

<220>
<221> misc_feature
<222> (92)..(92)
<223> Xaa is Met or Ile

<220>
<221> misc_feature
<222> (94)..(143)
<223> CDR H2; at least 3 and up to 50 amino acids can be present or absent; if present, Xaa can be any naturally occurring amino acid

<220>
<221> misc_feature
<222> (144)..(144)
<223> Xaa is Arg or Lys

<220>

<221> misc_feature
<222> (145)..(145)
<223> Xaa is Val, Ala, Ile, or Leu

<220>
<221> misc_feature
<222> (149)..(149)
<223> Xaa is Glu, Arg, Thr, Ala

<220>
<221> misc_feature
<222> (153)..(153)
<223> Xaa is Thr, Ser, Ile, Leu

<220>
<221> misc_feature
<222> (154)..(154)
<223> Xaa is Asp, Ser, Asn, Gly

<220>
<221> misc_feature
<222> (174)..(174)
<223> Xaa is Ala, Asn, or Ser

<220>
<221> misc_feature
<222> (176)..(225)
<223> CDR H3; at least 3 and up to 50 amino acids can be present or absent; if present, Xaa can be any naturally occurring amino acid

<400> 8

Xaa Val Gln Leu Val Gln Ser Gly Xaa Glu Xaa Xaa Xaa Pro Gly Ala
1 5 10 15

Ser Val Xaa Xaa Ser Cys Lys Ala Ser Xaa Xaa Xaa Xaa Xaa Xaa Xaa
20 25 30

Xaa Xaa Xaa Xaa Xaa Xaa Xaa Xaa Xaa Xaa Xaa Xaa Xaa Xaa Xaa Xaa
35 40 45

Xaa Xaa Xaa Xaa Xaa Xaa Xaa Xaa Xaa Xaa Xaa Xaa Xaa Xaa Xaa Xaa
50 55 60

Xaa Xaa Xaa Xaa Xaa Xaa Xaa Xaa Xaa Xaa Xaa Xaa Xaa Xaa Xaa Trp
65 70 75 80

Val Xaa Gln Xaa Pro Gly Xaa Gly Leu Glu Trp Xaa Gly Xaa Xaa Xaa
85 90 95

Xaa Xaa Xaa Xaa Xaa Xaa Xaa Xaa Xaa Xaa Xaa Xaa Xaa Xaa Xaa Xaa
100 105 110

Xaa Xaa Xaa Xaa Xaa Xaa Xaa Xaa Xaa Xaa Xaa Xaa Xaa Xaa Xaa Xaa
115 120 125

Xaa Xaa Xaa Xaa Xaa Xaa Xaa Xaa Xaa Xaa Xaa Xaa Xaa Xaa Xaa Xaa
130 135 140

Xaa Thr Met Thr Xaa Asp Thr Ser Xaa Xaa Thr Ala Tyr Met Glu Leu
145 150 155 160

Ser Ser Leu Arg Ser Glu Asp Thr Ala Val Tyr Tyr Cys Xaa Arg Xaa
165 170 175

Xaa Xaa Xaa Xaa Xaa Xaa Xaa Xaa Xaa Xaa Xaa Xaa Xaa Xaa Xaa Xaa
180 185 190

Xaa Xaa Xaa Xaa Xaa Xaa Xaa Xaa Xaa Xaa Xaa Xaa Xaa Xaa Xaa Xaa

195 200 205

Xaa Xaa Xaa Xaa Xaa Xaa Xaa Xaa Xaa Xaa Xaa Xaa Xaa Xaa Xaa Xaa
210 215 220

Xaa Trp Gly Gln Gly Thr Leu Val Thr Val Ser Ser
225 230 235

<210> 9
<211> 236
<212> PRT
<213> Unknown

<220>

<223> Fig. 11 - VH3 Family Heavy Chain

<220>
<221> misc_feature
<222> (1) .. (1)
<223> Xaa is Glu or Gln

<220>
<221> misc_feature
<222> (6)..(6)
<223> Xaa is Glu or Gln

<220>
<221> misc_feature
<222> (7)..(7)
<223> Xaa is Ser, Thr, or Ala

<220>
<221> misc_feature
<222> (27)..(29)
<223> Xaa can be any naturally occurring amino acid

<220>
<221> misc_feature
<222> (30)..(79)
<223> CDR H1; at least 3 and up to 50 amino acids can be present or absent; if present, Xaa can be any naturally occurring amino acid

<220>
<221> misc_feature
<222> (94)..(143)
<223> CDR H2; at least 3 and up to 50 amino acids can be present or absent; if present, Xaa can be any naturally occurring amino acid

<220>
<221> misc_feature
<222> (156)..(156)
<223> xaa is Leu, Val, Ala, or Phe

<220>
<221> misc_feature
<222> (170)..(170)
<223> Xaa is Val, Leu, Ile, Met, Phe, Arg, or Gln

<220>
<221> misc_feature
<222> (176)..(225)
<223> CDR H3; at least 3 and up to 50 amino acids can be present or absent; if present, Xaa can be any naturally occurring amino acid

<400> 9

```
Xaa Val Gln Leu Val Xaa Xaa Gly Pro Gly Leu Val Lys Pro Ser Glu
1               5                   10                  15

Thr Leu Arg Leu Ser Cys Ala Ala Ser Gly Xaa Xaa Xaa Xaa Xaa Xaa
            20                  25                  30

Xaa Xaa Xaa Xaa Xaa Xaa Xaa Xaa Xaa Xaa Xaa Xaa Xaa Xaa Xaa Xaa
        35                  40                  45

Xaa Xaa Xaa Xaa Xaa Xaa Xaa Xaa Xaa Xaa Xaa Xaa Xaa Xaa Xaa Xaa
    50                  55                  60

Xaa Xaa Xaa Xaa Xaa Xaa Xaa Xaa Xaa Xaa Xaa Xaa Xaa Xaa Xaa Trp
65                  70                  75                  80

Val Arg Gln Ala Pro Gly Lys Gly Leu Glu Trp Val Ser Xaa Xaa Xaa
                85                  90                  95

Xaa Xaa Xaa Xaa Xaa Xaa Xaa Xaa Xaa Xaa Xaa Xaa Xaa Xaa Xaa Xaa
            100                 105                 110

Xaa Xaa Xaa Xaa Xaa Xaa Xaa Xaa Xaa Xaa Xaa Xaa Xaa Xaa Xaa Xaa
        115                 120                 125

Xaa Xaa Xaa Xaa Xaa Xaa Xaa Xaa Xaa Xaa Xaa Xaa Xaa Xaa Xaa Arg
    130                 135                 140

Phe Thr Ile Ser Arg Asp Asn Ser Lys Asn Thr Xaa Tyr Leu Gln Met
145                 150                 155                 160

Asn Ser Leu Arg Ala Glu Asp Thr Ala Xaa Tyr Tyr Cys Ala Arg Xaa
                165                 170                 175

Xaa Xaa Xaa Xaa Xaa Xaa Xaa Xaa Xaa Xaa Xaa Xaa Xaa Xaa Xaa Xaa
            180                 185                 190

Xaa Xaa Xaa Xaa Xaa Xaa Xaa Xaa Xaa Xaa Xaa Xaa Xaa Xaa Xaa Xaa
        195                 200                 205

Xaa Xaa Xaa Xaa Xaa Xaa Xaa Xaa Xaa Xaa Xaa Xaa Xaa Xaa Xaa Xaa
    210                 215                 220

Xaa Trp Gly Gln Gly Thr Leu Val Thr Val Ser Ser
225                 230                 235
```

<210> 10
<211> 234
<212> PRT
<213> Unknown

<220>
<223> Fig. 12 - Vk1 Family Light Chain

<220>
<221> misc_feature
<222> (1) .. (1)
<223> Xaa is Glu, Asp, or Ile

<220>
<221> misc_feature
<222> (3)..(3)
<223> Xaa is Val, Gln, or Ile

<220>
<221> misc_feature
<222> (4)..(4)
<223> Xaa is Leu, Met, Val, or Ile

<220>
<221> misc_feature
<222> (24)..(24)
<223> Xaa is Gln or Arg

<220>
<221> misc_feature
<222> (25)..(74)
<223> CDR L1; at least 3 and up to 50 amino acids can be present or absent; if present, Xaa can be any naturally occurring amino acid

<220>
<221> misc_feature
<222> (79)..(79)
<223> Xaa is Arg, Lys, or Ile

<220>
<221> misc_feature
<222> (82)..(82)
<223> Xaa is Lys, Arg, Glu, Thr, Met, or Gln

<220>
<221> misc_feature
<222> (89)..(89)
<223> Xaa is ser, Tyr, Phe, or His

<220>
<221> misc_feature
<222> (90)..(139)
<223> CDR L2; at least 3 and up to 50 amino acids can be present or absent; if present, Xaa can be any naturally occurring amino acid

<220>
<221> misc_feature
<222> (152)..(153)
<223> Xaa can be any naturally occurring amino acid

<220>
<221> misc_feature
<222> (158)..(158)
<223> Xaa is Phe or Leu

<220>

<221> misc_feature
<222> (170)..(170)
<223> Xaa is Val, Thr, Ser, Gly, or Ile

<220>
<221> misc_feature
<222> (174)..(223)
<223> CDR L3; at least 3 and up to 50 amino acids can be present or absent; if present, Xaa can be any naturally occurring amino acid

<400> 10

```
Xaa Ile Xaa Xaa Thr Gln Ser Pro Ser Ser Leu Ser Ala Ser Val Gly
1               5                   10                  15

Asp Arg Val Thr Ile Thr Cys Xaa Xaa Xaa Xaa Xaa Xaa Xaa Xaa Xaa
            20                  25                  30

Xaa Xaa Xaa Xaa Xaa Xaa Xaa Xaa Xaa Xaa Xaa Xaa Xaa Xaa Xaa Xaa
        35                  40                  45

Xaa Xaa Xaa Xaa Xaa Xaa Xaa Xaa Xaa Xaa Xaa Xaa Xaa Xaa Xaa Xaa
    50                  55                  60

Xaa Xaa Xaa Xaa Xaa Xaa Xaa Xaa Xaa Xaa Trp Tyr Gln Gln Xaa Pro
65                  70                  75                  80

Gly Xaa Ala Pro Lys Leu Leu Ile Xaa Xaa Xaa Xaa Xaa Xaa Xaa Xaa
                85                  90                  95

Xaa Xaa Xaa Xaa Xaa Xaa Xaa Xaa Xaa Xaa Xaa Xaa Xaa Xaa Xaa Xaa
            100                 105                 110

Xaa Xaa Xaa Xaa Xaa Xaa Xaa Xaa Xaa Xaa Xaa Xaa Xaa Xaa Xaa Xaa
        115                 120                 125

Xaa Xaa Xaa Xaa Xaa Xaa Xaa Xaa Xaa Xaa Xaa Gly Val Pro Ser Arg
    130                 135                 140

Phe Ser Gly Ser Gly Ser Gly Xaa Xaa Thr Asp Phe Thr Xaa Thr Ile
145                 150                 155                 160

Ser Ser Leu Gln Pro Glu Asp Phe Ala Xaa Tyr Tyr Cys Xaa Xaa Xaa
                165                 170                 175

Xaa Xaa Xaa Xaa Xaa Xaa Xaa Xaa Xaa Xaa Xaa Xaa Xaa Xaa Xaa Xaa
            180                 185                 190

Xaa Xaa Xaa Xaa Xaa Xaa Xaa Xaa Xaa Xaa Xaa Xaa Xaa Xaa Xaa Xaa
        195                 200                 205

Xaa Xaa Xaa Xaa Xaa Xaa Xaa Xaa Xaa Xaa Xaa Xaa Xaa Xaa Xaa Phe
    210                 215                 220

Gly Gln Gly Thr Lys Val Glu Ile Lys Arg
225                 230
```

<210> 11
<211> 234
<212> PRT
<213> Unknown

<220>
<223> Fig. 13 - vk3 Family Light chain

<220>
<221> misc_feature
<222> (2)..(2)
<223> Xaa is Thr or Ile

<220>
<221> misc_feature
<222> (3)..(3)
<223> Xaa is Thr or Val

<220>
<221> misc_feature
<222> (10)..(10)
<223> Xaa is Ile or Thr

<220>
<221> miss_feature
<222> (12)..(12)
<223> Xaa is Tyr or ser

<220>
<221> misc_feature
<222> (18)..(18)
<223> Xaa is Ser or Arg

<220>
<221> misc_feature
<222> (20)..(20)
<223> Xaa is Ala or Thr

<220>
<221> misc_feature
<222> (25)..(74)
<223> CDR L1; at least 3 and up to 50 amino acids can be present or absent; if present, Xaa can be any naturally occurring amino acid

<220>
<221> misc_feature
<222> (88)..(88)
<223> Xaa is Met or Ile

<220>
<221> misc_feature
<222> (90)..(139)
<223> CDR L2; at least 3 and up to 50 amino acids can be present or absent; if present, Xaa can be any naturally occurring amino acid

<220>
<221> misc_feature
<222> (141)..(141)
<223> Xaa is Thr, Val, or Ile

<220>
<221> misc_feature
<222> (152)..(153)
<223> Xaa can be any naturally occurring amino acid

<220>

<221> misc_feature
<222> (161)..(161)
<223> Xaa is Asn or Ser

<220>
<221> misc_feature
<222> (168)..(168)
<223> Xaa is ser, Tyr, or Phe

<220>
<221> misc_feature
<222> (170)..(170)
<223> Xaa is Ala, Leu, or Val

<220>
<221> misc_feature
<222> (174)..(223)
<223> CDR L3; at least 3 and up to 50 amino acids can be present or absent; if present, Xaa can be any naturally occurring amino acid

<400> 11

Glu Xaa Xaa Leu Thr Gln Ser Pro Gly Xaa Leu Xaa Leu Ser Pro Gly
1               5                   10                  15

Glu Xaa Ala Xaa Leu Ser Cys Arg Xaa Xaa Xaa Xaa Xaa Xaa Xaa Xaa
            20                  25                  30

Xaa Xaa Xaa Xaa Xaa Xaa Xaa Xaa Xaa Xaa Xaa Xaa Xaa Xaa Xaa Xaa
        35                  40                  45

Xaa Xaa Xaa Xaa Xaa Xaa Xaa Xaa Xaa Xaa Xaa Xaa Xaa Xaa Xaa Xaa
    50                  55                  60

Xaa Xaa Xaa Xaa Xaa Xaa Xaa Xaa Xaa Xaa Trp Tyr Gln Gln Lys Pro
65                  70                  75                  80

Gly Gln Ala Pro Arg Leu Leu Xaa Tyr Xaa Xaa Xaa Xaa Xaa Xaa Xaa
                85                  90                  95

Xaa Xaa Xaa Xaa Xaa Xaa Xaa Xaa Xaa Xaa Xaa Xaa Xaa Xaa Xaa Xaa
            100                 105                 110

Xaa Xaa Xaa Xaa Xaa Xaa Xaa Xaa Xaa Xaa Xaa Xaa Xaa Xaa Xaa Xaa
        115                 120                 125

Xaa Xaa Xaa Xaa Xaa Xaa Xaa Xaa Xaa Xaa Xaa Gly Xaa Pro Asp Arg
    130                 135                 140

Phe Ser Gly Ser Gly Ser Gly Xaa Xaa Thr Asp Phe Thr Leu Thr Ile
145                 150                 155                 160

Xaa Arg Leu Glu Pro Glu Asp Xaa Ala Xaa Tyr Tyr Cys Xaa Xaa Xaa
                165                 170                 175

Xaa Xaa Xaa Xaa Xaa Xaa Xaa Xaa Xaa Xaa Xaa Xaa Xaa Xaa Xaa Xaa
            180                 185                 190

Xaa Xaa Xaa Xaa Xaa Xaa Xaa Xaa Xaa Xaa Xaa Xaa Xaa Xaa Xaa Xaa
        195                 200                 205

Xaa Xaa Xaa Xaa Xaa Xaa Xaa Xaa Xaa Xaa Xaa Xaa Xaa Xaa Xaa Phe
    210                 215                 220

Gly Gly Gly Thr Lys Leu Glu Ile Lys Arg
225                 230

<210> 12
<211> 234
<212> PRT
<213> Unknown

<220>
<223> Fig. 14 - VL1 Family Light Chain

<220>
<221> misc_feature
<222> (1)..(1)
<223> Xaa is Leu, Gln, Ser, or Glu

<220>
<221> misc_feature
<222> (2)..(2)
<223> Xaa is Ser, Ala, Pro, Ile, or Tyr

<220>
<221> misc_feature
<222> (4)..(4)
<223> Xaa is Val, Leu, or Met

<220>
<221> misc_feature
<222> (7)..(7)
<223> Xaa is Ser, Glu, or Pro

<220>
<221> misc_feature
<222> (8)..(8)
<223> Xaa can be any naturally occurring amino acid

<220>
<221> misc_feature
<222> (11)..(11)
<223> xaa is Ala or Val

<220>
<221> misc_feature
<222> (14)..(14)
<223> Xaa is Thr, Ser, or Ala

<220>
<221> misc_feature
<222> (25)..(74)
<223> CDR L1; at least 3 and up to 50 amino acids can be present or absent; if present, Xaa can be any naturally occurring amino acid

<220>
<221> misc_feature
<222> (78)..(78)
<223> Xaa is His or Gln

<220>
<221> misc_feature
<222> (85)..(85)
<223> Xaa is Thr, Lys, Ser, Asn, Gln, or Pro

<220>
<221> misc_feature
<222> (90)..(139)
<223> CDR L2; at least 3 and up to 50 amino acids can be present or absent; if present, Xaa can be any naturally occurring amino acid

<220>

<221> misc_feature
<222> (149)..(149)
<223> Xaa is Arg, Gln, or Lys

<220>
<221> misc_feature
<222> (152)..(153)
<223> Xaa can be any naturally occurring amino acid

<220>
<221> misc_feature
<222> (159)..(159)
<223> Xaa is Thr, Gly, Asp, or Ala

<220>
<221> misc_feature
<222> (170)..(170)
<223> Xaa is Val, Asp, Thr, His, or Glu

<220>
<221> misc_feature
<222> (174)..(223)
<223> CDR L3; at least 3 and up to 50 amino acids can be present or absent; if present, Xaa can be any naturally occurring amino acid

<400> 12

```
Xaa Xaa Val Xaa Thr Gln Xaa Xaa Pro Ser Xaa Ser Gly Xaa Pro Gly
1               5                   10                  15

Gln Arg Val Thr Ile Ser Cys Ser Xaa Xaa Xaa Xaa Xaa Xaa Xaa Xaa
            20                  25                  30

Xaa Xaa Xaa Xaa Xaa Xaa Xaa Xaa Xaa Xaa Xaa Xaa Xaa Xaa Xaa Xaa
        35                  40                  45

Xaa Xaa Xaa Xaa Xaa Xaa Xaa Xaa Xaa Xaa Xaa Xaa Xaa Xaa Xaa Xaa
    50                  55                  60

Xaa Xaa Xaa Xaa Xaa Xaa Xaa Xaa Xaa Xaa Trp Tyr Gln Xaa Leu Pro
65                  70                  75                  80

Gly Thr Ala Pro Xaa Leu Leu Ile Tyr Xaa Xaa Xaa Xaa Xaa Xaa Xaa
                85                  90                  95

Xaa Xaa Xaa Xaa Xaa Xaa Xaa Xaa Xaa Xaa Xaa Xaa Xaa Xaa Xaa Xaa
            100                 105                 110
```

```
Xaa Xaa Xaa Xaa Xaa Xaa Xaa Xaa Xaa Xaa Xaa Xaa Xaa Xaa Xaa Xaa
        115                 120                 125

Xaa Xaa Xaa Xaa Xaa Xaa Xaa Xaa Xaa Xaa Xaa Gly Val Pro Asp Arg
    130                 135                 140

Phe Ser Gly Ser Xaa Ser Gly Xaa Xaa Thr Ser Ala Ser Leu Xaa Ile
145                 150                 155                 160

Ser Gly Leu Gln Ser Glu Asp Glu Ala Xaa Tyr Tyr Cys Xaa Xaa Xaa
                165                 170                 175

Xaa Xaa Xaa Xaa Xaa Xaa Xaa Xaa Xaa Xaa Xaa Xaa Xaa Xaa Xaa Xaa
            180                 185                 190

Xaa Xaa Xaa Xaa Xaa Xaa Xaa Xaa Xaa Xaa Xaa Xaa Xaa Xaa Xaa Xaa
        195                 200                 205

Xaa Xaa Xaa Xaa Xaa Xaa Xaa Xaa Xaa Xaa Xaa Xaa Xaa Xaa Xaa Phe
    210                 215                 220

Gly Gly Gly Thr Lys Leu Thr Val Leu Gly
225                 230
```

## Description

This application claims priority to U.S. Provisional Application Serial No. 60/937,112, entitled "Sequence Based Engineering and Optimization of Single Chain Antibodies", filed on June 25, 2007. This application also claims priority to U.S. Provisional Application Serial No. 61/069,056, entitled "Methods of Modifying Antibodies, and Modified Antibodies with Improved Functional Properties", filed on March 12, 2008.

## Background of the Invention

Antibodies have proven to be very effective and successful therapeutic agents in the treatment of cancer, autoimmune diseases and other disorders. While full-length antibodies typically have been used clinically, there are a number of advantages that use of an antibody fragment can provide, such as increased tissue penetration, absence of Fc-effector function combined with the ability to add other effector functions and the likelihood of less systemic side effects resulting from a shorter *in vivo* half life systemically. The pharmacokinetic properties of antibody fragments indicate that they may be particularly well suited for local therapeutic approaches. Furthermore, antibody fragments can be easier to produce than full-length antibodies in certain expression systems.

One type of antibody fragment is a single chain antibody (scFv), which is composed of a heavy chain variable domain ($V_H$) conjugated to a light chain variable domain ($V_L$) via a linker sequence. Thus, scFvs lack all antibody constant region domains and the amino acid residues of the former variable/constant domain interface (interfacial residues) become solvent exposed. A scFv can be prepared from a full-length antibody (e.g., IgG molecule) through established recombinant engineering techniques. The transformation of a full length antibody into a scFv, however, often results in poor stability and solubility of the protein, low production yields and a high tendency to aggregate, which raises the risk of immunogenicity.

Accordingly, attempts have been made to improve properties such as solubility and stability of scFvs. For example, Nieba, L. et al. (Prot. Eng. (1997) 10:435-444) selected three amino acid residues known to be interfacial residues and mutated them. They observed increased periplasmic expression of the mutated scFv in bacteria, as well as a decreased rate of thermally induced aggregation, although thermodynamic stability and solubility were not significantly altered. Other studies in which site directed mutagenesis was carried out on particular amino acid residues within the scFv also have been reported (see *e.g.,* Tan, P.H. et al. (1988) Biophys. J. 75:1473-1482; Wörn, A. and Pluckthun, A. (1998) Biochem. 37:13120-13127; Wörn, A. and Plückthun, A. (1999) Biochem. 38:8739-8750). In these various studies, the amino acid residues selected for mutagenesis were chosen based on their known positions within the scFv structure (e.g., from molecular modeling studies).

In another approach, the complementarity determining regions (CDRs) from a very poorly expressed scFv were grafted into the framework regions of a scFv that had been demonstrated to have favorable properties (Jung, S. and Pluckthun, A. (1997) Prot. Eng. 10:959-966). The resultant scFv showed improved soluble expression and thermodynamic stability.

Progress in the engineering of scFvs to improve functional properties is reviewed in, for example, Wörn, A. and Pluckthun, A. (2001) J. Mol. Biol. 305:989-1010. New approaches, however, are still needed that allow for rational design of scFvs with superior functional properties, in particular approaches that assist the skilled artisan in selection of potentially problematic amino acid residues for engineering. Moreover, methods of engineering scFvs, and other types of antibodies, to thereby impart improved functional properties, such as increased stability and/or solubility properties, are still needed.

## Summary of the Invention

This invention provides methods of engineering immunobinders, such as scFv antibodies, based on sequence analysis of stable, soluble scFv frameworks that allowed for the identification of amino acids within a scFv sequence that are potentially problematic for stability and/or solubility and the identification of preferred amino acid residue substitutions at such amino acid positions. Thus, amino acid residues identified in accordance with the methods of the invention can be selected for mutation and engineered immunobinders, such as scFvs, that have been mutated can be prepared and screened for improved functional properties such as stability and/or solubility. The invention provides, and demonstrates the benefit of, a "functional consensus" approach to identify preferred amino acid substitutions within scFv frameworks based on the use of a database of functionally-selected scFv sequences.

Accordingly, the invention provides methods of engineering immunobinders (e.g., scFvs) by mutating particular framework amino acid positions to specified amino acid residues identified using the "functional consensus" approach described herein. Still further, the invention provides scFv framework scaffolds, designed based on the "functional consensus" approach described herein, that can be used as the framework sequence into which CDR sequences of interest can be inserted to create an immunobinder, *e.g.*, scFv, against a target antigen of interest.

Preferably, the immunobinder used in, or produced by, the engineering methods of the invention is a scFv, but other immunobinders, such as full-length immunogloblins, Fab fragments, single domain antibodies (e.g., Dabs) and Nanobodies also can be engineered according to the method. The invention also encompasses immunobinders prepared according to the engineering method, as well as compositions comprising the immunobinders and a pharmaceutically acceptable carrier.

In one aspect, the invention provides a method of engineering an immunobinder, the immunobinder comprising (i) a heavy chain variable region, or fragment thereof, the heavy chain variable region comprising $V_H$ framework residues and/or (ii) a light chain variable region, or fragment thereof, the light chain variable region comprising $V_L$ framework residues, the method comprising:

A) selecting one or more amino acid positions within the $V_H$ framework residues, the $V_L$ framework residues or the $V_H$ and $V_L$ framework residues for mutation; and
B) mutating the one or more amino acid positions selected for mutation, wherein the one or more amino acid positions selected for mutation, and the amino acid residue(s) inserted at the seleced position(s) are described in further detail below.

The amino acid position numbering set forth below uses the AHo numbering system; the corresponding positions using the Kabat numbering system are described further herein and the conversion tables for the AHo and Kabat numbering systems are set forth in Example 1. The amino acid residues are set forth using standard one letter abbreviation code.

In one embodiment, wherein if the one or more amino acid positions selected for mutation are of a heavy chain variable region, the mutating comprises one or more substitutions selected from the group consisting of:

(a) Q or E at amino acid position 1;
(b) Q or E at amino acid position 6;
(c) T, S or A at amino acid position 7, more preferably T or A, even more preferably T;
(d) A, T, P, V or D, more preferably T, P, V or D, at amino acid position 10,
(e) L or V, more preferably L, at amino acid position 12,
(f) V, R, Q, M or K, more preferably V, R, Q or M at amino acid position 13;
(g) R, M, E, Q or K, more preferably R, M, E or Q, even more preferably R or E, at amino acid position 14;
(h) L or V, more preferably L, at amino acid position 19;
(i) R, T, K or N, more preferably R, T or N, even more preferably N, at amino acid position 20;
(j) I, F, L or V, more preferably I, F or L, even more preferably I or L, at amino acid position 21;
(k) R or K, more preferably K, at amino acid position 45;
(l) T, P, V, A or R, more preferably T, P, V or R, even more preferably R, at amino acid position 47;
(m) K, Q, H or E, more preferably K, H or E, even more preferably K, at amino acid position 50;
(n) M or I, more preferably I, at amino acid position 55;
(o) K or R, more preferably K, at amino acid position 77;
(p) A, V, L or I, more preferably A, L or I, even more preferably A, at amino acid position 78;
(q) E, R, T or A, more preferably E, T or A, even more preferably E, at amino acid position 82;
(r) T, S, I or L, more preferably T, S or L, even more preferably T, at amino acid position 86;
(s) D, S, N or G, more preferably D, N or G, even more preferably N, at amino acid position 87;
(t) A, V, L or F, more preferably A, V or F, even more preferably V, at amino acid position 89;
(u) F, S, H, D or Y, more preferably F, S, H or D, at amino acid position 90;
(v) D, Q or E, more preferably D or Q, even more preferably D, at amino acid position 92;
(w) G, N, T or S, more preferably G, N or T, even more preferably G, at amino acid position 95;
(x) T, A, P, F or S, more preferably T, A, P or F, even more preferably F, at amino acid position 98;
(y) R, Q, V, I, M, F, or L, more preferably R, Q, I, M, F or L, even more preferably Y or L, at amino acid position 103; and
(z) N, S or A, more preferably N or S, even more preferably N, at amino acid position 107.

In another embodiment, wherein if the one or more amino acid positions selected for mutation are of a light chain variable region, the mutating comprises one or more substitutions selected from the group consisting of:

(aa) Q, D, L, E, S, or I, more preferably L, E, S or I, even more preferably L or E, at amino acid position 1;
(bb) S, A, Y, I, P or T, more preferably A, Y, I, P or T, even more preferably P or T at amino acid position 2;
(cc) Q, V, T or I, more preferably V, T or I, even more preferably V or T, at amino acid position 3;
(dd) V, L, I or M, more preferably V or L, at amino acid position 4;
(ee) S, E or P, more preferably S or E, even more preferably S, at amino acid position 7;
(ff) T or I, more preferably I, at amino acid position 10;

(gg) A or V, more preferably A, at amino acid position 11;
(hh) S or Y, more preferably Y, at amino acid position 12;
(ii) T, S or A, more preferably T or S, even more preferably T, at amino acid position 14;
(jj) S or R, more preferably S, at amino acid position 18;
(kk) T or A, more preferably A, at amino acid position 20;
(11) R or Q, more preferably Q, at amino acid position 24;
(mm) H or Q, more preferably H, at amino acid position 46;
(nn) K, R or I, more preferably R or I, even more preferably R, at amino acid position 47;
(oo) R, Q, K, E, T, or M, more preferably Q, K, E, T or M, at amino acid position 50;
(pp) K, T, S, N, Q or P, more preferably T, S, N, Q or P, at amino acid position 53;
(qq) I or M, more preferably M, at amino acid position 56;
(rr) H, S, F or Y, more preferably H, S or F, at amino acid position 57;
(ss) I, V or T, more preferably V or T, R, even more preferably T, at amino acid position 74;
(tt) R, Q or K, more preferably R or Q, even more preferably R, at amino acid position 82;
(uu) L or F, more preferably F, at amino acid position 91;
(w) G, D, T or A, more preferably G, D or T, even more preferably T, at amino acid position 92;
(xx) S or N, more preferably N, at amino acid position 94;
(yy) F, Y or S, more preferably Y or S, even more preferably S, at amino acid position 101; and
(zz) D, F, H, E, L, A, T, V, S, G or I, more preferably H, E, L, A, T ,V, S, G or I, even more preferably A or V, at amino acid position 103.

In one embodiment, the heavy chain variable region, or fragment thereof, is of the VH3 family and, thus, wherein if the one or more amino acid positions selected for mutation are of the VH3 family heavy chain variable region, the mutating comprises one or more substitutions selected from the group consisting of:

(i) E or Q at amino acid position 1, more preferably Q;
(ii) E or Q at amino acid position 6, more preferably Q;
(iii) T, S or A at amino acid position 7, more preferably T or A, even more preferably T;
(iv) A, V, L or F at amino acid position 89, more preferably A, V or F, even more preferably V; and
(v) R, Q, V, I, L, M or F at amino acid position 103, more preferably R, Q, I, L, M or F, even more preferably L;

In another embodiment, the heavy chain variable region, or fragment thereof, is of the VH1a family and, thus, wherein if the one or more amino acid positions selected for mutation are of the VH1a family heavy chain variable region, the mutating comprises one or more substitutions selected from the group consisting of:

(i) E or Q at amino acid position 1, more preferably E;
(ii) E or Q at amino acid position 6, more preferably E;
(iii) L or V at amino acid position 12, more preferably L;
(iv) M or K at amino acid position 13, more preferably M:
(v) E, Q or K at amino acid position 14, more preferably E or Q, even more preferably E;
(vi) L or V at amino acid position 19, more preferably L;
(vii) I or V at amino acid position 21, more preferably I;
(viii) F, S, H, D or Y at amino acid position 90, more preferably F, S, H or D;
(ix) D, Q or E at amino acid position 92, more preferably D or Q, even more preferably D;
(x) G, N, T or S at amino acid position 95, more preferably G, N or T, even more preferably G; and
(xi) T, A, P, F or S at amino acid position 98, more preferably T, A, P or F, even more preferably F.

In another embodiment, the heavy chain variable region, or fragment thereof, is of the VH1b family and, thus, wherein if the one or more amino acid positions selected for mutation are of the VH1b family heavy chain variable region, the mutating comprises one or more substitutions selected from the group consisting of:

(i) E or Q at amino acid position 1, more preferably E;
(ii) A, T, P, V or D at amino acid position 10, more preferably T, P, V or D;
(iii) L or V at amino acid position 12, more preferably L;
(iv) K, V, R, Q or M at amino acid position 13, more preferably V, R, Q or M;
(v) E, K, R or M at amino acid position 14, more preferably E, R or M, even more preferably R;
(vi) R, T, K or N at amino acid position 20, more preferably R, T or N, even more preferably N;
(vii) I, F, V or L at amino acid position 21, more preferably I, F or L, even more preferably L;

(viii) R or K at amino acid position 45, more preferably K;
(ix) T, P, V, A, R at amino acid position 47, more preferably T, P, V or R, even more preferably R;
(x) K, Q, H or E at amino acid position 50, more preferably K, H or E, even more preferably K;
(xi) M or I at amino acid position 55; more preferably I;
(xii) K or R at amino acid position 77, more preferably K;
(xiii) A, V, L or I at amino acid position 78, more preferably A, L or I, even more preferably A;
(xiv) E, R, T or A at amino acid position 82, more preferably E, T or A, even more preferably E;
(xv) T, S, I or L at amino acid position 86, more preferably T, S or L, even more preferably T;
(xvi) D, S, N or G at amino acid position 87, more preferably D, N or G, even more preferably N; and
(xvii) N, S or A at amino acid position 107, more preferably N or S, even more preferably N.

In another embodiment, the light chain variable region, or fragment thereof, is of the Vκ1 family and, thus, wherein if the one or more amino acid positions selected for mutation are of the Vκ1 family light chain variable region, the mutating comprises one or more substitutions selected from the group consisting of:

(i) D, E or I at amino acid position 1, more preferably E or I, even more preferably E;
(ii) Q, V or I at amino acid position 3, more preferably V or I, even more preferably V;
(iii) V, L, I or M at amino acid position 4, more preferably V, L or I, even more preferably L;
(iv) R or Q at amino acid position 24, more preferably Q;
(v) K, R or I at amino acid position 47, more preferably R or I, even more preferably R;
(vi) K, R, E, T, M or Q at amino acid position 50, more preferably K, E, T, M or Q;
(vii) H, S, F or Y at amino acid position 57, more preferably H, S or F, even more preferably S;
(viii) L or F at amino acid position 91, more preferably F; and
(ix) T, V, S, G or I, more preferably V, S, G or I, even more preferably V, at amino acid position 103.

In another embodiment, the light chain variable region, or fragment thereof, is of the Vκ3 family and, thus, wherein if the one or more amino acid positions selected for mutation are of the Vκ3 family light chain variable region, the mutating comprises one or more substitutions selected from the group consisting of:

(i) I or T at amino acid position 2, more preferably T;
(ii) V or T at amino acid position 3, more preferably T;
(iii) T or I at amino acid position 10, more preferably I;
(iv) S or Y at amino acid position 12, more preferably Y;
(v) S or R at amino acid position 18, more preferably S;
(vi) T or A at amino acid position 20, more preferably A;
(vii) I or M at amino acid position 56, more preferably M;
(viii) I, V or T at amino acid position 74, more preferably V or T, even more preferably T;
(ix) S or N at amino acid position 94, more preferably N;
(x) F, Y or S at amino acid position 101, more preferably Y or S, even more preferably S; and
(xi) V, L or A at amino acid position 103, more preferably L or A, even more preferably A.

In another embodiment, the light chain variable region, or fragment thereof, is of the Vλ1 family and, thus, wherein if the one or more amino acid positions selected for mutation are of the Vλ1 family light chain variable region, the mutating comprises one or more substitutions selected from the group consisting of:

(i) L, Q, S or E at amino acid position 1, more preferably L, S or E, even more preferably L;
(ii) S, A, P, I or Y at amino acid position 2, more preferably A, P, I or Y, even more preferably P;
(iii) V, M or L at amino acid position 4, more preferably V or M, even more preferably V;
(iv) S, E or P at amino acid position 7, more preferably S or E, even more preferably S;
(v) A or V at amino acid position 11, more preferably A;
(vi) T, S or A at amino acid position 14, more preferably T or S, even more preferably T;
(vii) H or Q at amino acid position 46, more preferably H;
(viii) K, T, S, N, Q or P at amino acid position 53, more preferably T, S, N, Q or P;
(ix) R, Q or K at amino acid position 82, more preferably R or Q, even more preferably R;
(x) G, T, D or A at amino acid position 92, more preferably G, T or D, even more preferably T; and
(xi) D, V, T, H or E at amino acid position 103, more preferably V, T, H or E, even more preferably V.

In another embodiment, the mutating further comprises one or more (preferably all) heavy chain substitutions selected from the group consisting of:

(i) serine (S) at amino acid position 12 using AHo or Kabat;
(ii) serine (S) at amino acid position 103 using AHo numbering (amino acid position 85 using Kabat numbering); and
(iii) serine (S) or threonine (T) at amino acid position 144 using AHo numbering (amino acid position 103 using Kabat numbering).

In another aspect, the invention provides isolated antibody framework scaffolds (*e.g.,* scFv scaffolds). For example, in various embodiments, the invention provides an isolated heavy chain framework scaffold comprising an amino acid sequence as shown in Figure 9 (SEQ ID NO:1), Figure 10 (SEQ ID NO:2) or Figure 11 (SEQ ID NO:3). In another exemplary embodiment, the invention provides an isolated light chain framework scaffold comprising an amino acid sequence as shown in Figure 12 (SEQ ID NO:4), Figure 13 (SEQ ID NO:5) or Figure 14 (SEQ ID NO:6). Such scaffolds can be used to engineer immunobinders, such as scFv antibodies. Accordingly, in another aspect, the invention provides a method of engineering an immunobinder, the immunobinder comprising heavy and/or light chain CDR1, CDR2 and CDR3 sequences, the method comprising inserting the heavy and/or light chain CDR1, CDR2 and CDR3 sequences, respectively, into a heavy chain framework scaffold. In certain exemplary embodiments, the heavy chain framework scaffold comprises an amino acid sequence as shown in Figure 9 (SEQ ID NO:1), Figure 10 (SEQ ID NO:2), Figure11 (SEQ ID NO:3), SEQ ID NO:7, SEQ ID NO:8 or SEQ ID NO:9. In a preferred embodiment, the heavy chain framework scaffold comprises an amino acid sequence as shown in Figure 9 (SEQ ID NO:1). In another preferred embodiment, the heavy chain framework scaffold comprises an amino acid sequence as shown in Figure 10 (SEQ ID NO:2). In another preferred embodiment, the heavy chain framework scaffold comprises an amino acid sequence as shown in Figure 11 (SEQ ID NO:3). In another preferred embodiment, the heavy chain framework scaffold comprises an amino acid sequence of SEQ ID NO:7. In another preferred embodiment, the heavy chain framework scaffold comprises an amino acid sequence of SEQ ID NO:8. In yet another preferred embodiment, the heavy chain framework scaffold comprises an amino acid sequence of SEQ ID NO:9. In other exemplary embodiments, the light chain framework scaffold comprises an amino acid sequence as shown in Figure 12 (SEQ ID NO:4), Figure 13 (SEQ ID NO:5), Figure 14 (SEQ ID NO:6), SEQ ID NO:10, SEQ ID NO:11 or SEQ ID NO: 12. In a preferred embodiment, the light chain framework scaffold comprises an amino acid sequence as shown in Figure 11 (SEQ ID NO:4). In another preferred embodiment, the light chain framework scaffold comprises an amino acid sequence as shown in Figure 12 (SEQ ID NO:5). In another preferred embodiment, the light chain framework scaffold comprises an amino acid sequence as shown in Figure 13 (SEQ ID NO:6). In another preferred embodiment, the light chain framework scaffold comprises an amino acid sequence as shown in SEQ ID NO:10. In another preferred embodiment, the light chain framework scaffold comprises an amino acid sequence as shown in SEQ ID NO:11. In yet another preffered embodiment, the light chain framework scaffold comprises an amino acid sequence as shown in SEQ ID NO:12. Preferably, the immunobinder is a scFv antibody, although other immunobinders as described herein (e.g., full-length antibodies, Fabs, Dabs or Nanobodies) can be engineered according to the methods of the invention. The invention also provides immunobinder compositions, such as scFv antibodies, engineered according to the methods of the invention.

**Brief Description of Figures**

Figure 1 is a flowchart diagram summarizing general sequence-based analyses of scFvs according to the methods of the invention.
In a first step, a sequence of a scFv to be improved in solubility and stability is provided (box 1), which is subsequently compared to antibody sequence databases (box 2), such as open source germ line sequence databases (e.g., Vbase, IMGT; box 3), open source mature antibody sequence databases (e.g., KDB; box 4) or databases of fully human stable and soluble scFv fragments (e.g., QC; box 5).
Applying an open source germ line sequence databases such as described in box 3 allows for the identification of highly conserved positions that were selected during evolution and are therefore believed to contribute to the stability of variable domains in the full-length antibody context (box 3'). A comparison against an open source mature antibody sequence databases 4 permits the identification of patterns that represent improvements of stability, solubility and/or binding that are independent of the respective CDRs (box 4'). Moreover, the comparison against a databases of fully human stable and soluble scFv fragments (box 5) leads to the identification of residues critical for stability and/or solubility specifically in the scFv format, as well as the identification of patterns that represent improvements of stability, solubility and/or binding independet of the respective CDRs, specifically in the scFv format, e.g. VL and VH combinations (box 5').
In a next step (box 6), a substitution of critical residues by most frequent suitable amino acid as identified in the respective database is made.
Finally (box 7), random or biased mutagenesis of critical residues and subsequent screening for improved stability and/or solubility in the yeast QC-system may be performed. The mutants may undergo again the above mentioned procedure (arrow to box 2).

Figure 2 is a flowchart diagram of an exemplary multi-step method for sequence-based analysis of scFvs.

In a first step (box 1), the frequency of every residue in the framework is determined by comparing the occurrence of different amino acids at each position, based on results provided by bioinformatic tools. In a second step, a degree of conservation at each position is defined, e.g. by using the Simpson's Index with the formula D = E *ni (ni -1) / N(N-1).* In a third step, the best substitution which minimize the overall free energy is determined (e.g., by applying Boltzmann's law: ΔΔGth =-RTln(*fparental/ fconsensus)).* Finally (step 4), the role of potential stabilizing mutations is determined. For this purpose, factors such as local and non-local interactions, canonical residues, interfaces, exposure degree and β-turns propensity may be taken into account.

Figure 3 is a schematic diagram of an exemplary Quality Control (QC) system for selection of stable and soluble scFvs in yeast. With this system, host cells capable of expressing stable and soluble scFvs in a reducing environment are selected due to the presence of an inducible reporter construct which expression is dependent on the presence of a stable and soluble scFv-AD-Gal11p fusion protein. Interaction of the fusion protein with Gal4 (1-100) forms a functional transcription factor which activates expression of a selectable marker (see Figure 3A). Unstable and/or insoluble scFvs are incapable of forming a functional transcription factor and inducing expression of the selectable marker and are therefore excluded from selection (Figure 3B). Selected scFvs are able to obtain a stable and soluble protein fold, even under reducing conditions, where disulphide bonds are not fold, whereas unstable and /or insoluble scFv tend to unfold, aggregate and/or degrade. Under oxidizing conditions, selected scFv still reveal superior solubility and stability characteristics.

Figure 4 is a schematic diagram of another exemplary Quality Control (QC) system. The overall concept for selecting soluble and scFv is the same as described for Figure 3, however in this version, the scFv is directly fused to a functional transcription factor comprising an activation domain (AD) and a DNA-binding domain (DBD). Figure 4A depicts an exemplary soluble and stable scFv which, when fused to a functional transcription factor, does not hinder the transcription of a selectable marker. In contrast, Figure 4B depicts the scenario whereby an unstable scFv is fused to the transcription factor giving rise to a non-functional fusion construct that is unable to activate transcription of the selectable marker

Figure 5 is a schematic diagram of the analysis of variability at particular framework (FW) positions within native germline sequences before somatic mutation (Figure 5A) and at the corresponding FW positions within mature antibody sequences after somatic mutation selected in the QC system (Figure 5B). Different variability values can be assigned to the respective FW positions (e.g., highly variable framework residues ("hvFR")) within the germline and QC sequences (i.e., "G" and "Q" values, respectively). If G >Q for a particular position, there is a restricted number of suitable stable FW residues at that position. If G<Q for a particular position, this may indicate that the residue has been naturally selected for optimal solubility and stability.

Figure 6 depicts the denaturation profile observed for ESBA105 variants following thermo-induced stress at a range of temperatures from 25 to 95°C. ESBA-105 variants having backmutations to germline consensus residues (V3Q, R47K, or V103T) are indicated by dashed lines. Variants comprising preferred substitutions identified by the methods of the invention (QC11.2, QC15.2, and QC23.2) are indicated by solid lines.

Figure 7 depicts a comparison of the thermal stability for a set of ESBA105 variants comprising either consensus backmutations (S-2, D-2, D-3), a mutation to alanine (D-1) or a QC residue (QC7.1, QC11.2, QC15.2, QC23.2). The thermal stability of each variant (in arbitrary unfolding units) is provided.

Figure 8 depicts the denaturation profile observed for ESBA212 variants following thermo-induced stress at a range of temperatures from 25 to 95°C. ESBA-212 variants having backmutations to germline consensus residues (V3Q or R47K) are indicated by dashed lines. The parent ESBA212 molecule is indicated by a solid line.

Figure 9 illustrates the scFv framework scaffold for the VH1a family. The first row shows the heavy chain variable region numbering using the Kabat system. The second row shows the heavy chain variable region numbering using the AHo system. The third row shows the scFv framework scaffold sequence (SEQ ID NO: 1), wherein at those positions marked as "X", the position can be occupied by any of the amino acid residues listed below the "X." The positions marked "x" and the regions marked as CDR1 H1, CDR H2 and CDR H3 can be occupied by any amino acid.

Figure 10 illustrates the scFv framework scaffold for the VH1b family. The first row shows the heavy chain variable region numbering using the Kabat system. The second row shows the heavy chain variable region numbering using the AHo system. The third row shows the scFv framework scaffold sequence (SEQ ID NO:2), wherein at those positions marked as "X", the position can be occupied by any of the amino acid residues listed below the "X." The positions marked "x" and the regions marked as CDR1 H1, CDR H2 and CDR H3 can be occupied by any amino acid.

Figure 11 illustrates the scFv framework scaffold for the VH3 family. The first row shows the heavy chain variable region numbering using the Kabat system. The second row shows the heavy chain variable region numbering using the AHo system. The third row shows the scFv framework scaffold sequence (SEQ ID NO:3), wherein at those positions marked as "X", the position can be occupied by any of the amino acid residues listed below the "X." The positions marked "x" and the regions marked as CDR1 H1, CDR H2 and CDR H3 can be occupied by any amino acid.

Figure 12 illustrates the scFv framework scaffold for the Vκ1 family. The first row shows the light chain variable region numbering using the Kabat system. The second row shows the light chain variable region numbering using the AHo system. The third row shows the scFv light chain framework scaffold sequence (SEQ ID NO:4), wherein at those positions marked as "X", the position can be occupied by any of the amino acid residues listed below the "X." The positions marked "." and the regions marked as CDR1 L1, CDR L2 and CDR L3 can be occupied by any amino acid.

Figure 13 illustrates the scFv framework scaffold for the Vk3 family. The first row shows the light chain variable region numbering using the Kabat system. The second row shows the light chain variable region numbering using the AHo system. The third row shows the scFv light chain framework scaffold sequence (SEQ ID NO:5), wherein at those positions marked as "X", the position can be occupied by any of the amino acid residues listed below the "X." The positions marked "." and regions marked as CDR1 L1, CDR L2 and CDR L3 can be occupied by any amino acid.

Figure 14 illustrates the scFv framework scaffold for the VL1 family. The first row shows the light chain variable region numbering using the Kabat system. The second row shows the light chain variable region numbering using the AHo system. The third row shows the scFv light chain framework scaffold sequence, wherein at those positions marked as "X", the position can be occupied by any of the amino acid residues listed below the "X." The positions marked "." and the regions marked as CDR1 L1, CDR L2 and CDR L3 can be occupied by any amino acid. In certain preferred embodiments, AHo positions 58 and 67-72 within CDR L1 are occupied by the following respective residues: D and NNQRPS.

Figure 15 depicts the PEG precipitation solubility curves of wild-type ESBA105 and solubility variants thereof.

Figure 16 depicts the thermal denaturation profiles for wild-type ESBA105 and solubility variants thereof as measured following thermochallenge at a broad range of temperatures (25-96°C).

Figure 17 depicts an SDS-PAGE gel which shows degradation behaviour of various ESBA105 solubility mutants after two weeks of incubation under conditions of thermal stress.

## Detailed Description of the Invention

The invention pertains to methods for sequence-based engineering and optimization of immunobinder properties, and in particular scFvs properties, including but not limited to stability, solubility and/or affinity. More specifically, the present invention discloses methods for optimizing scFv antibodies using antibody sequence analysis to identify amino acid positions within a scFv to be mutated to thereby improve one or more physical properties of the scFv. The invention also pertains to engineered immunobinders, e.g., scFvs, produced or obtainable according to the methods of the invention.

The invention is based, at least in part, on the analysis of the frequency of amino acids at each heavy and light chain framework position in multiple databases of antibody sequences. In particular, the frequency analysis of antibody sequence databases (e.g., germline antibody sequence databases or mature antibody databases, e.g., the Kabat database) has been compared to the frequency analysis of a database of scFv sequences that have been selected as having desired functional properties. By assigning a degree of variability to each framework position (e.g., using the Simpson's Index) and by comparing the degree of variability at each framework position within the different types of antibody sequence databases, it has now been possible to identify framework positions of importance to the functional properties (e.g., stability, solubility) of a scFv. This now allows for defining a "functional consensus" to the framework amino acid positions, in which framework positions that are either more or less tolerant of variability than the corresponding positions in immunoglobulin sequences (e.g., germline or mature immunoglobulin sequences) have been identified. Thus, the invention provides, and demonstrates the benefit of, a "functional consensus" approach based on the use of a database of functionally-selected scFv sequences. Still further, the invention provides methods of engineering immunobinders (e.g., scFvs) by mutating particular framework amino acid positions identified using the "functional consensus" approach described herein.

So that the invention may be more readily understood, certain terms are first defined. Unless otherwise defined, all technical and scientific terms used herein have the same meaning as commonly understood by one of ordinary skill in the art to which this invention belongs. Although methods and materials similar or equivalent to those described herein can be used in the practice or testing of the invention, suitable methods and materials are described below. All publications, patent applications, patents, and other references mentioned herein are incorporated by reference in their entirety. In the case of conflict, the present specification, including definitions, will control. In addition, the materials, methods, and examples are illustrative only and not intended to be limiting.

The term "antibody" as used herein is a synonym for "immunoglobulin". Antibodies according to the present invention may be whole immunoglobulins or fragments thereof, comprising at least one variable domain of an immunoglobulin, such as single variable domains, Fv (Skerra A. and Pluckthun, A. (1988) Science 240:1038-41), scFv (Bird, R.E. et al. (1988) Science 242:423-26; Huston, J.S. et al. (1988) Proc. Natl. Acad. Sci. USA 85:5879-83), Fab, (Fab')2 or other fragments well known to a person skilled in the art.

The term "antibody framework" as used herein refers to the part of the variable domain, either VL or VH, which serves as a scaffold for the antigen binding loops of this variable domain (Kabat, E.A. et al., (1991) Sequences of proteins of immunological interest. NIH Publication 91-3242).

The term "antibody CDR" as used herein refers to the complementarity determining regions of the antibody which consist of the antigen binding loops as defined by Kabat E.A. et al., (1991) Sequences of proteins of immunological interest. NIH Publication 91-3242). Each of the two variable domains of an antibody Fv fragment contain, for example, three CDRs.

The term "single chain antibody" or "scFv" is intended to refer to a molecule comprising an antibody heavy chain variable region ($V_H$) and an antibody light chain variable region ($V_L$) connected by a linker. Such scFv molecules can have the general structures: $NH_2$-$V_L$-linker-$V_H$-COOH or $NH_2$-$V_H$-linker-$V_L$-COOH.

As used herein, "identity" refers to the sequence matching between two polypeptides, molecules or between two nucleic acids. When a position in both of the two compared sequences is occupied by the same base or amino acid monomer subunit (for instance, if a position in each of the two DNA molecules is occupied by adenine, or a position in each of two polypeptides is occupied by a lysine), then the respective molecules are identical at that position. The "percentage identity" between two sequences is a function of the number of matching positions shared by the two sequences divided by the number of positions compared x 100. For instance, if 6 of 10 of the positions in two sequences are matched, then the two sequences have 60% identity. By way of example, the DNA sequences CTGACT and CAGGTT share 50% identity (3 of the 6 total positions are matched). Generally, a comparison is made when two sequences are aligned to give maximum identity. Such alignment can be provided using, for instance, the method of Needleman et al. (1970) J. Mol. Biol. 48: 443-453, implemented conveniently by computer programs such as the Align program (DNAstar, Inc.).

"Similar" sequences are those which, when aligned, share identical and similar amino acid residues, where similar residues are conservative substitutions for corresponding amino acid residues in an aligned reference sequence. In this regard, a "conservative substitution" of a residue in a reference sequence is a substitution by a residue that is physically or functionally similar to the corresponding reference residue, e.g., that has a similar size, shape, electric charge, chemical properties, including the ability to form covalent or hydrogen bonds, or the like. Thus, a "conservative substitution modified" sequence is one that differs from a reference sequence or a wild-type sequence in that one or more conservative substitutions are present. The "percentage similarity" between two sequences is a function of the number of positions that contain matching residues or conservative substitutions shared by the two sequences divided by the number of positions compared x 100. For instance, if 6 of 10 of the positions in two sequences are matched and 2 of 10 positions contain conservative substitutions, then the two sequences have 80% positive similarity.

"Amino acid consensus sequence" as used herein refers to an amino acid sequence that can be generated using a matrix of at least two, and preferably more, aligned amino acid sequences, and allowing for gaps in the alignment, such that it is possible to determine the most frequent amino acid residue at each position. The consensus sequence is that sequence which comprises the amino acids which are most frequently represented at each position. In the event that two or more amino acids are equally represented at a single position, the consensus sequence includes both or all of those amino acids.

The amino acid sequence of a protein can be analyzed at various levels. For example, conservation or variability can be exhibited at the single residue level, multiple residue level, multiple residue with gaps etc. Residues can exhibit conservation of the identical residue or can be conserved at the class level. Examples of amino acid classes include polar but uncharged R groups (Serine, Threonine, Asparagine and Glutamine); positively charged R groups (Lysine, Arginine, and Histidine); negatively charged R groups (Glutamic acid and Aspartic acid); hydrophobic R groups (Alanine, Isoleucine, Leucine, Methionine, Phenylalanine, Tryptophan, Valine and Tyrosine); and special amino acids (Cysteine, Glycine and Proline). Other classes are known to one of skill in the art and may be defined using structural determinations or other data to assess substitutability. In that sense, a substitutable amino acid can refer to any amino acid which can be substituted and maintain functional conservation at that position.

As used herein, when one amino acid sequence (*e.g.*, a first $V_H$ or $V_L$ sequence) is aligned with one or more additional amino acid sequences (*e.g.*, one or more VH or VL sequences in a database), an amino acid position in one sequence (e.g., the first $V_H$ or $V_L$ sequence) can be compared to a "corresponding position" in the one or more additional amino acid sequences. As used herein, the "corresponding position" represents the equivalent position in the sequence(s) being compared when the sequences are optimally aligned, *i.e.,* when the sequences are aligned to achieve the highest percent identity or percent similarity.

As used herein, the term "antibody database" refers to a collection of two or more antibody amino acid sequences (a "multiplicity" of sequences), and typically refers to a collection of tens, hundreds or even thousands of antibody amino acid sequences. An antibody database can store amino acid sequences of, for example, a collection of antibody $V_H$ regions, antibody $V_L$ regions or both, or can store a collection of scFv sequences comprised of $V_H$ and $V_L$ regions. Preferably, the database is stored in a searchable, fixed medium, such as on a computer within a searchable computer program. In one embodiment, the antibody database is a database comprising or consisting of germline antibody sequences. In another embodiment, the antibody database is a database comprising or consisting of mature (i.e., expressed) antibody sequences (e.g., a Kabat database of mature antibody sequences, e.g., a KBD database). In yet another embodiment, the antibody database comprises or consists of functionally selected sequences (e.g., sequences selected from a QC assay).

The term "immunobinder" refers to a molecule that contains all or a part of the antigen binding site of an antibody, *e.g*,. all or part of the heavy and/or light chain variable domain, such that the immunobinder specifically recognizes a target antigen. Non-limiting examples of immunobinders include full-length immunoglobulin molecules and scFvs, as well as antibody fragments, including but not limited to (i) a Fab fragment, a monovalent fragment consisting of the $V_L$, $V_H$, $C_L$ and $C_H1$ domains; (ii) a $F(ab')_2$ fragment, a bivalent fragment comprising two Fab fragments linked by a disulfide bridge at the hinge region; (iii) a Fab' fragment, which is essentially a Fab with part of the hinge region (see, FUNDAMENTAL IMMUNOLOGY (Paul ed., 3.sup.rd ed. 1993); (iv) a Fd fragment consisting of the $V_H$ and $C_H1$ domains; (v) a Fv fragment consisting of the $V_L$ and $V_H$ domains of a single arm of an antibody, (vi) a single domain antibody such as a Dab fragment (Ward et al., (1989) Nature 341:544-546), which consists of a $V_H$ or $V_L$ domain, a Camelid (see Hamers-Casterman, et al., Nature 363:446-448 (1993), and Dumoulin, et al., Protein Science 11:500-515 (2002)) or a Shark antibody (e.g., shark Ig-NARs Nanobodies®; and (vii) a nanobody, a heavy chain variable region containing a single variable domain and two constant domains.

As used herein, the term "functional property" is a property of a polypeptide (e.g., an immunobinder) for which an improvement (e.g., relative to a conventional polypeptide) is desirable and/or advantageous to one of skill in the art, e.g., in order to improve the manufacturing properties or therapeutic efficacy of the polypeptide. In one embodiment, the functional property is improved stability (e.g., thermal stability). In another embodiment, the functional property is improved solubility (e.g., under cellular conditions). In yet another embodiment, the functional property is non-aggregation. In still another embodiment, the functional property is an improvement in expression (e.g., in a prokaryotic cell). In yet another embodiment the functional property is an improvement in refolding yield following an inclusion body purification process. In certain embodiments, the functional property is not an improvement in antigen binding affinity.

<u>Sequence Based Analysis of scFvs</u>

The invention provides methods for analyzing a scFv sequence that allow for the identification of amino acid positions within the scFv sequence to be selected for mutation. The amino acid positions selected for mutation are ones that are predicted to influence functional properties of the scFv, such as solubility, stability and/or antigen binding, wherein mutation at such positions is predicted to improve the performance of the scFv. Thus, the invention allows for more focused engineering of scFvs to optimize performance than simply randomly mutating amino acid positions within the scFv sequence.

Certain aspects of the sequence-based analysis of scFv sequences are diagrammed schematically in the flowchart of Figure 1. As shown in this figure, the sequence of a scFv to be optimized is compared to the sequences in one or more antibody databases, including an antibody database composed of scFv sequences selected as being stable and soluble. This can allow for identification of residues critical for stability and/or solubility specifically in the scFv format, a well as identification of patterns that represent improvements in stability, solubility and/or binding independent of the respective CDRs, specifically in the scFv format (e.g., $V_L$ and $V_H$ combinations). Once critical residues have been identified, they can be substituted by, for example, the most frequent suitable amino acid as identified in the respective database and/or by random or biased mutagenesis.

Thus, in one aspect, the invention pertains to a method of identifying an amino acid position for mutation in a single chain antibody (scFv), the scFv having $V_H$ and $V_L$ amino acid sequences, the method comprising:

a) entering the scFv $V_H$, $V_L$ or $V_H$ and $V_L$ amino acid sequences into a database that comprises a multiplicity of antibody $V_H$, $V_L$ or $V_H$ and $V_L$ amino acid sequences such that the scFv $V_H$, $V_L$ or $V_H$ and $V_L$ amino acid sequences are aligned with the antibody $V_H$, $V_L$ or $V_H$ and $V_L$ amino acid sequences of the database;
b) comparing an amino acid position within the scFv $V_H$ or $V_L$ amino acid sequence with a corresponding position within the antibody $V_H$ or $V_L$ amino acid sequences of the database;
c) determining whether the amino acid position within the scFv $V_H$ or $V_L$ amino acid sequence is occupied by an amino acid residue that is conserved at the corresponding position within the antibody $V_H$ or $V_L$ amino acid sequences of the database; and
d) identifying the amino acid position within the scFv $V_H$ or $V_L$ amino acid sequence as an amino acid position for mutation when the amino acid position is occupied by an amino acid residue that is not conserved at the corresponding position within the antibody $V_H$ or $V_L$ amino acid sequences of the database.

Thus, in the method of the invention, the sequence of a scFv of interest (i.e., the sequence of the $V_H$, $V_L$ or both) is compared to the sequences of an antibody database and it is determined whether an amino acid position in the scFv of interest is occupied by an amino acid residue that is "conserved" in the corresponding position of the sequences in the database. If the amino acid position of the scFv sequence is occupied by an amino acid residue that is not "conserved" at the corresponding position within the sequences of the database, that amino acid position of the scFv is chosen for mutation. Preferably, the amino acid position that is analyzed is a framework amino acid position within the scFv of interest. Even more preferably, every framework amino acid position within the scFv of interest can be analyzed. In an alternative embodiment, one or more amino acid positions within one or more CDRs of the scFv of interest can be analyzed. In yet another embodiment, each amino acid position with the scFv of interest can be analyzed.

To determine whether an amino acid residue is "conserved" at a particular amino acid position within the sequences of the antibody database (*e.g.*, a framework position), the degree of conservation at the particular position can be calculated. There are a variety of different ways known in the art that amino acid diversity at a given position can be quantified, all of which can be applied to the methods of the present invention. Preferably, the degree of conservation is calculated using Simpson's diversity index, which is a measure of diversity. It takes into account the number of amino acids present at each position, as well as the relative abundance of each amino acid. The Simpson Index (S.I.) represents the probability that two randomly selected antibody sequences contain the same amino acid at certain positions. The Simpson Index takes into account two main factors when measuring conservation, richness and evenness. As used herein, "richness" is a measure of the number of different kinds of amino acids present in a particular position (*i.e.*, the number of different amino acid residues represented in the database at that position is a measure of richness). As used herein, "evenness" is a measure of the abundance of each of the amino acids present at the particular position (*i.e.*, the frequency with which amino acid residues occur that position within the sequences of the database is a measure of evenness).

While residue richness can be used as a measure on its own to examine degree of conservation at a particular position, it does not take into account the relative frequency of each amino acid residue present at a certain position. It gives as much weight to those amino acid residues that occur very infrequently at a particular position within the sequences of a database as it does to those residues that occur very frequently at the same position. Evenness is a measure of the relative abundance of the different amino acids making up the richness of a position. The Simpson Index takes both into account, richness and evenness, and thus is a preferred way to quantitate degree of conservation according to the present invention. In particular, low frequent residues at very conserved positions are considered as potentially problematic and thus can be chosen for mutation.

The formula for the Simpson index is $D = \sum n_i (n_i\text{-}1)/N(N\text{-}1)$, wherein N is the total number of sequences in the survey (*e.g.*, in the database) and $n_i$ is the frequency of each amino acid residue at the position being analyzed. The frequency of an amino acid event (i) in the database is the number ($n_i$) of times the amino acid occurred in the database. The counts $n_i$ themselves are given in relative frequencies, which means they are normalized by the total number of events. When maximum diversity occurs, the S.I. value is zero and when minimum diversity occurs, the S.I. value is 1. Thus, the S.I. range is 0-1, with an inverse relationship between diversity and the index value.

A flow chart summarizing the multiple steps for analysis of framework amino acid positions within the sequences of the database is described in further detail in Figure 2.

Accordingly, in a preferred embodiment of the above-described method, the corresponding position within the antibody $V_H$ or $V_L$ amino acid sequence of the database is assigned a degree of conservation using Simpson's Index. The S.I. value of that corresponding position can be used as an indicator of the conservation of that position.

In other embodiments, trusted alignments (i.e. sequence alignments for which protein structure similarity are considered) of closely related antibody sequences are used in the present invention to generate matrices of relative abundance of amino acids and degree of conservation of determined positions. These matrices are designed for use in antibody-antibody database comparisons. The observed frequency of each residue is calculated and compared to the expected frequencies (which are essentially the frequencies of each residue in the dataset for each position).

Analysis of a given scFv antibody with the described method provides information about biologically permissible mutations and unusual residues at certain positions in the given scFv antibody and allows the prediction of potential weakness within its framework. The routine can be used to engineer amino acid substitutions that "best" fit a set of amino acid-frequency data, using the S.I. value and the relative frequency as a criterion.

The sequence-based analysis described above can be applied to the $V_H$ region of the scFv, to the $V_L$ region of the scFv, or to both. Thus, in one embodiment, scFv $V_H$ amino acid sequence is entered into the database and aligned with antibody $V_H$ amino acid sequences of the database. In another embodiment, the scFv $V_L$ amino acid sequence is entered into the database and aligned with antibody $V_L$ amino acid sequences of the database. In yet another embodiment, the scFv $V_H$ and $V_L$ amino acid sequences are entered into the database and aligned with antibody $V_H$ and $V_L$ amino acid sequences of the database. Algorithms for aligning one sequence with a collection of other sequences in a database are well-established in the art. The sequences are aligned such that the highest percent identity or similarity between the sequences is achieved.

The methods of the invention can be used to analyze one amino acid position of interest within a scFv sequence or, more preferably, can be used to analyze multiple amino acid positions of interest. Thus, in step b) of the above-described method, multiple amino acid positions within the scFv $V_H$ or $V_L$ amino acid sequence can be compared with corresponding positions within the antibody $V_H$ or $V_L$ amino acid sequences of the database. Preferred positions to be analyzed are framework positions within the $V_H$ and/or $V_L$ sequences of the scFv (*e.g.*, each VH and VL framework position can be analyzed). Additionally or alternatively, one or more positions within one or more CDRs of the scFv can be analyzed (although it may not be preferred to mutate amino acid positions with the CDRs, since mutations within the CDRs are more likely to affect antigen binding activity than mutations within the framework regions). Still further, the methods of the invention allow for the analysis of each amino acid position within the scFv $V_H$, $V_L$ or $V_H$ and $V_L$ amino acid sequences.

In the methods of the invention, the sequence of a scFv of interest can be compared to the sequences within one or more of a variety of different types of antibody sequence databases. For example, in one embodiment, the antibody $V_H$, $V_L$ or $V_H$ and $V_L$ amino acid sequences of the database are germline antibody $V_H$, $V_L$ or $V_H$ and $V_L$ amino acid sequences. In another embodiment, the antibody $V_H$, $V_L$ or $V_H$ and $V_L$ amino acid sequences of the database are rearranged, affinity matured antibody $V_H$, $V_L$ or $V_H$ and $V_L$ amino acid sequences. In yet another preferred embodiment, the antibody $V_H$, $V_L$ or $V_H$ and $V_L$ amino acid sequences of the database are scFv antibody $V_H$, $V_L$ or $V_H$ and $V_L$ amino acid sequences selected as having at least one desirable functional property, such as scFv stability or scFv solubility (discussed further below).

Antibody sequence information can be obtained, compiled, and/or generated from sequence alignments of germ line sequences or from any other antibody sequence that occurs in nature. The sources of sequences may include but are not limited to one or more of the following databases:

- The Kabat database (.immuno.bme.nwu.Edu (as of October 2007); Johnson & Wu (2001) Nucleic Acids Res. 29: 205-206; Johnson & Wu (2000) Nucleic Acids Res. 28: 214-218). The raw data from 2000 are available by FTP in the US and mirrored in the UK.

- Kabatman contains a database that allows the user to search the Kabat sequence for sequence unusual features and enables the user to find canonical assignments for the CDRs in a specific antibody sequence.

- AAAAA Website (www.bioc.unizh.ch/antibody/ (as of October 2007)), an antibody page prepared by Annemarie Honegger that provides sequence information and structural data on antibodies.

- ABG: Directory of 3D structures of antibodies - The directory, created by the Antibody Group (ABG), allows the user to access the antibody structures compiled at Protein Data Bank (PDB). In the directory, each PDB entry has a hyperlink to the original source to make full information recovering easy

- ABG: Germline gene directories of the mouse VH and VK germline segments, part of the webpage of the Antibody Group at the Instituto de Biotecnologia, UNAM (National University of Mexico)

- IMGT ®, the international ImMunoGeneTics information system ® - created in 1989 by Marie-Paule Lefranc (Université Montpellier II, CNRS), IMGT is an integrated knowledge resource specialized in immunoglobulins, T cell receptors, and related proteins of the immune system for human and other vertebrate species. IMGT consists of sequence databases (IMGT/LIGM-DB, a comprehensive database of IG and TR from human and other vertebrates, with translation for fully annotated sequences, IMGT/MHC-DB, IMGT/PRIMER-DB), a genome database (IMGT/GENE-DB), a structure database (IMGT/3Dstructure-DB), a web resource (IMGT Repertoire) (IMGT, the international ImMunoGeneTics information system®; imgt.cines.fr (as of October 2007); Lefranc et al. (1999) Nucleic Acids Res. 27: 209-212; Ruiz et al. (2000) Nucleic Acids Res. 28: 219-221; Lefranc et al. (2001) Nucleic Acids Res. 29: 207-209; Lefranc et al. (2003) Nucleic Acids Res. 31: 307-310).

- V BASE - a comprehensive directory of all human germline variable region sequences compiled from over a thousand published sequences, including those in the current releases of the Genbank and EMBL data libraries.

In a preferred embodiment, the antibody sequence information is obtained from a scFv library having defined frameworks that have been selected for enhanced stability and solubility in a reducing environment. More specifically, a yeast Quality Control (QC) - System has been described (see *e.g.,* PCT Publication WO 2001/48017; U.S. Application Nos. 2001/0024831 and US 2003/0096306; US Patent Nos 7,258,985 and 7,258,986) that allows for the intracellular selection of scFv frameworks with enhanced stability and solubility in a reducing environment. In this system, a scFv library is transformed into host cells able to express a specific known antigen and only surviving in the presence of antigen-scFv interaction. The transformed host cells are cultivated under conditions suitable for expression of the antigen and the scFv and allowing for cell survival only in the presence of antigen-scFv interaction. Thus, scFvs expressed in the surviving cells and having defined frameworks that are stable and soluble in a reducing environment can be isolated.

Accordingly, the QC-System can be used to screen a large scFv library to thereby isolate those preferred scFvs having frameworks that are stable and soluble in a reducing environment and the sequences of those selected scFvs can be compiled into a scFv sequence database. Such a scFv database then can be used for comparison purposes with other scFv sequences of interest using the methods of the instant invention. Preferred scFv framework sequences that have previously selected and defined using the QC-System are described in further detail in PCT Publication WO 2003/097697 and U.S. Application No. 20060035320.

Variants of the original QC-System are known in the art. In one exemplary embodiment, which is illustrated schematically in Figure 3, a scFv library is fused to the activation domain (AD) of the Gal4 yeast transcription factor, which is in turn fused to a portion of the so-called Gal11p protein (11p). The scFv-AD-Gal11p fusion construct is then transformed into host cells that express the first 100 amino acids of Gal4 and thus contain the Gal4 DNA-binding domain (DBD; Gal4(1-100)). Gal11p is a point mutation that is known to directly bind to Gal4(1-100)(see Barberis et al., Cell, 81: 359 (1995)). The transformed host cells are cultivated under conditions which are suitable for expression of the scFv fusion protein and that allow for cell survival only in the case that the scFv fusion protein is stable and soluble enough to interact with Gal4(1-100) and thereby form a functional transcription factor containing an AD linked to a DBD (Figure 3A). Thus, scFvs expressed in the surviving cells and having defined frameworks that are stable and soluble in a reducing environment can be isolated. A further description of this exemplary QC system is described in Auf der Maur et al., Methods, 34: 215-224 (2004).

In another exemplary embodiment, a QC-system employed in the methods of the invention is depicted in Figure 4. In this version of the QC-system, the scFv or the scFv library is directly fused to a functional transcription factor and expressed in a yeast strain containing a selectable marker. The selectable marker will only by activated in the presence of a functional scFv-transcription factor fusion, which means that the construct as a whole needs to be stable and soluble (Figure 4A). In the event that the scFv is unstable, it will form aggregates and eventually be degraded, thereby also causing degradation of the transcription factor fused to it so that it is no longer able to activate the expression of the selectable marker (see Figure 4B).

In the methods of the invention, the sequence of a scFv of interest can be compared with all sequences within an antibody database or, alternatively, only a selected portion of the sequences in the database can be used for comparison purposes. That is, the database can be limited, or constrained, to only those sequences having a high percentage similarity or identity to the scFv of interest. Thus, in one embodiment of the method of the invention, the database is a constrained database in which only those antibody $V_H$, $V_L$ or $V_H$ and $V_L$ amino acid sequences having high similarity to the scFv antibody $V_H$, $V_L$ or $V_H$ and $V_L$ amino acid sequences are included in the database.

Once the scFv sequence of interest is entered into the database and compared to the antibody sequences within the database, sequence information is analyzed to provide information about the frequency and variability of amino acids of a given position and to predict potentially problematic amino acid positions, in particular potentially problematic amino acid positions within the framework of the scFv. Such information can also be used to design mutations that improve the properties of the scFv. For example antibody solubility can be improved by replacing solvent exposed hydrophobic residues by hydrophilic residues that otherwise occur frequently at this position.

In the method of the invention, there are a number of possible types of amino acid residues that can be "conserved" at a particular position within the antibody sequences of the database. For example, one particular amino acid residue may be found at that position at a very high frequency, indicating that this particular amino acid residue is preferred at that particular position. Accordingly, in one embodiment of the method, in step c), the amino acid residue that is conserved at the corresponding position within the antibody $V_H$ or $V_L$ amino acid sequences of the database is the amino acid residue that is most frequently at that position within the antibody $V_H$ or $V_L$ amino acid sequences of the database. In other embodiments, the position may be "conserved" with a particular type or class of amino acid residue (i.e., the position is not preferentially occupied by only a single particular amino acid residue, but rather is preferentially occupied by several different amino acid residues each of which is of the same type or class of residue). For example, in step c), the corresponding position within the antibody $V_H$ or $V_L$ amino acid sequences of the database may be conserved with: (i) hydrophobic amino acid residues, (ii) hydrophilic amino acid residues, (iii) amino acid residues capable of forming a hydrogen bond or (iv) amino acid residues having a propensity to form a $\beta$-sheet.

In step d) of the method, an amino acid position within the scFv $V_H$ or $V_L$ amino acid sequence is identified as an amino acid position for mutation when the amino acid position is occupied by an amino acid residue that is not conserved at the corresponding position within the antibody $V_H$ or $V_L$ amino acid sequences of the database. There are a number of possible situations that would identify an amino acid position as being occupied by an amino acid residue that is "not conserved" and thus as being potentially problematic. For example, if the corresponding amino acid position within the database is conserved with a hydrophobic residue and the position in the scFv is occupied by a hydrophilic residue, this position could be potentially problematic in the scFv and the position can be selected for mutation. Likewise, if the corresponding amino acid position within the database is conserved with a hydrophilic residue and the position in the scFv is occupied by a hydrophobic residue, this position could be potentially problematic in the scFv and the position can be selected for mutation. In still other instances, if the corresponding amino acid position within the database is conserved with amino acid residues that are capable of forming a hydrogen bond or that have a propensity to form a P sheet, and the position in the scFv is occupied by a residue that is not capable of forming a hydrogen bond or does not have a propensity to form a β sheet, respectively, this position could be potentially problematic in the scFv and the position can be selected for mutation.

In a preferred embodiment, the methods described in the present invention can be used alone or in combination to create combinatorial lists of amino acid substitutions to improve stability and or solubility of antibody single chain fragments.

Covariance Analysis

The invention also pertains to methods for analyzing covariance within the sequence of a scFv as compared to antibody sequences within a database. Residues which covary can be, for example, (i) a residue in a framework region (FR) and a residue in a CDR; (ii) a residue in one CDR and a residue in another CDR; or (iii) a residue in the $V_H$ and a residue in the $V_L$ domain. Residues which interact with each other in the tertiary structure of the antibody may covary such that preferred amino acid residues may be conserved at both positions of the covariant pair and if one residue is altered the other residue must be altered as well to maintain the antibody structure. Methods for conducting a covariance analysis on a set of amino acid sequences are known in the art. For example, Choulier, L. et al. (2000) Protein 41:475-484 describes applying a covariance analysis to human and mouse germline $V_\kappa$ and $V_H$ sequence alignments.

A covariance analysis can be combined with the above-described method for analyzing conserved amino acid positions (steps a)-d) in the method above), such that the method further comprises the steps:

e) carrying out a covariance analysis on the antibody $V_H$ or $V_L$ amino acid sequence of the database to identify a covariant pair of amino acid positions;
f) comparing the covariant pair of amino acid positions with corresponding positions within the scFv $V_H$ or $V_L$ amino acid sequence;
g) determining whether the corresponding positions within the scFv $V_H$ or $V_L$ amino acid sequence are occupied by amino acid residues that are conserved at the covariant pair of amino acid positions within the antibody $V_H$ or $V_L$ amino acid sequences of the database; and
h) identifying one or both of the corresponding positions within the scFv $V_H$ or $V_L$ amino acid sequence as an amino acid position for mutation when one or both of the corresponding positions within the scFv is occupied by an amino acid residue that is not conserved at the covariant pair of amino acid positions within the antibody $V_H$ or $V_L$ amino acid sequences of the database.

Additionally or alternatively, a covariance analysis can be conducted on its own, such that the invention provides a method comprising the steps:

a) carrying out a covariance analysis on antibody $V_H$ or $V_L$ amino acid sequences of a database to identify a covariant pair of amino acid positions;
b) comparing the covariant pair of amino acid positions with corresponding positions within a scFv $V_H$ or $V_L$ amino acid sequence;
c) determining whether the corresponding positions within the scFv $V_H$ or $V_L$ amino acid sequence are occupied by amino acid residues that are conserved at the covariant pair of amino acid positions within the antibody $V_H$ or $V_L$ amino acid sequences of the database; and
d) identifying one or both of the corresponding positions within the scFv $V_H$ or $V_L$ amino acid sequence as an amino acid position for mutation when one or both of the corresponding positions within the scFv is occupied by an amino acid residue that is not conserved at the covariant pair of amino acid positions within the antibody $V_H$ or $V_L$ amino acid sequences of the database.

The covariance analysis methods of the invention can be used to analyze one covariant pair, or more than one covariant pair. Thus, in one embodiment of the method, multiple covariant pairs of amino acid positions are identified within the antibody $V_H$ or $V_L$ amino acid sequence of the database and compared to the corresponding positions within the scFv $V_H$ or $V_L$ amino acid sequence.

The method can further comprise mutating one or both of the corresponding positions within the scFv that are occupied by an amino acid residue that is not conserved at the covariant pair of amino acid positions within the antibody $V_H$ or $V_L$ amino acid sequences of the database. In one embodiment, one of the corresponding positions within the scFv that is occupied by an amino acid residue that is not conserved at the covariant pair of amino acid positions is substituted with an amino acid residue that is most frequently at the covariant pair amino acid position. In another embodiment, both of the corresponding positions within the scFv that are occupied by amino acid residues that are not conserved at the covariant pair of amino acid positions are substituted with amino acid residues that are most frequently at the covariant pair amino acid positions.

Molecular Modeling

The sequence-based methods of the invention for analyzing scFvs for potentially problematic residues can be combined with other methods known in the art for analyzing antibody structure/function relationships. For example, in a preferred embodiment, the sequence-based analytical methods of the invention are combined with molecular modeling to identify additional potentially problematic residues. Methods and software for computer modeling of antibody structures, including scFv structures, are established in the art and can be combined with the sequence-based methods of the invention. Thus, in another embodiment, the sequence-based methods described above as set forth in steps a) - d) further comprise the steps of:

e) subjecting the scFv $V_H$, $V_L$ or $V_H$ and $V_L$ amino acid sequences to molecular modeling; and
f) identifying at least one additional amino acid position within the scFv $V_H$, $V_L$ or $V_H$ and $V_L$ amino acid sequences for mutation.

The method can further comprise mutating the at least one additional amino acid position within scFv $V_H$, $V_L$ or $V_H$ and $V_L$ amino acid sequences identified for mutation by molecular modeling.

"Functional Consensus" Versus "Conventional Consensus" Analysis

In a particularly preferred embodiment, the degree of variability at one or more framework positions is compared between a first database of antibody sequences (e.g., a germline database(s)(e.g., Vbase and/or IMGT) or a mature antibody database (e.g., KBD) and a second database of scFvs selected as having one or more desirable properties, e.g., a database of scFvs selected by QC screening in yeast, i.e., a QC database. As illustrated in Figure 5, a variability value (e.g., Simpson's Index value) can be assigned to framework positions within the first (e.g., germline) database, referred to as "G" values in Figure 5, and a variability value (e.g., Simpson's Index value) can be assigned to the corresponding framework positions within the second database (e.g., QC database), referred to as "Q" values in Figure 5. When the G value is greater than the Q value at a particular position (*i.e.,* more variability in the germline sequences at that position than in the selected scFv sequences), this indicates that there are a restricted number of stable scFv framework amino acid residues at that position, which stable scFv framework amino acid residues may be suitable for use with any CDRs. Alternatively, when the G value is less than the Q value at a particular position (i.e., more variability in the selected scFv sequences at that position than in the germline sequences), this indicates that this particular position is more tolerant of variability in the scFv and thus may represent a position at which amino acid subsititutions may optimize stability and/or solubility of the scFv. Table A presents a summary table of the number of amino acid positions, and highly variable framework residues (hvFR), at which either G is greater than Q or G is less than Q. As indicated in Table A, the variability in total number of amino acids (Aa #) and in highly variable framework residues (hvFRs) significantly increased between germline and QC-FWs. The sequences that were analyzed to generate Table A were about 90 scFv sequences that were selected using the QC assay (as described in WO03097697; herein referred to as "Q") and all germline VH and VL sequences retrieved from http://www.bioc.unizh.ch/antibody/Sequences/index.html in October 2007 (herein referred to as "G"). For the analysis of Table A, the VH and VL domains were not grouped according to their subtype.

Table A: Summary Table

| | Aa# | G<Q (#of cases) | G>Q (#of cases) | X/Y | #hvFR (Simpson <0.4) | G<Q (#of cases) | G>Q (#of cases) | X/Y |
|---|---|---|---|---|---|---|---|---|
| $V_L$ | 108 | 61 | 11 | 5.5 | 16 | 13 | 3 | 4.3 |
| $V_H$ | 116 | 50 | 18 | 2.8 | 27 | 22 | 5 | 4.4 |

In view of the foregoing, in yet another aspect, the invention provides a method of identifying one or more framework amino acid positions for mutation in a single chain antibody (scFv), the scFv having $V_H$ and $V_L$ amino acid sequences, the method comprising:

a) providing a first database of $V_H$, $V_L$ or $V_H$ and $V_L$ amino acid sequences (e.g., germline and/or mature antibody sequences);

b) providing a second database of scFv antibody $V_H$, $V_L$ or $V_H$ and $V_L$ amino acid sequences selected as having at least one desirable functional property;
c) determining amino acid variability at each framework position of the first database and at each framework position of the second database;
d) identifying one or more framework positions at which degree of amino acid variability differs between the first database and the second database to thereby identify one or more framework amino acid positions for mutation in a single chain antibody (scFv).

Preferably, the amino acid variability at each framework position is determined by assigning a degree of conservation using Simpson's Index. In one embodiment, the one or more framework amino acid positions is identified for mutation based on the one or more framework amino acid positions having a lower Simpson's Index value in the second (scFv) database as compared to the first database. In another embodiment, the one or more framework amino acid positions is identified for mutation based on the one or more framework amino acid positions having a higher Simpson's Index value in the second database as compared to the first database.

Variability analyses, and identification of residues for mutation, for three human $V_H$ families and three human $V_L$ families are described in further detail in Examples 2 and 3 below.

Enrichment / Exclusion Analysis

In another aspect, the invention provides methods for selecting preferred amino acid residue substitutions (or, alternatively, excluding particular amino acid substitutions) at a framework position of interest within an immunobinder (e.g., to improve a functional property such as stability and/or solubility). The methods of the invention compare the frequency of an amino acid residue at a framework position of interest in a first database of antibody sequences (e.g., germline database(s) such Vbase and/or IMGT or, more preferably, a mature antibody database such as the Kabat database (KBD)) with the frequency of the amino acid residue at a corresponding amino acid position in a second database of scFvs selected as having one or more desirable properties, e.g., a database of scFvs selected by QC screening in yeast, e.g., a QC database.

As described in detail in Example 4 below, antibody sequences (e.g., VH or VL sequences) from the first database (e.g., a database of mature antibody sequences) may be grouped according to their Kabat family subtype (e.g., Vh1b, VH3, etc.). Within each sequence subtype (i.e., subfamily), the frequency of each amino acid residue (e.g., A, V, etc.) at each amino acid position is determined as a percentage of all the analyzed sequences of that subtype. The same is done for all the sequences of the second database (e.g., a database of scFvs selected as having one or more desirable properties, e.g., by QC screening). For each subtype, the resulting percentages (relative frequencies) for each amino acid residue at a particular position are compared between the first and second databases. Where the relative frequency of a certain amino acid residue is increased in the second database (e.g., a QC database) relative to the first database (e.g., Kabat database), this indicates that the respective residue is favorably selected (i.e., an "enriched residue") and imparts favorable properties to the sequence. Conversely, where the relative frequency of the amino acid residue is decreased in the second database relative to the first database, this indicates that the respective residue is disfavored (i.e., an "excluded residue"). Accordingly, enriched residues are preferred residues for improving the functional properties (e.g., stability and/or solubility) of an immunobinder, while excluded residues are preferably avoided.

In view of the foregoing, in one embodiment, the invention provides a method of identifying a preferred amino acid residue for substitution in an immunobinder, the method comprising:

a) providing a first database of grouped $V_H$ or $V_L$ amino acid sequences (e.g., germline and/or mature antibody sequences grouped according to Kabat family subtype);
b) providing a second database of grouped scFv antibody $V_H$ or $V_L$ amino acid sequences selected as having at least one desirable functional property (e.g., according to QC assay);
c) determining amino acid frequency for an amino acid residue at a framework position of the first database and at a corresponding framework position of the second database;
d) identifying the amino acid residue as a preferred amino acid residue for substitution at a corresponding amino acid position of the immunobinder when the amino acid residue occurs at a higher frequency in the second database relative to the first database (i.e., an enriched residue).

The enrichment of an amino acid residue in the second (scFv) database (e.g., a QC database) can be quantified. For example, the ratio between the relative frequency of a residue within the second database (RF2) and the relative frequency of a residue within the first database (R1) can be determined. This ratio (RF2:RF1) may be termed an "enrichment factor" (EF). Accordingly, in certain embodiments, the amino acid residue in step (d) is identified if the ratio of the relative frequency of the amino acid residue between the first and second databases (herein, the "enrichment factor") is at least 1 (e.g., 1, 2, 3, 4, 5, 6, 7, 8, 9 or 10). In a preferred embodiment, the enrichment factor is greater than about 1.0 (e.g. 1.0, 1.1., 1.2, 1.3, 1.4 or 1.5). In yet another preferred embodiment, the enrichment factor is about 4.0 to about 6.0 (e.g., 4.0, 4.1, 4.2, 4.3, 4.4, 4.5, 4.6, 4.7, 4.8, 4.9, 5.0, 5.1, 5.2, 5.3, 5.4, 5.5, 5.6, 5.7, 5.8, 5.9 or 6.0). In another embodiment, the enrichment factor is about 6.0 to about 8.0 (e.g., 6.0, 6.1, 6.2, 6.3, 6.4, 6.5, 6.6, 6.7, 6.8, 6.9, 7.0, 7.1, 7.3, 7.4, 7.5, 7.6, 7.7, 7.8, 7.9 or 8.0). In other embodiments, the enrichment factor is greater than 10 (e.g., 10, 100, 1000, $10^4$, $10^5$, $10^6$, $10^7$, $10^8$, $10^9$ or more). In certain embodiments, infinite enrichment factors may be achieved.

In another embodiment, the invention provides a method of identifying an amino acid residue to be excluded at a particular position from an immunobinder, the method comprising:

a) providing a first database of grouped $V_H$ or $V_L$ amino acid sequences (e.g., germline and/or mature antibody sequences grouped according to Kabat family subtype);
b) providing a second database of grouped scFv antibody $V_H$ or $V_L$ amino acid sequences selected as having at least one desirable functional property (e.g., according to QC assay);
c) determining amino acid frequency for an amino acid residue at a framework position of the first database and at a corresponding framework position of the second database;
d) identifying the amino acid residue as a disfavored amino acid residue for substitution at corresponding amino acid position of the immunobinder when the amino acid residue occurs at a lower frequency in the second database relative to the first database, wherein said amino acid residue type is a disfavored amino acid residue (i.e., an excluded residue). In certain preferred embodiments, the disfavored amino acid residue in step (d) *supra* is identified if enrichment factor (EF) is less than 1.

Mutation of scFvs

In the methods of the invention, once one or more amino acid positions within a scFv have been identified as being potentially problematic with respect to the functional properties of the scFv, the method can further comprise mutating these one or more amino acid positions within the scFv $V_H$ or $V_L$ amino acid sequence. For example, an amino acid position identified for mutation can be substituted with an amino acid residue that is conserved or enriched at the corresponding position within the antibody $V_H$ or $V_L$ amino acid sequences of the database.

An amino acid position identified for mutation can be mutated using one of several possible mutagenesis methods well established in the art. For example, site directed mutagenesis can be used make a particular amino acid substitution at the amino acid position of interest. Site directed mutagenesis also can be used to create a set of mutated scFvs in which a limited repertoire of amino acid substitutions have been introduced at the amino acid position of interest.

Additionally or alternatively, the amino acid position(s) identified for mutation can be mutated by random mutagenesis or by biased mutagenesis to generate a library of mutated scFvs, followed by screening of the library of mutated scFvs and selection of scFvs, preferably selection of scFvs having at least one improved functional property. In a preferred embodiment, the library is screened using a yeast Quality Control-system (QC-system) (described in further detail above), which allows for selection of scFv frameworks having enhanced stability and/or solubility in a reducing environment.

Other suitable selection technologies for screening scFv libraries have been described in the art, including but not limited to display technologies such as phage display, ribosome display and yeast display (Jung et al. (1999) J. Mol. Biol. 294: 163-180; Wu et al. (1999) J. Mol. Biol. 294: 151- 162; Schier et al. (1996) J. Mol. Biol. 255: 28-43).

In one embodiment, an amino acid position identified for mutation is substituted with an amino acid residue that is most significantly enriched at the corresponding position within the antibody $V_H$ or $V_L$ amino acid sequences of the database. In another embodiment, the corresponding position within the antibody $V_H$ or $V_L$ amino acid sequences of the database is conserved with hydrophobic amino acid residues and the amino acid position identified for mutation within the scFv is substituted with a hydrophobic amino acid residue that is most significantly enriched at the corresponding position within the antibody $V_H$ or $V_L$ amino acid sequences of the database. In yet another embodiment, the corresponding position within the antibody $V_H$ or $V_L$ amino acid sequences of the database is conserved with hydrophilic amino acid residues and the amino acid position identified for mutation within the scFv is substituted with a hydrophilic amino acid residue that is most significantly enriched at the corresponding position within the antibody $V_H$ or $V_L$ amino acid sequences of the database. In yet another embodiment, the corresponding position within the antibody $V_H$ or $V_L$ amino acid sequences of the database is conserved with amino acid residues capable of forming a hydrogen bond and the amino acid position identified for mutation within the scFv is substituted with an amino acid residue capable of forming a hydrogen bond that is most significantly enriched at the corresponding position within the antibody $V_H$ or $V_L$ amino acid sequences of the database. In still another embodiment, the corresponding position within the antibody $V_H$ or $V_L$ amino acid sequences of the database is conserved with amino acid residues having a propensity to form a β-sheet and the amino acid position identified for mutation within the scFv is substituted with an amino acid residue having a propensity to form a β sheet that is most significantly enriched at the corresponding position within the antibody $V_H$ or $V_L$ amino acid sequences of the database.

In one embodiment, the best substitution that minimizes the overall free energy is selected as the mutation to be made at the amino acid position(s) of interest. The best substitution that minimizes the overall free energy can be determined using Boltzmann's Law. The formula for Boltzmann's Law is $\Delta\Delta G_{th} = RTln(f_{parental}/f_{consensus})$.

The role of potentially stabilizing mutations can be further determined by examining, for example, local and non-local interactions, canonical residues, interfaces, exposure degree and β-turn propensity. Molecular modeling methods known in the art can be applied, for example, in further examining the role of potentially stabilizing mutations. Molecular modeling methods also can be used to select "best fit" amino acid substitutions if a panel of possible substitutions are under consideration.

Depending on the particular amino acid position, further analysis may be warranted. For example, residues may be involved in the interaction between the heavy and the light chain or may interact with other residues through salt bridges or H bonding. In these cases special analysis might be required. In another embodiment of present invention, a potentially problematic residue for stability can be changed to one that is compatible with its counterpart in a covariant pair. Alternatively, the counterpart residue can be mutated in order to be compatible with the amino acid initially identified as being problematic.

Solubility Optimization

Residues potentially problematic for solubility in a scFv antibody include hydrophobic amino acids that are exposed to solvent in a scFv, but which in the context of a full-length antibody would be buried at the interface between variable and constant domains. In an engineered scFv, which lacks the constant domains, hydrophobic residues that participated in the interactions between the variable and constant domains become solvent exposed (see *e.g.,* Nieba et al. (1997) Protein Eng. 10:435-44). These residues on the surface of the scFv tend to cause aggregation and therefore solubility problems.

A number of strategies have been described to replace hydrophobic amino acids that are exposed to solvent on scFv antibodies. As is well known by those skilled in the art, modifying residues at certain positions affects biophysical properties of antibodies like stability, solubility, and affinity. In many cases these properties are interrelated, which means that the change of one single amino acid can affect several of above-mentioned properties. Therefore, mutating hydrophobic residues exposed to the solvent in a non-conservative manner may cause decreased stability and/or loss in affinity for its antigen.

Other approaches intend to solve solubility problems by exhaustive use of protein display technologies and or screening efforts. However, such methods are time-consuming, often fail to yield soluble protein or result in lower stability or reduction of the affinity of the antibody. In the present invention, methods are disclosed to design mutations of solvent exposed hydrophobic residues to residues with a higher hydrophilicity using a sequence based analysis. The potentially problematic residues can be replaced by choosing the most frequently represented hydrophilic amino acid at defined positions. If a residue is found to interact with any other residue in the antibody, the potentially problematic residue can be mutated, not to the most frequent residue but to one that is compatible with the second amino acid of the covariant pair. Alternatively, a second amino acid of the covariant pair can also be mutated in order to restore the combination of amino acids. Furthermore, the percentage of similarity between sequences can be taken into account to assist finding of an optimal combination of two interrelated amino acids.

Hydrophobic amino acids on the surface of the scFv are identified using several approaches, including but not limited to approaches based on solvent exposure, experimental information and sequence information, as well as molecular modeling.

In one embodiment of this invention, the solubility is improved by replacing hydrophobic residues exposed on the surface of the scFv antibody with the most frequent hydrophilic residues present at these positions in databases. This rationale rests on the fact that frequently occurring residues are likely to be unproblematic. As will be appreciated by those skilled in the art, conservative substitutions usually have a small effect in destabilizing the molecule, whereas non-conservative substitutions might be detrimental for the functional properties of the scFv.

Sometimes hydrophobic residues on the surface of the antibody may be involved in the interaction between the heavy and the light chain or may interact with other residues through salt bridges or H bonding. In these cases special analysis might be required. In another embodiment of the present invention, the potentially problematic residues for solubility can be mutated not to the most frequent residue but to a compatible one with the covariant pair or a second mutation can be performed to restore the combination of co-variant amino acids.

Additional methods may be used to design mutations at solvent exposed hydrophobic positions. In another embodiment of this invention, methods are disclosed that employ constraining of the database to those sequences that reveal the highest similarity to the scFv to be modified (discussed further above). By applying such a constrained reference database, the mutation is designed such that it best fits in the specific sequence context of the antibody to be optimized. In this situation, the chosen hydrophilic residue may in fact be poorly represented at its respective position when compared to a larger number of sequences (*i.e.,* the unconstrained database).

Stability Optimization

Single-chain antibody fragments contain a peptide linker that covalently joins the light and heavy variable domains. Although such a linker is effective to avoid having the variable domains come apart, and thereby makes the scFv superior over the Fv fragment, the scFv fragment still is more prone to unfolding and aggregation as compared to an Fab fragment or to a full-length antibody, in both of which the $V_H$ and the $V_L$ are only linked indirectly via the constant domains.

Another common problem in scFvs is exposure of hydrophobic residues on the surface of the scFv that lead to intermolecular aggregation. Furthermore, sometimes somatic mutations acquired during the process of affinity maturation place hydrophilic residues in the core of the β-sheet. Such mutations may be well tolerated in the IgG format or even in a Fab fragment but in an scFv this clearly contributes to destabilization and consequent unfolding.

Known factors that contribute to scFv destabilization include: solvent exposed hydrophobic residues on the surface of the scFv antibody; unusual hydrophilic residues buried in the core of the protein, as well as hydrophilic residues present in the hydrophobic interface between the heavy and the light chains. Furthermore, van der Waals packing interactions between nonpolar residues in the core are known to play an important role in protein stability (Monsellier E. and Bedouelle H. (2006) J. Mol. Biol. 362:580-93, Tan et al. (1998) Biophys. J. 75:1473-82; Wörn A. and Plückthun A. (1998) Biochemistry 37:13120-7).

Thus, in one embodiment, in order to increase the stability of scFv antibodies, unusual and/or unfavorable amino acids at very conserved positions are identified and mutated to amino acids that are more common at these conserved positions. Such unusual and/or unfavorable amino acids include: (i) solvent exposed hydrophobic residues on the surface of the scFv antibody; (ii) unusual hydrophilic residues buried in the core of the protein; (iii) hydrophilic residues present in the hydrophobic interface between the heavy and the light chains; and (iv) residues that disturb the VH/VL interface VH/VL by steric hindrance.

Thus, in one embodiment of this invention, an increase in stability can be achieved by substituting amino acids that are poorly represented at their positions by amino acids that occur most frequently at these positions. Frequency of occurrence generally provides an indication of biological acceptance.

Residues may be involved in the interaction between the heavy and the light chain or may interact with other residues through salt bridges, H bonding, or disulfide bonding. In these cases special analysis might be required. In another embodiment of present invention, a potentially problematic residue for stability can be changed to one that is compatible with its counterpart in a covariant pair. Alternatively, the counterpart residue can be mutated in order to be compatible with the amino acid initially identified as being problematic.

Additional methods may be used to design mutations to improve stability. In another embodiment of this invention, methods are disclosed that employ constraining of the database to those sequences that reveal the highest similarity to the scFv to be modified (discussed further above). By applying such a constrained reference database, the mutation is designed such that it best fits in the specific sequence context of the antibody to be optimized. The mutation uses the most frequent amino acid that is present in the selected subset of database sequences. In this situation, the chosen residue may in fact be poorly represented at its respective position when compared to a larger number of sequences (*i.e.*, the unconstrained database).

ScFv Compositions and Formulations

Another aspect of the invention pertains to scFv composition prepared according to the methods of invention. Thus, the invention provides engineered scFv compositions in which one or more mutations have been introduced into the amino acid sequence, as compared to an original scFv of interest, wherein the mutation(s) has been introduced into a position(s) predicted to influence one or more biological properties, such as stability or solubility, in particular one or more framework positions. In one embodiment, the scFv has been engineered to contain one mutated amino acid position (*e.g.,* one framework position). In other embodiments, the scFv has been engineered to contain two, three, four, five, six, seven, eight, nine, ten or more than ten mutated amino acid positions (e.g., framework positions).

Another aspect of the invention pertains to pharmaceutical formulations of the scFv compositions of the invention. Such formulations typically comprise the scFv composition and a pharmaceutically acceptable carrier. As used herein, "pharmaceutically acceptable carrier" includes any and all solvents, dispersion media, coatings, antibacterial and antifungal agents, isotonic and absorption delaying agents, and the like that are physiologically compatible. Preferably, the carrier is suitable for, for example, intravenous, intramuscular, subcutaneous, parenteral, spinal, epidermal administration (*e.g.*, by injection or infusion), or topical (e.g., to the eye or skin). Depending on the route of administration, the scFv may be coated in a material to protect the compound from the action of acids and other natural conditions that may inactivate the compound.

The pharmaceutical compounds of the invention may include one or more pharmaceutically acceptable salts. A "pharmaceutically acceptable salt" refers to a salt that retains the desired biological activity of the parent compound and does not impart any undesired toxicological effects (see *e.g.,* Berge, S. M., et al. (1977) J. Pharm. Sci. 66:1-19). Examples of such salts include acid addition salts and base addition salts. Acid addition salts include those derived from nontoxic inorganic acids, such as hydrochloric, nitric, phosphoric, sulfuric, hydrobromic, hydroiodic, phosphorous and the like, as well as from nontoxic organic acids such as aliphatic mono- and dicarboxylic acids, phenyl-substituted alkanoic acids, hydroxy alkanoic acids, aromatic acids, aliphatic and aromatic sulfonic acids and the like. Base addition salts include those derived from alkaline earth metals, such as sodium, potassium, magnesium, calcium and the like, as well as from nontoxic organic amines, such as N,N'-dibenzylethylenediamine, N-methylglucamine, chloroprocaine, choline, diethanolamine, ethylenediamine, procaine and the like.

A pharmaceutical composition of the invention also may include a pharmaceutically acceptable anti-oxidant. Examples of pharmaceutically acceptable antioxidants include: (1) water soluble antioxidants, such as ascorbic acid, cysteine hydrochloride, sodium bisulfate, sodium metabisulfite, sodium sulfite and the like; (2) oil-soluble antioxidants, such as ascorbyl palmitate, butylated hydroxyanisole (BHA), butylated hydroxytoluene (BHT), lecithin, propyl gallate, alpha-tocopherol, and the like; and (3) metal chelating agents, such as citric acid, ethylenediamine tetraacetic acid (EDTA), sorbitol, tartaric acid, phosphoric acid, and the like.

Examples of suitable aqueous and nonaqueous carriers that may be employed in the pharmaceutical compositions of the invention include water, ethanol, polyols (such as glycerol, propylene glycol, polyethylene glycol, and the like), and suitable mixtures thereof, vegetable oils, such as olive oil, and injectable organic esters, such as ethyl oleate. Proper fluidity can be maintained, for example, by the use of coating materials, such as lecithin, by the maintenance of the required particle size in the case of dispersions, and by the use of surfactants.

These compositions may also contain adjuvants such as preservatives, wetting agents, emulsifying agents and dispersing agents. Prevention of presence of microorganisms may be ensured both by sterilization procedures, *supra,* and by the inclusion of various antibacterial and antifungal agents, for example, paraben, chlorobutanol, phenol sorbic acid, and the like. It may also be desirable to include isotonic agents, such as sugars, sodium chloride, and the like into the compositions. In addition, prolonged absorption of the injectable pharmaceutical form may be brought about by the inclusion of agents that delay absorption such as aluminum monostearate and gelatin.

Pharmaceutically acceptable carriers include sterile aqueous solutions or dispersions and sterile powders for the extemporaneous preparation of sterile injectable solutions or dispersion. The use of such media and agents for pharmaceutically active substances is known in the art. Except insofar as any conventional media or agent is incompatible with the active compound, use thereof in the pharmaceutical compositions of the invention is contemplated. Supplementary active compounds can also be incorporated into the compositions.

Therapeutic compositions typically must be sterile and stable under the conditions of manufacture and storage. The composition can be formulated as a solution, microemulsion, liposome, or other ordered structure suitable to high drug concentration. The carrier can be a solvent or dispersion medium containing, for example, water, ethanol, polyol (for example, glycerol, propylene glycol, and liquid polyethylene glycol, and the like), and suitable mixtures thereof. The proper fluidity can be maintained, for example, by the use of a coating such as lecithin, by the maintenance of the required particle size in the case of dispersion and by the use of surfactants. In many cases, it will be preferable to include isotonic agents, for example, sugars, polyalcohols such as mannitol, sorbitol, or sodium chloride in the composition. Prolonged absorption of the injectable compositions can be brought about by including in the composition an agent that delays absorption, for example, monostearate salts and gelatin.

Sterile injectable solutions can be prepared by incorporating the active compound in the required amount in an appropriate solvent with one or a combination of ingredients enumerated above, as required, followed by sterilization microfiltration. Generally, dispersions are prepared by incorporating the active compound into a sterile vehicle that contains a basic dispersion medium and the required other ingredients from those enumerated above. In the case of sterile powders for the preparation of sterile injectable solutions, the preferred methods of preparation are vacuum drying and freeze-drying (lyophilization) that yield a powder of the active ingredient plus any additional desired ingredient from a previously sterile-filtered solution thereof.

The amount of active ingredient which can be combined with a carrier material to produce a single dosage form will vary depending upon the subject being treated, and the particular mode of administration. The amount of active ingredient which can be combined with a carrier material to produce a single dosage form will generally be that amount of the composition which produces a therapeutic effect. Generally, out of one hundred percent, this amount will range from about 0.01 percent to about ninety-nine percent of active ingredient, preferably from about 0.1 percent to about 70 percent, most preferably from about 1 percent to about 30 percent of active ingredient in combination with a pharmaceutically acceptable carrier.

Dosage regimens are adjusted to provide the optimum desired response (e.g., a therapeutic response). For example, a single bolus may be administered, several divided doses may be administered over time or the dose may be proportionally reduced or increased as indicated by the exigencies of the therapeutic situation. It is especially advantageous to formulate parenteral compositions in dosage unit form for ease of administration and uniformity of dosage. Dosage unit form as used herein refers to physically discrete units suited as unitary dosages for the subjects to be treated; each unit contains a predetermined quantity of active compound calculated to produce the desired therapeutic effect in association with the required pharmaceutical carrier. The specification for the dosage unit forms of the invention are dictated by and directly dependent on (a) the unique characteristics of the active compound and the particular therapeutic effect to be achieved, and (b) the limitations inherent in the art of compounding such an active compound for the treatment of sensitivity in individuals.

Immunobinder Engineering Based on "Functional Consensus" Approach

As described in detail in Examples 2 and 3, the "functional consensus" approach described herein, in which a database of scFv sequences selected for improved properties is used to analyze framework position variability, allows for the identification of amino acid positions that are either more or less tolerant of variability as compared to variability at these same positions in germline databases. As described in detail in Examples 5 and 6, back-mutation of certain amino acid positions within a sample scFv to the germline consensus residue has either a neutral or detrimental effect, whereas scFv variants that contain "functional consensus" residues exhibit increased thermal stability as compared to the wild-type scFv molecule. Accordingly, the framework positions identified herein through the functional consensus approach are preferred positions for scFv modification in order to alter, and preferably improve, the functional properties of the scFv. As set forth in Table 3-8 in Example 3, the following framework positions have been identified as preferred positions for modification in the indicated $V_H$ or $V_L$ sequences (the numbering used below is the AHo numbering system; conversion tables to convert the AHo numbering to the Kabat system numbering are set forth as Tables 1 and 2 in Example 1):

VH3: amino acid positions 1, 6, 7, 89 and 103;
VH1a: amino acid positions 1, 6, 12, 13, 14, 19, 21, 90, 92, 95 and 98;
VH1b: amino acid positions 1, 10, 12, 13, 14, 20, 21, 45, 47, 50, 55, 77, 78, 82, 86, 87 and 107;
Vκ1: amino acid positions 1, 3, 4, 24, 47, 50, 57, 91 and 103;
Vκ3: 2, 3, 10, 12, 18, 20, 56, 74, 94, 101 and 103; and
Vλ1: 1, 2, 4, 7, 11, 14, 46, 53, 82, 92 and 103.

Accordingly, one or more of these amino acid positions can be selected for engineering in immunobinders, such as scFv molecules, to thereby produce variant (*i.e.*, mutated) forms of the immunobinders. Thus, in yet another aspect, the invention provides a method of engineering an immunobinder, the method comprising:

a) selecting one or more amino acid positions within the immunobinder for mutation; and
b) mutating the one more more amino acid positions selected for mutation, wherein the one or more amino acid positions selected for mutation are selected from the group consisting of:

(i) amino acid positions 1, 6, 7, 89 and 103 of VH3 using AHo numbering (amino acid positions 1, 6, 7, 78 and 89 using Kabat numbering);
(ii) amino acid positions 1, 6, 12, 13, 14, 19, 21, 90, 92, 95 and 98 of VH1a using AHo numbering (amino acid positions 1,6, 11, 12, 13, 18, 20, 79, 81, 82b and 84 using Kabat numbering);
(iii) amino acid positions 1, 10, 12, 13, 14, 20, 21, 45, 47, 50, 55, 77, 78, 82, 86, 87 and 107 of VH1b using AHo numbering (amino acid positions 1, 9, 11, 12, 13, 19, 20, 38, 40, 43, 48, 66, 67, 71, 75, 76 and 93 using Kabat numbering);
(iv) amino acid positions 1, 3, 4, 24, 47, 50, 57, 91 and 103 of Vκ1 using AHo numbering (amino acid positions 1, 3, 4, 24, 39, 42, 49, 73 and 85 using Kabat numbering);
(v) amino acid positions 2, 3, 10, 12, 18, 20, 56, 74, 94, 101 and 103 of Vκ3 using AHo numbering (amino acid positions 2, 3, 10, 12, 18, 20, 48, 58, 76, 83 and 85 using Kabat numbering); and
(vi) amino acid positions 1, 2, 4, 7, 11, 14, 46, 53, 82, 92 and 103 of Vλ1 using AHo numbering (amino acid positions 1, 2, 4, 7, 11, 14, 38, 45, 66, 74 and 85 using Kabat numbering).

In a preferred embodiment, the one or more amino acid positions selected for mutation are selected from the group consisting of amino acid positions 1, 6, 7, 89 and 103 of VH3 using AHo numbering (amino acid positions 1, 6, 7, 78 and 89 using Kabat numbering).

In another preferred embodiment, the one or more amino acid positions selected for mutation are selected from the group consisting of amino acid positions 1, 6, 12, 13, 14, 19, 21, 90, 92, 95 and 98 of VH1a using AHo numbering (amino acid positions 1, 6, 11, 12, 13, 18, 20, 79, 81, 82b and 84 using Kabat numbering).

In another preferred embodiment, the one or more amino acid positions selected for mutation are selected from the group consisting of amino acid positions 1, 10, 12, 13, 14, 20, 21, 45, 47, 50, 55, 77, 78, 82, 86, 87 and 107 of VH1b using AHo numbering (amino acid positions 1, 9, 11, 12, 13, 19, 20, 38, 40, 43, 48, 66, 67, 71, 75, 76 and 93 using Kabat numbering).

In another preferred embodiment, the one or more amino acid positions selected for mutation are selected from the group consisting of amino acid positions 1, 3, 4, 24, 47, 50, 57, 91 and 103 of Vκ1 using AHo numbering (amino acid positions 1, 3, 4, 24, 39, 42, 49, 73 and 85 using Kabat numbering).

In another preferred embodiment, the one or more amino acid positions selected for mutation are selected from the group consisting of amino acid positions 2, 3, 10, 12, 18, 20, 56, 74, 94, 101 and 103 of Vκ3 using AHo numbering (amino acid positions 2, 3, 10, 12, 18, 20, 48, 58, 76, 83 and 85 using Kabat numbering).

In another preferred embodiment, one or more amino acid positions selected for mutation are selected from the group consisting of amino acid positions 1, 2, 4, 7, 11, 14, 46, 53, 82, 92 and 103 of Vλ1 using AHo numbering (amino acid positions 1, 2, 4, 7, 11, 14, 38, 45, 66, 74 and 85 using Kabat numbering).

In various embodiments, one, two, three, four, five, six, seven, eight, nine, ten, eleven, twelve, thirteen, fourteen, fifteen, sixteen, seventeen, eighteen, nineteen, twenty or more than twenty of the above-described amino acid positions are selected for mutation.

Preferably, the immunobinder is a scFv, but other immunobinders, such as full-length immunogloblins, Fab fragments or any other type of immunobinder described herein (e.g., Dabs or Nanobodies), also can be engineered according to the method. The invention also encompasses immunobinders prepared according to the engineering method, as well as compositions comprising the immunobinders and a pharmaceutically acceptable carrier. Moreover, the present invention further extends to immunobinders which were engineered according to anyone of the methods disclosed herein and are produced at commercial scale.

In certain exemplary embodiments, an immunobinder engineered according to the method of the invention is an art-recognized immunobinder which binds a target antigen of therapeutic importance or an immunobinder comprising variable regions (VL and/or VL regions) or one or more CDRs (e.g., CDRL1, CDRL2, CDRL3, CDRH1, CDRH2, and/or CDRH3) derived from the immunobinder of therapeutic importance. For example, immunobinders currently approved by the FDA or other regulatory authorities can be engineered according to the methods of the invention. More specifically, these exemplary immunobinders include, but are not limited to, anti-CD3 antibodies such as muromonab (Orthoclone® OKT3; Johnson&Johnson, Brunswick, NJ; see Arakawa et al. J. Biochem, (1996) 120:657-662; Kung and Goldstein et al., Science (1979), 206: 347-349), anti-CD11 antibodies such as efalizumab (Raptiva®, Genentech, South San Francisco, CA), anti-CD20 antibodies such as rituximab (Rituxan®/ Mabthera®, Genentech, South San Francisco, CA), tositumomab (Bexxar®, GlaxoSmithKline, London) or ibritumomab (Zevalin®, Biogen Idec, Cambridge MA)(see US Patent Nos. 5,736,137; 6,455,043; and 6,682,734), anti-CD25 (IL2Rα) antibodies such as daclizumab (Zenapax®, Roche, Basel, Switzerland) or basiliximab (Simulect®, Novartis, Basel, Switzerland), anti-CD33 antibodies such as gemtuzumab (Mylotarg®, Wyeth, Madison, NJ -see US Pat Nos. 5,714,350 and 6,350,861), anti-CD52 antibodies such as alemtuzumab (Campath®, Millennium Pharmacueticals, Cambridge, MA), anti-GpIIb/gIIa antibodies such as abciximab (ReoPro®, Centocor, Horsham, PA), anti-TNFα antibodies such as infliximab (Remicade®, Centocor, Horsham, PA) or adalimumab (Humira®, Abbott, Abbott Park, IL -see US Patent No. 6,258,562), anti-IgE antibodies such as omalizumab (Xolair®, Genentech, South San Francisco, CA), anti-RSV antibodies such as palivizumab (Synagis®, Medimmune, Gaithersburg, MD -see US Patent No. 5,824,307), anti-EpCAM antibodies such as edrecolomab (Panorex®, Centocor), anti-EGFR antibodies such as cetuximab (Erbitux®, Imclone Systems, New York, NY) or panitumumab (Vectibix®, Amgen, Thousand Oaks, CA), anti-HER2/neu antibodies such as trastuzumab (Herceptin®, Genentech), anti-a4 integrin antibodies such as natalizumab (Tysabri®, BiogenIdec), anti-C5 antibodies such as eculizumab (Soliris®, Alexion Pharmaceuticals, Chesire, CT) and anti-VEGF antibodies such as bevacizumab (Avastin®, Genentech -see US Patent No. 6,884,879) or ranibizumab (Lucentis®, Genentech).

Nothwithstanding the foregoing, in various embodiments, certain immunobinders are excluded from being used in the engineering methods of the invention and/or are excluded from being the immunobinder composition produced by the engineering methods. For example, in various embodiments, there is a proviso that the immunobinder is not any of the scFv antibodies, or variants thereof, as disclosed in PCT Publications WO 2006/131013 and WO 2008/006235, such as ESBA105 or variants thereof that are disclosed in PCT Publications WO 2006/131013 and WO 2008/006235.

In various other embodiments, if the immunobinder to be engineered according to the above-described methods is any of the scFv antibodies, or variants thereof, disclosed in PCT publications WO 2006/131013 or WO 2008/006235, then there can be the proviso that the list of possible amino acid positions that may be selected for substitution according to the engineering method does not include any or all of the following amino acid positions: AHo position 4 (Kabat 4) of Vκ1 or Vλ1; AHo position 101 (Kabat 83) of Vκ3; AHo position 12 (Kabat 11) of VH1a or VH1b; AHo position 50 (Kabat 43) of VH1b; AHo position 77 (Kabat 66) for VH1b; AHo position 78 (Kabat 67) for VH1b; AHo position 82 (Kabat 71) for VH1b; AHo position 86 (Kabat 75) for VH1b; AHo position 87 (Kabat 76) for VH1b; AHo position 89 (Kabat 78) for VH3; AHo position 90 (Kabat 79) for VH1a; and/or AHo position 107 (Kabat 93) for VH1b.

In still various other embodiments, for any immunobinder to be engineered according to the above-described methods, and/or any immunobinder produced according to the above-described methods, there can be the proviso that the list of possible amino acid positions that may be selected for substitution according to the engineering method does not include any or all of the following amino acid positions: AHo position 4 (Kabat 4) of Vκ1 or Vλ1; AHo position 101 (Kabat 83) of Vκ3; AHo position 12 (Kabat 11) of VH1a or VH1b; AHo position 50 (Kabat 43) of VH1b; AHo position 77 (Kabat 66) for VH1b; AHo position 78 (Kabat 67) for VH1b; AHo position 82 (Kabat 71) for VH1b; AHo position 86 (Kabat 75) for VH1b; AHo position 87 (Kabat 76) for VH1b; AHo position 89 (Kabat 78) for VH3; AHo position 90 (Kabat 79) for VH1a; and/or AHo position 107 (Kabat 93) for VH1b.

Mutation of Immunobinders at Exemplary and Preferred Positions

As described in detail in Example 7, the functional consensus approach described herein has been used successfully to identify particular amino acid residue substitutions that are enriched for in the selected scFv ("QC") database. For example, Tables 13-18 in Example 7 list exemplary and preferred amino acid substitutions at defined amino acid positions within VH3, VH1a, VH1b, Vκ1, Vκ3 or Vλ1 family frameworks. The exemplary substitutions include the consensus residue identified from analysis of the germline (IMGT and Vbase) and mature antibody (KDB) databases, as well as the amino acid residues identified as being preferentially enriched in the selected scFv framework database (QC). The most preferred substitution identified is that residue that exhibits the greatest enrichment at that position in the selected scFv framework database (QC).

Accordingly, the invention provides engineering methods in which one or more specified amino acid substitutions are introduced into an immunobinder, such as a scFv antibody. Such substitutions can be carried out using standard molecular biology methods, such as site-directed mutagenesis, PCR-mediated mutagenesis and the like.

In one embodiment, the invention provides a method of engineering an immunobinder, such as a scFv antibody, in which one or more amino acid substitutions are made at one or more amino acid positions, wherein the amino acid residue that is used for substitution into the immunobinder is selected from the exemplary and preferred amino acid residues identified in Tables 13-18 herein. Thus, the invention provides a method of engineering an immunobinder, the immunobinder comprising (i) a heavy chain variable region, or fragment thereof, of a VH3, VH1a or VH1b family, the heavy chain variable region comprising $V_H$ framework residues or (ii) a light chain variable region, or fragment thereof, of a Vκ1, Vκ3 or Vλ1 family, the light chain variable region comprising $V_L$ framework residues, the method comprising:

A) selecting one or more amino acid positions within the $V_H$ framework residues, the $V_L$ framework residues or the $V_H$ and $V_L$ framework residues for mutation; and
B) mutating the one or more amino acid positions selected for mutation,

a) wherein if the one or more amino acid positions selected for mutation are of a VH3 family heavy chain variable region, the mutating comprises one or more substitutions selected from the group consisting of:

(i) glutamic acid (E) or glutamine (Q) at amino acid position 1 using AHo or Kabat numbering system;
(ii) glutamic acid (E) or glutamine (Q) at amino acid position 6 using AHo or Kabat numbering system;
(iii) threonine (T), serine (S) or alanine (A) at amino acid position 7 using AHo or Kabat numbering system;
(iv) alanine (A), valine (V), leucine (L) or phenylalanine (F) at amino acid position 89 using AHo numbering system (amino acid position 78 using Kabat numbering system); and
(v) arginine (R), glutamine (Q), valine (V), isoleucine (I), leucine (L), methionine (M) or phenylalanine (F) at amino acid position 103 using AHo numbering system (amino acid position 89 using Kabat numbering);

b) wherein if the one or more amino acid positions selected for mutation are of a VH1a family heavy chain variable region, the mutating comprises one or more substitutions selected from the group consisting of:

(i) glutamic acid (E) or glutamine (Q) at amino acid position 1 using AHo or Kabat numbering system;
(ii) glutamic acid (E) or glutamine (Q) at amino acid position 6 using AHo or Kabat numbering system;
(iii) leucine (L) or valine (V) at amino acid position 12 using AHo numbering system (amino acid position 11 using Kabat numbering system);
(iv) methionine (M) or lysine (K) at amino acid position 13 using AHo numbering system (amino acid position 12 using Kabat numbering system):
(v) glutamic acid (E), glutamine (Q) or lysine (K) at amino acid position 14 using AHo numbering system (amino acid position 13 using Kabat numbering system);
(vi) leucine (L) or valine (V) at amino acid position 19 using AHo numbering system (amino acid position 18 using Kabat numbering system);
(vii) isoleucine (I) or valine (V) at amino acid position 21 using AHo numbering system (amino acid position 20 using Kabat numbering system);

(viii) phenylalanine (F), serine (S), histidine (H), aspartic acid (D) or tyrosine (Y) at amino acid position 90 using AHo numbering system (amino acid position 79 using Kabat numbering system);
(ix) aspartic acid (D), glutamine (Q) or glutamic acid (E) at amino acid position 92 using AHo numbering system (amino acid position 81 using Kabat numbering system);
(x) glycine (G), asparagine (N), threonine (T) or serine (S) at amino acid position 95 using AHo numbering system (amino acid position 82b using Kabat numbering system); and
(xi) threonine (T), alanine (A), proline (P), phenylalanine (F) or serine (S) at amino acid position 98 using AHo numbering (amino acid position 84 using Kabat numbering);

c) wherein if the one or more amino acid positions selected for mutation are of a VH1b family heavy chain variable region, the mutating comprises one or more substitutions selected from the group consisting of:

(i) glutamic acid (E) or glutamine (Q) at amino acid position 1 using AHo or Kabat numbering system;
(ii) alanine (A), threonine (T), proline (P), valine (V) or aspartic acid (D) at amino acid position 10 using AHo numbering system (amino acid position 9 using Kabat numbering system);
(iii) leucine (L) or valine (V) at amino acid position 12 using AHo numbering system (amino acid position 11 using Kabat numbering system);
(iv) lysine (K), valine (V), arginine (R), glutamine (Q) or methionine (M) at amino acid position 13 using AHo numbering system (amino acid position 12 using Kabat numbering system):
(v) glutamic acid (E), lysine (K), arginine (R) or methionine (M) at amino acid position 14 using AHo numbering system (amino acid position 13 using Kabat numbering system);
(vi) arginine (R), threonine (T), lysine (K) or asparagine (N) at amino acid position 20 using AHo numbering system (amino acid position 19 using Kabat numbering system);
(vii) isoleucine (I), phenylalanine (F), valine (V) or leucine (L) at amino acid position 21 using AHo numbering system (amino acid position 20 using Kabat numbering system);
(viii) arginine (R) or lysine (K) at amino acid position 45 using AHo numbering system (amino acid position 38 using Kabat numbering system);
(ix) threonine (T), proline (P), valine (V), alanine (A) or arginine (R) at amino acid position 47 using AHo numbering system (amino acid position 40 using Kabat numbering system);
(x) lysine (K), glutamine (Q), histidine (H) or glutamic acid (E) at amino acid position 50 using AHo numbering system (amino acid position 43 using Kabat numbering system);
(xi) methionine (M) or isoleucine (I) at amino acid position 55 using AHo numbering (amino acid position 48 using Kabat numbering);
(xii) lysine (K) or arginine (R) at amino acid position 77 using AHo numbering (amino acid position 66 using Kabat numbering);
(xiii) alanine (A), valine (V), leucine (L) or isoleucine (I) at amino acid position 78 using AHo numbering system (amino acid position 67 using Kabat numbering system);
(xiv) glutamic acid (E), arginine (R), threonine (T) or alanine (A) at amino acid position 82 using AHo numbering system (amino acid position 71 using Kabat numbering system);
(xv) threonine (T), serine (S), isoleucine (I) or leucine (L) at amino acid position 86 using AHo numbering system (amino acid position 75 using Kabat numbering system);
(xvi) aspartic acid (D), serine (S), asparagine (N) or glycine (G) at amino acid position 87 using AHo numbering system (amino acid position 76 using Kabat numbering system); and
(xvii) asparagine (N), serine (S) or alanine (A) at amino acid position 107 using AHo numbering system (amino acid position 93 using Kabat numbering system);

d) wherein if the one or more amino acid positions selected for mutation are of a Vκ1 family light chain variable region, the mutating comprises one or more substitutions selected from the group consisting of:

(i) aspartic acid (D), glutamic acid (E) or isoleucine (I) at amino acid position 1 using AHo or Kabat numbering system;
(ii) glutamine (Q), valine (V) or isoleucine (I) at amino acid position 3 using AHo or Kabat numbering system;
(iii) valine (V), leucine (L), isoleucine (I) or methionine (M) at amino acid position 4 using AHo or Kabat numbering system;
(iv) arginine (R) or glutamine (Q) at amino acid position 24 using AHo or Kabat numbering system;
(v) lysine (K), arginine (R) or isoleucine (I) at amino acid position 47 using AHo numbering system (amino acid position 39 using Kabat numbering system);
(vi) lysine (K), arginine (R), glutamic acid (E) threonine (T), methionine (M) or glutamine (Q) at amino acid position 50 using AHo numbering system (amino acid position 42 using Kabat numbering system);
(vii) histidine (H), serine (S), phenylalanine (F) or tyrosine (Y) at amino acid position 57 using AHo numbering system (amino acid position 49 using Kabat numbering system);
(viii) leucine (L) or phenylalanine (F) at amino acid position 91 using AHo numbering system (amino acid position 73 using Kabat numbering system); and
(ix) threonine (T), valine (V), serine (S), glycine (G) or isoleucine (I) at amino acid position 103 using AHo numbering system (amino acid position 85 using Kabat numbering system);

e) wherein if the one or more amino acid positions selected for mutation are of a Vκ3 family light chain variable region, the mutating comprises one or more substitutions selected from the group consisting of:

(i) isoleucine (I) or threonine (T) at amino acid position 2 using AHo or Kabat numbering system;
(ii) valine (V) or threonine (T) at amino acid position 3 using AHo or Kabat numbering system;
(iii) threonine (T) or isoleucine (I) at amino acid position 10 using AHo or Kabat numbering system;
(iv) serine (S) or tyrosine (Y) at amino acid position 12 using AHo or Kabat numbering system;
(v) serine (S) or arginine (R) at amino acid position 18 using AHo or Kabat numbering system;
(vi) threonine (T) or alanine (A) at amino acid position 20 using AHo or Kabat numbering system;
(vii) isoleucine (I) or methionine (M) at amino acid position 56 using AHo numbering system (amino acid position 48 using Kabat numbering system);
(viii) isoleucine (I), valine (V) or threonine (T) at amino acid position 74 using AHo numbering system (amino acid position 58 using Kabat numbering system);
(ix) serine (S) or asparagine (N) at amino acid position 94 using AHo numbering system (amino acid position 76 using Kabat numbering system);
(x) phenylalanine (F), tyrosine (Y) or serine (S) at amino acid position 101 using AHo numbering system (amino acid position 83 using Kabat numbering system); and
(xi) valine (V), leucine (L) or alanine (A) at amino acid position 103 using AHo numbering (amino acid position 85 using Kabat numbering); and

f) wherein if the one or more amino acid positions selected for mutation are of a Vλ1 family light chain variable region, the mutating comprises one or more substitutions selected from the group consisting of:

(i) leucine (L), glutamine (Q), serine (S) or glutamic acid (E) at amino acid position I using AHo or Kabat numbering system;
(ii) serine (S), alanine (A), proline (P), isoleucine (I) or tyrosine (Y) at amino acid position 2 using AHo or Kabat numbering system;
(iii) valine (V), methionine (M) or leucine (L) at amino acid position 4 using AHo or Kabat numbering system;
(iv) serine (S), glutamic acid (E), proline (P) at amino acid position 7 using AHo or Kabat numbering system;
(v) alanine (A) or valine (V) at amino acid position 11 using AHo or Kabat numbering system;
(vi) threonine (T), serine (S) or alanine (A) at amino acid position 14 using AHo or Kabat numbering system;
(vii) histidine (H) or glutamine (Q) at amino acid position 46 using AHo numbering system (amino acid position 38 using Kabat numbering system);
(viii) lysine (K), threonine (T), serine (S), asparagine (N), glutamine (Q) or proline (P) at amino acid position 53 using AHo numbering system (amino acid position 45 using Kabat numbering system);
(ix) arginine (R), glutamine (Q) or lysine (K) at amino acid position 82 using AHo numbering system (amino acid position 66 using Kabat numbering system);
(x) glycine (G), threonine (T), aspartic acid (D), alanine (A) at amino acid position 92 using AHo numbering system (amino acid position 74 using Kabat numbering system); and
(xi) aspartic acid (D), valine (V), threonine (T), histidine (H) or glutamic acid (E) at amino acid position 103 using AHo numbering (amino acid position 85 using Kabat numbering).

In a preferred embodiment, the immunobinder is a scFv antibody. In other embodiments, the immunobinder is, for example, a full-length immunoglobulin, Dab, Nanobody or a Fab fragment.

The invention also encompasses immunobinders prepared according to the above-described method. Preferably, the immunobinder is a scFv antibody. In other embodiments, the immunobinder is, for example, a full-length immunoglobulin, Dab, Nanobody or a Fab fragment. The invention also encompasses pharmaceutical compositions comprising the afore-mentioned immunobinder(s) and a pharmaceutically acceptable carrier.

In another embodiment, the invention provides a method of engineering an immunobinder, such as a scFv antibody, in which one or more amino acid substitutions are made at one or more amino acid positions, wherein the amino acid residue that is used for substitution into the immunobinder is selected from the exemplary and preferred amino acid residues identified in Tables 13-18 herein, but not including the consensus amino acid residue identified from analysis of the germline (IMGT and Vbase) and mature antibody (KDB) databases. That is, the substitutions are selected from those amino acid residues that exhibit enrichment in the selected scFv database (QC). Thus, in this embodiment, the invention provides a method of engineering an immunobinder, the immunobinder comprising (i) a heavy chain variable region, or fragment thereof, of a VH3, VH1a or VH1b family, the heavy chain variable region comprising $V_H$ framework residues or (ii) a light chain variable region, or fragment thereof, of a Vκ1, Vκ3 or Vλ1 family, the light chain variable region comprising $V_L$ framework residues, the method comprising:

A) selecting one or more amino acid positions within the $V_H$ framework residues, the $V_L$ framework residues or the $V_H$ and $V_L$ framework residues for mutation; and
B) mutating the one or more amino acid positions selected for mutation,

a) wherein if the one or more amino acid positions selected for mutation are of a VH3 family heavy chain variable region, the mutating comprises one or more substitutions selected from the group consisting of:

(i) glutamine (Q) at amino acid position 1 using AHo or Kabat numbering system;
(ii) glutamine (Q) at amino acid position 6 using AHo or Kabat numbering system;
(iii) threonine (T) or alanine (A) at amino acid position 7 using AHo or Kabat numbering system;
(iv) alanine (A), valine (V), or phenylalanine (F) at amino acid position 89 using AHo numbering system (amino acid position 78 using Kabat numbering system); and
(v) arginine (R), glutamine (Q), isoleucine (I), leucine (L), methionine (M) or phenylalanine (F) at amino acid position 103 using AHo numbering system (amino acid position 89 using Kabat numbering);

b) wherein if the one or more amino acid positions selected for mutation are of a VH1a family heavy chain variable region, the mutating comprises one or more substitutions selected from the group consisting of:

(i) glutamic acid (E) at amino acid position 1 using AHo or Kabat numbering system;
(ii) glutamic acid (E) at amino acid position 6 using AHo or Kabat numbering system;
(iii) leucine (L) at amino acid position 12 using AHo numbering system (amino acid position 11 using Kabat numbering system);
(iv) methionine (M) at amino acid position 13 using AHo numbering system (amino acid position 12 using Kabat numbering system):
(v) glutamic acid (E) or glutamine (Q) at amino acid position 14 using AHo numbering system (amino acid position 13 using Kabat numbering system);
(vi) leucine (L) at amino acid position 19 using AHo numbering system (amino acid position 18 using Kabat numbering system);
(vii) isoleucine (I) at amino acid position 21 using AHo numbering system (amino acid position 20 using Kabat numbering system);
(viii) phenylalanine (F), serine (S), histidine (H) or aspartic acid (D) at amino acid position 90 using AHo numbering system (amino acid position 79 using Kabat numbering system);
(ix) aspartic acid (D) or glutamine (Q) at amino acid position 92 using AHo numbering system (amino acid position 81 using Kabat numbering system);
(x) glycine (G), asparagine (N) or threonine (T) at amino acid position 95 using AHo numbering system (amino acid position 82b using Kabat numbering system); and
(xi) threonine (T), alanine (A), proline (P) or phenylalanine (F) at amino acid position 98 using AHo numbering (amino acid position 84 using Kabat numbering);

c) wherein if the one or more amino acid positions selected for mutation are of a VH1b family heavy chain variable region, the mutating comprises one or more substitutions selected from the group consisting of:

(i) glutamic acid (E) at amino acid position 1 using AHo or Kabat numbering system;
(ii) threonine (T), proline (P), valine (V) or aspartic acid (D) at amino acid position 10 using AHo numbering system (amino acid position 9 using Kabat numbering system);
(iii) leucine (L) at amino acid position 12 using AHo numbering system (amino acid position 11 using Kabat numbering system);
(iv) valine (V), arginine (R), glutamine (Q) or methionine (M) at amino acid position 13 using AHo numbering system (amino acid position 12 using Kabat numbering system):

(v) glutamic acid (E), arginine (R) or methionine (M) at amino acid position 14 using AHo numbering system (amino acid position 13 using Kabat numbering system);
(vi) arginine (R), threonine (T), or asparagine (N) at amino acid position 20 using AHo numbering system (amino acid position 19 using Kabat numbering system);
(vii) isoleucine (I), phenylalanine (F), or leucine (L) at amino acid position 21 using AHo numbering system (amino acid position 20 using Kabat numbering system);
(viii) lysine (K) at amino acid position 45 using AHo numbering system (amino acid position 38 using Kabat numbering system);
(ix) threonine (T), proline (P), valine (V) or arginine (R) at amino acid position 47 using AHo numbering system (amino acid position 40 using Kabat numbering system);
(x) lysine (K), histidine (H) or glutamic acid (E) at amino acid position 50 using AHo numbering system (amino acid position 43 using Kabat numbering system);
(xi) isoleucine (I) at amino acid position 55 using AHo numbering (amino acid position 48 using Kabat numbering);
(xii) lysine (K) at amino acid position 77 using AHo numbering (amino acid position 66 using Kabat numbering);
(xiii) alanine (A), leucine (L) or isoleucine (I) at amino acid position 78 using AHo numbering system (amino acid position 67 using Kabat numbering system);
(xiv) glutamic acid (E), threonine (T) or alanine (A) at amino acid position 82 using AHo numbering system (amino acid position 71 using Kabat numbering system);
(xv) threonine (T), serine (S) or leucine (L) at amino acid position 86 using AHo numbering system (amino acid position 75 using Kabat numbering system);
(xvi) aspartic acid (D), asparagine (N) or glycine (G) at amino acid position 87 using AHo numbering system (amino acid position 76 using Kabat numbering system); and
(xvii) asparagine (N) or serine (S) at amino acid position 107 using AHo numbering system (amino acid position 93 using Kabat numbering system);

d) wherein if the one or more amino acid positions selected for mutation are of a Vκ1 family light chain variable region, the mutating comprises one or more substitutions selected from the group consisting of:

(i) glutamic acid (E) or isoleucine (I) at amino acid position 1 using AHo or Kabat numbering system;
(ii) valine (V) or isoleucine (I) at amino acid position 3 using AHo or Kabat numbering system;
(iii) valine (V), leucine (L) or isoleucine (I) at amino acid position 4 using AHo or Kabat numbering system;
(iv) glutamine (Q) at amino acid position 24 using AHo or Kabat numbering system;
(v) arginine (R) or isoleucine (I) at amino acid position 47 using AHo numbering system (amino acid position 39 using Kabat numbering system);
(vi) lysine (K), glutamic acid (E) threonine (T), methionine (M) or glutamine (Q) at amino acid position 50 using AHo numbering system (amino acid position 42 using Kabat numbering system);
(vii) histidine (H), serine (S) or phenylalanine (F) at amino acid position 57 using AHo numbering system (amino acid position 49 using Kabat numbering system);
(viii) phenylalanine (F) at amino acid position 91 using AHo numbering system (amino acid position 73 using Kabat numbering system); and
(ix) valine (V), serine (S), glycine (G), isoleucine (I) at amino acid position 103 using AHo numbering system (amino acid position 85 using Kabat numbering system);

e) wherein if the one or more amino acid positions selected for mutation are of a Vκ3 family light chain variable region, the mutating comprises one or more substitutions selected from the group consisting of:

(i) threonine (T) at amino acid position 2 using AHo or Kabat numbering system;
(ii) threonine (T) at amino acid position 3 using AHo or Kabat numbering system;
(iii) isoleucine (I) at amino acid position 10 using AHo or Kabat numbering system;
(iv) tyrosine (Y) at amino acid position 12 using AHo or Kabat numbering system;
(v) serine (S) at amino acid position 18 using AHo or Kabat numbering system;
(vi) alanine (A) at amino acid position 20 using AHo or Kabat numbering system;
(vii) methionine (M) at amino acid position 56 using AHo numbering system (amino acid position 48 using Kabat numbering system);
(viii) valine (V) or threonine (T) at amino acid position 74 using AHo numbering system (amino acid position 58 using Kabat numbering system);

(ix) asparagine (N) at amino acid position 94 using AHo numbering system (amino acid position 76 using Kabat numbering system);
(x) tyrosine (Y) or serine (S) at amino acid position 101 using AHo numbering system (amino acid position 83 using Kabat numbering system); and
(xi) leucine (L) or alanine (A) at amino acid position 103 using AHo numbering (amino acid position 85 using Kabat numbering); and

f) wherein if the one or more amino acid positions selected for mutation are of a Vλ1 family light chain variable region, the mutating comprises one or more substitutions selected from the group consisting of:

(i) leucine (L), serine (S) or glutamic acid (E) at amino acid position 1 using AHo or Kabat numbering system;
(ii) alanine (A), proline (P), isoleucine (I) or tyrosine (Y) at amino acid position 2 using AHo or Kabat numbering system;
(iii) valine (V) or methionine (M) at amino acid position 4 using AHo or Kabat numbering system;
(iv) serine (S) or glutamic acid (E) at amino acid position 7 using AHo or Kabat numbering system;
(v) alanine (A) at amino acid position 11 using AHo or Kabat numbering system;
(vi) threonine (T) or serine (S) at amino acid position 14 using AHo or Kabat numbering system;
(vii) histidine (H) at amino acid position 46 using AHo numbering system (amino acid position 38 using Kabat numbering system);
(viii) threonine (T), serine (S), asparagine (N), glutamine (Q) or proline (P) at amino acid position 53 using AHo numbering system (amino acid position 45 using Kabat numbering system);
(ix) arginine (R) or glutamine (Q) at amino acid position 82 using AHo numbering system (amino acid position 66 using Kabat numbering system);
(x) glycine (G), threonine (T) or aspartic acid (D) at amino acid position 92 using AHo numbering system (amino acid position 74 using Kabat numbering system); and
(xi) valine (V), threonine (T), histidine (H) or glutamic acid (E) at amino acid position 103 using AHo numbering (amino acid position 85 using Kabat numbering).

In a preferred embodiment, the immunobinder is a scFv antibody. In other embodiments, the immunobinder is, for example, a full-length immunoglobulin, Dab, Nanobody or a Fab fragment.

The invention also encompasses immunobinders prepared according to the above-described method. Preferably, the immunobinder is a scFv antibody. In other embodiments, the immunobinder is, for example, a full-length immunoglobulin, Dab, Nanobody or a Fab fragment. The invention also encompasses pharmaceutical compositions comprising the aforementioned immunobinder(s) and a pharmaceutically acceptable carrier.

In yet another embodiment, the invention provides a method of engineering an immunobinder, such as a scFv antibody, in which one or more amino acid substitutions are made at one or more amino acid positions, wherein the amino acid residue that is used for substitution into the immunobinder is selected from the preferred amino acid residues identified in Tables 13-18 herein (*i.e.,* not including the consensus amino acid residue identified from analysis of the germline (IMGT and Vbase) and mature antibody (KDB) databases or the less enriched residues from the selected scFv database). That is, the substitutions are selected only from those amino acid residues that exhibit the greatest enrichment in the selected scFv database (QC). Thus, in this embodiment, the invention provides a method of engineering an immunobinder, the immunobinder comprising (i) a heavy chain variable region, or fragment thereof, of a VH3, VH1a or VH1b family, the heavy chain variable region comprising $V_H$ framework residues or (ii) a light chain variable region, or fragment thereof, of a Vκ1, Vκ3 or Vλ1 family, the light chain variable region comprising $V_L$ framework residues, the method comprising:

A) selecting one or more amino acid positions within the $V_H$ framework residues, the $V_L$ framework residues or the $V_H$ and $V_L$ framework residues for mutation; and
B) mutating the one or more amino acid positions selected for mutation,

a) wherein if the one or more amino acid positions selected for mutation are of a VH3 family heavy chain variable region, the mutating comprises one or more substitutions selected from the group consisting of:

(i) glutamine (Q) at amino acid position 1 using AHo or Kabat numbering system;
(ii) glutamine (Q) at amino acid position 6 using AHo or Kabat numbering system;
(iii) threonine (T) at amino acid position 7 using AHo or Kabat numbering system;
(iv) valine (V) at amino acid position 89 using AHo numbering system (amino acid position 78 using Kabat numbering system); and

(v) leucine (L) at amino acid position 103 using AHo numbering system (amino acid position 89 using Kabat numbering);

b) wherein if the one or more amino acid positions selected for mutation are of a VH1a family heavy chain variable region, the mutating comprises one or more substitutions selected from the group consisting of:

(i) glutamic acid (E) at amino acid position 1 using AHo or Kabat numbering system;
(ii) glutamic acid (E) at amino acid position 6 using AHo or Kabat numbering system;
(iii) leucine (L) at amino acid position 12 using AHo numbering system (amino acid position 11 using Kabat numbering system);
(iv) methionine (M) at amino acid position 13 using AHo numbering system (amino acid position 12 using Kabat numbering system):
(v) glutamic acid (E) at amino acid position 14 using AHo numbering system (amino acid position 13 using Kabat numbering system);
(vi) leucine (L) at amino acid position 19 using AHo numbering system (amino acid position 18 using Kabat numbering system);
(vii) isoleucine (I) at amino acid position 21 using AHo numbering system (amino acid position 20 using Kabat numbering system);
(viii) phenylalanine (F), serine (S), histidine (H) or aspartic acid (D) at amino acid position 90 using AHo numbering system (amino acid position 79 using Kabat numbering system);
(ix) aspartic acid (D) at amino acid position 92 using AHo numbering system (amino acid position 81 using Kabat numbering system);
(x) glycine (G) at amino acid position 95 using AHo numbering system (amino acid position 82b using Kabat numbering system); and
(xi) phenylalanine (F) at amino acid position 98 using AHo numbering (amino acid position 84 using Kabat numbering);

c) wherein if the one or more amino acid positions selected for mutation are of a VH1b family heavy chain variable region, the mutating comprises one or more substitutions selected from the group consisting of:

(i) glutamic acid (E) at amino acid position 1 using AHo or Kabat numbering system;
(ii) threonine (T), proline (P), valine (V) or aspartic acid (D) at amino acid position 10 using AHo numbering system (amino acid position 9 using Kabat numbering system);
(iii) leucine (L) at amino acid position 12 using AHo numbering system (amino acid position 11 using Kabat numbering system);
(iv) valine (V), arginine (R), glutamine (Q) or methionine (M) at amino acid position 13 using AHo numbering system (amino acid position 12 using Kabat numbering system):
(v) arginine (R) at amino acid position 14 using AHo numbering system (amino acid position 13 using Kabat numbering system);
(vi) asparagine (N) at amino acid position 20 using AHo numbering system (amino acid position 19 using Kabat numbering system);
(vii) leucine (L) at amino acid position 21 using AHo numbering system (amino acid position 20 using Kabat numbering system);
(viii) lysine (K) at amino acid position 45 using AHo numbering system (amino acid position 38 using Kabat numbering system);
(ix) arginine (R) at amino acid position 47 using AHo numbering system (amino acid position 40 using Kabat numbering system);
(x) lysine (K) at amino acid position 50 using AHo numbering system (amino acid position 43 using Kabat numbering system);
(xi) isoleucine (I) at amino acid position 55 using AHo numbering (amino acid position 48 using Kabat numbering);
(xii) lysine (K) at amino acid position 77 using AHo numbering (amino acid position 66 using Kabat numbering);
(xiii) alanine (A) at amino acid position 78 using AHo numbering system (amino acid position 67 using Kabat numbering system);
(xiv) glutamic acid (E) at amino acid position 82 using AHo numbering system (amino acid position 71 using Kabat numbering system);
(xv) threonine (T) at amino acid position 86 using AHo numbering system (amino acid position 75 using Kabat numbering system);
(xvi) asparagine (N) at amino acid position 87 using AHo numbering system (amino acid position 76 using Kabat numbering system); and
(xvii) asparagine (N) at amino acid position 107 using AHo numbering system (amino acid position 93 using Kabat numbering system);

d) wherein if the one or more amino acid positions selected for mutation are of a Vκ1 family light chain variable region, the mutating comprises one or more substitutions selected from the group consisting of:

(i) glutamic acid (E) at amino acid position 1 using AHo or Kabat numbering system;
(ii) valine (V) at amino acid position 3 using AHo or Kabat numbering system;
(iii) leucine (L) at amino acid position 4 using AHo or Kabat numbering system;
(iv) glutamine (Q) at amino acid position 24 using AHo or Kabat numbering system;
(v) arginine (R) at amino acid position 47 using AHo numbering system (amino acid position 39 using Kabat numbering system);
(vi) lysine (K), glutamic acid (E) threonine (T), methionine (M) or glutamine (Q) at amino acid position 50 using AHo numbering system (amino acid position 42 using Kabat numbering system);
(vii) serine (S) at amino acid position 57 using AHo numbering system (amino acid position 49 using Kabat numbering system);
(viii) phenylalanine (F) at amino acid position 91 using AHo numbering system (amino acid position 73 using Kabat numbering system); and
(ix) valine (V) at amino acid position 103 using AHo numbering system (amino acid position 85 using Kabat numbering system);

e) wherein if the one or more amino acid positions selected for mutation are of a Vκ3 family light chain variable region, the mutating comprises one or more substitutions selected from the group consisting of:

(i) threonine (T) at amino acid position 2 using AHo or Kabat numbering system;
(ii) threonine (T) at amino acid position 3 using AHo or Kabat numbering system;
(iii) isoleucine (I) at amino acid position 10 using AHo or Kabat numbering system;
(iv) tyrosine (Y) at amino acid position 12 using AHo or Kabat numbering system;
(v) serine (S) at amino acid position 18 using AHo or Kabat numbering system;
(vi) alanine (A) at amino acid position 20 using AHo or Kabat numbering system;
(vii) methionine (M) at amino acid position 56 using AHo numbering system (amino acid position 48 using Kabat numbering system);
(viii) threonine (T) at amino acid position 74 using AHo numbering system (amino acid position 58 using Kabat numbering system);
(ix) asparagine (N) at amino acid position 94 using AHo numbering system (amino acid position 76 using Kabat numbering system);
(x) serine (S) at amino acid position 101 using AHo numbering system (amino acid position 83 using Kabat numbering system); and
(xi) alanine (A) at amino acid position 103 using AHo numbering (amino acid position 85 using Kabat numbering); and

f) wherein if the one or more amino acid positions selected for mutation are of a Vλ1 family light chain variable region, the mutating comprises one or more substitutions selected from the group consisting of:

(i) leucine (L) at amino acid position 1 using AHo or Kabat numbering system;
(ii) proline (P) at amino acid position 2 using AHo or Kabat numbering system;
(iii) valine (V) at amino acid position 4 using AHo or Kabat numbering system;
(iv) serine (S) at amino acid position 7 using AHo or Kabat numbering system;
(v) alanine (A) at amino acid position 11 using AHo or Kabat numbering system;
(vi) threonine (T) at amino acid position 14 using AHo or Kabat numbering system;
(vii) histidine (H) at amino acid position 46 using AHo numbering system (amino acid position 38 using Kabat numbering system);
(viii) threonine (T), serine (S), asparagine (N), glutamine (Q) or proline (P) at amino acid position 53 using AHo numbering system (amino acid position 45 using Kabat numbering system);
(ix) arginine (R) at amino acid position 82 using AHo numbering system (amino acid position 66 using Kabat numbering system);
(x) threonine (T) at amino acid position 92 using AHo numbering system (amino acid position 74 using Kabat numbering system); and
(xi) valine (V) at amino acid position 103 using AHo numbering (amino acid position 85 using Kabat numbering).

In a preferred embodiment, the immunobinder is a scFv antibody. In other embodiments, the immunobinder is, for example, a full-length immunoglobulin, Dab, Nanobody or a Fab fragment.

The invention also encompasses immunobinders prepared according to the above-described method. Preferably, the immunobinder is a scFv antibody. In other embodiments, the immunobinder is, for example, a full-length immunoglobulin, Dab, Nanobody or a Fab fragment. The invention also encompasses pharmaceutical compositions comprising the afore-mentioned immunobinder(s) and a pharmaceutically acceptable carrier.

While the various engineering methods set forth above in this subsection provide a listing of all the exemplary and preferred substitutions as defined in Tables 13-18 herein for the VH3, VH1a, VH1b, Vκ1, Vκ3 and Vλ1 families, respectively, it should be understood that the invention encompasses methods in which only one or a few amino acid substitutions are made in one variable region selected from VH3, VH1a, VH1b, Vκ1, Vκ3 and Vλ1, as well as methods in which one, a few or many amino acid substitutions are made in one or more variable regions selected from a VH3, VH1a, VH1b, Vκ1, Vκ3 or Vλ1 family, such as in one heavy chain variable region selected from a VH3, VH1a or VH1b family and one light chain variable region selected from a Vκ1, Vκ3 or Vλ1 family in an immunobinder comprising one heavy and one light chain variable region (*e.g.*, a scFv). That is, any and all possible combinations of substitutions selected from the exemplary and preferred substitutions as defined in Tables 13-18 are intended to be encompassed by the engineering methods, and the resultant immunobinders made according to those methods.

For example, in various embodiments, the method comprises making one, two, three, four, five, six, seven, eight, nine, ten or more than ten of the specified amino acid substitions in a heavy chain variable region selected from a VH3, VH1a or VH1b family variable region. In other various embodiments, the method comprises making one, two, three, four, five, six, seven, eight, nine, ten or more than ten of the specified amino acid substitions in a light chain variable region selected from a Vκ1, Vκ3 or Vλ1 family variable region.

Nothwithstanding the foregoing, in various embodiments, certain immunobinders are excluded from being used in the engineering methods of the invention and/or are excluded from being the immunobinder composition produced by the engineering methods. For example, in various embodiments, there is a proviso that the immunobinder is not any of the scFv antibodies, or variants thereof, as disclosed in PCT Publications WO 2006/131013 and WO 2008/006235, such as ESBA105 or variants thereof that are disclosed in PCT Publications WO 2006/131013 and WO 2008/006235, the contents of each of which is expressly incorporated herein by reference.

In various other embodiments, if the immunobinder to be engineered according to the above-described methods is any of the scFv antibodies, or variants thereof, disclosed in PCT publications WO 2006/131013 or WO 2008/006235, then there can be the proviso that the list of possible amino acid positions that may be selected for substitution according to the engineering method does not include any or all of the following amino acid positions: AHo position 4 (Kabat 4) of Vκ1 or Vλ1; AHo position 101 (Kabat 83) of Vκ3; AHo position 12 (Kabat 11) of VH1a or VH1b; AHo position 50 (Kabat 43) of VH1b; AHo position 77 (Kabat 66) for VH1b; AHo position 78 (Kabat 67) for VH1b; AHo position 82 (Kabat 71) for VH1b; AHo position 86 (Kabat 75) for VH1b; AHo position 87 (Kabat 76) for VH1b; AHo position 89 (Kabat 78) for VH3; AHo position 90 (Kabat 79) for VH1a; and/or AHo position 107 (Kabat 93) for VH1b.

In still various other embodiments, for any immunobinder to be engineered according to the above-described methods, and/or any immunobinder produced according to the above-described methods, there can be the proviso that the list of possible amino acid positions that may be selected for substitution according to the engineering method does not include any or all of the following amino acid positions: AHo position 4 (Kabat 4) of Vκ1 or Vλ1; AHo position 101 (Kabat 83) of Vκ3; AHo position 12 (Kabat 11) of VH1a or VH1b; AHo position 50 (Kabat 43) of VH1b; AHo position 77 (Kabat 66) for VH1b; AHo position 78 (Kabat 67) for VH1b; AHo position 82 (Kabat 71) for VH1b; AHo position 86 (Kabat 75) for VH1b; AHo position 87 (Kabat 76) for VH1b; AHo position 89 (Kabat 78) for VH3; AHo position 90 (Kabat 79) for VH1a; and/or AHo position 107 (Kabat 93) for VH1b.

Framework Scaffolds

As described in detail in Example 8, the functional consensus approach described herein has been used successfully to design framework scaffold sequences that incorporate the exemplary and preferred amino acid substitutions identified for particular amino acid positions with variable region families. In these scaffolds, the CDR regions are not specified; rather, such scaffold sequences can be used as "templates" into which CDR sequences (CDRL1, CDRL2, CDRL3, CDRH1, CDRH2, and/or CDRH3) can be inserted to create variable regions likely to exhibit desirable stability and/or solubility properties due to the exemplary or preferred amino acid substitutions incorporated into the scaffold, based on the selected scFv sequences (selected based on their desirable stability and/or solubility properties). For example, a heavy chain framework scaffold sequence for the VH1a family is set forth in Figure 9 (SEQ ID NO:1), a heavy chain framework scaffold sequence for the VH1b family is set forth in Figure 10 (SEQ ID NO:2) a heavy chain framework scaffold sequence for the VH3 family is set forth in Figure 11 (SEQ ID NO:3), a light chain framework scaffold sequence for the Vk1 family is set forth in Figure 12 (SEQ ID NO:4), a light chain framework scaffold sequence for the Vk3 family is set forth in Figure 13 (SEQ ID NO:5) and a light chain framework scaffold sequence for the Vλ1 family is set forth in Figure 14 (SEQ ID NO:6).

Accordingly, in another aspect, the invention provides a method of engineering an immunobinder, the immunobinder comprising heavy chain CDR1, CDR2 and CDR3 sequences, the method comprising inserting the heavy chain CDR1, CDR2 and CDR3 sequences into a heavy chain framework scaffold, the heavy chain framework scaffold comprising an amino acid sequence as shown in Figure 9 (SEQ ID NO:1), Figure 10 (SEQ ID NO:2) or Figure 11 (SEQ ID NO:3). In one embodiment, the heavy chain framework scaffold comprises an amino acid sequence as shown in Figure 9 (SEQ ID NO:1). In another embodiment, the heavy chain framework scaffold comprises an amino acid sequence as shown in Figure 10 (SEQ ID NO:2). In yet another embodiment, the heavy chain framework scaffold comprises an amino acid sequence as shown in Figure 11 (SEQ ID NO:3).

Additionally or alternatively, the invention provides a method of engineering an immunobinder, the immunobinder comprising light chain CDR1, CDR2 and CDR3 sequences, the method comprising inserting the light chain CDR1, CDR2 and CDR3 sequences into a light chain framework scaffold, the light chain framework scaffold comprising an amino acid sequence as shown in Figure 12 (SEQ ID NO:4), Figure 13 (SEQ ID NO:5) or Figure 14 (SEQ ID NO:6). In one embodiment, the light chain framework scaffold comprises an amino acid sequence as shown in Figure 12 (SEQ ID NO:4). In another embodiment, the light chain framework scaffold comprises an amino acid sequence as shown in Figure 13 (SEQ ID NO:5). In yet another embodiment, the light chain framework scaffold comprises an amino acid sequence as shown in Figure 14 (SEQ ID NO:6).

Preferably, the immunobinder engineered according to the method is a scFv antibody, athough other immunobinders, such as full-length immunoglobulins and Fab fragments, also can be engineered according to the method. In certain exemplary embodiments, one or more of the CDRs (e.g., CDRL1, CDRL2, CDRL3, CDRH1, CDRH2, and/or CDRH3) are derived from any of the immunobinders of therapeutic importance discussed *supra.* The CDRs can be inserted into the framework scaffolds using standard molecular biology techniques.

The invention also encompasses immunobinders engineered according to the above-described method using framework scaffolds. Preferably, the immunobinder is a scFv antibody, athough other immunobinders, such as full-length immunoglobulins, Dabs, Nanobodies and Fab fragments, are also encompassed. Pharmaceutical compositions, comprising such immunobinders and a pharmaceutically acceptable carrier are also encompassed.

In yet another aspect, the invention provides an isolated heavy chain framework scaffolds comprising an amino acid sequence as shown in Figure 9, Figure 10 or Figure 11. Such heavy chain framework scaffolds can be prepared using standard molecular biology techniques.

Nothwithstanding the foregoing, in various embodiments, certain framework scaffold sequences may be excluded from being used in the scaffold-based engineering methods of the invention and/or are excluded from being the immunobinder composition produced by the scaffold-engineering methods. For example, in various embodiments, there is a proviso that the sequence of the framework scaffold is not any of the scFv framework sequences as disclosed in PCT Publication WO 2001/048017, PCT Publication WO 2003/097697, US Patent Publication No. 20010024831 and/or US Patent Publication US 20030096306, the contents of each of which is expressly incorporated herein by reference.

In various other embodiments of the above-described scaffold-based engineering methods, or immunobinders resulting therefrom, there can be the proviso that certain amino acid positions shown in Figures 9, 10 or 11 as being variable (*i.e.,* shown as "X", with the list of possible amino acid residues for that position listed below the "X") may be constrained from being variable. For example, in certain embodiments, there is the proviso that any or all of the following amino acid positions may be limited to only the amino acid residue that is listed first below the "X", or listed second below the "X", or (when present) listed third below the "X", or (when present) listed fourth below the "X" or (when present) listed fifth below the "X" or (when present) listed sixth below the "X": AHo position 12 (Kabat 11) of VH1a or VH1b; AHo position 50 (Kabat 43) of VH1b; AHo position 77 (Kabat 66) for VH1b; AHo position 78 (Kabat 67) for VH1b; AHo position 82 (Kabat 71) for VH1b; AHo position 86 (Kabat 75) for VH1b; AHo position 87 (Kabat 76) for VH1b; AHo position 89 (Kabat 78) for VH3; AHo position 90 (Kabat 79) for VH1a; and/or AHo position 107 (Kabat 93) for VH1b.

## Other Embodiments

It is understood that the invention also includes any of the methodologies, references, and/or compositions set forth in Appendices (A-C) of US Provisional Patent Application Serial No. 60/905,365 and Appendices (A-I) of US Provisional Patent Application Serial No. 60/937,112, including, but not limited to, identified databases, bioinformatics, in silico data manipulation and interpretation methods, functional assays, preferred sequences, preferred residue(s) positions / alterations, framework identification and selection, framework alterations, CDR alignment and integration, and preferred alterations/mutations.

Additional information regarding these methodologies and compositions can be found in U.S.S.N.s 60/819,378; and 60/899,907, and PCT Publication WO 2008/006235, entitled "scFv Antibodies Which Pass Epithelial And/Or Endothelial Layers" filed in July, 2006 and February 6, 2007 respectively; WO06131013A2 entitled "Stable And Soluble Antibodies Inhibiting TNFα" filed June 6, 2006; EP1506236A2 entitled "Immunoglobulin Frameworks Which Demonstrate Enhanced Stability In The Intracellular Environment And Methods Of Identifying Same" filed May 21,2003; EP1479694A2 entitled "Intrabodies ScFv with defined framework that is stable in a reducing environment" filed December 18,2000; EP1242457B1 entitled "Intrabodies With Defined Framework That Is Stable In A Reducing Environment And Applications Thereof" filed December 18, 2000; WO03097697A2 entitled "Immunoglobulin Frameworks Which Demonstrate Enhanced Stability In The Intracellular Environment And Methods Of Identifying Same" filed May 21, 2003; and WO0148017 entitled "Intrabodies With Defined Framework That Is Stable In A Reducing Environment And Applications Thereof" filed December 18, 2000; and Honegger et al., J. Mol. Biol. 309:657-670 (2001).

Further, it is understood that the invention also includes methodologies and compositions suitable for the discovery and/or improvement of other antibody formats, *e.g.*, full length antibodies or fragments thereof, for example Fabs, Dabs, and the like. Accordingly, the principles and residues identified herein as suitable for selection or alteration to achieve desired biophysical and/or therapeutic proprieties that can be applied to a wide range of immunobinders. In one embodiment, therapeutically relevant antibodies, for example, FDA-approved antibodies, are improved by modifying one or more residue positions as disclosed herein.

The invention is not limited to the engineering of immunobinders, however. For example, one skilled in the art will recognize that the methods of the invention can be applied to the engineering of other, non-immunoglobulin, binding molecules, including, but not limited to, fibronectin binding molecules such as Adnectins (see WO 01/64942 and US Patent Nos. 6,673,901, 6,703,199, 7,078,490, and 7,119,171), Affibodies (see *e.g.,* US Patents 6,740,734 and 6,602,977 and in WO 00/63243), Anticalins (also known as lipocalins) (see WO99/16873 and WO 05/019254), A domain proteins (see WO 02/088171 and WO 04/044011) and ankyrin repeat proteins such as Darpins or leucine-repeat proteins (see WO 02/20565 and WO 06/083275).

The present disclosure is further illustrated by the following examples, which should not be construed as further limiting. The contents of all figures and all references, patents and published patent applications cited throughout this application are expressly incorporated herein by reference in their entireties.

**EXAMPLE 1: Antibody Position Numbering Systems**

In this example, conversion tables are provided for two different numbering systems used to identify amino acid residue positions in antibody heavy and light chain variable regions. The Kabat numbering system is described further in Kabat *et al.* (Kabat, E. A., et al. (1991) Sequences of Proteins of Immunological Interest, Fifth Edition, U.S. Department of Health and Human Services, NIH Publication No. 91-3242). The AHo numbering system is described further in Honegger, A. and Plückthun, A. (2001) J. Mol. Biol 309:657-670).

Heavy Chain Variable Region Numbering

Table 1: Conversion table for the residue positions in the Heavy Chain Variable Domain

| Kabat | AHo | Kabat | AHo | Kabat | AHo |
|---|---|---|---|---|---|
| 1 | 1 | 44 | 51 | 87 | 101 |
| 2 | 2 | 45 | 52 | 88 | 102 |
| 3 | 3 | 46 | 53 | 89 | 103 |
| 4 | 4 | 47 | 54 | 90 | 104 |
| 5 | 5 | 48 | 55 | 91 | 105 |
| 6 | 6 | 49 | 56 | 92 | 106 |
| 7 | 7 | 50 | 57 | 93 | 107 |
| * | 8 | 51 | 58 | 94 | 108 |
| 8 | 9 | 52 | 59 | 95 | 109 |
| 9 | 10 | 52a | 60 | 96 | 110 |
| 10 | 11 | 52b | 61 | 97 | 111 |
| 11 | 12 | 52c | 62 | 98 | 112 |

(continued)

| Kabat | AHo | Kabat | AHo | Kabat | AHo |
|---|---|---|---|---|---|
| 12 | 13 | * | 63 | 99 | 113 |
| 13 | 14 | 53 | 64 | 100 | 114 |
| 14 | 15 | 54 | 65 | 100a | 115 |
| 15 | 16 | 55 | 66 | 100b | 116 |
| 16 | 17 | 56 | 67 | 100c | 117 |
| 17 | 18 | 57 | 68 | 100d | 118 |
| 18 | 19 | 58 | 69 | 100e | 119 |
| 19 | 20 | 59 | 70 | 100f | 120 |
| 20 | 21 | 60 | 71 | 100g | 121 |
| 21 | 22 | 61 | 72 | 100h | 122 |
| 22 | 23 | 62 | 73 | 100i | 123 |
| 23 | 24 | 63 | 74 | * | 124 |
| 24 | 25 | 64 | 75 | * | 125 |
| 25 | 26 | 65 | 76 | * | 126 |
| 26 | 27 | 66 | 77 | * | 127 |
| * | 28 | 67 | 78 | * | 128 |
| 27 | 29 | 68 | 79 | * | 129 |
| 28 | 30 | 69 | 80 | * | 130 |
| 29 | 31 | 70 | 81 | * | 131 |
| 30 | 32 | 71 | 82 | * | 132 |
| 31 | 33 | 72 | 83 | * | 133 |
| 32 | 34 | 73 | 84 | * | 134 |
| 33 | 35 | 74 | 85 | * | 135 |
| 34 | 36 | 75 | 86 | * | 136 |
| 35 | 37 | 76 | 87 | 101 | 137 |
| 35a | 38 | 77 | 88 | 102 | 138 |
| 35b | 39 | 78 | 89 | 103 | 139 |
| * | 40 | 79 | 90 | 104 | 140 |
| * | 41 | 80 | 91 | 105 | 141 |
| * | 42 | 81 | 92 | 106 | 142 |
| 36 | 43 | 82 | 93 | 107 | 143 |
| 37 | 44 | 82a | 94 | 108 | 144 |
| 38 | 45 | 82b | 95 | 109 | 145 |
| 39 | 46 | 82b | 96 | 110 | 146 |
| 40 | 47 | 83 | 97 | 111 | 147 |
| 41 | 48 | 84 | 98 | 112 | 148 |
| 42 | 49 | 85 | 99 | 113 | 149 |
| 43 | 50 | 86 | 100 | | |
| Column 1, Residue position in Kabat's numbering system. Column 2, Corresponding number in AHo's numbering system for the position indicated in column 1. Column 3, Residue position in Kabat's numbering system. Column 4, Corresponding number in AHo's numbering system for the position indicated in column 3. Column 5, Residue position in Kabat's numbering system. Column 6, Corresponding number in AHo's numbering system for the position indicated in column 5 | | | | | |

Light Chain Variable Region Numbering

Table 2: Conversion table for the residue positions in the Light Chain Variable Domain

| Kabat | AHo | Kabat | AHo | Kabat | AHo |
|---|---|---|---|---|---|
| 1 | 1 | 43 | 51 | 83 | 101 |
| 2 | 2 | 44 | 52 | 84 | 102 |
| 3 | 3 | 45 | 53 | 85 | 103 |
| 4 | 4 | 46 | 54 | 86 | 104 |
| 5 | 5 | 47 | 55 | 87 | 105 |
| 6 | 6 | 48 | 56 | 88 | 106 |
| 7 | 7 | 49 | 57 | 89 | 107 |
| 8 | 8 | 50 | 58 | 90 | 108 |
| 9 | 9 | * | 59 | 91 | 109 |
| 10 | 10 | * | 60 | 92 | 110 |
| 11 | 11 | * | 61 | 93 | 111 |
| 12 | 12 | * | 62 | 94 | 112 |
| 13 | 13 | * | 63 | 95 | 113 |
| 14 | 14 | * | 64 | 95a | 114 |
| 15 | 15 | * | 65 | 95b | 115 |
| 16 | 16 | * | 66 | 95c | 116 |
| 17 | 17 | 51 | 67 | 95d | 117 |
| 18 | 18 | 52 | 68 | 95e | 118 |
| 19 | 19 | 53 | 69 | 95f | 119 |
| 20 | 20 | 54 | 70 | * | 120 |
| 21 | 21 | 55 | 71 | * | 121 |
| 22 | 22 | 56 | 72 | * | 122 |
| 23 | 23 | 57 | 73 | * | 123 |
| 24 | 24 | 58 | 74 | * | 124 |
| 25 | 25 | 59 | 75 | * | 125 |
| 26 | 26 | 60 | 76 | * | 126 |
| 27 | 27 | 61 | 77 | * | 127 |
| * | 28 | 62 | 78 | * | 128 |
| 27a | 29 | 63 | 79 | * | 129 |
| 27b | 30 | 64 | 80 | * | 130 |
| 27c | 31 | 65 | 81 | * | 131 |
| 27d | 32 | 66 | 82 | * | 132 |
| 27e | 33 | 67 | 83 | * | 133 |
| 27f | 34 | 68 | 84 | * | 134 |
| * | 35 | * | 85 | * | 135 |
| 28 | 36 | * | 86 | * | 136 |
| 29 | 37 | 69 | 87 | 96 | 137 |
| 30 | 38 | 70 | 88 | 97 | 138 |
| 31 | 39 | 71 | 89 | 98 | 139 |
| 32 | 40 | 72 | 90 | 99 | 140 |
| 33 | 41 | 73 | 91 | 100 | 141 |
| 34 | 42 | 74 | 92 | 101 | 142 |
| 35 | 43 | 75 | 93 | 102 | 143 |
| 36 | 44 | 76 | 94 | 103 | 144 |
| 37 | 45 | 77 | 95 | 104 | 145 |
| 38 | 46 | 78 | 96 | 105 | 146 |
| 39 | 47 | 79 | 97 | 106 | 147 |
| 40 | 48 | 80 | 98 | 107 | 148 |
| 41 | 49 | 81 | 99 | 108 | 149 |

(continued)

| Kabat | AHo | Kabat | AHo | Kabat | AHo |
|---|---|---|---|---|---|
| 42 | 50 | 82 | 100 | | |
| Column 1, Residue position in Kabat's numbering system. Column 2, Corresponding number in AHo's numbering system for the position indicated in column 1. Column 3, Residue position in Kabat's numbering system. Column 4, Corresponding number in AHo's numbering system for the position indicated in column 3. Column 5, Residue position in Kabat's numbering system. Column 6, Corresponding number in AHo's numbering system for the position indicated in column 5 | | | | | |

**EXAMPLE 2: Sequence-Based Analysis of scFv Sequences**

In this example, the sequence-based analysis of scFv sequences is described in detail. A flowchart summarizing the process of the analysis is shown in Figure 1.

Collection and Alignment of Human Immunoglobulin Sequences

Sequences of variable domains of human mature antibodies and germlines were collected from different databases and entered into a customized database as one letter code amino acid sequences. The antibody sequences were aligned using an EXCEL implementation of the Needleman-Wunsch sequence alignment algorithm (Needleman et al., J Mol Biol., 48(3):443-53 (1970)). The database was then sub-divided into four different arrays (according to the original data source) to facilitate the subsequent analysis and comparison, as follows:

VBase: Human germline sequences
IMGT: Human germline sequences
KDB database: Mature antibodies
QC database: ESBATech's internal database comprising selected scFv _______frameworks selected by Quality Control screening

The QC screening system, and scFv framework sequences having desirable functional properties selected therefrom, are described further in , for example, PCT Publication WO 2001/48017; U.S. Application No. 20010024831; US 20030096306; US Patent Nos. 7,258,985 and 7,258,986; PCT Publication WO 2003/097697 and U.S. Application No. 20060035320.

The introduction of gaps and the nomenclature of residue positions were done following AHo's numbering system for immunoglobulin variable domain (Honegger, A. and Plückthun, A. (2001) J. Mol. Biol. 309:657-670). Subsequently, framework regions and CDRs regions were identified according to Kabat *et al.* (Kabat, E. A., et al. (1991) Sequences of Proteins of Immunological Interest, Fifth Edition, U.S. Department of Health and Human Services, NIH Publication No. 91-3242). Sequences in the KDB database less than 70% complete or containing multiple undetermined residues in the framework regions were discarded. Sequences with more than 95% identity to any other sequence within the database were also excluded to avoid random noise in the analysis.

Assignment of Sequences to Subgroups

The antibody sequences were classified into distinct families by clustering the antibodies according to classification methods based on sequence homology (Tomlinson, I.M. et al. (1992) J. Mol. Biol. 227:776-798; Williams, S.C. and Winter, G. (1993) Eur. J. Immunol. 23:1456-1461); Cox, J.P. et al. (1994) Eur. J. Immunol. 24:827-836). The percentage of homology to the family consensus was constrained to 70% similarity. In cases where sequences showed conflicts between two or more different germline families, or the percentage of homology was below 70% (to any family), the nearest germline counterpart was determined, CDRs length, canonical classes and defining subtype residues were analyzed in detail to correctly assign the family.

Statistical Analysis

Once the family clusters were defined, statistical analysis were performed for hits identified in the "Quality Control ("QC") screening" (such QC screening is described in detail in PCT Publication WO 2003/097697). Analyses were only possible for the most represented families (VH3, VH1a, VH1b, Vk1, Vk3 and Vλ1) since a minimum number of sequences are needed for the analysis. The residue frequencies, fi(r), for each position, i, was calculated by the number of times that particular residue-type was observed within the data set divided by the total number of sequences. The positional entropy, N(i), was calculated as a measure of every residue position's variability (Shenkin, P.S. et al. (1991) Proteins 11:297-313; Larson, S.M. and Davidson, A.R. (2000) Protein Sci. 9:2170-2180; Demarest, S.J. et al. (2004) J. Mol. Biol. 335:41-48) using the Simpson's index which is a mathematical measure of diversity in a system providing more information about amino acids composition than simply richness. The degree of diversity for each position, i, was calculated taking into account the number of different amino acids present, as well as the relative abundance of each residue.

$$D = \frac{\sum_{i=1}^{r} n(n-1)}{N(N-1)}$$

Where: D is the Simpson's Index, N is the total number of amino acids, r is the number of different amino acids present at each position and n is the number of residues of a particular amino acid type.

The QC database of the selected Fv frameworks (selected by the QC screening) was screened using different criteria to define the unique features. The different arrays in the sequence database were used to define the degree of variability of residue positions within the Fv frameworks and to identify variation-tolerant positions not common in nature which are present in the selected Fv frameworks. A difference in the positional entropy scores equal or more than 10% was defined as a threshold. Additional positions were selected if the residue at a given position was occupied by an amino acid infrequently observed in the other sequence arrays, *i.e.,* infrequently observed in the germlines databases (VBase and IMGT) and the KDB database. If the behavior of a residue was found to be truly different, (low or none represented in any of the other sequence arrays), the residue position was defined as unique.

The rationale behind the identification of unique features of the selected Fv framework sequences is the proven superior properties of the frameworks and the potential use of these findings for improved scaffolding. We assumed that highly conserved positions in nature showing a certain degree of variability in the selected frameworks should tolerate random mutagenesis and present an increased probability of finding alternative amino acids superior to the native residue in a scFv format. In addition a pronounced preference for an uncommon amino acid is an indication of natural selection toward certain residue. Based on these two statistical guidelines different residues within the heavy and light chains were chosen as either floating positions (variability-tolerant) or preferred substitutions (unusual residues).

**EXAMPLE 3: Identification of Variability-Tolerant and Unusual Residue Positions**

Using the sequence-based scFv analysis approach described above in Example 2, three heavy chain variable region families (VH3, VH1a and VH1b) and three light chain variable region families (Vκ1, Vκ3 and Vλ1) were analyzed to identify variability-tolerant amino acid positions. In particular, the degree of diversity, as calculated using the Simpson's Index, was determined for each amino acid position for sequences within four different databases, Vbase, IMGT, KDB and QC (selected scFvs), as described above. Variant-tolerant and unusual residue amino acid positions were identified based on differences in the Simpson's Index values at those positions for the Vbase and IMGT germline databases as compared to the QC selected scFv database. Additionally, for the identified positions of interest, the germline consensus residue was identified and the frequency of that consensus residue in the QC and KDB databases was determined.

The variability analysis results for the heavy chain variable region familes VH3, VH1a and VH1b are shown below in Tables 3, 4 and 5, respectively. For each table, the columns are as follows: column 1: amino acid residue position using the AHo numbering system (conversion to the Kabat numbering system can be accomplished using the conversion table set forth as Table 1 in Example 1); columns 2 to 5: calculated diversity for each antibody array in the database for the residue position indicated in column 1; column 6: consensus residue of the corresponding germline family and KDB; column 7: relative residue frequency in the KDB database for the consensus residue in column 6; and column 8: relative residue frequency in the QC selected scFv database for the consensus residue in column 6.

Table 3: Variability analysis of residues and corresponding frequencies of the consensus amino acid identified in the germline for the VH3 family.

| *Residue position* | *IMGT germline* | *VBase germline* | QC *selected scFv* | *KDBseq* | *Consensus residue* | *f(cons KDB)* | *f (cons QC)* |
|---|---|---|---|---|---|---|---|
| 1 | 0.68 | 0.65 | 0.50 | 0.53 | E | 66.67 | 53.57 |
| 6 | 1.00 | 1.00 | 0.57 | 0.86 | E | 92.56 | 68.97 |
| 7 | 1.00 | 0.91 | 0.65 | 0.93 | S | 96.33 | 77.59 |
| 89 | 0.86 | 0.83 | 0.55 | 0.71 | L | 84.06 | 70.18 |

(continued)

| Residue position | IMGT germline | VBase germline | QC selected scFv | KDBseq | Consensus residue | f(cons KDB) | f (cons QC) |
|---|---|---|---|---|---|---|---|
| 103 | 0.73 | 0.76 | 0.38 | 0.76 | V | 86.85 | 55.36 |

Table 4: Variability analysis of residues and corresponding frequencies of the consensus amino acid identified in the germline for the VH1a family.

| Residue position | IMGT germline | VBase germline | QC selected scFv | KDBseq | Consensus residue | f(cons KDB) | f (cons QC) |
|---|---|---|---|---|---|---|---|
| 1 | 0.82 | 0.83 | 0.62 | 0.77 | Q | 86.60 | 75.00 |
| 6 | 1.00 | 1.00 | 0.51 | 0.74 | Q | 84.31 | 58.30 |
| 12 | 1.00 | 1.00 | 0.72 | 0.93 | V | 96.29 | 83.30 |
| 13 | 1.00 | 1.00 | 0.72 | 0.86 | K | 92.59 | 83.30 |
| 14 | 1.00 | 1.00 | 0.60 | 0.93 | K | 96.29 | 75.00 |
| 19 | 1.00 | 1.00 | 0.72 | 1.00 | V | 100.00 | 83.30 |
| 21 | 0.83 | 0.83 | 0.72 | 0.96 | V | 98.14 | 83.30 |
| 90 | 1.00 | 1.00 | 0.47 | 0.89 | Y | 94.44 | 66.60 |
| 92 | 0.83 | 1.00 | 0.60 | 0.93 | E | 96.29 | 75.00 |
| 95 | 0.83 | 0.83 | 0.49 | 0.70 | S | 83.33 | 66.60 |
| 98 | 1.00 | 1.00 | 0.39 | 0.83 | S | 90.74 | 38.30 |

Table 5: Variability analysis of residues and corresponding frequencies of the consensus amino acid identified in the germline for the VH1b family.

| Residue position | IMGT germline | VBase germline | QC selected scFv | KDBseq | Consensus residue | f(cons KDB) | f (cons QC) |
|---|---|---|---|---|---|---|---|
| 1 | 0.82 | 0.83 | 0.58 | 0.92 | Q | 95.65 | 70.59 |
| 10 | 0.82 | 0.83 | 0.52 | 0.73 | A | 85.00 | 70.59 |
| 12 | 1.00 | 1.00 | 0.64 | 0.86 | V | 92.59 | 76.47 |
| 13 | 1.00 | 1.00 | 0.52 | 0.86 | K | 92.59 | 70.59 |
| 14 | 1.00 | 1.00 | 0.54 | 0.88 | K | 93.83 | 70.59 |
| 20 | 1.00 | 1.00 | 0.61 | 0.86 | K | 92.59 | 76.47 |
| 21 | 0.83 | 0.83 | 0.47 | 0.84 | V | 91.36 | 64.71 |
| 45 | 0.70 | 0.83 | 0.64 | 0.90 | R | 95.06 | 76.47 |
| 47 | 0.83 | 1.00 | 0.31 | 0.95 | A | 97.53 | 47.06 |
| 50 | 0.70 | 0.70 | 0.48 | 0.76 | Q | 86.42 | 64.71 |
| 55 | 0.83 | 0.83 | 0.64 | 0.82 | M | 90.12 | 76.47 |
| 77 | 1.00 | 1.00 | 0.64 | 1.00 | R | 100.00 | 76.47 |
| 78 | 0.83 | 1.00 | 0.32 | 0.76 | A | 86.42 | 47.06 |
| 82 | 0.45 | 0.39 | 0.25 | 0.36 | R | 55.56 | 29.41 |
| 86 | 0.45 | 0.45 | 0.37 | 0.27 | I | 24.69 | 17.65 |
| 87 | 0.57 | 0.70 | 0.30 | 0.53 | S | 70.37 | 25.00 |
| 107 | 1.00 | 1.00 | 0.60 | 0.90 | A | 95.00 | 75.00 |

The variability analysis results for the light chain variable region familes Vκ1, Vκ3 and Vλ1 are shown below in Tables 6, 7 and 8, respectively. For each table, the columns are as follows: column 1: amino acid residue position using the AHo numbering system (conversion to the Kabat numbering system can be accomplished using the conversion table set forth as Table 1 in Example 1); columns 2 to 5: calculated diversity for each antibody array in the database for the residue position indicated in column 1; column 6: consensus residue of the corresponding germline family and KDB; column 7: relative residue frequency in the KDB database for the consensus residue in column 6; and column 8: relative residue frequency in the QC selected scFv database for the consensus residue in column 6.

Table 6: Variability analysis of residues and corresponding frequencies of the consensus amino acid identified in the germline for the Vk1 family.

| *Residue position* | *IMGT germline* | *VBase germline* | *QC selected scFv* | *KDBseq* | *Consensus residue* | *f(cons KDB)* | *f (cons QC)* |
|---|---|---|---|---|---|---|---|
| 1 | 0.52 | 0.47 | 0.61 | 0.68 | D | 81.5 | 23.3 |
| 3 | 0.76 | 0.72 | 0.66 | 0.55 | Q | 72.0 | 18.6 |
| 4 | 0.65 | 0.73 | 0.57 | 0.62 | M | 76.0 | 23.3 |
| 24 | 0.69 | 0.72 | 0.64 | 0.74 | R | 85.3 | 76.7 |
| 47 | 1.00 | 1.00 | 0.69 | 0.88 | K | 94.0 | 81.4 |
| 50 | 1.00 | 1.00 | 0.60 | 0.79 | R | 89.0 | 76.7 |
| 57 | 1.00 | 1.00 | 0.58 | 0.79 | Y | 88.6 | 74.4 |
| 91 | 0.83 | 0.81 | 0.70 | 0.77 | L | 86.6 | 81.4 |
| 103 | 0.91 | 1.00 | 0.67 | 0.90 | T | 81.4 | 95.7 |

Table 7: Variability analysis of residues and corresponding frequencies of the consensus amino acid identified in the germline for the Vk3 family.

| *Residue position* | *IMGT germline* | *VBase germline* | *QC selected scFv* | *KDBseq* | *Consensus residue* | *f(cons KDB)* | *f (cons QC)* |
|---|---|---|---|---|---|---|---|
| +2 | 1.00 | 1.00 | 0.72 | 0.69 | I | 82.47 | 83.33 |
| 3 | 1.00 | 1.00 | 0.72 | 0.64 | V | 77.93 | 83.33 |
| 10 | 1.00 | 1.00 | 0.72 | 0.93 | T | 96.19 | 83.33 |
| 12 | 1.00 | 1.00 | 0.72 | 0.98 | S | 98.84 | 83.33 |
| 18 | 1.00 | 1.00 | 0.72 | 0.92 | R | 95.86 | 83.33 |
| 20 | 1.00 | 1.00 | 0.68 | 0.95 | T | 97.30 | 66.67 |
| 56 | 1.00 | 1.00 | 0.72 | 0.91 | I | 95.31 | 83.33 |
| 74 | 1.00 | 1.00 | 0.50 | 0.86 | I | 92.61 | 66.67 |
| 94 | 1.00 | 1.00 | 0.72 | 0.82 | S | 90.29 | 83.33 |
| 101 | 1.00 | 1.00 | 0.50 | 0.91 | F | 95.14 | 66.67 |
| 103 | 1.00 | 1.00 | 0.50 | 0.82 | F | 90.47 | 66.67 |

Table 8: Variability analysis of residues and corresponding frequencies of the consensus amino acid identified in the germline for the Vλ1 family.

| *Residue position* | *IMGT germline* | *VBase germline* | *QC selected scFv* | *KDBseq* | *Consensus residue* | *f(cons KDB)* | *f(cons QC)* |
|---|---|---|---|---|---|---|---|
| 1 | 1.00 | 1.00 | 0.45 | 0.70 | Q | 81.10 | 62.50 |
| 2 | 1.00 | 1.00 | 0.27 | 0.73 | S | 85.13 | 37.50 |
| 4 | 1.00 | 1.00 | 0.60 | 0.85 | L | 92.00 | 75.00 |
| 7 | 1.00 | 1.00 | 0.77 | 0.99 | P | 99.32 | 87.50 |
| 11 | 0.59 | 0.52 | 0.53 | 0.51 | V | 59.88 | 37.50 |
| 14 | 0.59 | 0.52 | 0.49 | 0.51 | A | 59.95 | 31.25 |
| 46 | 1.00 | 1.00 | 0.70 | 0.80 | Q | 89.00 | 81.25 |
| 53 | 1.00 | 1.00 | 0.49 | 0.90 | K | 94.63 | 68.75 |
| 82 | 1.00 | 1.00 | 0.60 | 0.90 | K | 94.88 | 75.00 |
| 92 | 0.59 | 0.68 | 0.51 | 0.54 | A | 69.82 | 68.75 |
| 103 | 1.00 | 1.00 | 0.50 | 0.86 | D | 92.84 | 68.75 |

As set forth in Tables 3-8 above, it was found that a subset of residue positions in the QC system selected scFv frameworks were strongly biased towards certain residues not present or under-represented in the germlines (VBase and IMGT) and in mature antibodies (KDB), suggested that the stability of scFv can be rationally improved based on the unique features of the framework sequences selected in the Quality Control Yeast Screening System.

**EXAMPLE 4: Selection of Preferred Residues**

In order to select preferred amino acid residue substitutions (or, alternatively, exclude amino acid residues) at a particular amino acid position known to improve the functional properties (e.g., stability and/or solubility) of a scFv, VH and VL sequences from the Kabat database of matured antibody sequences were grouped according to their family subtype (e.g., VH1b, VH3, etc.). Within each subfamily of sequences, the frequency of each amino acid residue at each amino acid position was determined as a percentage of all the analyzed sequences of one group of subtypes. The same was done for all the sequences of the QC database consisting of antibodies that were preselected for enhanced stability and/or solubility by the so-called QC system. For each subtype, the resulting percentages (relative frequencies) for each amino acid residue obtained for the Kabat sequences and for the QC sequences were compared at each corresponding position. In the event that the relative frequency of a certain amino acid residue was increased in the QC database relative to the Kabat database, the respective residue was considered a preferred residue at the given position to improve the stability and / or solubility of a scFv. Conversely, in the case that the relative frequency of a certain amino acid residue was decreased in the QC database as compared to the Kabat database, the respective residue was considered unfavorable at that position in the context of an scFv format.

Table 9 depicts an exemplary analysis of the residue frequency at amino acid position H78 (AHo numbering; Kabat position H67) for the VH1b subtype in the different databases. The columns in Table 9 are as follows: column 1: residue type; column 2: residue frequency in IMGT germline database; column 3: residue frequency in Vbase germline database; column 4: residue frequency in a QC database; column 5: residue frequency in a Kabat database.

Table 9: Relative residue frequency at position 78 (AHo numbering) for the VH1b subtype in two germline databases, a QC database, and a Kabat database of mature antibodies.

| *Residue* | *IMGT_germ* | *Vbase_germ* | ***QC database*** | *KDB_VH1B* |
|---|---|---|---|---|
| D | | | | |
| E | | | | |
| K | | | | |
| R | | | | |
| H | | | | |
| T | | | | |
| S | | | | |
| N | | | | |
| Q | | | | |
| G | | | | |
| A | | | **24** | 2 |
| C | | | | |
| P | | | | |
| V | 91 | 100 | **47** | 86 |
| I | | | **18** | 1 |
| L | | | **12** | |
| M | | | | |
| F | 9 | | | 10 |
| Y | | | | |
| W | | | | |
| **Consensus** | **V** | **V** | **V** | **V** |
| **% Agree** | **91** | **100** | **47** | **86** |
| **# of Seq*** | **11** | **11** | **17** | **81** |

*Number of sequences collected for the analysis of residue frequency

In the QC database, an alanine (A) residue was observed at a frequency of 24%, a factor of 12 above the 2% frequency observed for the same residue in a mature Kabat database (KDB_VH1B). Accordingly, an alanine residue at position H78 (AHo numbering) is considered a preferred residue at that position for enhancing the functional properties (e.g., stability and/or solubility) of a scFv. In contrast, a valine (V) residue was observed in the QC database at a relative frequency of 47%, much lower than the 86% frequency observed in the mature Kabat database and the more than 90% frequency observed for the same residue in germline databases (91% in IMGT-germ and 100% in Vbase germ). Therefore, a valine residue (V) was considered to be an unfavorable residue at position H78 in the context of an scFv format.

**EXAMPLE 5: Comparison of ESBA105 scfv Variants from Two Different Approaches**

In this example, the stability of scFv variants prepared by two different approaches was compared. The parental scFv antibody was ESBA 105, which has previously been described (see *e.g.,* PCT Publications WO 2006/131013 and WO 2008/006235). One set of ESBA 105 variants was selected using the Quality Control Yeast Screening System ("QC variants"), which variants also have been previously described (see *e.g.,* PCT Publications WO 2006/131013 and WO 2008/006235). The other set of variants was prepared by back-mutating certain amino acid positions to the preferred germline consensus sequence identified by the sequence analysis described in Examples 2 and 3 above. The back-mutations were selected by searching within the amino acid sequences for positions that were conserved in the germline sequence but that contained an unusual or low frequency amino acid in the selected scFv (referred to as the germline consensus engineering approach).

All of the variants were tested for stability by subjecting the molecules to a thermal induced stress. By challenging at a broad range of temperatures (25-95°C) it was possible to determine approximate midpoints of the thermal unfolding transitions (TM) for every variant. Thermostability measurements for the wild type molecules and the variants were performed with the FT-IR ATR spectroscopy where the IR light was guided through an interferometer. The measured signal is the interferogram, performing a Fourier transformation on this signal the final spectrum is identical to that from conventional (dispersive) infrared spectroscopy.

The thermal unfolding results are summarized below in Table 10 and graphically depicted in Figure 6. The columns in Table 10 are as follows: column 1: ESBA 105 variants; column 2: domain containing the mutation; column 3: mutation(s) in AHo numbering; column 4: TM midpoints calculated from the thermal unfolding curves in Figure 6; column 5: relative activity compared to the parental ESBA 105; column 5: mutagenesis strategy for the variant specified in column 1.

Table 10: Comparison of ESBA105 variants from two different approaches and their contribution to overall stability measured in FT-IR (Midpoints calculated for the thermal unfolding transitions).

| Variant | Domain | Mutation | TM°C | Binding Activity | Description |
|---|---|---|---|---|---|
| **E105** | | | 61.53 | | Parental molecule |
| **ESBA105_QC11.2** | VH | F78L | 66.26 | 1 | QCvariant |
| **ESBA105_QC15.2** | VH | K50R, F78I | 65.47 | 1 | QCvariant |
| **ESBA105_QC23.2** | VH | F78L | 66.53 | 1 | QCvariant |
| **ESBA105_VL R47K** | VL | R47K | 62.4 | 0.9 | back-mutated to consensus |
| **ESBA105_VL V103T** | VL | V103T | 60.7 | 1 | back-mutated to consensus |
| **ESBA105_VL V3Q** | VL | V3Q | 61.9 | 1.2 | back-mutated to consensus |

As compared to the QC variants, the back mutations to the germline consensus had negative or no effect on the thermostability and activity of ESBA 105. Thus, these results contradict the consensus engineering approach which has been used by others to improve stability in different antibodies and formats (see *e.g.,* Steipe, B et al. (1994) J. Mol. Biol. 240:188-192; Ohage, E. and Steipe, B. (1999) J. Mol. Biol 291:1119-1128; Knappik, A. et al. (2000) J. Mol. Biol. 296:57-86, Ewert, S. et al. (2003) Biochemistry 42:1517-1528; and Monsellier, E. and Bedouelle, H. (2006) J. Mol. Biol. 362:580-593).

In a separate experiment, the above QC variants (QC11.2, QC 15.2, and QC23.2) and an additional QC variant (QC7.1) were compared with a second set variants having either consensus backmutations (S-2, D-2, and D-3) or backmutation to alanine (D-1)(see table 11). The identity of the residue at selected framework positions are indicated in table 11 and the measured thermal stability (in arbitrary unfolding units) is depicted in Figure 7.

Table 11: The identity of the framework residues at selected framework positions of ESBA105 variants comprising either consensus backmutations (S-2, D-2, D-3), a mutation to alanine (D-1) or a QC residue (QC7.1, QC11.2, QC15.2, QC23.2) is provided. Residues which differ from the parental ESBA105 antibody are depicted in bold italics. Amino acid positions are provided in Kabat numbering.

| | VL-CL interface | VH-CH interface | Outer loop | Outer loop | VH-CH interface |
|---|---|---|---|---|---|
| | L83 | H43 | H67 | H69 | H78 |
| Original | V | K | F | F | V |
| QC7.1 | *E* | K | F | F | *A* |
| QC11.2 | V | K | *L* | F | V |
| QC15.2 | V | *R* | *I* | F | V |
| QC23.2 | V | K | *L* | F | V |
| S-2 | V | K | *V* | F | V |
| D-1 | V | K | *A* | F | V |
| D-2 | V | K | *V* | *L* | V |
| D-3 | V | K | F | *L* | V |

Although some consensus variants (S-2 and D-1) exhibited a marked enhancement in thermal stability, this enhancement was less than the enhancement in thermal stability achieved by each of the four QC variants.

Accordingly, the results herein demonstrate that the selection pressure applied in the "Quality Control Yeast Screening System" yields a sub-population of scaffolds which do contain common features seldom observed in nature (yet still human) and presumably responsible for the superior biophysical properties of these frameworks. By challenging at 60°C different variants of ESBA105, it was possible to reconfirm the superior properties of the preferred substitutions identified in the selected scFv framework database. Thus, the "functional consensus" approach described herein based on the selected scFv sequences obtained from the QC yeast screening system has been demonstrated to yield scFv variants having superior thermal stability than variants prepared using the germline consensus approach.

## EXAMPLE 6: ESBA212 scFv Variants

In this example, the stability of germline consensus variants of a scFv antibody (ESBA212) with a different binding specificity than ESBA105 were compared. All ESBA212 variants were prepared by back-mutating certain amino acid positions to the preferred germline consensus sequence identified by the sequence analysis described in Examples 2 and 3 above. The back-mutations were selected by searching within the amino acid sequences for positions that were conserved in the germline sequence but that contained an unusual or low frequency amino acid in the selected scFv (referred to as the germline consensus engineering approach). As in Example 5, all of the variants were tested for stability by subjecting the molecules to a thermal induced stress.

The thermal unfolding results for the ESBA212 variants are summarized below in Table 11 and graphically depicted in Figure 8. The columns in Table 11 are as follows: column 1: ESBA 212 variants; column 2: domain containing the mutation; column 3: mutation(s) in AHo numbering; column 4: TM midpoints calculated from the thermal unfolding curves in Figure 7; column 5: relative activity compared to the parental ESBA 212; column 5: mutagenesis strategy for the variant specified in column 1.

Table 12: Comparison of ESBA212 variants back-mutated to the germline consensus residue and their contribution to overall stability measured in FT-IR (Midpoints calculated for the thermal unfolding transitions).

| **Variant** | **Domain** | **Mutation** | **TM°C** | **Binding Activity** | **Description** |
|---|---|---|---|---|---|
| **ESBA212** | | | 63.66 | | Parental molecule |
| **ESBA212_VL R47K** | VL | R47K | 59.94 | 2.8 | back-mutated to consensus |

(continued)

| Variant | Domain | Mutation | TM°C | Binding Activity | Description |
|---|---|---|---|---|---|
| **ESBA212_VL** V3Q | VL | V3Q | 63.6 | 1.1 | back-mutated to consensus |

As observed for the unrelated ESBA105 scFv antibody, back mutations to the germline consensus had negative or no effect on the thermostability and activity of ESBA212. Thus, these results serve to further highlight the inadequacy of conventional consensus-based approaches. These deficiencies can be addressed by employing the functional consensus methodology of the invention.

**<u>EXAMPLE 7</u>: Exemplary and Preferred Amino Acids Substitutions at Identified scFv Framework Positions**

Using the sequence-based scFv analysis approach described above in Example 2, 3 and 4, it was possible to identify exemplary and preferred amino acid substitutions at amino acid residue positions within the scFv frameworks in the QC selected scFv database that exhibited differences in variability as compared to the germline databases. This analysis was performed by determining the frequency of each of the twenty amino acids at each particular framework position of interest within the two germline databases (IMGT and Vbase), the QC selected scFv database and the mature antibody database (KDB), as described in Example 4 for AHo position 78 (Kabat position 67) for the VH1b heavy chain family as a representative example. Exemplary and preferred amino acid substitutions were identified for three heavy chain variable region families, VH3, VH1a and VH1b, and for three light chain variable region families, Vκ1, Vκ3 and Vλ1.

The results are summarized below in Tables 13-18. For each table, column one shows the residue position using the AHo numbering system, column two shows the germline consensus residue, column three shows the exemplary substitutions found in the QC selected scFv frameworks, column 4 shows the preferred residue found in the QC selected scFv frameworks and columns 5 to 8 show the relative residue frequency in the four different databases for the preferred substitution (shown in column 4) at the residue position indicated in column 1.

Table 13: Exemplary and preferred amino acid substitutions of residue positions identified as unique features of the QC selected scFv frameworks of the family VH3.

| *Residue position* | *Consensus residue* | *Substitutions* | *Preferred substitution* | *IMGT germline* | *VBase germline* | *QC selected scFv* | *KDBseq* |
|---|---|---|---|---|---|---|---|
| 1 | E | E, Q | Q | 15.38 | 22.73 | 46.43 | 28.13 |
| 6 | E | E, Q | Q | 0.00 | 0.00 | 31.03 | 6.98 |
| 7 | S | T, S, A | T | 0.00 | 4.55 | 20.69 | 0.46 |
| 89 | L | A, V, L, F | V | 0.00 | 0.00 | 22.81 | 6.37 |
| 103 | V | R, Q, V, I, L, M, F | L | 11.54 | 13.64 | 25.00 | 9.96 |

Table 14: Exemplary and preferred amino acid substitutions of residue positions identified as unique features of the QC selected scFv frameworks of the family VH1a.

| *Residue position* | *Consensus residue* | *Substitutions* | *Preferred substitution* | *IMGT germline* | *VBase germline* | *QCselected scFv* | *KDBseq* |
|---|---|---|---|---|---|---|---|
| 1 | Q | E, Q | E | 10.00 | 9.09 | 25.00 | 0.00 |
| 6 | Q | E, Q | E | 0.00 | 0.00 | 41.67 | 15.69 |
| 12 | V | L, V | L | 0.00 | 0.00 | 16.67 | 0.00 |
| 13 | K | M, K | M | 0.00 | 0.00 | 16.67 | 0.00 |
| 14 | K | E, Q, K | E | 0.00 | 0.00 | 16.67 | 1.85 |
| 19 | V | L, V | L | 0.00 | 0.00 | 16.67 | 0.00 |
| 21 | V | I, V | I | 9.09 | 9.09 | 16.67 | 0.00 |
| 90 | Y | F, S, H, D, Y | Nd | | | | |

(continued)

| *Residue position* | *Consensus residue* | *Substitutions* | *Preferred substitution* | *IMGT germline* | *VBase germline* | *QCselected scFv* | *KDBseq* |
|---|---|---|---|---|---|---|---|
| 92 | E | D, Q, E | D | 9.09 | 0.00 | 16.67 | 1.85 |
| 95 | S | G, N, T, S | G | 0.00 | 0.00 | 16.67 | 7.41 |
| 98 | S | T, A, P, F, S | F | 0.00 | 0.00 | 16.67 | 1.85 |

Table 15: Exemplary and preferred amino acid substitutions of residue positions identified as unique features of the QC selected scFv frameworks of the family VH1b.

| *Residue position* | *Consensus residue* | *Substitutions* | *Preferred substitution* | *IMGT germline* | *VBase germline* | *QC selected scFv* | *KDBseq* |
|---|---|---|---|---|---|---|---|
| 1 | Q | Q, E | E | 10.00 | 9.09 | 29.41 | 1.45 |
| 10 | A | A, T, P, V,D | T | 0.00 | 0.00 | 11.76 | 2.50 |
| 12 | V | V, L | L | 0.00 | 0.00 | 23.53 | 7.41 |
| 13 | K | K, V, R, Q, M | V | 0.00 | 0.00 | 11.76 | 0.00 |
| 14 | K | E, K, R, M | R | 0.00 | 0.00 | 17.65 | 2.47 |
| 20 | K | R, , T, K, N | N | 0.00 | 0.00 | 11.76 | 0.00 |
| 21 | V | I, F, V, L | L | 0.00 | 0.00 | 17.65 | 2.47 |
| 45 | R | R, K | K | 0.00 | 0.00 | 23.53 | 0.00 |
| 47 | A | T, P, V, A, R | R | 0.00 | 0.00 | 23.53 | 0.00 |
| 50 | Q | K, Q, H, E | K | 18.18 | 18.18 | 23.53 | 2.47 |
| 55 | M | M, I | I | 9.09 | 9.09 | 23.53 | 3.70 |
| 77 | R | K, R | K | 0.00 | 0.00 | 23.53 | 0.00 |
| 78 | V | A, V, L, I | A | 0.00 | 0.00 | 23.53 | 2.47 |
| 82 | R | E, R, T, A | E CONS | 9.09 | 9.09 | 29.41 | 1.23 |
| 86 | I | T, S, I, L | T CONS | 63.64 | 63.64 | 52.94 | 29.63 |
| 87 | S | D, S, N ,G | N CONS | 0.00 | 0.00 | 37.50 | 18.52 |
| 107 | A | N, S, A | N | 0.00 | 0.00 | 18.75 | 0.00 |

Table 16: Exemplary and preferred amino acid substitutions of residue positions identified as unique features of the QC selected scFv frameworks of the family Vκ1.

| *Residue position* | *Consensus residue* | *Substitutions* | *Preferred substitution* | *IMGT germline* | *VBase germline* | *QC selected scFv* | *KDBseq* |
|---|---|---|---|---|---|---|---|
| 1 | D | D, E, I | E | 0% | 0% | 74% | 10% |
| 3 | Q | Q, V, I | V | 0% | 0% | 79% | 8% |
| 4 | M | V,L,I,M | L | 23% | 16% | 72% | 21% |
| 24 | R | R, Q | Q | 9% | 11% | 23% | 11% |
| 47 | K | K, R, I | R | 0% | 0% | 16% | 2% |
| 50 | R | K, R, E, T, M, Q | nd | | | | |
| 57 | Y | H, S, F, Y | S | 0% | 0% | 14% | 5% |
| 91 | L | L, F | F | 9% | 11% | 19% | 12% |
| 103 | T | V, S, G, I | V | 0% | 0% | 9% | 1% |

Table 17: Exemplary and preferred amino acid substitutions of residue positions identified as unique features of QC selected scFv frameworks of the family Vκ3.

| *Residue position* | *Consensus residue* | *Substitutions* | *Preferred substitution* | *IMGT germlilne* | *VBase germline* | *QC selected scFv* | *KDBseq* |
|---|---|---|---|---|---|---|---|
| 2 | I | I, T | T | 0% | 0% | 17% | 1% |
| 3 | V | V, T | T | 0% | 0% | 17% | 0% |
| 10 | T | T, I | I | 0% | 0% | 17% | 1% |
| 12 | S | S, Y | Y | 0% | 0% | 17% | 0% |
| 18 | R | S, R | S | 0% | 0% | 17% | 1% |
| 20 | T | T, A | A | 0% | 0% | 17% | 1% |
| 56 | I | I, M | M | 0% | 0% | 17% | 2% |
| 74 | I | I, V, T | T | 0% | 0% | 17% | 1% |
| 94 | S | S, N | N | 0% | 0% | 17% | 3% |
| 101 | F | F, Y, S | S | 0% | 0% | 17% | 2% |
| 103 | F | F, L, A | A | 0% | 0% | 17% | 0% |

Table 18: Exemplary and preferred amino acid substitutions of residue positions identified as unique features of the QC selected scFv frameworks of the family Vλ1.

| *Residue position* | *Consensus residue* | *Substitutions* | *Preferred substitutton* | *IMGT germline* | *VBase germline* | *QC selected scFv* | *KDBseq* |
|---|---|---|---|---|---|---|---|
| 1 | Q | L, Q, S, E | L | 0.00 | 0.00 | 18.75 | 0.79 |
| 2 | S | S, A, P, I, Y | P | 0.00 | 0.00 | 31.25 | 0.37 |
| 4 | L | V, M, L | V | 0.00 | 0.00 | 18.75 | 5.45 |
| 7 | P | S, E, P | S | 0.00 | 0.00 | 6.25 | 0.68 |
| 11 | V | A, V | A | 28.57 | 40.00 | 62.50 | 38.95 |
| 14 | A | T, S, A | T | 28.57 | 40.00 | 62.50 | 38.22 |
| 46 | Q | H, Q | H | 0.00 | 0.00 | 18.75 | 9.21 |
| 53 | K | K, T, S, N, Q, P | nd | | | | |
| 82 | K | R, Q, K | R | 0.00 | 0.00 | 18.75 | 3.32 |
| 92 | A | G, T, D, A | T | 0.00 | 0.00 | 12.50 | 0.51 |
| 103 | D | D, V, T, H, E | V | 0.00 | 0.00 | 12.50 | 0.26 |

As demonstrated by the results shown in Tables 13-18, it was found that a subset of residue position in the QC selected scFv frameworks were strongly biased towards certain residues not present or under-represented in the germline sequences and in mature antibody sequences and therefore apparently not used in the Ig format or derived fragments. Thus, the exemplary and preferred substitutions identified in the QC selected scFv frameworks represent amino acid residues likely to contribute to the desirable functional properties (*e.g.*, stability, solubility) exhibited by the QC selected scFv frameworks.

**EXAMPLE 8: scFv Framework Scaffolds based on Functional Consensus**

Based on the exemplary and preferred amino acid substitutions identified in Example 7, scFv framework scaffolds were designed based on the functional consensus approach described herein. In these scFv framework scaffolds, the CDR1, CDR2 and CDR3 sequences are not defined, since these scaffolds represent framework sequences into which essentially any CDR1, CDR2 and CDR3 sequences can be inserted. Furthermore, in the scFv framework scaffolds, those amino acid positions which have been identified as being amenable to variability (as set forth in the tables of Example 7) are allowed to be occupied by any of exemplary or preferred amino acid substitutions identified for that position.

Heavy chain framework scaffolds are depicted in Figures 9-11 (see also tables 19-21). Thus, for the VH1a family, the scFv framework scaffold is illustrated in Figure 9 (see also table 19). For the VH1b family, the scFv framework scaffold is illustrated in Figure 10 (see also table 20). For the VH3 family, the scFv framework is illustrated in Figure 11 (see also table 21). For the alignments in each of these figures, the first row shows the heavy chain variable region numbering using the Kabat system and the second row shows the heavy chain variable region numbering using the AHo system. The third row shows the scFv framework scaffold sequence, wherein at those positions marked as "X", the position can be occupied by any of the amino acid residues listed below the "X." Furthermore, the positions marked *"x"* (*i.e.,* Kabat 26, 27, 28, 29 and AHo 27, 29, 30, 31 in Figures ) and the regions marked as CDRs can be occupied by any amino acid. For the variable positions marked as "X", the first amino acid residue listed below the "X" represents the germline consensus residue, the second amino acid residue listed below the "X" represents the preferred amino acid substitution at that position and the additional amino acid residues listed below the "X" (if any) represent other exemplary amino acid subsititutions at that position.

Tab. 19: VH1 Family Heavy chain Framework Scaffold

| | | | | | | | | | | | | | | | | | | | | | | | | | |
|---|---|---|---|---|---|---|---|---|---|---|---|---|---|---|---|---|---|---|---|---|---|---|---|---|---|
| 1 | 2 | 3 | 4 | 5 | 6 | 7 | * | 8 | 9 | 10 | 11 | 12 | 13 | 14 | 15 | 16 | 17 | 18 | 19 | 20 | 21 | 22 | 23 | 24 | 25 |
| 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
| X | V | Q | L | V | X | S | | G | A | E | X | X | X | P | G | S | S | X | K | X | S | C | K | A | S |
| Q | | | | | Q | | | | | | V | K | K | | | | | V | | V | | | | | |
| E | | | | | E | | | | | | L | M | E | | | | | L | | I | | | | | |
| | | | | | | | | | | | | | Q | | | | | | | | | | | | |

| | | | | | | | | | | | | | | | | | | | | | | | | | |
|---|---|---|---|---|---|---|---|---|---|---|---|---|---|---|---|---|---|---|---|---|---|---|---|---|---|
| 26 | * | 27 | 28 | 29 | 30 | 31 | 32 | 33 | 34 | 35 | 35a | 35b | * | * | * | 36 | 37 | 38 | 39 | 40 | 41 | 42 | 43 | 44 | 45 |
| 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
| x | | x | x | x | CDR H1 | | | | | | | | | | | W | V | R | Q | A | P | G | Q | G | L |

| | | | | | | | | | | | | | | | | | | | | | | | | | | |
|---|---|---|---|---|---|---|---|---|---|---|---|---|---|---|---|---|---|---|---|---|---|---|---|---|---|---|
| 46 | 47 | 48 | 49 | 50 | 51 | 52 | 52a | 52b | 52c | * | 53 | 54 | 55 | 56 | 57 | 58 | 59 | 60 | 61 | 62 | 63 | 64 | 65 | 66 | 67 | 68 |
| 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 | 79 |
| E | W | M | G | CDR H2 | | | | | | | | | | | | | | | | | | | | R | V | T |

| | | | | | | | | | | | | | | | | | | | | | | | | | |
|---|---|---|---|---|---|---|---|---|---|---|---|---|---|---|---|---|---|---|---|---|---|---|---|---|---|
| 69 | 70 | 71 | 72 | 73 | 74 | 75 | 76 | 77 | 78 | 79 | 80 | 81 | 82 | 82a | 82b | 82b | 83 | 84 | 85 | 86 | 87 | 88 | 89 | 90 | 91 |
| 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 | 105 |
| I | T | A | D | E | S | T | S | T | A | X | M | X | L | S | X | L | R | X | E | D | T | A | V | Y | Y |
| | | | | | | | | | | Y | | E | | | S | | | S | | | | | | | |
| | | | | | | | | | | F | | D | | | G | | | F | | | | | | | |
| | | | | | | | | | | S | | Q | | | T | | | T | | | | | | | |
| | | | | | | | | | | H | | | | | N | | | A | | | | | | | |
| | | | | | | | | | | D | | | | | | | | P | | | | | | | |

| | | | | | | | | | | | | | | | | | | | | | | |
|---|---|---|---|---|---|---|---|---|---|---|---|---|---|---|---|---|---|---|---|---|---|---|
| 92 | 93 | 94 | 95 | 96 | 97 | 98 | 99 | 100 | 100a | 100b | 100c | 100d | 100e | 100f | 100g | 100h | 100i | * | * | * | * | * |
| 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 |
| C | A | R | CDR H3 | | | | | | | | | | | | | | | | | | | |

| | | | | | | | | | | | | | | | | | | | | |
|---|---|---|---|---|---|---|---|---|---|---|---|---|---|---|---|---|---|---|---|---|
| * | * | * | * | * | * | * | * | 101 | 102 | 103 | 104 | 105 | 106 | 107 | 108 | 109 | 110 | 111 | 112 | 113 |
| 129 | 130 | 131 | 132 | 133 | 134 | 135 | 136 | 137 | 138 | 139 | 140 | 141 | 142 | 143 | 144 | 145 | 146 | 147 | 148 | 149 |
| CDR H3 | | | | | | | | | | W | G | Q | G | T | L | V | T | V | S | S |

Tab. 20: VH1B Family Heavy chain Framework Scaffold

| | | | | | | | | | | | | | | | | | | | | | | | | | |
|---|---|---|---|---|---|---|---|---|---|---|---|---|---|---|---|---|---|---|---|---|---|---|---|---|---|
| 1 | 2 | 3 | 4 | 5 | 6 | 7 | * | 8 | 9 | 10 | 11 | 12 | 13 | 14 | 15 | 16 | 17 | 18 | 19 | 20 | 21 | 22 | 23 | 24 | 25 |
| 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
| X | V | Q | L | V | Q | S | | G | X | E | X | X | X | P | G | A | S | V | X | X | S | C | K | A | S |
| Q | | | | | | | | | A | | V | K | K | | | | | | K | V | | | | | |
| E | | | | | | | | | T | | L | V | R | | | | | | N | L | | | | | |
| | | | | | | | | | D | | | R | E | | | | | | R | I | | | | | |
| | | | | | | | | | P | | | Q | M | | | | | | T | F | | | | | |
| | | | | | | | | | V | | | M | | | | | | | | | | | | | |

| | | | | | | | | | | | | | | | | | | | | | | | | | |
|---|---|---|---|---|---|---|---|---|---|---|---|---|---|---|---|---|---|---|---|---|---|---|---|---|---|
| 26 | * | 27 | 28 | 29 | 30 | 31 | 32 | 33 | 34 | 35 | 35a | 35b | * | * | * | 36 | 37 | 38 | 39 | 40 | 41 | 42 | 43 | 44 | 45 |
| 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
| x | | x | x | x | CDR H1 | | | | | | | | | | | W | V | X | Q | X | P | G | X | G | L |
| | | | | | | | | | | | | | | | | | | R | | A | | | Q | | |
| | | | | | | | | | | | | | | | | | | K | | R | | | K | | |
| | | | | | | | | | | | | | | | | | | | | T | | | E | | |
| | | | | | | | | | | | | | | | | | | | | V | | | H | | |
| | | | | | | | | | | | | | | | | | | | | P | | | | | |

| | | | | | | | | | | | | | | | | | | | | | | | | | | |
|---|---|---|---|---|---|---|---|---|---|---|---|---|---|---|---|---|---|---|---|---|---|---|---|---|---|---|
| 46 | 47 | 48 | 49 | 50 | 51 | 52 | 52a | 52b | 52c | * | 53 | 54 | 55 | 56 | 57 | 58 | 59 | 60 | 61 | 62 | 63 | 64 | 65 | 66 | 67 | 68 |
| 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 | 79 |
| E | W | X | G | CDR H2 | | | | | | | | | | | | | | | | | | | | X | X | T |
| | | M | | | | | | | | | | | | | | | | | | | | | | R | V | |
| | | I | | | | | | | | | | | | | | | | | | | | | | K | A | |
| | | | | | | | | | | | | | | | | | | | | | | | | | I | |
| | | | | | | | | | | | | | | | | | | | | | | | | | L | |

| | | | | | | | | | | | | | | | | | | | | | | | | | |
|---|---|---|---|---|---|---|---|---|---|---|---|---|---|---|---|---|---|---|---|---|---|---|---|---|---|
| 69 | 70 | 71 | 72 | 73 | 74 | 75 | 76 | 77 | 78 | 79 | 80 | 81 | 82 | 82a | 82b | 82b | 83 | 84 | 85 | 86 | 87 | 88 | 89 | 90 | 91 |
| 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 | 105 |
| M | T | E | D | T | S | T | N | T | A | Y | M | E | L | S | S | L | R | S | E | D | T | A | V | Y | Y |

| | | | | | | | | | | | | | | | | | | | | | | |
|---|---|---|---|---|---|---|---|---|---|---|---|---|---|---|---|---|---|---|---|---|---|---|
| 92 | 93 | 94 | 95 | 96 | 97 | 98 | 99 | 100 | 100a | 100b | 100c | 100d | 100e | 100f | 100g | 100h | 100i | * | * | * | * | * |
| 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 |
| C | X | R | CDR H3 | | | | | | | | | | | | | | | | | | | |
| | A | | | | | | | | | | | | | | | | | | | | | |
| | N | | | | | | | | | | | | | | | | | | | | | |
| | S | | | | | | | | | | | | | | | | | | | | | |

| | | | | | | | | | | | | | | | | | | | | |
|---|---|---|---|---|---|---|---|---|---|---|---|---|---|---|---|---|---|---|---|---|
| * | * | * | * | * | * | * | * | 101 | 102 | 103 | 104 | 105 | 106 | 107 | 108 | 109 | 110 | 111 | 112 | 113 |
| 129 | 130 | 131 | 132 | 133 | 134 | 135 | 136 | 137 | 138 | 139 | 140 | 141 | 142 | 143 | 144 | 145 | 146 | 147 | 148 | 149 |
| CDR H3 | | | | | | | | | | W | G | Q | G | T | L | V | T | V | S | S |

Tab. 21: VH3 Family Heavy chain Framework Scaffold

| | | | | | | | | | | | | | | | | | | | | | | | | | |
|---|---|---|---|---|---|---|---|---|---|---|---|---|---|---|---|---|---|---|---|---|---|---|---|---|---|
| 1 | 2 | 3 | 4 | 5 | 6 | 7 | * | 8 | 9 | 10 | 11 | 12 | 13 | 14 | 15 | 16 | 17 | 18 | 19 | 20 | 21 | 22 | 23 | 24 | 25 |
| 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
| X | V | Q | L | V | X | X | | G | P | G | L | V | K | P | S | E | T | L | R | L | S | C | A | A | S |
| E | | | | | E | S | | | | | | | | | | | | | | | | | | | |
| Q | | | | | Q | T | | | | | | | | | | | | | | | | | | | |
| | | | | | | A | | | | | | | | | | | | | | | | | | | |

| | | | | | | | | | | | | | | | | | | | | | | | | | |
|---|---|---|---|---|---|---|---|---|---|---|---|---|---|---|---|---|---|---|---|---|---|---|---|---|---|
| 26 | * | 27 | 28 | 29 | 30 | 31 | 32 | 33 | 34 | 35 | 35a | 35b | * | * | * | 36 | 37 | 38 | 39 | 40 | 41 | 42 | 43 | 44 | 45 |
| 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
| G | | x | x | x | CDR H1 | | | | | | | | | | | W | V | R | Q | A | P | G | K | G | L |

| | | | | | | | | | | | | | | | | | | | | | | | | | | |
|---|---|---|---|---|---|---|---|---|---|---|---|---|---|---|---|---|---|---|---|---|---|---|---|---|---|---|
| 46 | 47 | 48 | 49 | 50 | 51 | 52 | 52a | 52b | 52c | * | 53 | 54 | 55 | 56 | 57 | 58 | 59 | 60 | 61 | 62 | 63 | 64 | 65 | 66 | 67 | 68 |
| 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 | 79 |
| E | W | V | S | CDR H2 | | | | | | | | | | | | | | | | | | | | R | F | T |

| | | | | | | | | | | | | | | | | | | | | | | | | | |
|---|---|---|---|---|---|---|---|---|---|---|---|---|---|---|---|---|---|---|---|---|---|---|---|---|---|
| 69 | 70 | 71 | 72 | 73 | 74 | 75 | 76 | 77 | 78 | 79 | 80 | 81 | 82 | 82a | 82b | 82b | 83 | 84 | 85 | 86 | 87 | 88 | 89 | 90 | 91 |
| 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 | 105 |
| I | S | R | D | N | S | K | N | T | X | Y | L | Q | M | N | S | L | R | A | E | D | T | A | X | Y | Y |
| | | | | | | | | | L | | | | | | | | | | | | | | V | | |
| | | | | | | | | | V | | | | | | | | | | | | | | L | | |
| | | | | | | | | | A | | | | | | | | | | | | | | I | | |
| | | | | | | | | | F | | | | | | | | | | | | | | M | | |
| | | | | | | | | | | | | | | | | | | | | | | | F | | |
| | | | | | | | | | | | | | | | | | | | | | | | R | | |
| | | | | | | | | | | | | | | | | | | | | | | | Q | | |

| | | | | | | | | | | | | | | | | | | | | | | |
|---|---|---|---|---|---|---|---|---|---|---|---|---|---|---|---|---|---|---|---|---|---|---|
| 92 | 93 | 94 | 95 | 96 | 97 | 98 | 99 | 100 | 100a | 100b | 100c | 100d | 100e | 100f | 100g | 100h | 100i | * | * | * | * | * |
| 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 |
| C | A | R | CDR H3 | | | | | | | | | | | | | | | | | | | |

| | | | | | | | | | | | | | | | | | | | | |
|---|---|---|---|---|---|---|---|---|---|---|---|---|---|---|---|---|---|---|---|---|
| * | * | * | * | * | * | * | * | 101 | 102 | 103 | 104 | 105 | 106 | 107 | 108 | 109 | 110 | 111 | 112 | 113 |
| 129 | 130 | 131 | 132 | 133 | 134 | 135 | 136 | 137 | 138 | 139 | 140 | 141 | 142 | 143 | 144 | 145 | 146 | 147 | 148 | 149 |
| CDR H3 | | | | | | | | | | W | G | Q | G | T | L | V | T | V | S | S |

Light chain framework scaffolds are depicted in Figures 12-14 (see also tables 22-24). For the Vk1 family, the scFv framework scaffold is illustrated in Figure 12 (see also table 22). For the Vk3 family, the scFv framework scaffold is illustrated in Figure 13 (see also table 23). For the Vλ1 family, the scFv framework is illustrated in Figure 14 (see also table 24). For the alignments in each of these figures, the first row shows the light chain variable region numbering using the Kabat system and the second row shows the light chain variable region numbering using the AHo system. The third row shows the scFv framework scaffold sequence, wherein at those positions marked as "X", the position can be occupied by any of the amino acid residues listed below the "X." Furthermore, framework positions marked "."and the regions marked as CDRs can be occupied by any amino acid.

Table 22: Vκ1 Family Light Chain Framework Scaffold.

| 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 | 27 | | 27a | 27b | 27c | 27d | 27e |
|---|---|---|---|---|---|---|---|---|---|---|---|---|---|---|---|---|---|---|---|---|---|---|---|---|---|---|---|---|---|---|---|---|
| 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 | 27 | 28 | 29 | 30 | 31 | 32 | 33 |
| X | I | X | X | T | Q | S | P | S | S | L | S | A | S | V | G | D | R | V | T | I | T | C | X | | | | | | | | | |
| E | | V | L | | | | | | | | | | | | | | | | | | | | Q | | | | | | | | | |
| D | | Q | M | | | | | | | | | | | | | | | | | | | | R | CDR L1 | | | | | | | | |
| I | | I | V | | | | | | | | | | | | | | | | | | | | | | | | | | | | | |
| | | | I | | | | | | | | | | | | | | | | | | | | | | | | | | | | | |

| 27f | | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | | | | | | | | |
|---|---|---|---|---|---|---|---|---|---|---|---|---|---|---|---|---|---|---|---|---|---|---|---|---|---|---|---|---|---|---|---|---|
| 34 | 35 | 36 | 37 | 38 | 39 | 40 | 41 | 42 | 43 | 44 | 45 | 46 | 47 | 48 | 49 | 50 | 51 | 52 | 53 | 54 | 55 | 56 | 57 | 58 | 59 | 60 | 61 | 62 | 63 | 64 | 65 | 66 |
| | | | | | | | | | W | Y | Q | Q | R | P | G | K | A | P | K | L | L | I | S | | | | | | | | | |
| | | | | | | | | | | | | | K | | | R | | | | | | | Y | | | | | | | | | |
| CDR L1 | | | | | | | | | | | | | I | | | E | | | | | | | F | CDR L2 | | | | | | | | |
| | | | | | | | | | | | | | | | | T | | | | | | | H | | | | | | | | | |
| | | | | | | | | | | | | | | | | M | | | | | | | | | | | | | | | | |
| | | | | | | | | | | | | | | | | Q | | | | | | | | | | | | | | | | |

| 51 | 52 | 53 | 54 | 55 | 56 | 57 | 58 | 59 | 60 | 61 | 62 | 63 | 64 | 65 | 66 | 67 | 68 | | | 69 | 70 | 71 | 72 | 73 | 74 | 75 | 76 | 77 | 78 | 79 | 80 | 81 |
|---|---|---|---|---|---|---|---|---|---|---|---|---|---|---|---|---|---|---|---|---|---|---|---|---|---|---|---|---|---|---|---|---|
| 67 | 68 | 69 | 70 | 71 | 72 | 73 | 74 | 75 | 76 | 77 | 78 | 79 | 80 | 81 | 82 | 83 | 84 | 85 | 86 | 87 | 88 | 89 | 90 | 91 | 92 | 93 | 94 | 95 | 96 | 97 | 98 | 99 |
| CDR L2 | | | | | | G | V | P | S | R | F | S | G | S | G | S | G | . | . | T | D | F | T | F | T | I | S | S | L | Q | P | E |
| | | | | | | | | | | | | | | | | | | | | | | | | L | | | | | | | | |

| 82 | 83 | 84 | 85 | 86 | 87 | 88 | 89 | 90 | 91 | 92 | 93 | 94 | 95 | 95a | 95b | 95c | 95d | 95e | 95f | | | | | | | | | | | | | |
|---|---|---|---|---|---|---|---|---|---|---|---|---|---|---|---|---|---|---|---|---|---|---|---|---|---|---|---|---|---|---|---|---|
| 100 | 101 | 102 | 103 | 104 | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 | 131 | 132 |
| D | F | A | V | Y | Y | C | | | | | | | | | | | | | | | | | | | | | | | | | | |
| | | | T | | | | | | | | | | | | | | | | | | | | | | | | | | | | | |
| | | | S | | | | | | | | | | | | | | | | | | | CDR L3 | | | | | | | | | | |
| | | | G | | | | | | | | | | | | | | | | | | | | | | | | | | | | | |
| | | | I | | | | | | | | | | | | | | | | | | | | | | | | | | | | | |

| | | | | 96 | 97 | 98 | 99 | 100 | 101 | 102 | 103 | 104 | 105 | 106 | 107 | 108 |
|---|---|---|---|---|---|---|---|---|---|---|---|---|---|---|---|---|
| 133 | 134 | 135 | 136 | 137 | 138 | 139 | 140 | 141 | 142 | 143 | 144 | 145 | 146 | 147 | 148 | 149 |
| CDR L3 | | | | | | F | G | Q | G | T | K | V | E | I | K | R |

Tab. 23: Vκ3 Family Light Chain Framework Scaffold

| 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 | 27 | | 27a | 27b | 27c | 27d | 27e |
|---|---|---|---|---|---|---|---|---|---|---|---|---|---|---|---|---|---|---|---|---|---|---|---|---|---|---|---|---|---|---|---|---|
| 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 | 27 | 28 | 29 | 30 | 31 | 32 | 33 |
| E | X | X | L | T | Q | S | P | G | X | L | X | L | S | P | G | E | X | A | X | L | S | C | R | CDR L1 | | | | | | | | |
| | T | T | | | | | | | I | | Y | | | | | | S | | A | | | | | | | | | | | | | |
| | I | V | | | | | | | T | | S | | | | | | R | | T | | | | | | | | | | | | | |

| 27f | | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | | | | | | | | |
|---|---|---|---|---|---|---|---|---|---|---|---|---|---|---|---|---|---|---|---|---|---|---|---|---|---|---|---|---|---|---|---|---|
| 34 | 35 | 36 | 37 | 38 | 39 | 40 | 41 | 42 | 43 | 44 | 45 | 46 | 47 | 48 | 49 | 50 | 51 | 52 | 53 | 54 | 55 | 56 | 57 | 58 | 59 | 60 | 61 | 62 | 63 | 64 | 65 | 66 |
| CDR L1 | | | | | | | | | W | Y | Q | Q | K | P | G | Q | A | P | R | L | L | M | Y | CDR L2 | | | | | | | | |
| | | | | | | | | | | | | | | | | | | | | | | I | | | | | | | | | | |

| 51 | 52 | 53 | 54 | 55 | 56 | 57 | 58 | 59 | 60 | 61 | 62 | 63 | 64 | 65 | 66 | 67 | 68 | | | 69 | 70 | 71 | 72 | 73 | 74 | 75 | 76 | 77 | 78 | 79 | 80 | 81 |
|---|---|---|---|---|---|---|---|---|---|---|---|---|---|---|---|---|---|---|---|---|---|---|---|---|---|---|---|---|---|---|---|---|
| 67 | 68 | 69 | 70 | 71 | 72 | 73 | 74 | 75 | 76 | 77 | 78 | 79 | 80 | 81 | 82 | 83 | 84 | 85 | 86 | 87 | 88 | 89 | 90 | 91 | 92 | 93 | 94 | 95 | 96 | 97 | 98 | 99 |
| | | | | | | G | I | P | D | R | F | S | G | S | G | S | G | | | T | D | F | T | L | T | I | N | R | L | E | P | E |
| CDR L2 | | | | | | | T | | | | | | | | | | | | | | | | | | | | S | | | | | |
| | | | | | | | V | | | | | | | | | | | | | | | | | | | | | | | | | |

| 82 | 83 | 84 | 85 | 86 | 87 | 88 | 89 | 90 | 91 | 92 | 93 | 94 | 95 | 95a | 95b | 95c | 95d | 95e | 95f | | | | | | | | | | | | | |
|---|---|---|---|---|---|---|---|---|---|---|---|---|---|---|---|---|---|---|---|---|---|---|---|---|---|---|---|---|---|---|---|---|
| 100 | 101 | 102 | 103 | 104 | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 | 131 | 132 |
| D | S | A | A | Y | Y | C | | | | | | | | | | | | | | | CDR L3 | | | | | | | | | | | |
| | Y | | L | | | | | | | | | | | | | | | | | | | | | | | | | | | | | |
| | F | | V | | | | | | | | | | | | | | | | | | | | | | | | | | | | | |

| | | | | 96 | 97 | 98 | 99 | 100 | 101 | 102 | 103 | 104 | 105 | 106 | 107 | 108 |
|---|---|---|---|---|---|---|---|---|---|---|---|---|---|---|---|---|
| 133 | 134 | 135 | 136 | 137 | 138 | 139 | 140 | 141 | 142 | 143 | 144 | 145 | 146 | 147 | 148 | 149 |
| CDR L3 | | | | | | F | G | G | G | T | K | L | E | I | K | R |

Tab. 24: VL1 Family Light Chain Framework Scaffold

| 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 | 27 | | 27a | 27b | 27c | 27d | 27e |
|---|---|---|---|---|---|---|---|---|---|---|---|---|---|---|---|---|---|---|---|---|---|---|---|---|---|---|---|---|---|---|---|---|
| 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 | 27 | 28 | 29 | 30 | 31 | 32 | 33 |
| X | X | V | X | T | Q | X | . | P | S | X | S | G | X | P | G | Q | R | V | T | I | S | C | S | | | | | | | | | |
| L | S | | V | | | S | | | | A | | | T | | | | | | | | | | | | | | | | | | | |
| Q | A | | L | | | E | | | | V | | | S | | | | | | | | | | | CDR L1 | | | | | | | | |
| S | P | | M | | | P | | | | | | | A | | | | | | | | | | | | | | | | | | | |
| E | I | | | | | | | | | | | | | | | | | | | | | | | | | | | | | | | |
| | Y | | | | | | | | | | | | | | | | | | | | | | | | | | | | | | | |

| 27f | | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | | | | | | | | |
|---|---|---|---|---|---|---|---|---|---|---|---|---|---|---|---|---|---|---|---|---|---|---|---|---|---|---|---|---|---|---|---|---|
| 34 | 35 | 36 | 37 | 38 | 39 | 40 | 41 | 42 | 43 | 44 | 45 | 46 | 47 | 48 | 49 | 50 | 51 | 52 | 53 | 54 | 55 | 56 | 57 | 58 | 59 | 60 | 61 | 62 | 63 | 64 | 65 | 66 |
| | | | | | | | | | W | Y | Q | X | L | P | G | T | A | P | X | L | L | I | Y | D | | | | | | | | |
| | | | | | | | | | | | | H | | | | | | | T | | | | | | CDR L2 | | | | | | | |
| | | | | | | | | | | | | Q | | | | | | | K | | | | | | | | | | | | | |
| CDR L1 | | | | | | | | | | | | | | | | | | | S | | | | | | | | | | | | | |
| | | | | | | | | | | | | | | | | | | | N | | | | | | | | | | | | | |
| | | | | | | | | | | | | | | | | | | | Q | | | | | | | | | | | | | |
| | | | | | | | | | | | | | | | | | | | P | | | | | | | | | | | | | |

| 51 | 52 | 53 | 54 | 55 | 56 | 57 | 58 | 59 | 60 | 61 | 62 | 63 | 64 | 65 | 66 | 67 | 68 | | | 69 | 70 | 71 | 72 | 73 | 74 | 75 | 76 | 77 | 78 | 79 | 80 | 81 |
|---|---|---|---|---|---|---|---|---|---|---|---|---|---|---|---|---|---|---|---|---|---|---|---|---|---|---|---|---|---|---|---|---|
| 67 | 68 | 69 | 70 | 71 | 72 | 73 | 74 | 75 | 76 | 77 | 78 | 79 | 80 | 81 | 82 | 83 | 84 | 85 | 86 | 87 | 88 | 89 | 90 | 91 | 92 | 93 | 94 | 95 | 96 | 97 | 98 | 99 |
| N | N | Q | R | P | S | G | V | P | D | R | F | S | G | S | X | S | G | . | . | T | S | A | S | L | X | I | S | G | L | Q | S | E |
| | | | | | | | | | | | | | | | R | | | | | | | | | | T | | | | | | | |
| CDR L2 | | | | | | | | | | | | | | | Q | | | | | | | | | | G | | | | | | | |
| | | | | | | | | | | | | | | | K | | | | | | | | | | D | | | | | | | |
| | | | | | | | | | | | | | | | | | | | | | | | | | A | | | | | | | |

| 82 | 83 | 84 | 85 | 86 | 87 | 88 | 89 | 90 | 91 | 92 | 93 | 94 | 95 | 95a | 95b | 95c | 95d | 95e | 95f | | | | | | | | | | | | | |
|---|---|---|---|---|---|---|---|---|---|---|---|---|---|---|---|---|---|---|---|---|---|---|---|---|---|---|---|---|---|---|---|---|
| 100 | 101 | 102 | 103 | 104 | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 | 131 | 132 |
| D | E | A | X | Y | Y | C | | | | | | | | | | | | | | | | | | | | | | | | | | |
| | | | V | | | | | | | | | | | | | | | | | | | | | | | | | | | | | |
| | | | D | | | | | | | | | | | | | | | | | | | CDR L3 | | | | | | | | | | |
| | | | T | | | | | | | | | | | | | | | | | | | | | | | | | | | | | |
| | | | H | | | | | | | | | | | | | | | | | | | | | | | | | | | | | |
| | | | E | | | | | | | | | | | | | | | | | | | | | | | | | | | | | |

| | | | | 96 | 97 | 98 | 99 | 100 | 101 | 102 | 103 | 104 | 105 | 106 | 107 | 108 |
|---|---|---|---|---|---|---|---|---|---|---|---|---|---|---|---|---|
| 133 | 134 | 135 | 136 | 137 | 138 | 139 | 140 | 141 | 142 | 143 | 144 | 145 | 146 | 147 | 148 | 149 |
| CDR L3 | | | | | | F | G | G | G | T | K | L | T | V | L | G |

**EXAMPLE 9: Generation of scFvs with Improved Solubility**

In this example, a structural modeling and sequence analysis based approach was used to identify mutations in scFv framework regions that result in improved solubility.

a) Structural analysis

The 3D structure of the ESBA105 scFv was modeled using the automated protein structure homology-modeling server, accessible via the ExPASy web server. The structure was analyzed according to the relative surface accessible to the solvent (rSAS) and residues were classified as follows: (1) Exposed for residues showing a rSAS ≥ 50%; and (2) partially exposed for residues with a 50% ≤ rSAS ≥ 25%. Hydrophobic residues with an rSAS ≥ 25% were considered as hydrophobic patches. To validate the solvent accessible area of each hydrophobic patch found, calculations were done from 27 PDB files with high homology to ESBA105 and a resolution higher than 2.7 Å. The average rSAS and standard deviation were calculated for the hydrophobic patches and examined in detail for each of them (see Table 25).

Table 25: Assessment of the hydrophobic patches.

| Residue | Domain | Surface exposed to the solvent % | STDE % | rSAS | Sequence Variability | VH/Antigen Interface | VH/VL Interface | VH/CH Interface | |
|---|---|---|---|---|---|---|---|---|---|
| 2 | VH | 23.06 | 19.26 | 10-25% | 10-25% | >0-20% | >0-20% | 0 | |
| 4 | VH | 0.66 | 1.26 | 0-10% | 0-10% | 0 | | 0 | |
| 5 | VH | 61.85 | 12.96 | 50-75% | 10-25% | 0 | >0-20% | 0 | |
| 12 | VH | 70.27 | 9.17 | 50-75% | 10-25% | 0 | 0 | 60-80% | |
| 103 | VH | 35.85 | 5.85 | 25-50% | 10-25% | 0 | >0-2% | >0-2% | |
| 144 | VH | 62.17 | 7.82 | 50-75% | 10-25% | 0 | 0 | >0-2% | |
| 15 | VL | 49.59 | 9.77 | 25-50% | 10-25% | 0 | 0 | 0 | |
| 147 | VL | 31.19 | 23.32 | 25-50% | 10-25% | 0 | 0 | 60-80% | |

Column 1, residue position in AHo's numbering system. Column 2, Domain for the position indicated in column 1. Column 3, Average solvent accessible area calculations from 27 PDB files. Column 4, Standard deviations of column 3. Columns 5 to 9, Structural role of the hydrophobic patches retrieved from AHo's.

Most of the hydrophobic patches identified in ESBA105 corresponded to the variable-constant domain (VH/CH) interface. This correlated with previous findings of solvent exposed hydrophobic residues in a scFv format (Nieba et al., 1997). Two of the hydrophobic patches (VH 2 and VH 5) also contributed to the VL-VH interaction and were therefore excluded from subsequent analysis.

b) Design of solubility mutations

A total of 122 VL and 137 VH sequences were retrieved from Annemarie Honegger's antibody web-page (www.bioc.uzh.ch/antibody). The sequences originally corresponded to 393 antibody structures in Fv or Fab format extracted from the Protein Data Bank (PDB) (www.rcsb.org/pdb/home/home.do). Sequences were used for the analysis regardless of species or subgroup in order to increase the probability of finding alternative amino acids with higher hydrophilicity than the native residue. Sequences having more than 95% identity to any other sequence within the database were excluded to reduce bias. The sequences were aligned and analyzed for residues frequency. Sequence analysis tools and algorithms were applied to identify and select hydrophilic mutations to disrupt the hydrophobic patches in ESBA105. The sequences were aligned following AHo's numbering system for immunoglobulin variable domain (Honegger and Plückthun 2001). The analysis was constrained to the framework regions.

The residues frequency, f(r), for each position, i, in the customized database was calculated by the number of times that particular residue is observed within the data set divided by the total number of sequences. In a first step, the frequency of occurrence of the different amino-acids was calculated for each hydrophobic patch. The residue frequency for each hydrophobic patch identified in ESBA105 was analyzed from the customized database described above. Table 26 reports the residue frequency at the hydrophobic patches divided by the totality of the residues present in the database.

Table 26. Residue frequency of 259 sequences from mature antibodies in a scFv or Fab format for the hydrophobic patches identified in ESBA105

| Residue | VH 4 | VH 12 | VH 103 | VH 144 | VL15 | VL 147 |
|---|---|---|---|---|---|---|
| **A** | 0.23046215 | 0 | 0 | 0 | 3.8647343 | 0.176821923 |
| **C** | 0 | 0 | 0 | 0 | 0 | 0 |
| **D** | 0 | 0 | 0 | 0 | 0 | 0 |
| **E** | 0 | 0 | 0 | 0 | 0 | 0 |
| **F** | 0.483091787 | 0 | 0.483091787 | 0 | 0 | 0 |
| **G** | 0 | 0 | 0 | 0 | 0 | 0 |
| **H** | 0 | 0 | 0 | 0 | 0 | 0 |
| **I** | 0 | 2.415458937 | 9.661835749 | 0 | 5.314009662 | 70.38834951 |
| **K** | 0 | 0 | 0 | 0 | 0 | 0 |
| **L** | 96.61835749 | 89.85507246 | 7.246376812 | 27.0531401 | 45.89371981 | 15.53398058 |
| **M** | 0 | 0 | 10.62801932 | 1.93236715 | 0 | 0.970873786 |
| **N** | 0 | 0 | 0 | 0 | 0 | 0 |
| **P** | *0.966183575* | 0 | 0 | *0.966183575* | *21.73913043* | *0.485436893* |
| **Q** | 0 | 0 | 0 | 0.483091787 | 0 | 0 |
| **R** | 0 | 0 | 7.246376812 | 0 | 0 | 0 |
| **S** | 0 | 0.966183575 | 0 | 18.84057971 | 0 | 0 |
| **T** | 0 | 0 | 15.4589372 | 50.72463768 | 0.966183575 | 0 |
| **V** | 1.93236715 | 6.763285024 | 49.27536232 | 0 | 22.22222222 | 12.62135922 |
| **W** | 0 | 0 | 0 | 0 | 0 | 0 |
| **Y** | 0 | 0 | 0 | 0 | 0 | 0 |
| Column 1, Residue type. Columns 2 to 5, relative frequency of residues for the hydrophobic patches in the heavy chain. Column 6 and 7, relative frequency of residues for the hydrophobic patches in the light chain | | | | | | |

In the second step the frequency of hydrophilic residues at the hydrophobic patches was used to design the solubility mutations by selecting the most abundant hydrophilic residue at each hydrophobic patch. Table 27 reports the solubility mutants identitied using this approach. The hydrophobicity of the parental and mutant residues were calculated as average hydrophobicity of values published in several papers and expressed in function of the level of exposure of the side chain to the solvent.

Table 27. Different solubility mutations introduced in ESBA105 to disrupt the hydrophobic patches

| Residue | Domain | Surface exposed to the solvent % | Parental residue | Hydophobicity of parental residue | Solubility mutation | Hydophobicity of mutations |
|---|---|---|---|---|---|---|
| 4 | VH | 0.66 | L | 85.2 | A | 42.7 |
| 12 | VH | 70.27 | V | 73.2 | S | 28 |
| 103 | VH | 35.85 | V | 73.2 | T | 32.8 |
| 144* | VH | 62.17 | V | 73.2 | S | 28 |
| 15 | VL | 49.59 | V | 73.2 | T | 32.8 |
| 147 | VL | 31.19 | L | 85.2 | A | 42.7 |

*The hydrophobic patch at position 144 was exchanged not by the most abundant hydrophilic residue in the database but for Ser since this was already contained in the CDR's donor of ESBA105.
Column 1, residue position in AHo's numbering system. Column 2, Domain for the position indicated in column 1. Column 3, Average solvent accessible area calculations from 27 PDB files. Column 4, parental residues in ESBA105. Column 5, Average hydrophobicities of column 4, retrieved from AHo's. Columns 6, Most abundant hydrophilic residue at the position indicated in column 1. Average hydrophobicity of column 6 retrieved from AHo's.

c) Testing of Solubility ESBA105 Variants

The solubility mutations were introduced alone or in multiple combinations and tested for refolding yield, expression, activity and stability and aggregation patterns. Table 28 shows the various combinations of solubility mutations introduced in each ESBA 105 optimized variant based on potential contribution to solubility and the level of risk that the mutation would alter antigen binding.

Table 28: Design of solubility variants for ESBA105.

| Hydrophobic surface residue | Domain | Parental residue | Mutants** | | | |
|---|---|---|---|---|---|---|
| | | | Opt 1_0 | Opt 0_2 | Opt 1_2 | Opt 2_4 |
| 15 | VL | V | X | | X | X |
| 147* | VL | V | | | | X |
| 4* | VH | L | | | | X |
| 12 | VH | V | | X | X | X |
| 103* | VH | V | | | | X |
| 144 | VH | L | | X | X | X |

*Tested separately in a second round
**The underscore separates the number of mutations contained in the light and the heavy chain respectively.
Column 1, residue position in AHo's numbering system. Column 2, Domain for the position indicated in column 1. Column 3, Parental residue in ESBA105 at the different hydrophobic patches. Column 4, Different variants containing solubility mutations at the positions indicated,

i. Solubility measurements

Maximal solubilities of ESBA105 and variants were determined by measuring the protein concentration in the supernatants of centrifugated PEG-Protein mixtures. A starting concentration of 20mg/ml was mixed 1:1 with PEG solutions ranging from 30 to 50% saturation. These conditions were chosen based on the solubility profile observed for the wild-type ESBA105 after empirical determination of linear dependence of Log S versus Peg concentration (% w/v). Solubility curves of several examples of variant ESBA105 that exhibited superior solubility are depicted in Figure 15. A complete list of solubility values is also provided in Table 29.

Table 29. Estimated maximal solubility and activity of the mutants in comparison with the parental ESBA05.

| Molecule | E105 | E105 Opt 1_0 | E105 Opt 0_2 | E105 Opt 1_2 | E105 VH V103T | E105 VL V147A |
|---|---|---|---|---|---|---|
| INTERCEPT | 1.956 | 2.228 | 2.179 | 2.163 | 2.223 | 2.047 |
| Maximal solubility | 90.36 | 169.04 | 151.01 | 145.55 | 167.11 | 111.43 |
| Activity relative to ESBA105 | 1 | 1.4 | 1.5 | 1.5 | 1.2 | 2 |

ii. Thermostability Measurements

Thermostability measurements for the parental ESBA105 and the solubility follow ups were performed using FT-IR ATR spectroscopy. The molecules were thermochallenged to a broad range of temperatures (25 to 95°C). The denaturation profile was obtained by applying a Fourier transformation to the interferogram signals (see Figure 16). The denaturation profiles were used to approximate midpoints of the thermal unfolding transitions (TM) for every ESBA105 variant applying the Boltzmann sigmoidal model (Table 30).

Table 30: Midpoints of the thermal unfolding transitions (TM) for every solubility variant.

| | ESBA105 | E105 Opt1.0 | E105 Opt1.2 | E105 Opt0.2 | E105 VH V103T | E105 VL V147A |
|---|---|---|---|---|---|---|
| Boltzmann sigmoidal | | | | | | |
| Best-fit values | | | | | | |
| BOTTOM | 0.3604 | -0.405 | 0.7032 | 0.4516 | 0.4691 | -0.6873 |
| TOP | 100.4 | 99.3 | 98.84 | 99.04 | 99.2 | 99.16 |
| **V50** | **61.53** | **59.91** | **59.39** | **60.86** | **62.08** | **55.89** |
| SLOPE | 2.935 | 2.886 | 3.117 | 2.667 | 2.682 | 3.551 |
| Std. Error | | | | | | |
| BOTTOM | 0.5206 | 0.3471 | 0.6652 | 0.4953 | 0.3938 | 0.4754 |
| TOP | 0.5361 | 0.3266 | 0.6116 | 0.4891 | 0.4167 | 0.3714 |
| V50 | 0.1047 | 0.06658 | 0.1328 | 0.0949 | 0.07811 | 0.0919 |
| SLOPE | 0.09039 | 0.05744 | 0.1146 | 0.08199 | 0.06751 | 0.08235 |
| 95% Confidence Intervals | | | | | | |
| BOTTOM | -0.7432 to 1.464 | -1.141 to 0.3309 | -0.7071 to 2.114 | -0.5984 to 1.502 | -0.3658 to 1.304 | -1.695 to 0.3206 |
| TOP | 99.25 to 101.5 | 98.61 to 99.99 | 97.54 to 100.1 | 98.01 to 100.1 | 98.32 to 100.1 | 98.38 to 99.95 |
| V50 | 61.31 to 61.75 | 59.77 to 60.06 | 59.11 to 59.67 | 60.66 to 61.06 | 61.91 to 62.24 | 55.70 to 56.09 |
| SLOPE | 2.743 to 3.127 | 2.764 to 3.007 | 2.874 to 3.360 | 2.494 to 2.841 | 2.539 to 2.825 | 3.376 to 3.725 |
| Goodness of Fit | | | | | | |
| Degrees of Freedom | 16 | 16 | 16 | 16 | 16 | 16 |
| $R^2$ | 0.9993 | 0.9997 | 0.999 | 0.9994 | 0.9996 | 0.9996 |
| Absolute Sum of Squares | 26.18 | 10.8 | 37.2 | 24 | 16.14 | 15.11 |
| Sy.x | 1.279 | 0.8217 | 1.525 | 1.225 | 1.004 | 0.9719 |

iii. Aggregation measurements

ESBA105 and its solubility variants were also analyzed on a time-dependent test to assess degradation and aggregation behavior. For this purpose soluble proteins (20 mg/ml) were incubated at an elevated temperature (40°C) in phosphate buffers at pH6.5. Control samples were kept at -80°C. The samples were analyzed after an incubation period of two weeks for degradation (SDS-PAGE) and aggregation (SEC). This allowed for the discarding of variants that were prone to degradation (see Figure 17) or which exhibited a tendency to form soluble or insoluble aggregates (see Table 31).

Table 31: Insoluble aggregation measurements.

| Protein | Protein loss (Insoluble aggregates) |
|---|---|
| ESBA 105 | 1.14% |
| ESBA 105 Opt 1_0 | 8.17% |
| ESBA 105 Opt 0_2 | 4.45% |
| ESBA 105 Opt 1_2 | 46.60% |
| ESBA 105 VH V103T | -1.95% |

iv. Expression and refolding of solubility variants

The solubility mutants were also tested for expression and refolding yield relative to the parent ESBA105 molecule. The results of these studies are shown in Table 31.

Table 31. Expression and refolding of solubility variants.

| | Hydrophobic surface residue | | | | | | | Expression relative, to ESBA105 | Refolding Yield mg/L |
|---|---|---|---|---|---|---|---|---|---|
| | VH | | | | VL | | | | |
| ESBA 105 | L4 | V12 | V103 | L144 | V15 | F52 | V147 | 1.0 | 34 |
| Opt 1.0 | | | | | T | | | 1.15 | 12.5 |
| Opt 0_2 | | S | | S | | | | 1.10 | 35 |
| Opt 1_2 | | S | | S | T | | | 0.96 | 44 |
| Opt 2_4 | A | S | T | S | T | | A | 1.20 | not producible |
| VH L4A | | | | | | | | 1.0 | not producible |
| VH V103T | | | T | | | | | 1.1 | 55 |
| VL V147A | | | | | | | A | 1.2 | 20 |

Although all the hydrophilic solubility mutants exhibited improved solubility in comparison to the parental ESBA105 molecule, only some of these molecules exhibited suitable for other biophysical properties. For example, many variants had a reduced thermostability and/or refolding yield relative to the parental ESBA105 molecule. In particular, hydrophilic replacement at position VL147 severely diminished stability. Solubility mutations that did not significantly affect thermal stability were therefore combined and subjected to further thermal stress to confirm their properties.

Three mutants containing a combination of four different solubility mutations (Opt1.0, Opt0.2 and VH:V103T) significantly improved the solubility of ESBA105 without affecting reproducibility, activity or thermal stability. However, a mutant having the combined mutations of Opt1.0 and Opt0.2 in ESBA105 (Opt 1_2) exhibited an increased amount of insoluble aggregates after incubation for 2 weeks at 40° C (see Table 29). This might be explained by the role of the Val at position VL 15 in a beta sheet turn, since Val has the greatest beta sheet propensity of all amino acid. This result demonstrated that a single solubility mutation at position VL 15 is tolerated, but not in combination with solubility mutants that disrupt other hydrophobic patches. Therefore, the mutations contained in Opt0_2 and VH:V103T were selected as best performers to improve solubility properties of scFv molecules.

**EXAMPLE 10: Generation of scFvs enhanced solubility and stability**

ESBA105 variants identified by solubility design were further optimized by substitution with stabilizing mutations identified by Quality Control (QC) assay. A total of 4 constructs were created which contained between 1 and 3 of the solubility mutations identified in Example 9 above, in combination with all stabilizing mutations found in QC 7.1 and 15.2 (i.e., D31N and V83E in the VL domain and V78A, K43 and F67L in the VH domain). All optimized constructs yielded more soluble protein than a wild-type scFv (see Table 33). The best construct consistently exhibited a greater than 2-fold increase in solubility over wild-type. Neither the activity nor the stability of the scFv molecules was significantly impacted by the combination of stabilizing and solubility enhancing mutations.

Table 33: ScFvs with optimized solubility and stability

| Protein | VL/VH Mutations | FTIR Tm (°C) | PEG solubility (mg/ml) | Activity relative to E105 | kD |
|---|---|---|---|---|---|
| QC7.1D-N-15.2 | VL: D31N; V83E VH: V78A; K43R; F67L | 69.0 | 90 | 1.7 | $9.06 \times 10^{-10}$ |
| QC7.1D-N-15.2 VH V103T | VL: D31N; V83E VH: V78A; K43R; F67L; V103T | 68.9 | 106 | 1.5 | $8.79 \times 10^{-10}$ |
| QC7.1D-N-15.2 Opt 0_2 | VL: D31N; V83E VH: V12S; V78A; K43R; F67L; L144S | 66.6 | 121 | 1.2 | $8.12 \times 10^{-10}$ |
| QC7.1D-N-15.2 VH V103T Opt 0_2 | VL: D31N; V83E VH: V12S; V78A; K43R; F67L; V103T; L144S | 67.3 | 186 | 1.5 | $1.34 \times 10^{-9}$ |

The solubility values for all 4 variants were used to deconvolute the contribution each mutation to the solubility of the scFv. All mutations appeared to contribute to the solubility of the scFv in an additive manner even though several of these residues are relatively close to one another both in primary sequence and within the 3D structure. The analysis indicated that a combination of three solubility-enhancing mutations in the VH domain (V12S, L144S, V103T (or V103S)) account for ~60% of scFv solubility. Since hydrophobic patches are conserved in the variable domains of all immunobinders, this optimal combination of mutations can be used to improve the solubility of virtually any scFv or other immunobinder molecule.

## Claims

1. A method of engineering an immunobinder, the immunobinder comprising (i) a heavy chain variable region, or fragment thereof, the heavy chain variable region comprising $V_H$ framework residues and/or (ii) a light chain variable region, or fragment thereof, the light chain variable region comprising $V_L$ framework residues, the method comprising:

    A) selecting one or more amino acid positions within the $V_H$ framework residues, the $V_L$ framework residues or the $V_H$ and $V_L$ framework residues for mutation; and
    B) mutating the one or more amino acid positions selected for mutation,

        a) wherein if the one or more heavy chain amino acid positions selected for mutation are of a human VH3 family heavy chain variable region, the mutating comprises one or more substitutions selected from the group consisting of:

            (i) glutamine (Q) at amino acid position 1 using AHo or Kabat numbering system;
            (ii) glutamine (Q) at amino acid position 6 using AHo or Kabat numbering system;
            (iii) threonine (T) or alanine (A) at amino acid position 7 using AHo or Kabat numbering system;
            (iv) alanine (A), valine (V), or phenylalanine (F) at amino acid position 89 using AHo numbering system (amino acid position 78 using Kabat numbering system); and
            (v) arginine (R), glutamine (Q), isoleucine (I), leucine (L), methionine (M) or phenylalanine (F) at amino acid position 103 using AHo numbering system (amino acid position 89 using Kabat numbering);

        b) wherein if the one or more heavy chain amino acid positions selected for mutation are of a human VH1a family heavy chain variable region, the mutating comprises one or more substitutions selected from the group consisting of:

            (i) glutamic acid (E) at amino acid position 1 using AHo or Kabat numbering system;
            (ii) glutamic acid (E) at amino acid position 6 using AHo or Kabat numbering system;
            (iii) leucine (L) at amino acid position 12 using AHo numbering system (amino acid position 11 using Kabat numbering system);

(iv) methionine (M) at amino acid position 13 using AHo numbering system (amino acid position 12 using Kabat numbering system):
(v) glutamic acid (E) or glutamine (Q) at amino acid position 14 using AHo numbering system (amino acid position 13 using Kabat numbering system);
(vi) leucine (L) at amino acid position 19 using AHo numbering system (amino acid position 18 using Kabat numbering system);
(vii) isoleucine (I) at amino acid position 21 using AHo numbering system (amino acid position 20 using Kabat numbering system);
(viii) phenylalanine (F), serine (S), histidine (H) or aspartic acid (D) at amino acid position 90 using AHo numbering system (amino acid position 79 using Kabat numbering system);
(ix) aspartic acid (D) or glutamine (Q) at amino acid position 92 using AHo numbering system (amino acid position 81 using Kabat numbering system);
(x) glycine (G), asparagine (N) or threonine (T) at amino acid position 95 using AHo numbering system (amino acid position 82b using Kabat numbering system); and
(xi) threonine (T), alanine (A), proline (P) or phenylalanine (F) at amino acid position 98 using AHo numbering (amino acid position 84 using Kabat numbering);

c) wherein if the one or more heavy chain amino acid positions selected for mutation are of a human VH1b family heavy chain variable region, the mutating comprises one or more substitutions selected from the group consisting of:

(i) glutamic acid (E) at amino acid position 1 using AHo or Kabat numbering system;
(ii) threonine (T), proline (P), valine (V) or aspartic acid (D) at amino acid position 10 using AHo numbering system (amino acid position 9 using Kabat numbering system);
(iii) leucine (L) at amino acid position 12 using AHo numbering system (amino acid position 11 using Kabat numbering system);
(iv) valine (V), arginine (R), glutamine (Q) or methionine (M) at amino acid position 13 using AHo numbering system (amino acid position 12 using Kabat numbering system):
(v) glutamic acid (E), arginine (R) or methionine (M) at amino acid position 14 using AHo numbering system (amino acid position 13 using Kabat numbering system);
(vi) arginine (R), threonine (T), or asparagine (N) at amino acid position 20 using AHo numbering system (amino acid position 19 using Kabat numbering system);
(vii) isoleucine (I), phenylalanine (F), or leucine (L) at amino acid position 21 using AHo numbering system (amino acid position 20 using Kabat numbering system);
(viii) lysine (K) at amino acid position 45 using AHo numbering system (amino acid position 38 using Kabat numbering system);
(ix) threonine (T), proline (P), valine (V) or arginine (R) at amino acid position 47 using AHo numbering system (amino acid position 40 using Kabat numbering system);
(x) lysine (K), histidine (H) or glutamic acid (E) at amino acid position 50 using AHo numbering system (amino acid position 43 using Kabat numbering system);
(xi) isoleucine (I) at amino acid position 55 using AHo numbering (amino acid position 48 using Kabat numbering);
(xii) lysine (K) at amino acid position 77 using AHo numbering (amino acid position 66 using Kabat numbering);
(xiii) alanine (A), leucine (L) or isoleucine (I) at amino acid position 78 using AHo numbering system (amino acid position 67 using Kabat numbering system);
(xiv) glutamic acid (E), threonine (T) or alanine (A) at amino acid position 82 using AHo numbering system (amino acid position 71 using Kabat numbering system);
(xv) threonine (T), serine (S) or leucine (L) at amino acid position 86 using AHo numbering system (amino acid position 75 using Kabat numbering system);
(xvi) aspartic acid (D), asparagine (N) or glycine (G) at amino acid position 87 using AHo numbering system (amino acid position 76 using Kabat numbering system); and
(xvii) asparagine (N) or serine (S) at amino acid position 107 using AHo numbering system (amino acid position 93 using Kabat numbering system);

d) wherein if the one or more light chain amino acid positions selected for mutation are of a human Vκ1 family light chain variable region, the mutating comprises one or more substitutions selected from the group consisting of:

(i) glutamic acid (E) or isoleucine (I) at amino acid position 1 using AHo or Kabat numbering system;
(ii) valine (V) or isoleucine (I) at amino acid position 3 using AHo or Kabat numbering system;
(iii) valine (V), leucine (L) or isoleucine (I) at amino acid position 4 using AHo or Kabat numbering system;
(iv) glutamine (Q) at amino acid position 24 using AHo or Kabat numbering system;
(v) arginine (R) or isoleucine (I) at amino acid position 47 using AHo numbering system (amino acid position 39 using Kabat numbering system);
(vi) arginine (R), glutamic acid (E) threonine (T), methionine (M) or glutamine (Q) at amino acid position 50 using AHo numbering system (amino acid position 42 using Kabat numbering system);
(vii) histidine (H), serine (S) or phenylalanine (F) at amino acid position 57 using AHo numbering system (amino acid position 49 using Kabat numbering system);
(viii) phenylalanine (F) at amino acid position 91 using AHo numbering system (amino acid position 73 using Kabat numbering system); and
(ix) valine (V), serine (S), glycine (G) or isoleucine (I) at amino acid position 103 using AHo numbering system (amino acid position 85 using Kabat numbering system);

e) wherein if the one or more light chain amino acid positions selected for mutation are of a human Vκ3 family light chain variable region, the mutating comprises one or more substitutions selected from the group consisting of:

(i) threonine (T) at amino acid position 2 using AHo or Kabat numbering system;
(ii) threonine (T) at amino acid position 3 using AHo or Kabat numbering system;
(iii) isoleucine (I) at amino acid position 10 using AHo or Kabat numbering system;
(iv) tyrosine (Y) at amino acid position 12 using AHo or Kabat numbering system;
(v) serine (S) at amino acid position 18 using AHo or Kabat numbering system;
(vi) alanine (A) at amino acid position 20 using AHo or Kabat numbering system;
(vii) methionine (M) at amino acid position 56 using AHo numbering system (amino acid position 48 using Kabat numbering system);
(viii) valine (V) or threonine (T) at amino acid position 74 using AHo numbering system (amino acid position 58 using Kabat numbering system);
(ix) asparagine (N) at amino acid position 94 using AHo numbering system (amino acid position 76 using Kabat numbering system);
(x) tyrosine (Y) or serine (S) at amino acid position 101 using AHo numbering system (amino acid position 83 using Kabat numbering system); and
(xi) leucine (L) or alanine (A) at amino acid position 103 using AHo numbering (amino acid position 85 using Kabat numbering);

f) wherein if the one or more light chain amino acid positions selected for mutation are of a human Vλ1 family light chain variable region, the mutating comprises one or more substitutions selected from the group consisting of:

(i) leucine (L), serine (S) or glutamic acid (E) at amino acid position 1 using AHo or Kabat numbering system;
(ii) alanine (A), proline (P), isoleucine (I) or tyrosine (Y) at amino acid position 2 using AHo or Kabat numbering system;
(iii) valine (V) or methionine (M) at amino acid position 4 using AHo or Kabat numbering system;
(iv) glutamic acid (E) at amino acid position 7 using AHo or Kabat numbering system;
(v) alanine (A) at amino acid position 11 using AHo or Kabat numbering system;
(vi) threonine (T) or serine (S) at amino acid position 14 using AHo or Kabat numbering system;
(vii) histidine (H) at amino acid position 46 using AHo numbering system (amino acid position 38 using Kabat numbering system);
(viii) threonine (T), serine (S), asparagine (N), glutamine (Q) or proline (P) at amino acid position 53 using AHo numbering system (amino acid position 45 using Kabat numbering system);
(ix) arginine (R) or glutamine (Q) at amino acid position 82 using AHo numbering system (amino acid position 66 using Kabat numbering system);
(x) glycine (G), threonine (T) or aspartic acid (D) at amino acid position 92 using AHo numbering system (amino acid position 74 using Kabat numbering system); and
(xi) valine (V), threonine (T), histidine (H) or glutamic acid (E) at amino acid position 103 using AHo numbering (amino acid position 85 using Kabat numbering).

2. The method of claim 1, wherein the mutating further comprises one or more heavy chain substitutions selected from the group consisting of:

(i) serine (S) at amino acid position 12 using AHo or Kabat;
(ii) serine (S) at amino acid position 103 using AHo numbering (amino acid position 85 using Kabat numbering); and
(iii) serine (S) or threonine (T) at amino acid position 144 using AHo numbering (amino acid position 103 using Kabat numbering).

3. The method of claim 1, wherein the mutating further comprises the following heavy chain substitutions:

(i) serine (S) at amino acid position 12 using AHo or Kabat;
(ii) serine (S) at amino acid position 103 using AHo numbering (amino acid position 85 using Kabat numbering); and
(iii) serine (S) or threonine (T) at amino acid position 144 using AHo numbering (amino acid position 103 using Kabat numbering).

4. The method of any one of claims 1-3, wherein the immunobinder is a scFv antibody, a full-length immunoglobulin, a Fab fragment, a Dab or a Nanobody.

5. The method of claim 1, wherein if the one or more heavy chain amino acid positions selected for mutation are of a VH3 family heavy chain variable region, the mutating comprises one or more substitutions selected from the group consisting of:

(i) glutamine (Q) at amino acid position 1 using AHo or Kabat numbering system;
(ii) glutamine (Q) at amino acid position 6 using AHo or Kabat numbering system;
(iii) threonine (T) at amino acid position 7 using AHo or Kabat numbering system;
(iv) valine (V) at amino acid position 89 using AHo numbering system (amino acid position 78 using Kabat numbering system); and
(v) leucine (L) at amino acid position 103 using AHo numbering system (amino acid position 89 using Kabat numbering).

6. The method of claim 1, wherein if the one or more heavy chain amino acid positions selected for mutation are of a VH1a family heavy chain variable region, the mutating comprises one or more substitutions selected from the group consisting of:

(i) glutamic acid (E) at amino acid position 1 using AHo or Kabat numbering system;
(ii) glutamic acid (E) at amino acid position 6 using AHo or Kabat numbering system;
(iii) leucine (L) at amino acid position 12 using AHo numbering system (amino acid position 11 using Kabat numbering system);
(iv) methionine (M) at amino acid position 13 using AHo numbering system (amino acid position 12 using Kabat numbering system):
(v) glutamic acid (E) at amino acid position 14 using AHo numbering system (amino acid position 13 using Kabat numbering system);
(vi) leucine (L) at amino acid position 19 using AHo numbering system (amino acid position 18 using Kabat numbering system);
(vii) isoleucine (I) at amino acid position 21 using AHo numbering system (amino acid position 20 using Kabat numbering system);
(viii) phenylalanine (F), serine (S), histidine (H) or aspartic acid (D) at amino acid position 90 using AHo numbering system (amino acid position 79 using Kabat numbering system);
(ix) aspartic acid (D) at amino acid position 92 using AHo numbering system (amino acid position 81 using Kabat numbering system);
(x) glycine (G) at amino acid position 95 using AHo numbering system (amino acid position 82b using Kabat numbering system); and
(xi) phenylalanine (F) at amino acid position 98 using AHo numbering (amino acid position 84 using Kabat numbering);

7. The method of claim 1, wherein if the one or more heavy chain amino acid positions selected for mutation are of a VH1b family heavy chain variable region, the mutating comprises one or more substitutions selected from the group consisting of:

(i) glutamic acid (E) at amino acid position 1 using AHo or Kabat numbering system;
(ii) threonine (T), at amino acid position 10 using AHo numbering system (amino acid position 9 using Kabat numbering system);
(iii) leucine (L) at amino acid position 12 using AHo numbering system (amino acid position 11 using Kabat numbering system);
(iv) valine (V), at amino acid position 13 using AHo numbering system (amino acid position 12 using Kabat numbering system):
(v) arginine (R) at amino acid position 14 using AHo numbering system (amino acid position 13 using Kabat numbering system);
(vi) asparagine (N) at amino acid position 20 using AHo numbering system (amino acid position 19 using Kabat numbering system);
(vii) leucine (L) at amino acid position 21 using AHo numbering system (amino acid position 20 using Kabat numbering system);
(viii) lysine (K) at amino acid position 45 using AHo numbering system (amino acid position 38 using Kabat numbering system);
(ix) arginine (R) at amino acid position 47 using AHo numbering system (amino acid position 40 using Kabat numbering system);
(x) lysine (K) at amino acid position 50 using AHo numbering system (amino acid position 43 using Kabat numbering system);
(xi) isoleucine (I) at amino acid position 55 using AHo numbering (amino acid position 48 using Kabat numbering);
(xii) lysine (K) at amino acid position 77 using AHo numbering (amino acid position 66 using Kabat numbering);
(xiii) alanine (A) at amino acid position 78 using AHo numbering system (amino acid position 67 using Kabat numbering system);
(xiv) glutamic acid (E) at amino acid position 82 using AHo numbering system (amino acid position 71 using Kabat numbering system);
(xv) threonine (T) at amino acid position 86 using AHo numbering system (amino acid position 75 using Kabat numbering system);
(xvi) asparagine (N) at amino acid position 87 using AHo numbering system (amino acid position 76 using Kabat numbering system); and
(xvii) asparagine (N) at amino acid position 107 using AHo numbering system (amino acid position 93 using Kabat numbering system).

**8.** The method of claim 1, wherein if the one or more light chain amino acid positions selected for mutation are of a Vκ1 family light chain variable region, the mutating comprises one or more substitutions selected from the group consisting of:

(i) glutamic acid (E) at amino acid position 1 using AHo or Kabat numbering system;
(ii) valine (V) at amino acid position 3 using AHo or Kabat numbering system;
(iii) leucine (L) at amino acid position 4 using AHo or Kabat numbering system;
(iv) glutamine (Q) at amino acid position 24 using AHo or Kabat numbering system;
(v) arginine (R) at amino acid position 47 using AHo numbering system (amino acid position 39 using Kabat numbering system);
(vi) arginine (R), glutamic acid (E) threonine (T), methionine (M) or glutamine (Q) at amino acid position 50 using AHo numbering system (amino acid position 42 using Kabat numbering system);
(vii) serine (S) at amino acid position 57 using AHo numbering system (amino acid position 49 using Kabat numbering system); and
(viii) phenylalanine (F) at amino acid position 91 using AHo numbering system (amino acid position 73 using Kabat numbering system);
(ix) valine (V) at amino acid position 103 using AHo numbering system (amino acid position 85 using Kabat numbering system).

**9.** The method of claim 1, wherein if the one or more light chain amino acid positions selected for mutation are of a Vκ3 family light chain variable region, the mutating comprises one or more substitutions selected from the group consisting of:

(i) threonine (T) at amino acid position 2 using AHo or Kabat numbering system;
(ii) threonine (T) at amino acid position 3 using AHo or Kabat numbering system;
(iii) isoleucine (I) at amino acid position 10 using AHo or Kabat numbering system;
(iv) tyrosine (Y) at amino acid position 12 using AHo or Kabat numbering system;
(v) serine (S) at amino acid position 18 using AHo or Kabat numbering system;
(vi) alanine (A) at amino acid position 20 using AHo or Kabat numbering system;
(vii) methionine (M) at amino acid position 56 using AHo numbering system (amino acid position 48 using Kabat numbering system);
(viii) threonine (T) at amino acid position 74 using AHo numbering system (amino acid position 58 using Kabat numbering system);
(ix) asparagine (N) at amino acid position 94 using AHo numbering system (amino acid position 76 using Kabat numbering system);
(x) serine (S) at amino acid position 101 using AHo numbering system (amino acid position 83 using Kabat numbering system); and
(xi) alanine (A) at amino acid position 103 using AHo numbering (amino acid position 85 using Kabat numbering).

**10.** The method of claim 1, wherein if the one or more light chain amino acid positions selected for mutation are of a Vλ1 family light chain variable region, the mutating comprises one or more substitutions selected from the group consisting of:

(i) leucine (L) at amino acid position 1 using AHo or Kabat numbering system;
(ii) proline (P) at amino acid position 2 using AHo or Kabat numbering system;
(iii) valine (V) at amino acid position 4 using AHo or Kabat numbering system;
(iv) serine (S) at amino acid position 7 using AHo or Kabat numbering system;
(v) alanine (A) at amino acid position 11 using AHo or Kabat numbering system;
(vi) threonine (T) at amino acid position 14 using AHo or Kabat numbering system;
(vii) histidine (H) at amino acid position 46 using AHo numbering system (amino acid position 38 using Kabat numbering system);
(viii) threonine (T), serine (S), asparagine (N), glutamine (Q) or proline (P) at amino acid position 53 using AHo numbering system (amino acid position 45 using Kabat numbering system);
(ix) arginine (R) at amino acid position 82 using AHo numbering system (amino acid position 66 using Kabat numbering system);
(x) threonine (T) at amino acid position 92 using AHo numbering system (amino acid position 74 using Kabat numbering system); and
(xi) valine (V) at amino acid position 103 using AHo numbering (amino acid position 85 using Kabat numbering).

**11.** The method of any one of claims 5-10, wherein the mutating further comprises one or more heavy chain substitutions selected from the group consisting of:

(i) serine (S) at amino acid position 12 using AHo or Kabat;
(ii) serine (S) at amino acid position 103 using AHo numbering (amino acid position 85 using Kabat numbering); and
(iii) serine (S) or threonine (T) at amino acid position 144 using AHo numbering (amino acid position 103 using Kabat numbering).

**12.** The method of any one of claims 5-10, wherein the mutating further comprises the following heavy chain substitutions:

(i) serine (S) at amino acid position 12 using AHo or Kabat;
(ii) serine (S) at amino acid position 103 using AHo numbering (amino acid position 85 using Kabat numbering); and
(iii) serine (S) or threonine (T) at amino acid position 144 using AHo numbering (amino acid position 103 using Kabat numbering).

**13.** The method of any one of claims 8-12, wherein the immunobinder is a scFv antibody, a full-length immunoglobulin, a Fab fragment, a Dab or a Nanobody.

**14.** A method of engineering an immunobinder, the immunobinder comprising heavy chain CDR1, CDR2 and CDR3 sequences, the method comprising inserting the heavy chain CDR1, CDR2 and CDR3 sequences into a heavy chain framework scaffold, the heavy chain framework scaffold comprising an amino acid sequence as shown in Figure 9 (SEQ ID NO:1), Figure 10 (SEQ ID NO:2), Figure 11 (SEQ ID NO:3), SEQ ID NO: 7, SEQ ID NO:8, or SEQ ID NO:9, with the proviso that the amino acid sequence is not the germline consensus sequence..

**15.** The method of claim 14, wherein the heavy chain framework scaffold comprises an amino acid sequence as shown in Figure 9 (SEQ ID NO: 1).

**16.** The method of claim 14, wherein the heavy chain framework scaffold comprises an amino acid sequence as shown in Figure 10 (SEQ ID NO:2).

**17.** The method of claim 14, wherein the heavy chain framework scaffold comprises an amino acid sequence as shown in Figure 11 (SEQ ID NO:3).

**18.** A method of engineering an immunobinder, the immunobinder comprising light chain CDR1, CDR2 and CDR3 sequences, the method comprising inserting the light chain CDR1, CDR2 and CDR3 sequences into a light chain framework scaffold, the light chain framework scaffold comprising an amino acid sequence as shown in Figure 12 (SEQ ID NO:4), Figure 13 (SEQ ID NO:5), Figure 14 (SEQ ID NO:6), SEQ ID NO: 10, SEQ ID NO: 11, or SEQ ID NO: 12, with the proviso that the amino acid sequence is not the germline consensus sequence.

**19.** The method of claim 18, wherein the light chain framework scaffold comprises an amino acid sequence as shown in Figure 12 (SEQ ID NO:4).

**20.** The method of claim 18, wherein the light chain framework scaffold comprises an amino acid sequence as shown in Figure 13 (SEQ ID NO:5).

**21.** The method of claim 18, wherein the light chain framework scaffold comprises an amino acid sequence as shown in Figure 14 (SEQ ID NO:6).

**22.** The method of any one of claims 18-21, wherein the immunobinder is a scFv antibody.

**23.** An isolated heavy chain framework scaffold comprising an amino acid sequence as shown in Figure 9 (SEQ ID NO: 1), Figure 10 (SEQ ID NO:2) or Figure 11 (SEQ ID NO:3) with the proviso that the amino acid sequence is not the germline consensus sequence.

**24.** An isolated light chain framework scaffold comprising an amino acid sequence as shown in Figure 12 (SEQ ID NO:4), Figure 13 (SEQ ID NO:5) or Figure 14 (SEQ ID NO:6) with the proviso that the amino acid sequence is not the germline consensus sequence.

## Patentansprüche

**1.** Verfahren zur Herstellung eines Immunbinders, wobei der Immunbinder umfasst (i) die variable Region einer schweren Kette, oder ein Fragment davon, wobei die variable Region der schweren Kette $V_H$-Frameworkreste umfasst, und/oder (ii) die variable Region einer leichten Kette, oder ein Fragment davon, wobei die variable Region der leichten Kette $V_L$-Frameworkreste umfasst, und wobei das Verfahren umfasst:

A) Auswählen einer oder mehrerer Aminosäurepositionen innerhalb der $V_H$-Frameworkreste, der $V_L$-Frameworkreste oder der $V_H$- und $V_L$-Frameworkreste zur Mutation; und
B) Mutieren der zur Mutation ausgewählten einen oder mehreren Aminosäurepositionen,

a) wobei dann, wenn die eine oder mehreren zur Mutation ausgewählten Aminosäurepositionen der schweren Kette von der variablen Region einer schweren Kette der menschlichen VH3-Familie stammen, das Mutieren eine oder mehrere Substitutionen umfasst, welche ausgewählt sind aus der Gruppe bestehend aus:

(i) Glutamin (Q) an Aminosäureposition 1 unter Verwendung des AHo- oder Kabat-Nummerierungssystems;
(ii) Glutamin (Q) an Aminosäureposition 6 unter Verwendung des AHo- oder Kabat-Nummerierungssystems;
(iii) Threonin (T) oder Alanin (A) an Aminosäureposition 7 unter Verwendung des AHo- oder Kabat-Nummerierungssystems;
(iv) Alanin (A), Valin (V), oder Phenylalanin (F) an Aminosäureposition 89 unter Verwendung des AHo-Nummerierungssystems (Aminosäureposition 78 unter Verwendung des Kabat-Nummerierungssystems); und
(v) Arginin (R), Glutamin (Q), Isoleucin (I), Leucin (L), Methionin (M) oder Phenylalanin (F) an Aminosäureposition 103 unter Verwendung des AHo-Nummerierungssystems (Aminosäureposition 89 unter Verwendung der Kabat-Nummerierung);

b) wobei dann, wenn die eine oder mehreren zur Mutation ausgewählten Aminosäurepositionen der schweren Kette von der variablen Region einer schweren Kette der menschlichen VH1a-Familie stammen, das Mutieren eine oder mehreren Substitutionen umfasst, welche ausgewählt sind aus der Gruppe bestehend aus:

(i) Glutaminsäure (E) an Aminosäureposition 1 unter Verwendung des AHo- oder Kabat-Nummerierungssystems;
(ii) Glutaminsäure (E) an Aminosäureposition 6 unter Verwendung des AHo- oder Kabat-Nummerierungssystems;
(iii) Leucin (L) an Aminosäureposition 12 unter Verwendung des AHo-Nummerierungssystems (Aminosäureposition 11 unter Verwendung des Kabat-Nummerierungssystems);
(iv) Methionin (M) an Aminosäureposition 13 unter Verwendung des AHo-Nummerierungssystems (Aminosäureposition 12 unter Verwendung des Kabat-Nummerierungssystems);
(v) Glutaminsäure (E) oder Glutamin (Q) an Aminosäureposition 14 unter Verwendung des AHo-Nummerierungssystems (Aminosäureposition 13 unter Verwendung des Kabat-Nummerierungssystems);
(vi) Leucin (L) an Aminosäureposition 19 unter Verwendung des AHo-Nummerierungssystems (Aminosäureposition 18 unter Verwendung des Kabat-Nummerierungssystems);
(vii) Isoleucin (I) an Aminosäureposition 21 unter Verwendung des AHo-Nummerierungssystems (Aminosäureposition 20 unter Verwendung des Kabat-Nummerierungssystems);
(viii) Phenylalanin (F), Serin (S), Histidin (H) oder Asparaginsäure (D) an Aminosäureposition 90 unter Verwendung des AHo-Nummerierungssystems (Aminosäureposition 79 unter Verwendung des Kabat-Nummerierungssystems);
(ix) Asparaginsäure (D) oder Glutamin (Q) an Aminosäureposition 92 unter Verwendung des AHo-Nummerierungssystems (Aminosäureposition 81 unter Verwendung des Kabat-Nummerierungssystems);
(x) Glycin (G), Asparagin (N) oder Threonin (T) an Aminosäureposition 95 unter Verwendung des AHo-Nummerierungssystems (Aminosäureposition 82b unter Verwendung des Kabat-Nummerierungssystems); und
(xi) Threonin (T), Alanin (A), Prolin (P) oder Phenylalanin (F) an Aminosäureposition 98 unter Verwendung der AHo-Nummerierung (Aminosäureposition 84 unter Verwendung der Kabat-Nummerierung);

c) wobei dann, wenn die eine oder mehreren zur Mutation ausgewählten Aminosäurepositionen der schweren Kette von der variablen Region einer schweren Kette der menschlichen VH1b-Familie stammen, das Mutieren eine oder mehrere Substitutionen umfasst, welche ausgewählt sind aus der Gruppe bestehend aus:

(i) Glutaminsäure (E) an Aminosäureposition 1 unter Verwendung des AHo- oder des Kabat-Nummerierungssystems;
(ii) Threonin (T), Prolin (P), Valin (V) oder Asparaginsäure (D) an Aminosäureposition 10 unter Verwendung des AHo-Nummerierungssystems (Aminosäureposition 9 unter Verwendung des Kabat-Nummerierungssystems);
(iii) Leucin (L) an Aminosäureposition 12 unter Verwendung des AHo-Nummerierungssystems (Aminosäureposition 11 unter Verwendung des Kabat-Nummerierungssystems);
(iv) Valine (V), Arginin (R), Glutamin (Q) oder Methionin (M) an Aminosäureposition 13 unter Verwendung des AHo-Nummerierungssystems (Aminosäureposition 12 unter Verwendung des Kabat-Nummerierungssystems):
(v) Glutaminsäure (E), Arginin (R) oder Methionin (M) an Aminosäureposition 14 unter Verwendung des AHo-Nummerierungssystems (Aminosäureposition 13 unter Ver-wendung des Kabat-Nummerierungssystems);

(vi) Arginin (R), Threonin (T), oder Asparagin (N) an Aminosäureposition 20 unter Verwendung des AHo-Nummerierungssystems (Aminosäureposition 19 unter Verwendung des Kabat-Nummerierungssystems);

(vii) Isoleucin (I), Phenylalanin (F), oder Leucin (L) an Aminosäureposition 21 unter Verwendung des AHo-Nummerierungssystems (Aminosäureposition 20 unter Ver-wendung des Kabat-Nummerierungssystems);

(viii) Lysin (K) an Aminosäureposition 45 unter Verwendung des AHo-Nummerierungssystems (Aminosäureposition 38 unter Verwendung des Kabat-Nummerierungssystems);

(ix) Threonin (T), Prolin (P), Valin (V) oder Arginin (R) an Aminosäureposition 47 unter Verwendung des AHo-Nummerierungssystems (Aminosäureposition 40 unter Ver-wendung des Kabat-Nummerierungssystems);

(x) Lysin (K), Histidin (H) oder Glutaminsäure (E) an Aminosäureposition 50 unter Verwendung des AHo-Nummerierungssystems (Aminosäureposition 43 unter Verwendung des Kabat-Nummerierungssystems);

(xi) Isoleucin (I) an Aminosäureposition 55 unter Verwendung der AHo-Nummerierung (Aminosäureposition 48 unter Verwendung der Kabat-Nummerierung);

(xii) Lysin (K) an Aminosäureposition 77 unter Verwendung der AHo-Nummerierung (Aminosäureposition 66 unter Verwendung der Kabat-Nummerierung);

(xiii) Alanin (A), Leucin (L) oder Isoleucin (I) an Aminosäureposition 78 unter Verwendung des AHo-Nummerierungssystems (Aminosäureposition 67 unter Verwendung des Kabat-Nummerierungssystems);

(xiv) Glutaminsäure (E), Threonin (T) oder Alanin (A) an Aminosäureposition 82 unter Verwendung des AHo-Nummerierungssystems (Aminosäureposition 71 unter Verwendung des Kabat-Nummerierungssystems);

(xv) Threonin (T), Serin (S) oder Leucin (L) an Aminosäureposition 86 unter Verwendung des AHo-Nummerierungssystems (Aminosäureposition 75 unter Verwendung des Kabat-Nummerierungssystems);

(xvi) Asparaginsäure (D), Asparagin (N) oder Glycin (G) an Aminosäureposition 87 unter Verwendung des AHo-Nummerierungssystems (Aminosäureposition 76 unter Verwendung des Kabat-Nummerierungssystems); und

(xvii) Asparagin (N) oder Serin (S) an Aminosäureposition 107 unter Verwendung des AHo-Nummerierungssystems (Aminosäureposition 93 unter Verwendung des Kabat-Nummerierungssystems);

d) wobei dann, wenn die eine oder mehreren zur Mutation ausgewählten Aminosäurepositionen der leichte Kette von der variablen Region einer leichten Kette der menschlichen Vκ1-Familie stammen, das Mutieren eine oder mehrere Substitutionen umfasst, welche ausgewählt sind aus der Gruppe bestehend aus:

(i) Glutaminsäure (E) oder Isoleucin (I) an Aminosäureposition 1 unter Verwendung des AHo- oder Kabat-Nummerierungssystems;

(ii) Valin (V) oder Isoleucin (I) an Aminosäureposition 3 unter Verwendung des AHo- oder Kabat-Nummerierungssystems;

(iii) Valin (V), Leucin (L) oder Isoleucin (I) an Aminosäureposition 4 unter Verwendung des AHo- oder Kabat-Nummerierungssystems;

(iv) Glutamin (Q) an Aminosäureposition 24 unter Verwendung des AHo- oder Kabat-Nummerierungssystems;

(v) Arginin (R) oder Isoleucin (I) an Aminosäureposition 47 unter Verwendung des AHo-Nummerierungssystems (Aminosäureposition 39 unter Verwendung des Kabat-Nummerierungssystems);

(vi) Arginin (R), Glutaminsäure (E) Threonin (T), Methionin (M) oder Glutamin (Q) an Aminosäureposition 50 unter Verwendung des AHo-Nummerierungssystems (Aminosäureposition 42 unter Verwendung des Kabat-Nummerierungssystems);

(vii) Histidin (H), Serin (S) oder Phenylalanin (F) an Aminosäureposition 57 unter Verwendung des AHo-Nummerierungssystems (Aminosäureposition 49 unter Verwendung des Kabat-Nummerierungssystems);

(viii) Phenylalanin (F) an Aminosäureposition 91 unter Verwendung des AHo-Nummerierungssystems (Aminosäureposition 73 unter Verwendung des Kabat-Nummerierungssystems); und

(ix) Valin (V), Serin (S), Glycin (G) oder Isoleucin (I) an Aminosäureposition 103 unter Verwendung des AHo-Nummerierungssystems (Aminosäureposition 85 unter Verwendung des Kabat-Nummerierungssystems);

e) wobei dann, wenn die eine oder mehreren zur Mutation ausgewählten Aminosäurepositionen der leichte Kette von der variablen Region einer schweren Kette der menschlichen Vκ3-Familie stammen, das Mutieren eine oder mehrere Substitutionen umfasst, welche ausgewählt sind aus der Gruppe bestehend aus:

(i) Threonin (T) an Aminosäureposition 2 unter Verwendung des AHo- oder Kabat-Nummerierungssystems;
(ii) Threonin (T) an Aminosäureposition 3 unter Verwendung des AHo- oder Kabat-Nummerierungssystems;
(iii) Isoleucin (I) an Aminosäureposition 10 unter Verwendung des AHo- oder Kabat-Nummerierungssystems;
(iv) Tyrosin (Y) an Aminosäureposition 12 unter Verwendung des AHo- oder Kabat- Nummerierungssystems;
(v) Serin (S) an Aminosäureposition 18 unter Verwendung des AHo- oder Kabat-Nummerierungssystems;
(vi) Alanin (A) an Aminosäureposition 20 unter Verwendung des AHo- oder Kabat-Nummerierungssystems;
(vii) Methionin (M) an Aminosäureposition 56 unter Verwendung des AHo-Nummerierungssystems (Aminosäureposition 48 unter Verwendung des Kabat-Nummerierungssystems);
(viii) Valin (V) oder Threonin (T) an Aminosäureposition 74 unter Verwendung des AHo-Nummerierungssystems (Aminosäureposition 58 unter Verwendung des Kabat-Nummerierungssystems);
(ix) Asparagin (N) an Aminosäureposition 94 unter Verwendung des AHo-Nummerierungssystems (Aminosäureposition 76 unter Verwendung des Kabat-Nummerierungssystems);
(x) Tyrosin (Y) oder Serin (S) an Aminosäureposition 101 unter Verwendung des AHo-Nummerierungssystems (Aminosäureposition 83 unter Verwendung des Kabat-Nummerierungssystems); und
(xi) Leucin (L) oder Alanin (A) an Aminosäureposition 103 unter Verwendung der AHo-Nummerierung (Aminosäureposition 85 unter Verwendung der Kabat-Nummerierung);

f) wobei dann, wenn die eine oder mehreren zur Mutation ausgewählten Aminosäurepositionen der leichte Kette von der variablen Region einer leichten Kette der menschlichen Vλ1-Familie stammen, das Mutieren eine oder mehrere Substitutionen umfasst, welche ausgewählt sind aus der Gruppe bestehend aus:

(i) Leucin (L), Serin (S) oder Glutaminsäure (E) an Aminosäureposition 1 unter Verwendung des AHo- oder Kabat-Nummerierungssystems;
(ii) Alanin (A), Prolin (P), Isoleucin (I) oder Tyrosin (Y) an Aminosäureposition 2 unter Verwendung des AHo- oder Kabat-Nummerierungssystems;
(iii) Valin (V) oder Methionin (M) an Aminosäureposition 4 unter Verwendung des AHo- oder Kabat-Nummerierungssystems;
(iv) Glutaminsäure (E) an Aminosäureposition 7 unter Verwendung des AHo- oder Kabat-Nummerierungssystems;
(v) Alanin (A) an Aminosäureposition 11 unter Verwendung des AHo- oder Kabat-Nummerierungssystems;
(vi) Threonin (T) oder Serin (S) an Aminosäureposition 14 unter Verwendung des AHo- oder Kabat-Nummerierungssystems;
(vii) Histidin (H) an Aminosäureposition 46 unter Verwendung des AHo-Nummerierungssystems (Aminosäureposition 38 unter Verwendung des Kabat-Nummerierungssystems);
(viii) Threonin (T), Serin (S), Asparagin (N), Glutamin (Q) oder Prolin (P) an Aminosäureposition 53 unter Verwendung des AHo-Nummerierungssystems (Aminosäureposition 45 unter Verwendung des Kabat-Nummerierungssystems);
(ix) Arginin (R) oder Glutamin (Q) an Aminosäureposition 82 unter Verwendung des AHo-Nummerierungssystems (Aminosäureposition 66 unter Verwendung des Kabat-Nummerierungssystems);
(x) Glycin (G), Threonin (T) oder Asparaginsäure (D) an Aminosäureposition 92 unter Verwendung des AHo-Nummerierungssystems (Aminosäureposition 74 unter Verwendung des Kabat-Nummerierungssystems); und
(xi) Valin (V), Threonin (T), Histidin (H) oder Glutaminsäure (E) an Aminosäureposition 103 unter Verwendung der AHo-Nummerierung (Aminosäureposition 85 unter Verwendung der Kabat-Nummerierung).

**2.** Verfahren nach Anspruch 1, wobei das Mutieren weiterhin eine oder mehrere Substitutionen in der schweren Kette umfasst, welche ausgewählt sind aus der Gruppe bestehend aus:

(i) Serin (S) an Aminosäureposition 12 unter Verwendung der AHo- oder Kabat-Nummerierung;
(ii) Serin (S) an Aminosäureposition 103 unter Verwendung der AHo-Nummerierung (Aminosäureposition 85 unter Verwendung der Kabat-Nummerierung); und
(iii) Serin (S) oder Threonin (T) an Aminosäureposition 144 unter Verwendung der AHo-Nummerierung (Aminosäureposition 103 unter Verwendung der Kabat-Nummerierung).

**3.** Verfahren nach Anspruch 1, wobei das Mutieren weiterhin die folgenden Substitutionen in der schweren Kette umfasst:

(i) Serin (S) an Aminosäureposition 12 unter Verwendung der AHo- oder Kabat-Nummerierung;
(ii) Serin (S) an Aminosäureposition 103 unter Verwendung der AHo-Nummerierung (Aminosäureposition 85 unter Verwendung der Kabat-Nummerierung); und
(iii) Serin (S) oder Threonin (T) an Aminosäureposition 144 unter Verwendung der AHo-Nummerierung (Aminosäureposition 103 unter Verwendung der Kabat-Nummerierung).

**4.** Verfahren nach einem der Ansprüche 1 bis 3, wobei der Immunbinder ein scFv-Antikörper, ein Volllängen-Immunglobulin, ein Fab-Fragment, ein Dab oder ein Nanobody ist.

**5.** Verfahren nach Anspruch 1, wobei dann, wenn die eine oder mehreren zur Mutation ausgewählten Aminosäurepositionen der schweren Kette von der variablen Region einer schweren Kette der VH3-Familie stammen, das Mutieren eine oder mehrere Substitutionen umfasst, welche ausgewählt sind aus der Gruppe bestehend aus:

(i) Glutamin (Q) an Aminosäureposition 1 unter Verwendung des AHo- oder Kabat-Nummerierungssystems;
(ii) Glutamin (Q) an Aminosäureposition 6 unter Verwendung des AHo- oder Kabat-Nummerierungssystems;
(iii) Threonin (T) an Aminosäureposition 7 unter Verwendung des AHo- oder Kabat-Nummerierungssystems;
(iv) Valin (V) an Aminosäureposition 89 unter Verwendung des AHo-Nummerierungssystems (Aminosäureposition 78 unter Verwendung des Kabat-Nummerierungssystems); und
(v) Leucin (L) an Aminosäureposition 103 unter Verwendung des AHo-Nummerierungssystems (Aminosäureposition 89 unter Verwendung der Kabat-Nummerierung).

**6.** Verfahren nach Anspruch 1, wobei dann, wenn die eine oder mehreren zur Mutation ausgewählten Aminosäurepositionen von der variablen Region einer schweren Kette der VH1a-Familie stammen, das Mutieren eine oder mehrere Substitutionen umfasst, welche ausgewählt sind aus der Gruppe bestehend aus:

(i) Glutaminsäure (E) an Aminosäureposition 1 unter Verwendung des AHo- oder Kabat-Nummerierungssystems;
(ii) Glutaminsäure (E) an Aminosäureposition 6 unter Verwendung des AHo- oder Kabat-Nummerierungssystems;
(iii) Leucin (L) an Aminosäureposition 12 unter Verwendung des AHo-Nummerierungssystems (Aminosäureposition 11 unter Verwendung des Kabat-Nummerierungssystems);
(iv) Methionin (M) an Aminosäureposition 13 unter Verwendung des AHo-Nummerierungssystems (Aminosäureposition 12 unter Verwendung des Kabat-Nummerierungssystems):
(v) Glutaminsäure (E) an Aminosäureposition 14 unter Verwendung des AHo-Nummerierungssystems (Aminosäureposition 13 unter Verwendung des Kabat-Nummerierungssystems);
(vi) Leucin (L) an Aminosäureposition 19 unter Verwendung des AHo-Nummerierungssystems (Aminosäureposition 18 unter Verwendung des Kabat-Nummerierungssystems);
(vii) Isoleucin (I) an Aminosäureposition 21 unter Verwendung des AHo-Nummerierungssystems (Aminosäureposition 20 unter Verwendung des Kabat-Nummerierungssystems);
(viii) Phenylalanin (F), Serin (S), Histidin (H) oder Asparaginsäure (D) an Aminosäureposition 90 unter Verwendung des AHo-Nummerierungssystems (Aminosäureposition 79 unter Verwendung des Kabat-Nummerierungssystems);
(ix) Asparaginsäure (D) an Aminosäureposition 92 unter Verwendung des AHo-Nummerierungssystems (Aminosäureposition 81 unter Verwendung des Kabat-Nummerierungssystems);
(x) Glycin (G) an Aminosäureposition 95 unter Verwendung des AHo-Nummerierungssystems (Aminosäureposition 82b unter Verwendung des Kabat-Nummerierungssystems); und

(xi) Phenylalanin (F) an Aminosäureposition 98 unter Verwendung der AHo-Nummerierung (Aminosäureposition 84 unter Verwendung der Kabat-Nummerierung).

**7.** Verfahren nach Anspruch 1, wobei dann, wenn die eine oder mehreren zur Mutation ausgewählten Aminosäurepositionen der schweren Kette von der variablen Region einer schweren Kette der VH1b-Familie stammen, das Mutieren eine oder mehrere Substitutionen umfasst, welche ausgewählt sind aus der Gruppe bestehend aus:

(i) Glutaminsäure (E) an Aminosäureposition 1 unter Verwendung des AHo- oder Kabat-Nummerierungssystems;
(ii) Threonin (T) an Aminosäureposition 10 unter Verwendung des AHo-Nummerierungssystems (Aminosäureposition 9 unter Verwendung des Kabat-Nummerierungssystems);
(iii) Leucin (L) an Aminosäureposition 12 unter Verwendung des AHo-Nummerierungssystems (Aminosäureposition 11 unter Verwendung des Kabat-Nummerierungssystems);
(iv) Valin (V) an Aminosäureposition 13 unter Verwendung des AHo-Nummerierungssystems (Aminosäureposition 12 unter Verwendung des Kabat-Nummerierungssystems):
(v) Arginin (R) an Aminosäureposition 14 unter Verwendung des AHo-Nummerierungssystems (Aminosäureposition 13 unter Verwendung des Kabat-Nummerierungssystems);
(vi) Asparagin (N) an Aminosäureposition 20 unter Verwendung des AHo-Nummerierungssystems (Aminosäureposition 19 unter Verwendung des Kabat-Nummerierungssystems);
(vii) Leucin (L) an Aminosäureposition 21 unter Verwendung des AHo-Nummerierungssystems (Aminosäureposition 20 unter Verwendung des Kabat-Nummerierungssystems);
(viii) Lysin (K) an Aminosäureposition 45 unter Verwendung des AHo-Nummerierungssystems (Aminosäureposition 38 unter Verwendung des Kabat-Nummerierungssystems);
(ix) Arginin (R) an Aminosäureposition 47 unter Verwendung des AHo-Nummerierungssystems (Aminosäureposition 40 unter Verwendung des Kabat-Nummerierungssystems);
(x) Lysin (K) an Aminosäureposition 50 unter Verwendung des AHo-Nummerierungssystems (Aminosäureposition 43 unter Verwendung des Kabat-Nummerierungssystems);
(xi) Isoleucin (I) an Aminosäureposition 55 unter Verwendung der AHo-Nummerierung (Aminosäureposition 48 unter Verwendung der Kabat-Nummerierung);
(xii) Lysin (K) an Aminosäureposition 77 unter Verwendung der AHo-Nummerierung (Aminosäureposition 66 unter Verwendung der Kabat-Nummerierung);
(xiii) Alanin (A) an Aminosäureposition 78 unter Verwendung des AHo-Nummerierungssystems (Aminosäureposition 67 unter Verwendung des Kabat-Nummerierungssystems);
(xiv) Glutaminsäure (E) an Aminosäureposition 82 unter Verwendung des AHo-Nummerierungssystems (Aminosäureposition 71 unter Verwendung des Kabat-Nummerierungssystems);
(xv) Threonin (T) an Aminosäureposition 86 unter Verwendung des AHo-Nummerierungssystems (Aminosäureposition 75 unter Verwendung des Kabat-Nummerierungssystems);
(xvi) Asparagin (N) an Aminosäureposition 87 unter Verwendung des AHo-Nummerierungssystems (Aminosäureposition 76 unter Verwendung des Kabat-Nummerierungssystems); und
(xvii) Asparagin (N) an Aminosäureposition 107 unter Verwendung des AHo-Nummerierungssystems (Aminosäureposition 93 unter Verwendung des Kabat-Nummerierungssystems).

**8.** Verfahren nach Anspruch 1, wobei dann, wenn die eine oder mehreren zur Mutation ausgewählten Aminosäurepositionen der leichten Kette von der variablen Region einer leichten Kette der Vλ1-Familie stammen, das Mutieren eine oder mehrere Substitutionen umfasst, welche ausgewählt sind aus der Gruppe bestehend aus:

(i) Glutaminsäure (E) an Aminosäureposition 1 unter Verwendung des AHo- oder Kabat-Nummerierungssystems;
(ii) Valin (V) an Aminosäureposition 3 unter Verwendung des AHo- oder Kabat-Nummerierungssystems;
(iii) Leucin (L) an Aminosäureposition 4 unter Verwendung des AHo- oder Kabat-Nummerierungssystems;
(iv) Glutamin (Q) an Aminosäureposition 24 unter Verwendung des AHo- oder Kabat-Nummerierungssystems;
(v) Arginin (R) an Aminosäureposition 47 unter Verwendung des AHo-Nummerierungssystems (Aminosäureposition 39 unter Verwendung des Kabat-Nummerierungssystems);
(vi) Arginin (R), Glutaminsäure (E), Threonin (T), Methionin (M) oder Glutamin (Q) an Aminosäureposition 50 unter Verwendung des AHo-Nummerierungssystems (Aminosäureposition 42 unter Verwendung des Kabat-Nummerierungssystems);
(vii) Serin (S) an Aminosäureposition 57 unter Verwendung des AHo-Nummerierungssystems (Aminosäureposition 49 unter Verwendung des Kabat-Nummerierungssystems); und

(viii) Phenylalanin (F) an Aminosäureposition 91 unter Verwendung des AHo-Nummerierungssystems (Aminosäureposition 73 unter Verwendung des Kabat-Nummerierungssystems);
(ix) Valin (V) an Aminosäureposition 103 unter Verwendung des AHo-Nummerierungssystems (Aminosäureposition 85 unter Verwendung des Kabat-Nummerierungssystems).

**9.** Verfahren nach Anspruch 1, wobei dann, wenn die eine oder mehreren zur Mutation ausgewählten Aminosäurepositionen der leichten Kette von der variablen Region einer leichten Kette der Vκ3-Familie stammen, das Mutieren eine oder mehrere Substitutionen umfasst, welche ausgewählt sind aus der Gruppe bestehend aus:

(i) Threonin (T) an Aminosäureposition 2 unter Verwendung des AHo- oder Kabat-Nummerierungssystems;
(ii) Threonin (T) an Aminosäureposition 3 unter Verwendung des AHo- oder Kabat-Nummerierungssystems;
(iii) Isoleucin (I) an Aminosäureposition 10 unter Verwendung des AHo- oder Kabat-Nummerierungssystems;
(iv) Tyrosin (Y) an Aminosäureposition 12 unter Verwendung des AHo- oder Kabat-Nummerierungssystems;
(v) Serin (S) an Aminosäureposition 18 unter Verwendung des AHo- oder Kabat-Nummerierungssystems;
(vi) Alanin (A) an Aminosäureposition 20 unter Verwendung des AHo- oder Kabat-Nummerierungssystems;
(vii) Methionin (M) an Aminosäureposition 56 unter Verwendung des AHo-Nummerierungssystems (Aminosäureposition 48 unter Verwendung des Kabat-Nummerierungssystems);
(viii) Threonin (T) an Aminosäureposition 74 unter Verwendung des AHo-Nummerierungssystems (Aminosäureposition 58 unter Verwendung des Kabat-Nummerierungssystems);
(ix) Asparagin (N) an Aminosäureposition 94 unter Verwendung des AHo-Nummerierungssystems (Aminosäureposition 76 unter Verwendung des Kabat-Nummerierungssystems);
(x) Serin (S) an Aminosäureposition 101 unter Verwendung des AHo-Nummerierungssystems (Aminosäureposition 83 unter Verwendung des Kabat-Nummerierungssystems); und
(xi) Alanin (A) an Aminosäureposition 103 unter Verwendung der AHo-Nummerierung (Aminosäureposition 85 unter Verwendung der Kabat-Nummerierung).

**10.** Verfahren nach Anspruch 1, wobei dann, wenn die eine oder mehreren zur Mutation ausgewählten Aminosäurepositionen der leichten Kette von der variablen Region einer leichten Kette der Vλ1-Familie stammen, das Mutieren eine oder mehrere Substitutionen umfasst, welche ausgewählt sind aus der Gruppe bestehend aus:

(i) Leucin (L) an Aminosäureposition 1 unter Verwendung des AHo- oder Kabat-Nummerierungssystems;
(ii) Prolin (P) an Aminosäureposition 2 unter Verwendung des AHo- oder Kabat-Nummerierungssystems;
(iii) Valin (V) an Aminosäureposition 4 unter Verwendung des AHo- oder Kabat-Nummerierungssystems;
(iv) Serin (S) an Aminosäureposition 7 unter Verwendung des AHo- oder Kabat-Nummerierungssystems;
(v) Alanin (A) an Aminosäureposition 11 unter Verwendung des AHo- oder Kabat-Nummerierungssystems;
(vi) Threonin (T) an Aminosäureposition 14 unter Verwendung des AHo- oder Kabat-Nummerierungssystems;
(vii) Histidin (H) an Aminosäureposition 46 unter Verwendung des AHo-Nummerierungssystems (Aminosäureposition 38 unter Verwendung des Kabat-Nummerierungssystems);
(viii) Threonin (T), Serin (S), Asparagin (N), Glutamin (Q) oder Prolin (P) an Aminosäureposition 53 unter Verwendung des AHo-Nummerierungssystems (Aminosäureposition 45 unter Verwendung des Kabat-Nummerierungssystems);
(ix) Arginin (R) an Aminosäureposition 82 unter Verwendung des AHo-Nummerierungssystems (Aminosäureposition 66 unter Verwendung des Kabat-Nummerierungssystems);
(x) Threonin (T) an Aminosäureposition 92 unter Verwendung des AHo-Nummerierungssystems (Aminosäureposition 74 unter Verwendung des Kabat-Nummerierungssystems); und
(xi) Valin (V) an Aminosäureposition 103 unter Verwendung des AHo-Nummerierungssystems (Aminosäureposition 85 unter Verwendung der Kabat-Nummerierung).

**11.** Verfahren nach einem der Ansprüche 5 bis 10, wobei das Mutieren weiterhin eine oder mehrere Substitutionen in der schweren Kette umfasst, welche ausgewählt sind aus der Gruppe bestehend aus:

(i) Serin (S) an Aminosäureposition 12 unter Verwendung der AHo- oder Kabat-Nummerierung;
(ii) Serin (S) an Aminosäureposition 103 unter Verwendung der AHo-Nummerierung (Aminosäureposition 85 unter Verwendung der Kabat-Nummerierung); und
(iii) Serin (S) oder Threonin (T) an Aminosäureposition 144 unter Verwendung der AHo-Nummerierung (Aminosäureposition 103 unter Verwendung der Kabat-Nummerierung).

**12.** Verfahren nach einem der Ansprüche 5 bis 10, wobei das Mutieren weiterhin die folgenden Substitutionen in der schweren Kette umfasst:

(i) Serin (S) an Aminosäureposition 12 unter Verwendung der AHo- oder Kabat-Nummerierung;
(ii) Serin (S) an Aminosäureposition 103 unter Verwendung der AHo-Nummerierung (Aminosäureposition 85 unter Verwendung der Kabat-Nummerierung); und
(iii) Serin (S) oder Threonin (T) an Aminosäureposition 144 unter Verwendung der AHo-Nummerierung (Aminosäureposition 103 unter Verwendung der Kabat-Nummerierung).

**13.** Verfahren nach einem der Ansprüche 8 bis 12, wobei der Immunbinder ein scFv-Antikörper, ein Volllängen-Immunglobulin, ein Fab-Fragment, ein Dab oder ein Nanobody ist.

**14.** Verfahren zur Herstellung eines Immunbinders, wobei der Immunbinder CDR1-, CDR2-, und CDR3-Sequenzen einer schweren Kette umfasst, wobei das Verfahren das Einfügen der CDR1-, CDR2- und CDR3-Sequenzen der schweren Kette in ein Framework-Gerüst einer schweren Kette umfasst, wobei das Framework-Gerüst der schweren Kette eine Aminosäuresequenz wie in Fig. 9 (SEQ ID NO:1), Figur 10 (SEQ ID NO:2), Fig. 11 (SEQ ID NO:3), SEQ ID NO:7, SEQ ID NO:8, oder SEQ ID NO:9 gezeigt umfasst, mit der Maßgabe, dass es sich bei der Aminosäuresequenz nicht um die Konsensussequenz der Keimbahn handelt.

**15.** Verfahren nach Anspruch 14, wobei das Framework-Gerüst der schweren Kette eine Aminosäuresequenz wie in Fig. 9 (SEQ ID NO:1) gezeigt umfasst.

**16.** Verfahren nach Anspruch 14, wobei das Framework- Gerüst der schweren Kette eine Aminosäuresequenz wie in Fig. 10 (SEQ ID NO:2) gezeigt umfasst.

**17.** Verfahren nach Anspruch 14, wobei das Framework-Gerüst der schweren Kette eine Aminosäuresequenz wie in Fig. 11 (SEQ ID NO:3) gezeigt umfasst.

**18.** Verfahren zur Herstellung eines Immunobinders, wobei der Immunbinder CDR1-, CDR2-, und CDR3-Sequenzen einer leichten Kette umfasst, wobei das Verfahren das Einfügen der CDR1-, CDR2- und CDR3-Sequenzen der leichten Kette in ein Framework-Gerüst einer leichten Kette umfasst, wobei das Framework-Gerüst der leichten Kette eine Aminosäuresequenz wie in Fig. 12 (SEQ ID NO:4), Figur 13 (SEQ ID NO:5), Fig. 14 (SEQ ID NO:6), SEQ ID NO:10, SEQ ID NO:11, oder SEQ ID NO:12 gezeigt umfasst, mit der Maßgabe, dass es sich bei der Aminosäuresequenz nicht um die Konsensussequenz der Keimbahn handelt.

**19.** Verfahren nach Anspruch 18, wobei das Framework-Gerüst der leichten Kette eine Aminosäuresequenz wie in Fig. 12 (SEQ ID NO:4) gezeigt umfasst.

**20.** Verfahren nach Anspruch 18, wobei das Framework-Gerüst der leichten Kette eine Aminosäuresequenz wie in Fig. 13 (SEQ ID NO:5) gezeigt umfasst.

**21.** Verfahren nach Anspruch 18, wobei das Framework-Gerüst der leichten Kette eine Aminosäuresequenz wie in Fig. 14 (SEQ ID NO:6) gezeigt umfasst.

**22.** Verfahren nach einem der Ansprüche 18 bis 21, wobei der Immunbinder ein scFv-Antikörper ist.

**23.** Isoliertes Framework-Gerüst einer schweren Kette umfassend eine Aminosäuresequenz wie in Fig. 9 (SEQ ID NO:1), Fig. 10 (SEQ ID NO:2) oder Fig. 11 (SEQ ID NO:3) gezeigt, mit der Maßgabe, dass es sich bei der Aminosäuresequenz nicht um die Konsensussequenz der Keimbahn handelt.

**24.** Isoliertes Framework-Gerüst einer leichten Kette umfassend eine Aminosäuresequenz wie in Fig. 12 (SEQ ID NO:4), Fig. 13 (SEQ ID NO:5), oder Fig. 14 (SEQ ID NO:6) gezeigt, mit der Maßgabe, dass es sich bei der Aminosäuresequenz nicht um die Konsensussequenz der Keimbahn handelt.

## Revendications

**1.** Procédé d'ingénierie d'un immunoliant, l'immunoliant comprenant (i) une région variable de chaîne lourde, ou son fragment, la région variable de chaîne lourde comprenant des résidus de région structurale de la $V_H$ et/ou (ii) une région variable de chaîne légère, ou son fragment, la région variable de la chaîne légère comprenant des résidus de la région structurale de la $V_L$, le procédé comprenant :

A) la sélection d'une ou plusieurs positions d'acide aminé à l'intérieur des résidus de la région structurale de la $V_H$, les résidus de la région structurale de la $V_L$ ou les résidus de la région structurale de la $V_H$ et de la $V_L$ pour mutation ; et
B) la mutation de la une ou plusieurs positions d'acide aminé sélectionnées pour la mutation,

a) où si la une ou plusieurs positions d'acide aminé de chaîne lourde sélectionnées pour mutation sont une région variable de chaîne lourde de la famille VH3 humaine, la mutation comprend une ou plusieurs substitutions sélectionnées dans le groupe constitué de :

(i) la glutamine (Q) à la position d'acide aminé 1 en utilisant le système de numérotation AHo ou Kabat ;
(ii) la glutamine (Q) à la position d'acide aminé 6 en utilisant le système de numérotation AHo ou Kabat ;
(iii) la thréonine (T) ou l'alanine (A) à la position d'acide aminé 7 en utilisant le système de numérotation AHo ou Kabat ;
(iv) l'alanine (A), la valine (V) ou la phénylalanine (F) à la position d'acide aminé 89 en utilisant le système de numérotation AHo (position d'acide aminé 78 en utilisant le système de numérotation Kabat) ; et
(v) l'arginine (R), la glutamine (Q), l'isoleucine (I), la leucine (L), la méthionine (M) ou la phénylalanine (F) à la position d'acide aminé 103 en utilisant le système de numérotation AHo (position d'acide aminé 89 en utilisant la numérotation Kabat) ;

b) où si la une ou plusieurs positions d'acide aminé de chaîne lourde sélectionnées pour la mutation sont une région variable de chaîne lourde de la famille VH1a humaine, la mutation comprend une ou plusieurs substitutions sélectionnées dans le groupe constitué de :

(i) l'acide glutamique (E) à la position d'acide aminé 1 en utilisant le système de numérotation AHo ou Kabat ;
(ii) l'acide glutamique (E) à la position d'acide aminé 6 en utilisant le système de numérotation AHo ou Kabat ;
(iii) la leucine (L) à la position d'acide aminé 12 en utilisant le système de numérotation AHo (position d'acide aminé 11 en utilisant le système de numérotation Kabat) ;
(iv) la méthionine (M) à la position d'acide aminé 13 en utilisant le système de numérotation AHo (position d'acide aminé 12 en utilisant le système de numérotation Kabat) ;
(v) l'acide glutamique (E) ou la glutamine (Q) à la position d'acide aminé 14 en utilisant le système de numérotation AHo (position d'acide aminé 13 en utilisant le système de numérotation Kabat) ;
(vi) la leucine (L) à la position d'acide aminé 19 en utilisant le système de numérotation AHo (position d'acide aminé 18 en utilisant le système de numérotation Kabat ) ;
(vii) l'isoleucine (I) à la position d'acide aminé 21 en utilisant le système de numérotation AHo (position d'acide aminé 20 en utilisant le système de numérotation Kabat);
(viii) la phénylalanine (F), la sérine (S), l'histidine (H) ou l'acide aspartique (D) à la position d'acide aminé 90 en utilisant le système de numérotation AHo (position d'acide aminé 79 en utilisant le système de numérotation Kabat) ;
(ix) l'acide aspartique (D) ou la glutamine (Q) à la position d'acide aminé 92 en utilisant le système de numérotation AHo (position d'acide aminé 81 en utilisant le système de numérotation Kabat) ;
(x) la glycine (G), l'asparagine (N) ou la thréonine (T) à la position d'acide aminé 95 en utilisant le système de numérotation AHo (position d'acide aminé 82b en utilisant le système de numérotation Kabat) ; et
(xi) la thréonine (T), l'alanine (A), la proline (P) ou la phénylalanine (F) à la position d'acide aminé 98 en utilisant le système de numérotation AHo (position d'acide aminé 84 en utilisant le système de numérotation Kabat) ;

c) où si la une ou plusieurs positions d'acide aminé de chaîne lourde sélectionnées pour mutation sont une région variable de chaîne lourde de la famille VHI1b humaine, la mutation comprend une ou plusieurs substitutions sélectionnées dans le groupe constitué de :

(i) l'acide glutamique (E) à la position d'acide aminé 1 en utilisant le système de numérotation AHo ou Kabat ;
(ii) la thréonine (T), la proline (P), la valine (V) ou l'acide aspartique (D) à la position d'acide aminé 10 en utilisant le système de numérotation AHo (position d'acide aminé 9 en utilisant le système de numérotation Kabat) ;
(iii) la leucine (L) à la position d'acide aminé 12 en utilisant le système de numérotation AHo (position d'acide aminé 11 en utilisant le système de numérotation Kabat) ;
(iv) la valine (V), l'arginine (R), la glutamine (Q) ou méthionine (M) à la position d'acide aminé 13 en utilisant le système de numérotation AHo (position d'acide aminé 12 en utilisant le système de numérotation Kabat) ;
(v) l'acide glutamique (E), l'arginine (R) ou la méthionine (M) à la position d'acide aminé 14 en utilisant le système de numérotation AHo (position d'acide aminé 13 en utilisant le système de numérotation Kabat) ;
(vi) l'arginine (R), la thréonine (T) ou l'asparagine (N) à la position d'acide aminé 20 en utilisant le système de numérotation AHo (position d'acide aminé 19 en utilisant le système de numérotation Kabat) ;
(vii) l'isoleucine (I), la phénylalanine (F) ou la leucine (L) à la position d'acide aminé 21 en utilisant le système de numérotation AHo (position d'acide aminé 20 en utilisant le système de numérotation Kabat) ;
(viii) la lysine (K) à la position d'acide aminé 45 en utilisant le système de numérotation AHo (position d'acide aminé 38 en utilisant le système de numérotation Kabat) ;
(ix) la thréonine (T), la proline (P), la valine (V) ou l'arginine (R) à la position d'acide aminé 47 en utilisant le système de numérotation AHo (position d'acide aminé 40 en utilisant le système de numérotation Kabat) ;
(x) la lysine (K), l'histidine (H) ou l'acide glutamique (E) à la position d'acide aminé 50 en utilisant le système de numérotation AHo (position d'acide aminé 43 en utilisant le système de numérotation Kabat) ;
(xi) l'isoleucine (I) à la position d'acide aminé 55 en utilisant le système de numérotation AHo (position d'acide aminé 48 en utilisant le système de numérotation Kabat) ;
(xii) la lysine (K) à la position d'acide aminé 77 en utilisant le système de numérotation AHo (position d'acide aminé 66 en utilisant le système de numérotation Kabat) ;
(xiii) l'alanine (A), la leucine (L) ou l'isoleucine (I) à la position d'acide aminé 78 en utilisant le système de numérotation AHo (position d'acide aminé 67 en utilisant le système de numérotation Kabat) ;
(xiv) l'acide glutamique (E), la thréonine (T) ou l'alanine (A) à la position d'acide aminé 82 en utilisant le système de numérotation AHo (position d'acide aminé 71 en utilisant le système de numérotation Kabat) ;
(xv) la thréonine (T), la sérine (S) ou la leucine (L) à la position d'acide aminé 85 en utilisant le système de numérotation AHo (position d'acide aminé 75 en utilisant le système de numérotation Kabat) ;
(xvi) l'acide aspartique (D), l'asparagine (N) ou la glycine (G) à la position d'acide aminé 87 en utilisant le système de numérotation AHo (position d'acide aminé 76 en utilisant le système de numérotation Kabat) ;
(xvii) l'asparagine (N) ou la sérine (S) à la position d'acide aminé 107 en utilisant le système de numérotation AHo (position d'acide aminé 93 en utilisant le système de numérotation Kabat) ;

d) où si la une ou plusieurs positions d'acide aminé de chaîne légère sélectionnées pour mutation sont une région variable de chaîne légère de la famille Vκ1 humaine, la mutation comprend une ou plusieurs substitutions sélectionnées dans le groupe constitué de :

(i) l'acide glutamique (E) ou l'isoleucine (I) à la position d'acide aminé 1 en utilisant le système de numérotation AHo ou Kabat ;
(ii) la valine (V) ou l'isoleucine (I) à la position d'acide aminé 3 en utilisant le système de numérotation AHo ou ion Kabat ;
(iii) la valine (V), la leucine (L) ou l'isoleucine (I) à la position d'acide aminé 4 en utilisant le système de numérotation AHo ou Kabat ;
(iv) la glutamine (Q) à la position d'acide aminé 24 en utilisant le système de numérotation AHo ou Kabat ;
(v) l'arginine (R) ou l'isoleucine (I) à la position d'acide aminé 47 en utilisant le système de numérotation AHo (position d'acide aminé 39 en utilisant le système de numérotation Kabat) ;
(vi) l'arginine (R), l'acide glutamique (E), la thréonine (T), la méthionine (M) ou la glutamine (Q) à la position d'acide aminé 50 en utilisant le système de numérotation AHo (position d'acide aminé 42 en utilisant le système de numérotation Kabat) ;
(vii) l'histidine (H), la sérine (S) ou la phénylalanine (F) à la position d'acide aminé 57 en utilisant le système de numérotation AHo (position d'acide aminé 49 en utilisant le système de numérotation Kabat) ;
(viii) la phénylalanine (F) à la position d'acide aminé 91 en utilisant le système de numérotation AHo (position d'acide aminé 73 en utilisant le système de numérotation Kabat) ; et
(ix) la valine (V), la sérine (S), la glycine (G) ou l'isoleucine (I) à la position d'acide aminé 103 en utilisant le système de numérotation AHo (position d'acide aminé 85 en utilisant le système de numérotation Kabat) ;

e) où si la une ou plusieurs positions d'acide aminé de chaîne légère sélectionnées pour mutation sont une région variable de chaîne légère de la famille Vκ3 humaine, la mutation comprend une ou plusieurs substitutions sélectionnées dans le groupe constitué de :

(i) la thréonine (T) à la position d'acide aminé 2 en utilisant le système de numérotation AHo ou Kabat ;
(ii) la thréonine (T) à la position d'acide aminé 3 en utilisant le système de numérotation AHo ou Kabat ;
(iii) l'isoleucine (I) à la position d'acide aminé 10 en utilisant le système de numérotation AHo ou Kabat ;
(iv) la tyrosine (Y) à la position d'acide aminé 12 en utilisant le système de numérotation AHo ou Kabat ;
(v) la sérine (S) à la position d'acide aminé 18 en utilisant le système de numérotation AHo ou Kabat ;
(vi) l'alanine (A) à la position d'acide aminé 20 en utilisant le système de numérotation AHo ou Kabat ;
(vii) la méthionine (M) à la position d'acide aminé 56 en utilisant le système de numérotation AHo (position d'acide aminé 48 en utilisant le système de numérotation Kabat) ;
(viii) la valine (V) ou la thréonine (T) à la position d'acide aminé 74 en utilisant le système de numérotation AHo (position d'acide aminé 48 en utilisant le système de numérotation Kabat) ;
(ix) l'asparagine (N) à la position d'acide aminé 94 en utilisant le système de numérotation AHo (position d'acide aminé 76 en utilisant le système de numérotation Kabat) ;
(x) la tyrosine (Y) ou la sérine à la position d'acide aminé 101 en utilisant le système de numérotation AHo (position d'acide aminé 83 en utilisant le système de numérotation Kabat) ;
(xi) la leucine (L) ou l'alanine (A) à la position d'acide aminé 103 en utilisant le système de numérotation AHo (position d'acide aminé 85 en utilisant le système de numérotation Kabat) ;

f) où si la une ou plusieurs positions d'acide aminé de chaîne légère sélectionnées pour mutation sont une région variable de chaîne légère de la famille Vλ1 humaine, la mutation comprend une ou plusieurs substitutions sélectionnées dans le groupe constitué de :

(i) la leucine (L), la sérine (S) ou l'acide glutamique (E) à la position d'acide aminé 1 en utilisant le système de numérotation AHo ou Kabat ;
(ii) l'alanine (A), la proline (P), l'isoleucine (I) ou la tyrosine (Y) à la position d'acide aminé 2 en utilisant le système de numérotation AHo ou Kabat ;
(iii) la valine (V) ou la méthionine (M) à la position d'acide aminé 4 en utilisant le système de numérotation AHo ou Kabat ;
(iv) l'acide glutamique (E) à la position d'acide aminé 7 en utilisant le système de numérotation AHo ou Kabat ;
(v) l'alanine (A) à la position d'acide aminé 11 en utilisant le système de numérotation AHo ou Kabat ;
(vi) la thréonine (T) ou la sérine (S) à la position d'acide aminé 14 en utilisant le système de numérotation AHo ou Kabat ;
(vii) l'histidine (H) à la position d'acide aminé 46 en utilisant le système de numérotation AHo (position d'acide aminé 38 en utilisant le système de numérotation Kabat) ;
(viii) la thréonine (T), la sérine (S), l'asparagine (N), la glutamine (Q) ou la proline (P) à la position d'acide aminé 53 en utilisant le système de numérotation AHo (position d'acide aminé 45 en utilisant le système de numérotation Kabat) ;
(ix) l'arginine (R) ou la glutamine (Q) à la position d'acide aminé 82 en utilisant le système de numérotation AHo (position d'acide aminé 66 en utilisant le système de numérotation Kabat) ;
(x) la glycine (G), la thréonine (T) ou l'acide aspartique (D) ou la sérine à la position d'acide aminé 92 en utilisant le système de numérotation AHo (position d'acide aminé 74 en utilisant le système de numérotation Kabat) ; et
(xi) la valine (V), la thréonine (T), l'histidine (H) or l'acide glutamique (E) à la position d'acide aminé 103 en utilisant le système de numérotation AHo (position d'acide aminé 85 en utilisant le système de numérotation Kabat) ;

2. Procédé selon la revendication 1, où la mutation comprend en outre une ou plusieurs substitutions de chaîne lourde sélectionnées dans le groupe constitué de :

(i) la sérine (S) à la position d'acide aminé 12 en utilisant AHo ou Kabat ;
(ii) la sérine (S) à la position d'acide aminé 103 en utilisant la numérotation AHo (position d'acide aminé 85 en utilisant la numérotation Kabat) ; et
(iii) la sérine (S) ou la thréonine (T) à la position d'acide aminé 144 en utilisant la numérotation AHo (position d'acide aminé 103 en utilisant la numérotation Kabat).

3. Procédé selon la revendication 1, où la mutation comprend en outre les substitutions suivantes de chaîne lourde :

(i) la sérine (S) à la position d'acide aminé 12 en utilisant AHo ou Kabat ;
(ii) la sérine (S) à la position d'acide aminé 103 en utilisant la numérotation AHo (position d'acide aminé 85 en utilisant la numérotation Kabat) ; et
(iii) la sérine (S) ou la thréonine (T) à la position d'acide aminé 144 en utilisant la numérotation AHo (position d'acide aminé 103 en utilisant la numérotation Kabat).

4. Procédé selon l'une quelconque des revendications 1 à 3, où l'immunoliant est un anticorps scFv, une immunoglobuline de pleine longueur, un fragment Fab, un Dab ou un Nanocorps.

5. Procédé selon la revendication 1, où si la une ou plusieurs positions d'acide aminé de chaîne lourde sélectionnées pour mutation sont une région variable de chaîne lourde de la famille VH3, la mutation comprend une ou plusieurs substitutions sélectionnées dans le groupe constitué de :

(i) la glutamine (Q) à la position d'acide aminé 1 en utilisant AHo ou Kabat ;
(ii) la glutamine (Q) à la position d'acide aminé 6 en utilisant la numérotation AHo ou Kabat ;
(iii) la thréonine (T) à la position d'acide aminé 7 en utilisant la numérotation AHo ou Kabat ;
(iv) la valine (V) à la position d'acide aminé 89 en utilisant la numérotation AHo (position d'acide aminé 78 en utilisant la numérotation Kabat) ; et
(v) la leucine (L) à la position d'acide aminé 103 en utilisant la numérotation AHo (position d'acide aminé 89 en utilisant la numérotation Kabat).

6. Procédé selon la revendication 1, où si la une ou plusieurs positions d'acide aminé de chaîne lourde sélectionnées pour mutation sont une région variable de chaîne lourde de la famille VH1a, la mutation comprend une ou plusieurs substitutions sélectionnées dans le groupe constitué de :

(i) l'acide glutamique (E) à la position d'acide aminé 1 en utilisant AHo ou Kabat ;
(ii) l'acide glutamique (E) à la position d'acide aminé 6 en utilisant la numérotation AHo ou Kabat ;
(iii) la leucine (L) à la position d'acide aminé 12 en utilisant la numérotation AHo (position d'acide aminé 11 en utilisant la numérotation Kabat) ;
(iv) la méthionine (M) à la position d'acide aminé 12 en utilisant la numérotation AHo (position d'acide aminé 11 en utilisant la numérotation Kabat) ;
(v) l'acide glutamique (E) à la position d'acide aminé 14 en utilisant la numérotation AHo (position d'acide aminé 13 en utilisant la numérotation Kabat) ;
(vi) la leucine (L) à la position d'acide aminé 19 en utilisant la numérotation AHo (position d'acide aminé 18 en utilisant la numérotation Kabat) ;
(vii) l'isoleucine (I) à la position d'acide aminé 21 en utilisant la numérotation AHo (position d'acide aminé 20 en utilisant la numérotation Kabat) ;
(viii) la phénylalanine (F), la sérine (S), l'histidine (H) ou l'aspect aspartique (D) à la position d'acide aminé 90 en utilisant la numérotation AHo (position d'acide aminé 79 en utilisant la numérotation Kabat) ;
(ix) l'acide aspartique (D) à la position d'acide aminé 92 en utilisant la numérotation AHo (position d'acide aminé 81 en utilisant la numérotation Kabat) ;
(x) la glycine (G) à la position d'acide aminé 95 en utilisant la numérotation AHo (position d'acide aminé 82b en utilisant la numérotation Kabat) ; et
(xi) la phénylalanine (F) à la position d'acide aminé 98 en utilisant la numérotation AHo (position d'acide aminé 84 en utilisant la numérotation Kabat).

7. Procédé selon la revendication 1, où si la une ou plusieurs positions d'acide aminé de chaîne lourde sélectionnées pour mutation sont une région variable de chaîne lourde de la famille VH1b, la mutation comprend une ou plusieurs substitutions sélectionnées dans le groupe constitué de :

(i) l'acide glutamique (E) à la position d'acide aminé 1 en utilisant AHo ou Kabat ;
(ii) la thréonine (T) à la position d'acide aminé 10 en utilisant la numérotation AHo (position d'acide aminé 11 en utilisant la numérotation Kabat) ;
(iii) la leucine à la position d'acide aminé 12 en utilisant la numérotation AHo (position d'acide aminé 11 en utilisant la numérotation Kabat) ;
(iv) la valine (V) à la position d'acide aminé 13 en utilisant la numérotation AHo (position d'acide aminé 12 en utilisant la numérotation Kabat) ;
(v) l'arginine (R) à la position d'acide aminé 14 en utilisant la numérotation AHo (position d'acide aminé 13 en utilisant la numérotation Kabat) ;
(vi) l'asparagine (N) à la position d'acide aminé 20 en utilisant la numérotation AHo (position d'acide aminé 19 en utilisant la numérotation Kabat) ;
(vii) la leucine (L) à la position d'acide aminé 21 en utilisant la numérotation AHo (position d'acide aminé 20 en utilisant la numérotation Kabat) ;
(viii) la lysine (K) à la position d'acide aminé 45 en utilisant la numérotation AHo (position d'acide aminé 38 en utilisant la numérotation Kabat) ;
(ix) l'arginine (R) à la position d'acide aminé 47 en utilisant la numérotation AHo (position d'acide aminé 40 en utilisant la numérotation Kabat) ; et
(x) la lysine (K) à la position d'acide aminé 50 en utilisant la numérotation AHo (position d'acide aminé 43 en utilisant la numérotation Kabat) ;
(xi) l'isoleucine (I) à la position d'acide aminé 55 en utilisant la numérotation AHo (position d'acide aminé 48 en utilisant la numérotation Kabat) ;
(xii) la lysine (K) à la position d'acide aminé 77 en utilisant la numérotation AHo (position d'acide aminé 66 en utilisant la numérotation Kabat) ;
(xiii) l'alanine (A) à la position d'acide aminé 78 en utilisant la numérotation AHo (position d'acide aminé 67 en utilisant la numérotation Kabat) ;
(xiv) l'acide glutamique (E) à la position d'acide aminé 82 en utilisant la numérotation AHo (position d'acide aminé 71 en utilisant la numérotation Kabat) ;
(xv) la thréonine (T) à la position d'acide aminé 86 en utilisant la numérotation AHo (position d'acide aminé 75 en utilisant la numérotation Kabat) ;
(xvi) l'asparagine (N) à la position d'acide aminé 87 en utilisant la numérotation AHo (position d'acide aminé 76 en utilisant la numérotation Kabat) ; et
(xvii) l'asparagine (N) à la position d'acide aminé 107 en utilisant la numérotation AHo (position d'acide aminé 93 en utilisant la numérotation Kabat).

8. Procédé selon la revendication 1, où si la une ou plusieurs positions d'acide aminé de chaîne légère sélectionnées pour mutation sont une région variable de chaîne légère de la famille Vκ1, la mutation comprend une ou plusieurs substitutions sélectionnées dans le groupe constitué de :

(i) l'acide glutamique (E) à la position d'acide aminé 1 en utilisant la numérotation AHo ou Kabat ;
(ii) la valine (V) à la position d'acide aminé 3 en utilisant la numérotation AHo ou Kabat ;
(iii) la leucine (L) à la position d'acide aminé 4 en utilisant la numérotation AHo ou Kabat ;
(iv) la glutamine (Q) à la position d'acide aminé 24 en utilisant la numérotation AHo ou Kabat ;
(v) l'arginine (R) à la position d'acide aminé 47 en utilisant la numérotation AHo (position d'acide aminé 39 en utilisant la numérotation Kabat) ;
(vi) l'arginine (R), l'acide glutamique (E), la thréonine (T), la méthionine (M) ou la glutamine (Q) à la position d'acide aminé 50 en utilisant la numérotation AHo (position d'acide aminé 42 en utilisant la numérotation Kabat) ;
(vii) la sérine (S) à la position d'acide aminé 57 en utilisant la numérotation AHo (position d'acide aminé 49 en utilisant la numérotation Kabat) ;
(viii) la phénylalanine (F) à la position d'acide aminé 91 en utilisant la numérotation AHo (position d'acide aminé 73 en utilisant la numérotation Kabat) ;
(ix) la valine (V) à la position d'acide aminé 103 en utilisant la numérotation AHo (position d'acide aminé 85 en utilisant la numérotation Kabat).

9. Procédé selon la revendication 1, où si la une ou plusieurs positions d'acide aminé de chaîne légère sélectionnées pour mutation sont une région variable de chaîne légère de la famille Vκ3, la mutation comprend une ou plusieurs substitutions sélectionnées dans le groupe constitué de :

(i) la thréonine (T) à la position d'acide aminé 2 en utilisant la numérotation AHo ou Kabat ;
(ii) la thréonine (T) à la position d'acide aminé 3 en utilisant la numérotation AHo ou Kabat ;
(iii) l'isoleucine (I) à la position d'acide aminé 10 en utilisant la numérotation AHo ou Kabat ;
(iv) la tyrosine (Y) à la position d'acide aminé 12 en utilisant la numérotation AHo ou Kabat ;
(v) la sérine (S) à la position d'acide aminé 18 en utilisant la numérotation AHo ou Kabat ;
(vi) l'alanine (A) à la position d'acide aminé 20 en utilisant la numérotation AHo ou Kabat ;
(vii) la méthionine (M) à la position d'acide aminé 56 en utilisant la numérotation AHo (position d'acide aminé 48 en utilisant la numérotation Kabat) ;
(viii) la thréonine (T) à la position d'acide aminé 74 en utilisant la numérotation AHo (position d'acide aminé 58 en utilisant la numérotation Kabat) ;
(ix) l'asparagine (N) à la position d'acide aminé 94 en utilisant la numérotation AHo (position d'acide aminé 76 en utilisant la numérotation Kabat) ;
(x) la sérine (S) à la position d'acide aminé 101 en utilisant la numérotation AHo (position d'acide aminé 83 en utilisant la numérotation Kabat) ; et
(xi) l'alanine (A) à la position d'acide aminé 103 en utilisant la numérotation AHo (position d'acide aminé 85 en utilisant la numérotation Kabat).

**10.** Procédé selon la revendication 1, où si la une ou plusieurs positions d'acide aminé de chaîne légère sélectionnées pour mutation sont une région variable de chaîne légère de la famille Vλ3, la mutation comprend une ou plusieurs substitutions sélectionnées dans le groupe constitué de :

(i) la leucine (L) à la position d'acide aminé 1 en utilisant la numérotation AHo ou Kabat ;
(ii) la proline (P) à la position d'acide aminé 2 en utilisant la numérotation AHo ou Kabat ;
(iii) la valine (V) à la position d'acide aminé 4 en utilisant la numérotation AHo ou Kabat ;
(iv) la sérine (S) à la position d'acide aminé 7 en utilisant la numérotation AHo ou Kabat ;
(v) l'alanine (A) à la position d'acide aminé 11 en utilisant la numérotation AHo ou Kabat ;
(vi) la thréonine (T) à la position d'acide aminé 14 en utilisant la numérotation AHo ou Kabat ;
(vii) l'histidine (H) à la position d'acide aminé 46 en utilisant la numérotation AHo (position d'acide aminé 38 en utilisant la numérotation Kabat) ;
(viii) la thréonine (T), la sérine (S), l'asparagine (N), la glutamine (Q) ou la proline (P) à la position d'acide aminé 53 en utilisant la numérotation AHo (position d'acide aminé 45 en utilisant la numérotation Kabat) ;
(ix) l'arginine (R) à la position d'acide aminé 82 en utilisant la numérotation AHo (position d'acide aminé 66 en utilisant la numérotation Kabat) ;
(x) la thréonine (T) à la position d'acide aminé 92 en utilisant la numérotation AHo (position d'acide aminé 74 en utilisant la numérotation Kabat) ; et
(xi) la valine (V) à la position d'acide aminé 103 en utilisant la numérotation AHo (position d'acide aminé 85 en utilisant la numérotation Kabat).

**11.** Procédé selon l'une quelconque des revendications 5 à 10, où la mutation comprend en outre une ou plusieurs substitutions de chaîne lourde sélectionnées dans le groupe constitué de :

(i) la sérine (S) à la position d'acide aminé 12 en utilisant la numérotation AHo ou Kabat ;
(ii) la sérine (S) à la position d'acide aminé 103 en utilisant la numérotation AHo (position d'acide aminé 85 en utilisant la numérotation Kabat) ; et
(iii) la sérine (S) ou la thréonine (T) à la position d'acide aminé 144 en utilisant la numérotation AHo (position d'acide aminé 103 en utilisant la numérotation Kabat).

**12.** Procédé selon l'une quelconque des revendications 5 à 10, où la mutation comprend en outre les mutations suivantes de chaîne lourde :

(i) la sérine (S) à la position d'acide aminé 12 en utilisant la numérotation AHo ou Kabat ;
(ii) la sérine (S) à la position d'acide aminé 103 en utilisant la numérotation AHo (position d'acide aminé 85 en utilisant la numérotation Kabat) ; et
(iii) la sérine (S) ou la thréonine (T) à la position d'acide aminé 144 en utilisant la numérotation AHo (position d'acide aminé 103 en utilisant la numérotation Kabat).

**13.** Procédé selon l'une quelconque des revendications 8 à 12, où l'immunoliant est un anticorps scFv, et une immunoglobuline de pleine longueur, un fragment Fab, un Dab ou un Nanocorps.

**14.** Procédé d'ingénierie d'un immunoliant, l'immunoliant comprenant les séquences CDR1, CDR2 et CDR3 de chaîne lourde, le procédé comprenant l'insertion des séquences CDR1, CDR2 et CDR3 de chaîne lourde dans un squelette de région structurale de chaîne lourde, le squelette de région structurale de chaîne lourde comprenant une séquence d'acides aminés telle que présentée en figure 9 (SEQ ID N° 1), figure 10 (SEQ ID N° 2), figure 11 (SEQ ID N° 3), SEQ ID N° 7, SEQ ID N° 8 ou SEQ ID N° 9), à condition que la séquence d'acides aminés ne soit pas la séquence consensus de lignée germinale.

**15.** Procédé selon la revendication 4, où le squelette de région structurale de chaîne lourde comprend une séquence d'acides aminés telle que présentée en figure 9 (SEQ ID N° 1).

**16.** Procédé selon la revendication 14, où le squelette de région structurale de chaîne lourde comprend une séquence d'acides aminés telle que présentée en figure 10 (SEQ ID N° 2).

**17.** Procédé selon la revendication 14, où le squelette de région structurale de chaîne lourde comprend une séquence d'acides aminés telle que présentée en figure 11 (SEQ ID N° 3).

**18.** Procédé d'ingénierie d'un immunoliant, l'immunoliant comprenant des séquences CDR1, CDR2 et CDR3 de chaîne légère, le procédé comprenant l'insertion des séquences CDR1, CDR2 et CDR3 de chaîne légère dans un squelette de région structurale de chaîne légère, le squelette de région structurale de chaîne légère comprenant une séquence d'acides aminés telle que présentée en figure 12 (SEQ ID N° 4), figure 13 (SEQ ID N° 5), figure 14 SEQ ID N° 6), SEQ ID N° 10, SEQ ID N° 11 ou SEQ ID N° 12, à condition que la séquence d'acides aminés ne soit pas la séquence consensus de lignée germinale.

**19.** Procédé selon la revendication 18, où le squelette de région structurale de chaîne légère comprend une séquence d'acides aminés telle que présentée en figure 12 (SEQ ID N° 4).

**20.** Procédé selon la revendication 18, où le squelette de région structurale de chaîne légère comprend une séquence d'acides aminés telle que présentée en figure 13 (SEQ ID N° 5).

**21.** Procédé selon la revendication 18, où le squelette de région structurale de chaîne légère comprend une séquence d'acides aminés telle que présentée en figure 14 (SEQ ID N° 6).

**22.** Procédé selon l'une quelconque des revendications 18 à 21, où l'immunoliant est un anticorps scFv.

**23.** Squelette de région structurale de chaîne lourde isolé comprenant une séquence d'acides aminés telle que présentée en figure 9 SEQ ID N° 1), figure 10 (SEQ ID N° 2) ou figure 11 (SEQ ID N° 3) à condition que la séquence d'acides aminés ne soit pas la séquence consensus de lignée germinale.

**24.** Squelette de région structurale de chaîne lourde isolé comprenant une séquence d'acides aminés telle que présentée en figure 12 SEQ ID N° 4), figure 13 (SEQ ID N° 5) ou figure 14 (SEQ ID N° 6) à condition que la séquence d'acides aminés ne soit pas la séquence consensus de lignée germinale.

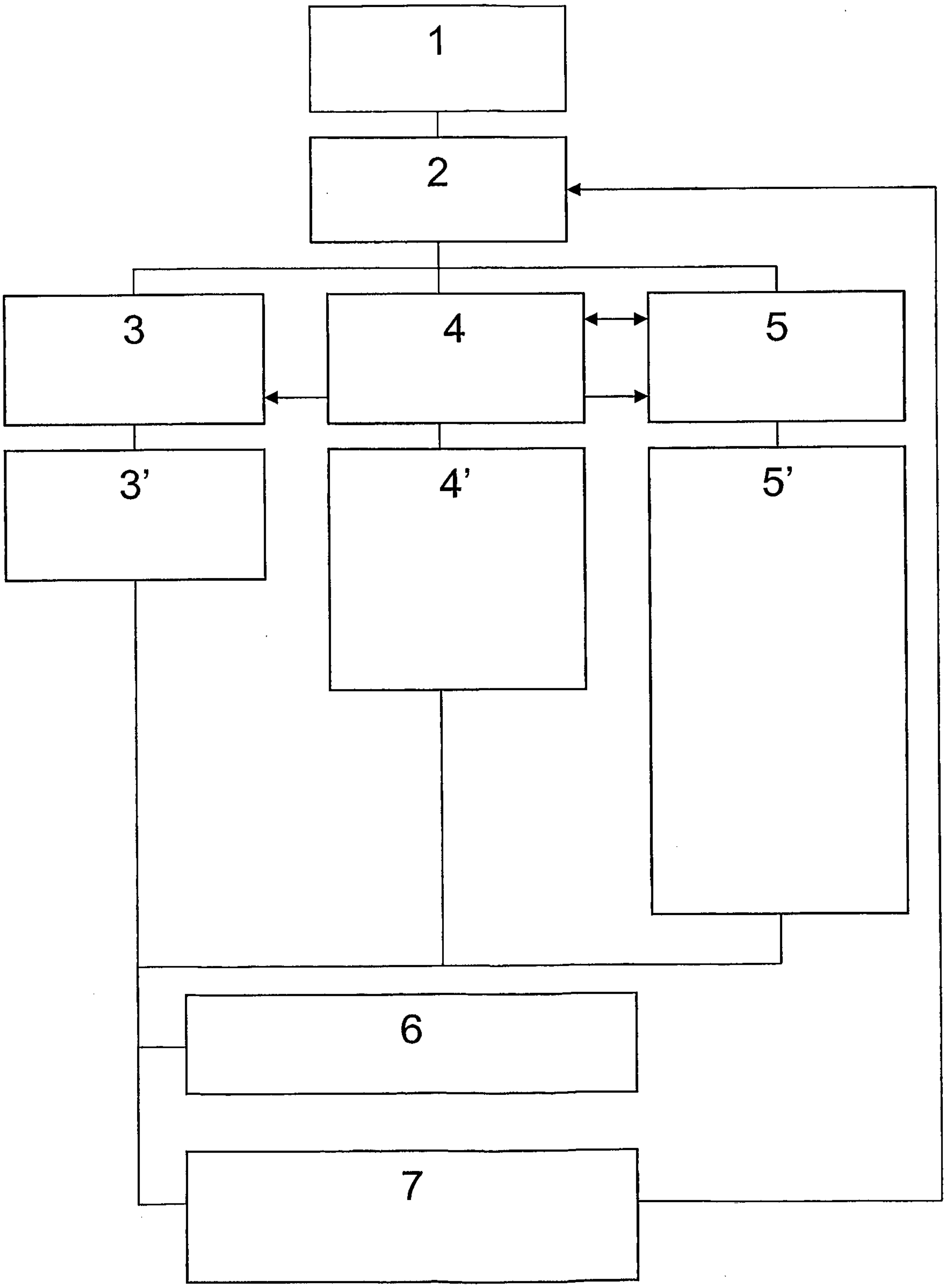


Fig. 1

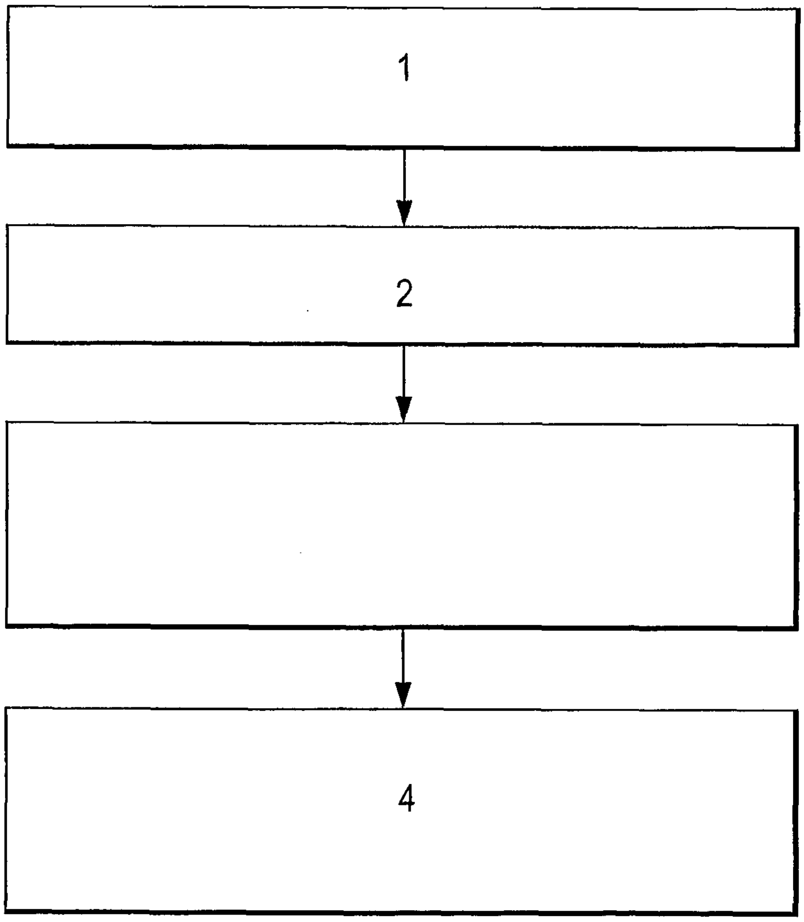


Fig. 2

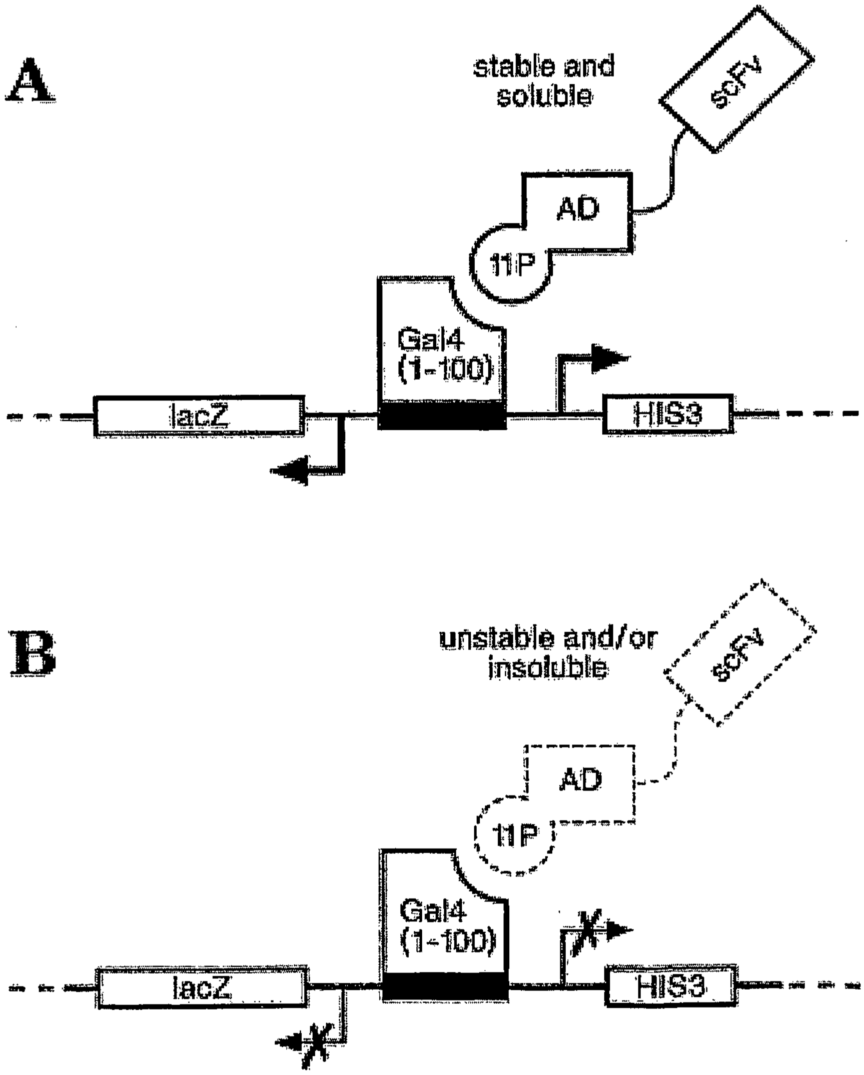
A
stable and
soluble
scFv
AD
11P
Gal4
(1-100)
lacZ
HIS3
B
unstable and/or
insoluble
scFv
AD
11P
Gal4
(1-100)
lacZ
HIS3

*Fig. 3*

A

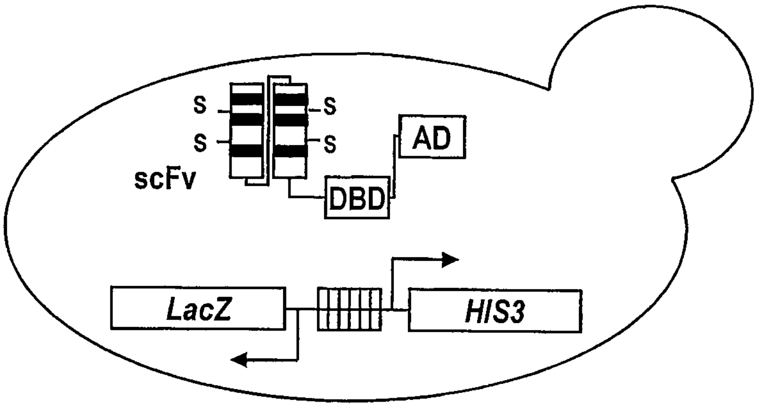


B

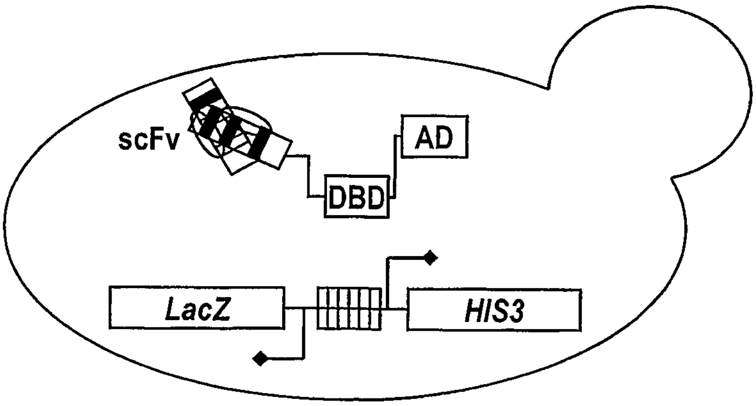


*Fig. 4*

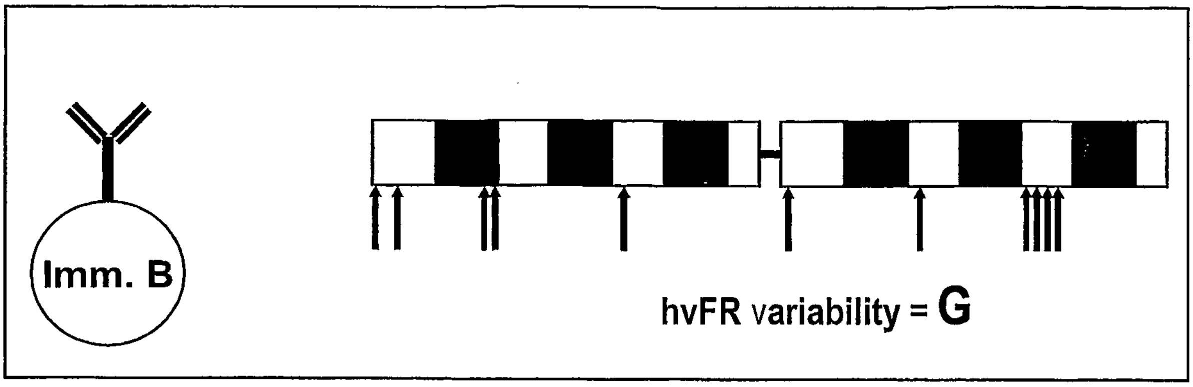


*Fig. 5A*

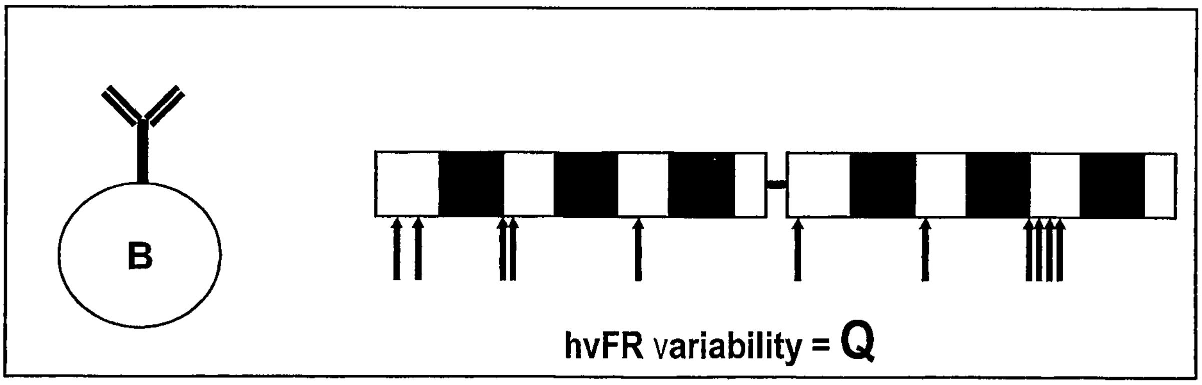


*Fig. 5B*

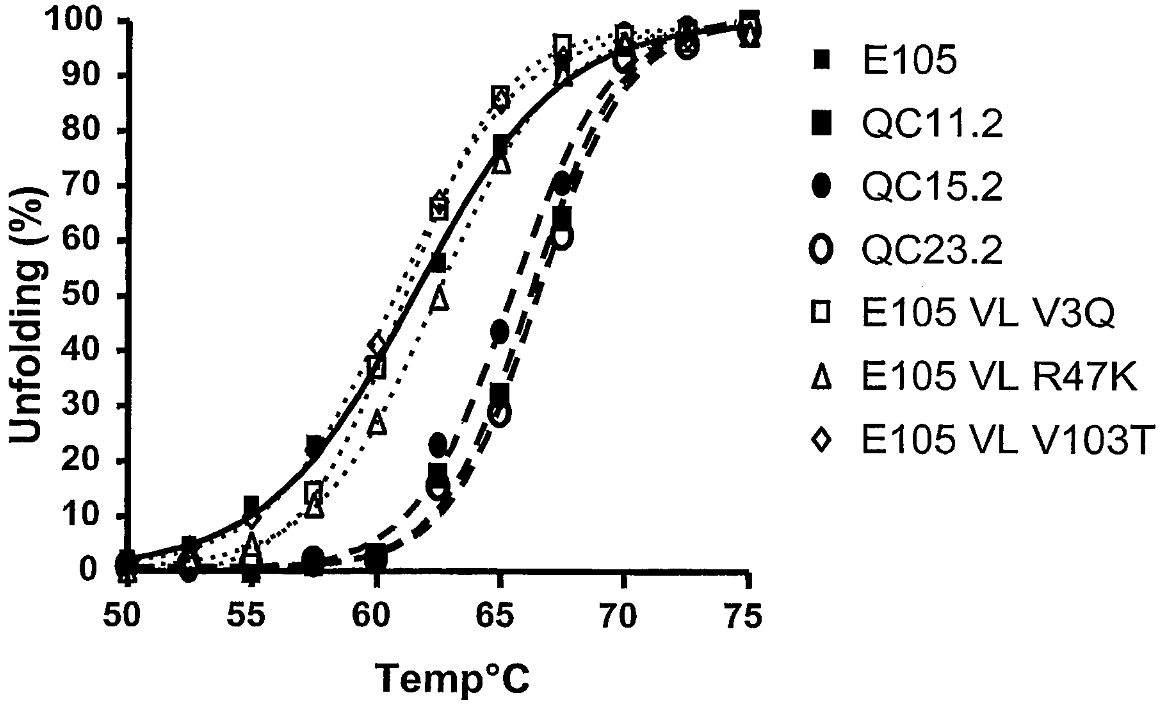
100
90
80
70
60
50
40
30
20
10
0
Unfolding (%)
50
55
60
65
70
75
Temp°C
E105
QC11.2
QC15.2
QC23.2
E105 VL V3Q
E105 VL R47K
E105 VL V103T

*Fig. 6*

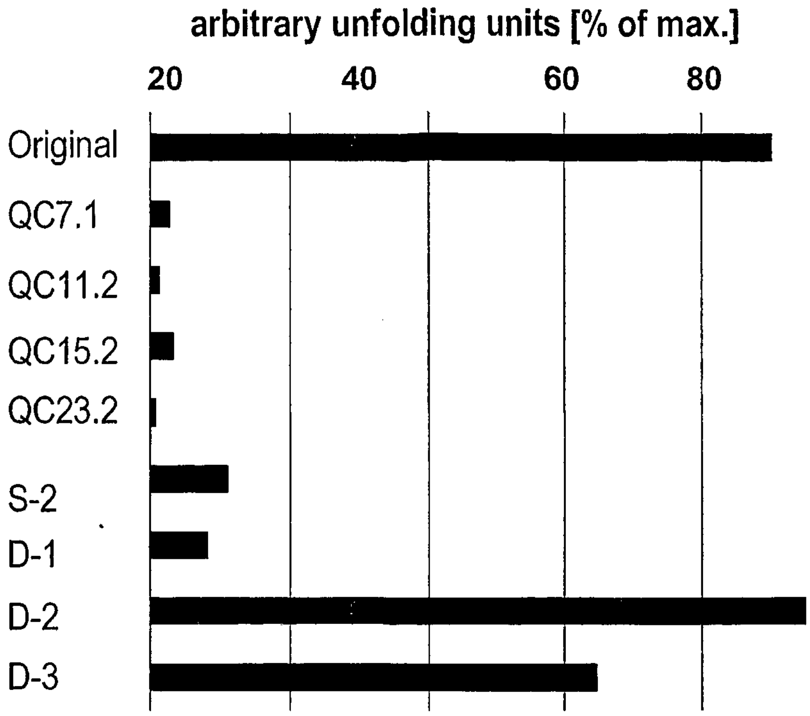
arbitrary unfolding units [% of max.]
20
40
60
80
Original
QC7.1
QC11.2
QC15.2
QC23.2
S-2
D-1
D-2
D-3

*Fig. 7*

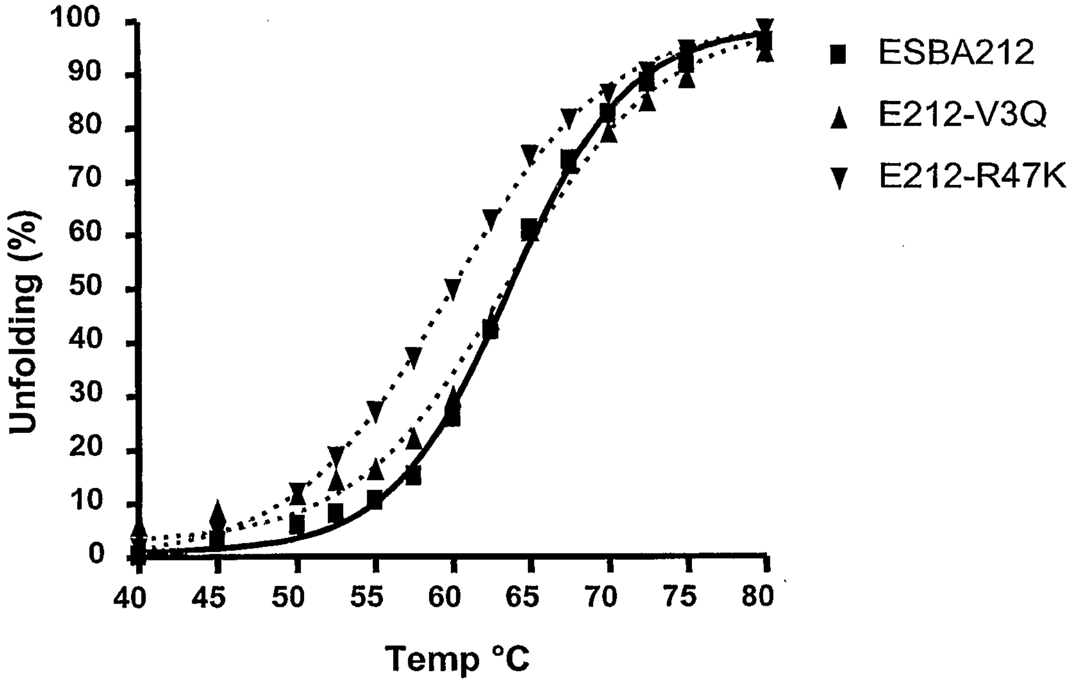
100
90
80
70
60
50
40
30
20
10
0
Unfolding (%)
40
45
50
55
60
65
70
75
80
Temp °C
ESBA212
E212-V3Q
E212-R47K

*Fig. 8*

## VH1 Family Heavy Chain Framework Scaffold

| | | | | | | | | | | | | | | | | | | | | | | | | | |
|---|---|---|---|---|---|---|---|---|---|---|---|---|---|---|---|---|---|---|---|---|---|---|---|---|---|
| 1 | 2 | 3 | 4 | 5 | 6 | 7 | * | 8 | 9 | 10 | 11 | 12 | 13 | 14 | 15 | 16 | 17 | 18 | 19 | 20 | 21 | 22 | 23 | 24 | 25 |
| 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
| X | V | Q | L | V | X | S | | G | A | E | X | X | X | P | G | S | S | X | K | X | S | C | K | A | S |
| Q | | | | | Q | | | | | | V | K | K | | | | | V | | V | | | | | |
| E | | | | | E | | | | | | L | M | E | | | | | L | | I | | | | | |
| | | | | | | | | | | | | | Q | | | | | | | | | | | | |

| | | | | | | | | | | | | | | | | | | | | | | | | | |
|---|---|---|---|---|---|---|---|---|---|---|---|---|---|---|---|---|---|---|---|---|---|---|---|---|---|
| 26 | * | 27 | 28 | 29 | 30 | 31 | 32 | 33 | 34 | 35 | 35a | 35b | * | * | * | 36 | 37 | 38 | 39 | 40 | 41 | 42 | 43 | 44 | 45 |
| 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
| x | | x | x | x | CDR H1 | | | | | | | | | | | W | V | R | Q | A | P | G | Q | G | L |

| | | | | | | | | | | | | | | | | | | | | | | | | | | |
|---|---|---|---|---|---|---|---|---|---|---|---|---|---|---|---|---|---|---|---|---|---|---|---|---|---|---|
| 46 | 47 | 48 | 49 | 50 | 51 | 52 | 52a | 52b | 52c | * | 53 | 54 | 55 | 56 | 57 | 58 | 59 | 60 | 61 | 62 | 63 | 64 | 65 | 66 | 67 | 68 |
| 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 | 79 |
| E | W | M | G | CDR H2 | | | | | | | | | | | | | | | | | | | | R | V | T |

| | | | | | | | | | | | | | | | | | | | | | | | | | |
|---|---|---|---|---|---|---|---|---|---|---|---|---|---|---|---|---|---|---|---|---|---|---|---|---|---|
| 69 | 70 | 71 | 72 | 73 | 74 | 75 | 76 | 77 | 78 | 79 | 80 | 81 | 82 | 82a | 82b | 82b | 83 | 84 | 85 | 86 | 87 | 88 | 89 | 90 | 91 |
| 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 | 105 |
| I | T | A | D | E | S | T | S | T | A | X | M | X | L | S | X | L | R | X | E | D | T | A | V | Y | Y |
| | | | | | | | | | | Y | | E | | | S | | | S | | | | | | | |
| | | | | | | | | | | F | | D | | | G | | | F | | | | | | | |
| | | | | | | | | | | S | | Q | | | T | | | T | | | | | | | |
| | | | | | | | | | | H | | | | | N | | | A | | | | | | | |
| | | | | | | | | | | D | | | | | | | | P | | | | | | | |

| | | | | | | | | | | | | | | | | | | | | | | |
|---|---|---|---|---|---|---|---|---|---|---|---|---|---|---|---|---|---|---|---|---|---|---|
| 92 | 93 | 94 | 95 | 96 | 97 | 98 | 99 | 100 | 100a | 100b | 100c | 100d | 100e | 100f | 100g | 100h | 100i | * | * | * | * | * |
| 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 |
| C | A | R | CDR H3 | | | | | | | | | | | | | | | | | | | |

| | | | | | | | | | | | | | | | | | | | | |
|---|---|---|---|---|---|---|---|---|---|---|---|---|---|---|---|---|---|---|---|---|
| * | * | * | * | * | * | * | * | 101 | 102 | 103 | 104 | 105 | 106 | 107 | 108 | 109 | 110 | 111 | 112 | 113 |
| 129 | 130 | 131 | 132 | 133 | 134 | 135 | 136 | 137 | 138 | 139 | 140 | 141 | 142 | 143 | 144 | 145 | 146 | 147 | 148 | 149 |
| CDR H3 | | | | | | | | | | W | G | Q | G | T | L | V | T | V | S | S |

*Fig. 9*

## VH1B Family Heavy Chain Framework Scaffold

| | | | | | | | | | | | | | | | | | | | | | | | | | |
|---|---|---|---|---|---|---|---|---|---|---|---|---|---|---|---|---|---|---|---|---|---|---|---|---|---|
| 1 | 2 | 3 | 4 | 5 | 6 | 7 | * | 8 | 9 | 10 | 11 | 12 | 13 | 14 | 15 | 16 | 17 | 18 | 19 | 20 | 21 | 22 | 23 | 24 | 25 |
| 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
| X | V | Q | L | V | Q | S | | G | X | E | X | X | X | P | G | A | S | V | X | X | S | C | K | A | S |
| Q | | | | | | | | | A | | V | K | K | | | | | | K | V | | | | | |
| E | | | | | | | | | T | | L | V | R | | | | | | N | L | | | | | |
| | | | | | | | | | D | | | R | E | | | | | | R | I | | | | | |
| | | | | | | | | | P | | | Q | M | | | | | | T | F | | | | | |
| | | | | | | | | | V | | | M | | | | | | | | | | | | | |

| | | | | | | | | | | | | | | | | | | | | | | | | | |
|---|---|---|---|---|---|---|---|---|---|---|---|---|---|---|---|---|---|---|---|---|---|---|---|---|---|
| 26 | * | 27 | 28 | 29 | 30 | 31 | 32 | 33 | 34 | 35 | 35a | 35b | * | * | * | 36 | 37 | 38 | 39 | 40 | 41 | 42 | 43 | 44 | 45 |
| 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
| x | | x | x | x | CDR H1 | | | | | | | | | | | W | V | X | Q | X | P | G | X | G | L |
| | | | | | | | | | | | | | | | | | | R | | A | | | Q | | |
| | | | | | | | | | | | | | | | | | | K | | R | | | K | | |
| | | | | | | | | | | | | | | | | | | | | T | | | E | | |
| | | | | | | | | | | | | | | | | | | | | V | | | H | | |
| | | | | | | | | | | | | | | | | | | | | P | | | | | |

| | | | | | | | | | | | | | | | | | | | | | | | | | | |
|---|---|---|---|---|---|---|---|---|---|---|---|---|---|---|---|---|---|---|---|---|---|---|---|---|---|---|
| 46 | 47 | 48 | 49 | 50 | 51 | 52 | 52a | 52b | 52c | * | 53 | 54 | 55 | 56 | 57 | 58 | 59 | 60 | 61 | 62 | 63 | 64 | 65 | 66 | 67 | 68 |
| 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 | 79 |
| E | W | X | G | CDR H2 | | | | | | | | | | | | | | | | | | | | X | X | T |
| | | M | | | | | | | | | | | | | | | | | | | | | | R | V | |
| | | I | | | | | | | | | | | | | | | | | | | | | | K | A | |
| | | | | | | | | | | | | | | | | | | | | | | | | | I | |
| | | | | | | | | | | | | | | | | | | | | | | | | | L | |

| | | | | | | | | | | | | | | | | | | | | | | | | | |
|---|---|---|---|---|---|---|---|---|---|---|---|---|---|---|---|---|---|---|---|---|---|---|---|---|---|
| 69 | 70 | 71 | 72 | 73 | 74 | 75 | 76 | 77 | 78 | 79 | 80 | 81 | 82 | 82a | 82b | 82b | 83 | 84 | 85 | 86 | 87 | 88 | 89 | 90 | 91 |
| 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 | 105 |
| M | T | E | D | T | S | T | N | T | A | Y | M | E | L | S | S | L | R | S | E | D | T | A | V | Y | Y |

| | | | | | | | | | | | | | | | | | | | | | | |
|---|---|---|---|---|---|---|---|---|---|---|---|---|---|---|---|---|---|---|---|---|---|---|
| 92 | 93 | 94 | 95 | 96 | 97 | 98 | 99 | 100 | 100a | 100b | 100c | 100d | 100e | 100f | 100g | 100h | 100i | * | * | * | * | * |
| 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 |
| C | X | R | CDR H3 | | | | | | | | | | | | | | | | | | | |
| | A | | | | | | | | | | | | | | | | | | | | | |
| | N | | | | | | | | | | | | | | | | | | | | | |
| | S | | | | | | | | | | | | | | | | | | | | | |

| | | | | | | | | | | | | | | | | | | | | |
|---|---|---|---|---|---|---|---|---|---|---|---|---|---|---|---|---|---|---|---|---|
| * | * | * | * | * | * | * | * | 101 | 102 | 103 | 104 | 105 | 106 | 107 | 108 | 109 | 110 | 111 | 112 | 113 |
| 129 | 130 | 131 | 132 | 133 | 134 | 135 | 136 | 137 | 138 | 139 | 140 | 141 | 142 | 143 | 144 | 145 | 146 | 147 | 148 | 149 |
| CDR H3 | | | | | | | | | | W | G | Q | G | T | L | V | T | V | S | S |

*Fig. 10*

# VH3 Family Heavy Chain Framework Scaffold

| | | | | | | | | | | | | | | | | | | | | | | | | | |
|---|---|---|---|---|---|---|---|---|---|---|---|---|---|---|---|---|---|---|---|---|---|---|---|---|---|
| 1 | 2 | 3 | 4 | 5 | 6 | 7 | * | 8 | 9 | 10 | 11 | 12 | 13 | 14 | 15 | 16 | 17 | 18 | 19 | 20 | 21 | 22 | 23 | 24 | 25 |
| 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
| X | V | Q | L | V | X | X | | G | P | G | L | V | K | P | S | E | T | L | R | L | S | C | A | A | S |
| E | | | | | E | S | | | | | | | | | | | | | | | | | | | |
| Q | | | | | Q | T | | | | | | | | | | | | | | | | | | | |
| | | | | | | A | | | | | | | | | | | | | | | | | | | |

| | | | | | | | | | | | | | | | | | | | | | | | | | |
|---|---|---|---|---|---|---|---|---|---|---|---|---|---|---|---|---|---|---|---|---|---|---|---|---|---|
| 26 | * | 27 | 28 | 29 | 30 | 31 | 32 | 33 | 34 | 35 | 35a | 35b | * | * | * | 36 | 37 | 38 | 39 | 40 | 41 | 42 | 43 | 44 | 45 |
| 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
| G | | x | x | x | CDR H1 | | | | | | | | | | | W | V | R | Q | A | P | G | K | G | L |

| | | | | | | | | | | | | | | | | | | | | | | | | | | |
|---|---|---|---|---|---|---|---|---|---|---|---|---|---|---|---|---|---|---|---|---|---|---|---|---|---|---|
| 46 | 47 | 48 | 49 | 50 | 51 | 52 | 52a | 52b | 52c | * | 53 | 54 | 55 | 56 | 57 | 58 | 59 | 60 | 61 | 62 | 63 | 64 | 65 | 66 | 67 | 68 |
| 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 | 79 |
| E | W | V | S | CDR H2 | | | | | | | | | | | | | | | | | | | | R | F | T |

| | | | | | | | | | | | | | | | | | | | | | | | | | |
|---|---|---|---|---|---|---|---|---|---|---|---|---|---|---|---|---|---|---|---|---|---|---|---|---|---|
| 69 | 70 | 71 | 72 | 73 | 74 | 75 | 76 | 77 | 78 | 79 | 80 | 81 | 82 | 82a | 82b | 82b | 83 | 84 | 85 | 86 | 87 | 88 | 89 | 90 | 91 |
| 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 | 105 |
| I | S | R | D | N | S | K | N | T | X | Y | L | Q | M | N | S | L | R | A | E | D | T | A | X | Y | Y |
| | | | | | | | | | L | | | | | | | | | | | | | | V | | |
| | | | | | | | | | V | | | | | | | | | | | | | | L | | |
| | | | | | | | | | A | | | | | | | | | | | | | | I | | |
| | | | | | | | | | F | | | | | | | | | | | | | | M | | |
| | | | | | | | | | | | | | | | | | | | | | | | F | | |
| | | | | | | | | | | | | | | | | | | | | | | | R | | |
| | | | | | | | | | | | | | | | | | | | | | | | Q | | |

| | | | | | | | | | | | | | | | | | | | | | | |
|---|---|---|---|---|---|---|---|---|---|---|---|---|---|---|---|---|---|---|---|---|---|---|
| 92 | 93 | 94 | 95 | 96 | 97 | 98 | 99 | 100 | 100a | 100b | 100c | 100d | 100e | 100f | 100g | 100h | 100i | * | * | * | * | * |
| 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 |
| C | A | R | CDR H3 | | | | | | | | | | | | | | | | | | | |

| | | | | | | | | | | | | | | | | | | | | |
|---|---|---|---|---|---|---|---|---|---|---|---|---|---|---|---|---|---|---|---|---|
| * | * | * | * | * | * | * | * | 101 | 102 | 103 | 104 | 105 | 106 | 107 | 108 | 109 | 110 | 111 | 112 | 113 |
| 129 | 130 | 131 | 132 | 133 | 134 | 135 | 136 | 137 | 138 | 139 | 140 | 141 | 142 | 143 | 144 | 145 | 146 | 147 | 148 | 149 |
| CDR H3 | | | | | | | | | | W | G | Q | G | T | L | V | T | V | S | S |

*Fig. 11*

| | | | | | | | | | | | | | | | | | | | | | | | | | | | | | | | | | |
|---|---|---|---|---|---|---|---|---|---|---|---|---|---|---|---|---|---|---|---|---|---|---|---|---|---|---|---|---|---|---|---|---|---|
| 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 | 27 | | 27a | 27b | 27c | 27d | 27e |
| 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 | 27 | 28 | 29 | 30 | 31 | 32 | 33 |
| X | I | X | X | T | Q | S | P | S | S | L | S | A | S | V | G | D | R | V | T | I | T | C | X | | | | | | | | | |
| E | | V | L | | | | | | | | | | | | | | | | | | | | Q | CDR L1 | | | | | | | | |
| D | | Q | M | | | | | | | | | | | | | | | | | | | | R | | | | | | | | | |
| I | | I | V | | | | | | | | | | | | | | | | | | | | | | | | | | | | | |
| | | | I | | | | | | | | | | | | | | | | | | | | | | | | | | | | | |

| | | | | | | | | | | | | | | | | | | | | | | | | | | | | | | | | |
|---|---|---|---|---|---|---|---|---|---|---|---|---|---|---|---|---|---|---|---|---|---|---|---|---|---|---|---|---|---|---|---|---|
| 27f | | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | | | | | | | | |
| 34 | 35 | 36 | 37 | 38 | 39 | 40 | 41 | 42 | 43 | 44 | 45 | 46 | 47 | 48 | 49 | 50 | 51 | 52 | 53 | 54 | 55 | 56 | 57 | 58 | 59 | 60 | 61 | 62 | 63 | 64 | 65 | 66 |
| | | | | | | | | | W | Y | Q | Q | R | P | G | K | A | P | K | L | L | I | S | | | | | | | | | |
| CDR L1 | | | | | | | | | | | | | K | | | R | | | | | | | Y | CDR L2 | | | | | | | | |
| | | | | | | | | | | | | | I | | | E | | | | | | | F | | | | | | | | | |
| | | | | | | | | | | | | | | | | T | | | | | | | H | | | | | | | | | |
| | | | | | | | | | | | | | | | | M | | | | | | | | | | | | | | | | |
| | | | | | | | | | | | | | | | | Q | | | | | | | | | | | | | | | | |

| | | | | | | | | | | | | | | | | | | | | | | | | | | | | | | | | |
|---|---|---|---|---|---|---|---|---|---|---|---|---|---|---|---|---|---|---|---|---|---|---|---|---|---|---|---|---|---|---|---|---|
| 51 | 52 | 53 | 54 | 55 | 56 | 57 | 58 | 59 | 60 | 61 | 62 | 63 | 64 | 65 | 66 | 67 | 68 | | | 69 | 70 | 71 | 72 | 73 | 74 | 75 | 76 | 77 | 78 | 79 | 80 | 81 |
| 67 | 68 | 69 | 70 | 71 | 72 | 73 | 74 | 75 | 76 | 77 | 78 | 79 | 80 | 81 | 82 | 83 | 84 | 85 | 86 | 87 | 88 | 89 | 90 | 91 | 92 | 93 | 94 | 95 | 96 | 97 | 98 | 99 |
| CDR L2 | | | | | | G | V | P | S | R | F | S | G | S | G | S | G | . | . | T | D | F | T | F | T | I | S | S | L | Q | P | E |
| | | | | | | | | | | | | | | | | | | | | | | | | L | | | | | | | | |

| | | | | | | | | | | | | | | | | | | | | | | | | | | | | | | | | |
|---|---|---|---|---|---|---|---|---|---|---|---|---|---|---|---|---|---|---|---|---|---|---|---|---|---|---|---|---|---|---|---|---|
| 82 | 83 | 84 | 85 | 86 | 87 | 88 | 89 | 90 | 91 | 92 | 93 | 94 | 95 | 95a | 95b | 95c | 95d | 95e | 95f | | | | | | | | | | | | | |
| 100 | 101 | 102 | 103 | 104 | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 | 131 | 132 |
| D | F | A | V | Y | Y | C | | | | | | | | | | | | | | | | | | | | | | | | | | |
| | | | T | | | | | | | | | | | | | | | | | | | | | | | | | | | | | |
| | | | S | | | | | | | | | | | | | | | | | | | | | | CDR L3 | | | | | | | |
| | | | G | | | | | | | | | | | | | | | | | | | | | | | | | | | | | |
| | | | I | | | | | | | | | | | | | | | | | | | | | | | | | | | | | |

| | | | | | | | | | | | | | | | | |
|---|---|---|---|---|---|---|---|---|---|---|---|---|---|---|---|---|
| | | | | 96 | 97 | 98 | 99 | 100 | 101 | 102 | 103 | 104 | 105 | 106 | 107 | 108 |
| 133 | 134 | 135 | 136 | 137 | 138 | 139 | 140 | 141 | 142 | 143 | 144 | 145 | 146 | 147 | 148 | 149 |
| CDR L3 | | | | | | F | G | Q | G | T | K | V | E | I | K | R |

*Fig. 12*

| | | | | | | | | | | | | | | | | | | | | | | | | | | | | | | | | |
|---|---|---|---|---|---|---|---|---|---|---|---|---|---|---|---|---|---|---|---|---|---|---|---|---|---|---|---|---|---|---|---|---|
| 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 | 27 | | 27a | 27b | 27c | 27d | 27e |
| 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 | 27 | 28 | 29 | 30 | 31 | 32 | 33 |
| E | X | X | L | T | Q | S | P | G | X | L | X | L | S | P | G | E | X | A | X | L | S | C | R | | | | | CDR L1 | | | | |
| | T | T | | | | | | | I | | Y | | | | | | S | | A | | | | | | | | | | | | | |
| | I | V | | | | | | | T | | S | | | | | | R | | T | | | | | | | | | | | | | |

| | | | | | | | | | | | | | | | | | | | | | | | | | | | | | | | | |
|---|---|---|---|---|---|---|---|---|---|---|---|---|---|---|---|---|---|---|---|---|---|---|---|---|---|---|---|---|---|---|---|---|
| 27f | | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | | | | | | | | |
| 34 | 35 | 36 | 37 | 38 | 39 | 40 | 41 | 42 | 43 | 44 | 45 | 46 | 47 | 48 | 49 | 50 | 51 | 52 | 53 | 54 | 55 | 56 | 57 | 58 | 59 | 60 | 61 | 62 | 63 | 64 | 65 | 66 |
| | | CDR L1 | | | | | | | W | Y | Q | Q | K | P | G | Q | A | P | R | L | L | M | Y | | | | | | CDR L2 | | | |
| | | | | | | | | | | | | | | | | | | | | | | I | | | | | | | | | | |

| | | | | | | | | | | | | | | | | | | | | | | | | | | | | | | | | |
|---|---|---|---|---|---|---|---|---|---|---|---|---|---|---|---|---|---|---|---|---|---|---|---|---|---|---|---|---|---|---|---|---|
| 51 | 52 | 53 | 54 | 55 | 56 | 57 | 58 | 59 | 60 | 61 | 62 | 63 | 64 | 65 | 66 | 67 | 68 | | | 69 | 70 | 71 | 72 | 73 | 74 | 75 | 76 | 77 | 78 | 79 | 80 | 81 |
| 67 | 68 | 69 | 70 | 71 | 72 | 73 | 74 | 75 | 76 | 77 | 78 | 79 | 80 | 81 | 82 | 83 | 84 | 85 | 86 | 87 | 88 | 89 | 90 | 91 | 92 | 93 | 94 | 95 | 96 | 97 | 98 | 99 |
| | | | | | | G | I | P | D | R | F | S | G | S | G | S | G | | | T | D | F | T | L | T | I | N | R | L | E | P | E |
| | CDR L2 | | | | | | T | | | | | | | | | | | | | | | | | | | | S | | | | | |
| | | | | | | | V | | | | | | | | | | | | | | | | | | | | | | | | | |

| | | | | | | | | | | | | | | | | | | | | | | | | | | | | | | | | |
|---|---|---|---|---|---|---|---|---|---|---|---|---|---|---|---|---|---|---|---|---|---|---|---|---|---|---|---|---|---|---|---|---|
| 82 | 83 | 84 | 85 | 86 | 87 | 88 | 89 | 90 | 91 | 92 | 93 | 94 | 95 | 95a | 95b | 95c | 95d | 95e | 95f | | | | | | | | | | | | | |
| 100 | 101 | 102 | 103 | 104 | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 | 131 | 132 |
| D | S | A | A | Y | Y | C | | | | | | | | | | | | | | | CDR L3 | | | | | | | | | | | |
| | Y | | L | | | | | | | | | | | | | | | | | | | | | | | | | | | | | |
| | F | | V | | | | | | | | | | | | | | | | | | | | | | | | | | | | | |

| | | | | | | | | | | | | | | | | |
|---|---|---|---|---|---|---|---|---|---|---|---|---|---|---|---|---|
| | | | | 96 | 97 | 98 | 99 | 100 | 101 | 102 | 103 | 104 | 105 | 106 | 107 | 108 |
| 133 | 134 | 135 | 136 | 137 | 138 | 139 | 140 | 141 | 142 | 143 | 144 | 145 | 146 | 147 | 148 | 149 |
| | CDR L3 | | | | | F | G | G | G | T | K | L | E | I | K | R |

Fig. 13

| 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 | 27 | | 27a | 27b | 27c | 27d | 27e |
|---|---|---|---|---|---|---|---|---|---|---|---|---|---|---|---|---|---|---|---|---|---|---|---|---|---|---|---|---|---|---|---|---|
| 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 | 27 | 28 | 29 | 30 | 31 | 32 | 33 |
| X | X | V | X | T | Q | X | . | P | S | X | S | G | X | P | G | Q | R | V | T | I | S | C | S | | | | | | | | | |
| L | S | | V | | | S | | | | A | | | T | | | | | | | | | | | | | | | CDR L1 | | | | |
| Q | A | | L | | | E | | | | V | | | S | | | | | | | | | | | | | | | | | | | |
| S | P | | M | | | P | | | | | | | A | | | | | | | | | | | | | | | | | | | |
| E | I | | | | | | | | | | | | | | | | | | | | | | | | | | | | | | | |
| | Y | | | | | | | | | | | | | | | | | | | | | | | | | | | | | | | |

| 27f | | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | | | | | | | | |
|---|---|---|---|---|---|---|---|---|---|---|---|---|---|---|---|---|---|---|---|---|---|---|---|---|---|---|---|---|---|---|---|---|
| 34 | 35 | 36 | 37 | 38 | 39 | 40 | 41 | 42 | 43 | 44 | 45 | 46 | 47 | 48 | 49 | 50 | 51 | 52 | 53 | 54 | 55 | 56 | 57 | 58 | 59 | 60 | 61 | 62 | 63 | 64 | 65 | 66 |
| | | | | | | | | | W | Y | Q | X | L | P | G | T | A | P | X | L | L | I | Y | D | | | | | CDR L2 | | | |
| | | | | | | | | | | | | H | | | | | | | T | | | | | | | | | | | | | |
| | | | | | | | | | | | | Q | | | | | | | K | | | | | | | | | | | | | |
| | | CDR L1 | | | | | | | | | | | | | | | | | S | | | | | | | | | | | | | |
| | | | | | | | | | | | | | | | | | | | N | | | | | | | | | | | | | |
| | | | | | | | | | | | | | | | | | | | Q | | | | | | | | | | | | | |
| | | | | | | | | | | | | | | | | | | | P | | | | | | | | | | | | | |

| 51 | 52 | 53 | 54 | 55 | 56 | 57 | 58 | 59 | 60 | 61 | 62 | 63 | 64 | 65 | 66 | 67 | 68 | | | 69 | 70 | 71 | 72 | 73 | 74 | 75 | 76 | 77 | 78 | 79 | 80 | 81 |
|---|---|---|---|---|---|---|---|---|---|---|---|---|---|---|---|---|---|---|---|---|---|---|---|---|---|---|---|---|---|---|---|---|
| 67 | 68 | 69 | 70 | 71 | 72 | 73 | 74 | 75 | 76 | 77 | 78 | 79 | 80 | 81 | 82 | 83 | 84 | 85 | 86 | 87 | 88 | 89 | 90 | 91 | 92 | 93 | 94 | 95 | 96 | 97 | 98 | 99 |
| N | N | Q | R | P | S | G | V | P | D | R | F | S | G | S | X | S | G | . | . | T | S | A | S | L | X | I | S | G | L | Q | S | E |
| | | | | | | | | | | | | | | | R | | | | | | | | | | T | | | | | | | |
| | CDR L2 | | | | | | | | | | | | | | Q | | | | | | | | | | G | | | | | | | |
| | | | | | | | | | | | | | | | K | | | | | | | | | | D | | | | | | | |
| | | | | | | | | | | | | | | | | | | | | | | | | | A | | | | | | | |

| 82 | 83 | 84 | 85 | 86 | 87 | 88 | 89 | 90 | 91 | 92 | 93 | 94 | 95 | 95a | 95b | 95c | 95d | 95e | 95f | | | | | | | | | | | | | |
|---|---|---|---|---|---|---|---|---|---|---|---|---|---|---|---|---|---|---|---|---|---|---|---|---|---|---|---|---|---|---|---|---|
| 100 | 101 | 102 | 103 | 104 | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 | 131 | 132 |
| D | E | A | X | Y | Y | C | | | | | | | | | | | | | | | | | | | | | | | | | | |
| | | | V | | | | | | | | | | | | | | | | | | | CDR L3 | | | | | | | | | | |
| | | | D | | | | | | | | | | | | | | | | | | | | | | | | | | | | | |
| | | | T | | | | | | | | | | | | | | | | | | | | | | | | | | | | | |
| | | | H | | | | | | | | | | | | | | | | | | | | | | | | | | | | | |
| | | | E | | | | | | | | | | | | | | | | | | | | | | | | | | | | | |

| | | | | 96 | 97 | 98 | 99 | 100 | 101 | 102 | 103 | 104 | 105 | 106 | 107 | 108 |
|---|---|---|---|---|---|---|---|---|---|---|---|---|---|---|---|---|
| 133 | 134 | 135 | 136 | 137 | 138 | 139 | 140 | 141 | 142 | 143 | 144 | 145 | 146 | 147 | 148 | 149 |
| CDR L3 | | | | | | F | G | G | G | T | K | L | T | V | L | G |

*Fig. 14*

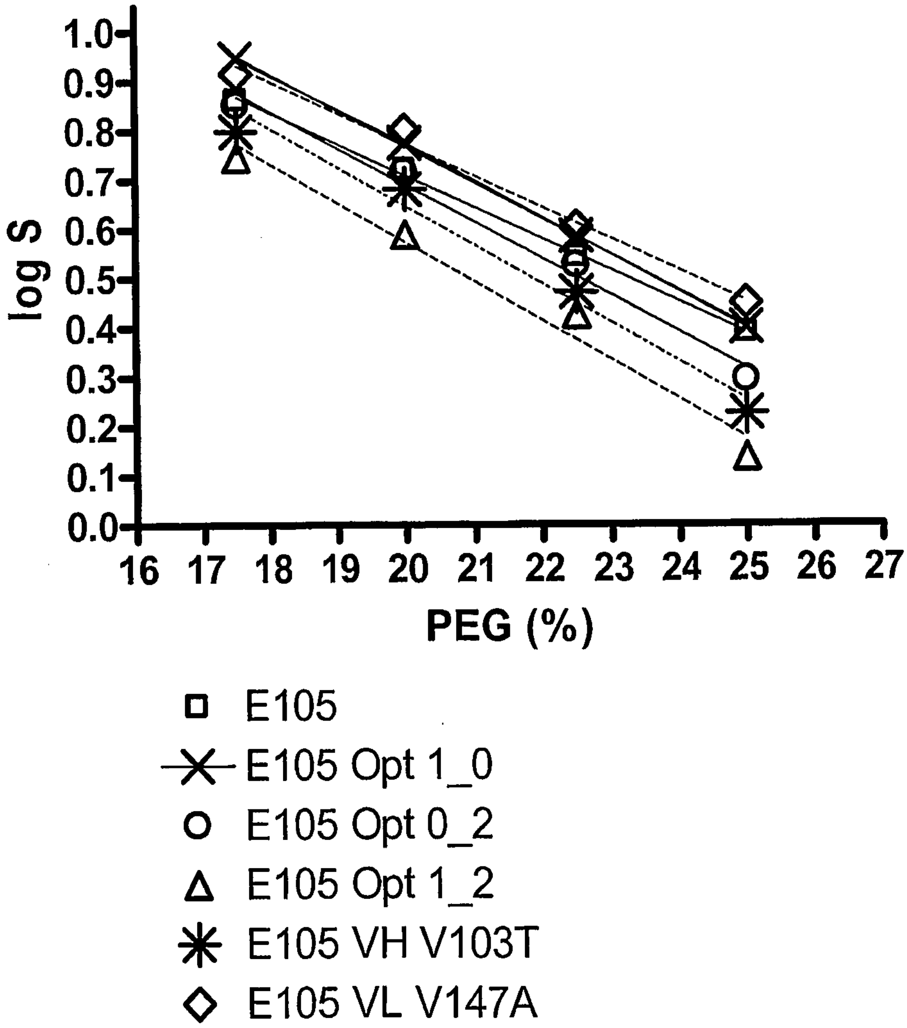
log S
1.0
0.9
0.8
0.7
0.6
0.5
0.4
0.3
0.2
0.1
0.0
16
17
18
19
20
21
22
23
24
25
26
27
PEG (%)
E105
E105 Opt 1_0
E105 Opt 0_2
E105 Opt 1_2
E105 VH V103T
E105 VL V147A

*Fig. 15*

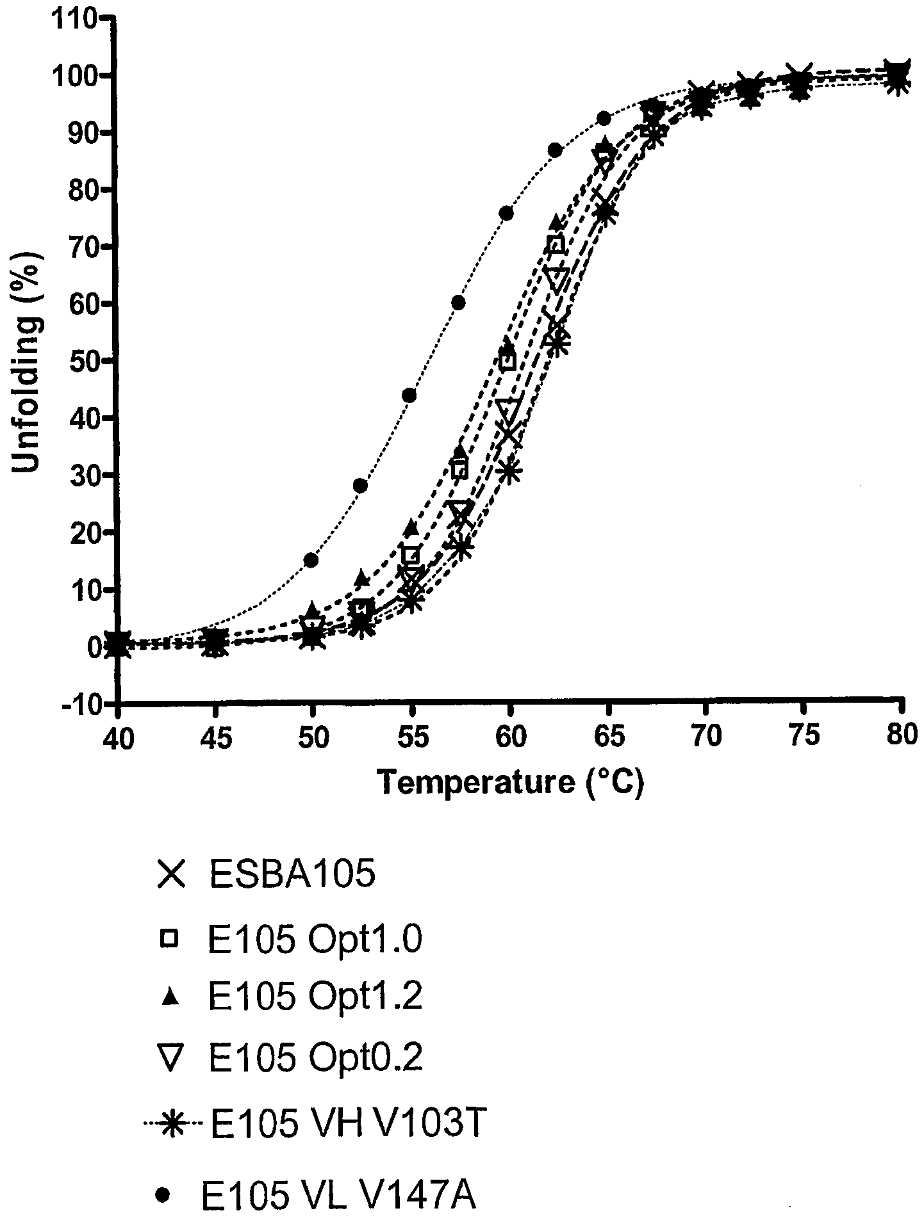

110
100
90
80
70
60
50
40
30
20
10
0
-10
Unfolding (%)
40
45
50
55
60
65
70
75
80
Temperature (°C)
ESBA105
E105 Opt1.0
E105 Opt1.2
E105 Opt0.2
E105 VH V103T
E105 VL V147A


*Fig. 16*

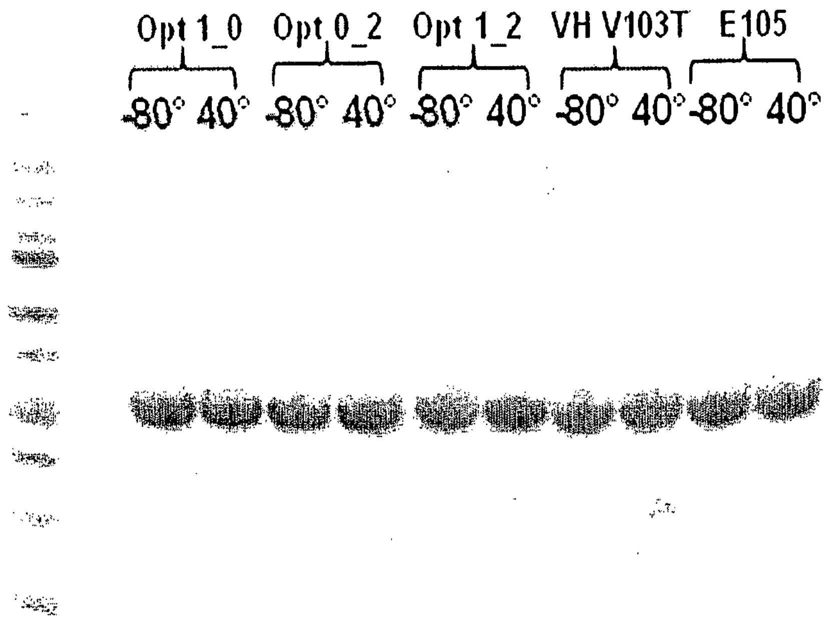


*Fig. 17*

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 93711207 P
- US 61069056 A
- WO 200148017 A
- US 20010024831 A
- US 20030096306 A
- US 7258985 B
- US 7258986 B
- WO 2003097697 A
- US 20060035320 A
- WO 03097697 A
- US 5736137 A
- US 6455043 B
- US 6682734 B
- US 5714350 A
- US 6350861 B
- US 6258562 B
- US 5824307 A
- US 6884879 B
- WO 2006131013 A
- WO 2008006235 A
- WO 2001048017 A
- US 905365 P
- US 937112 P
- US 60819378 B
- US 60899907 B
- WO 06131013 A2
- EP 1506236 A2
- EP 1479694 A2
- EP 1242457 B1
- WO 03097697 A2
- WO 0148017 A
- WO 0164942 A
- US 6673901 B
- US 6703199 B
- US 7078490 B
- US 7119171 B
- US 6740734 B
- US 6602977 B
- WO 0063243 A
- WO 9916873 A
- WO 05019254 A
- WO 02088171 A
- WO 04044011 A
- WO 0220565 A
- WO 06083275 A


### Non-patent literature cited in the description


- **NIEBA, L. et al.** *Prot. Eng.,* 1997, vol. 10, 435-444
- **TAN, P.H. et al.** *Biophys. J.,* 1988, vol. 75, 1473-1482
- **WÖRN, A. ; PLUCKTHUN, A.** *Biochem.,* 1998, vol. 37, 13120-13127
- **WÖRN, A. ; PLÜCKTHUN, A.** *Biochem.,* 1999, vol. 38, 8739-8750
- **JUNG, S. ; PLUCKTHUN, A.** *Prot. Eng.,* 1997, vol. 10, 959-966
- **WÖRN, A. ; PLUCKTHUN, A.** *J. Mol. Biol.,* 2001, vol. 305, 989-1010
- **SKERRA A. ; PLUCKTHUN, A.** *Science,* 1988, vol. 240, 1038-41
- **BIRD, R.E. et al.** *Science,* 1988, vol. 242, 423-26
- **HUSTON, J.S. et al.** *Proc. Natl. Acad. Sci. USA,* 1988, vol. 85, 5879-83
- **KABAT, E.A. et al.** Sequences of proteins of immunological interest. *NIH Publication,* 1991, vol. 91, 3242
- **KABAT E.A. et al.** Sequences of proteins of immunological interest. *NIH Publication,* 1991, vol. 91, 3242
- **NEEDLEMAN et al.** *J. Mol. Biol.,* 1970, vol. 48, 443-453
- FUNDAMENTAL IMMUNOLOGY. 1993
- **WARD et al.** *Nature,* 1989, vol. 341, 544-546
- **HAMERS-CASTERMAN et al.** *Nature,* 1993, vol. 363, 446-448
- **DUMOULIN et al.** *Protein Science,* 2002, vol. 11, 500-515
- **JOHNSON ; WU.** *Nucleic Acids Res.,* 2001, vol. 29, 205-206
- **JOHNSON ; WU.** *Nucleic Acids Res.,* 2000, vol. 28, 214-218
- **LEFRANC et al.** 27. *Nucleic Acids Res.,* 1999, 209-212
- **RUIZ et al.** *Nucleic Acids Res.,* 2000, vol. 28, 219-221
- **LEFRANC et al.** *Nucleic Acids Res.,* 2001, vol. 29, 207-209

- **LEFRANC et al.** *Nucleic Acids Res.,* 2003, vol. 31, 307-310
- **BARBERIS et al.** *Cell,* 1995, vol. 81, 359
- **AUF DER MAUR et al.** *Methods,* 2004, vol. 34, 215-224
- **CHOULIER, L. et al.** *Protein,* 2000, vol. 41, 475-484
- **JUNG et al.** *J. Mol. Biol.,* 1999, vol. 294, 163-180
- **WU et al.** *J. Mol. Biol.,* 1999, vol. 294, 151-162
- **SCHIER et al.** *J. Mol. Biol.,* 1996, vol. 255, 28-43
- **NIEBA et al.** *Protein Eng.,* 1997, vol. 10, 435-44
- **MONSELLIER E. ; BEDOUELLE H.** *J. Mol. Biol.,* 2006, vol. 362, 580-93
- **TAN et al.** *Biophys. J.,* 1998, vol. 75, 1473-82
- **WÖRN A. ; PLÜCKTHUN A.** *Biochemistry,* 1998, vol. 37, 13120-7
- **BERGE, S. M. et al.** *J. Pharm. Sci.,* 1977, vol. 66, 1-19
- **ARAKAWA et al.** *J. Biochem,* 1996, vol. 120, 657-662
- **KUNG ; GOLDSTEIN et al.** *Science,* 1979, vol. 206, 347-349
- **HONEGGER et al.** *J. Mol. Biol.,* 2001, vol. 309, 657-670
- **KABAT, E. A. et al.** Sequences of Proteins of Immunological Interest. Department of Health and Human Services, 1991
- **HONEGGER, A. ; PLÜCKTHUN, A.** *J. Mol. Biol,* 2001, vol. 309, 657-670
- **NEEDLEMAN et al.** *J Mol Biol.,* 1970, vol. 48 (3), 443-53
- **HONEGGER, A. ; PLÜCKTHUN, A.** *J. Mol. Biol.,* 2001, vol. 309, 657-670
- **KABAT, E. A. et al.** Sequences of Proteins of Immunological Interest. U.S. Department of Health and Human Services, 1991
- **TOMLINSON, I.M. et al.** *J. Mol. Biol.,* 1992, vol. 227, 776-798
- **WILLIAMS, S.C. ; WINTER, G.** *Eur. J. Immunol.,* 1993, vol. 23, 1456-1461
- **COX, J.P. et al.** *Eur. J. Immunol.,* 1994, vol. 24, 827-836
- **SHENKIN, P.S. et al.** *Proteins,* 1991, vol. 11, 297-313
- **LARSON, S.M. ; DAVIDSON, A.R.** *Protein Sci.,* 2000, vol. 9, 2170-2180
- **DEMAREST, S.J. et al.** *J. Mol. Biol.,* 2004, vol. 335, 41-48
- **STEIPE, B et al.** *J. Mol. Biol.,* 1994, vol. 240, 188-192
- **OHAGE, E. ; STEIPE, B.** *J. Mol. Biol,* 1999, vol. 291, 1119-1128
- **KNAPPIK, A. et al.** *J. Mol. Biol.,* 2000, vol. 296, 57-86
- **EWERT, S. et al.** *Biochemistry,* 2003, vol. 42, 1517-1528
- **MONSELLIER, E. ; BEDOUELLE, H.** *J. Mol. Biol.,* 2006, vol. 362, 580-593